(19) Europäisches Patentamt
European Patent Office
Office européen des brevets

(11) EP 4 166 158 A1

(12) **EUROPEAN PATENT APPLICATION**
published in accordance with Art. 153(4) EPC

(43) Date of publication:
**19.04.2023 Bulletin 2023/16**

(21) Application number: **21821552.3**

(22) Date of filing: **10.06.2021**

(51) International Patent Classification (IPC):
***A61K 39/00*** (2006.01) ***A61K 39/215*** (2006.01)
***A61K 48/00*** (2006.01) ***A61P 31/12*** (2006.01)
***A61P 31/14*** (2006.01) ***C07K 14/165*** (2006.01)
***A61K 9/107*** (2006.01) ***A61K 9/14*** (2006.01)
***A61K 47/14*** (2017.01) ***A61K 47/18*** (2017.01)
***A61K 47/24*** (2006.01) ***A61K 47/28*** (2006.01)
***C12N 15/50*** (2006.01) ***C12N 15/88*** (2006.01)
***A61K 31/7105*** (2006.01)

(52) Cooperative Patent Classification (CPC):
**A61K 9/107; A61K 9/14; A61K 31/7105; A61K 39/00; A61K 39/215; A61K 47/14; A61K 47/18; A61K 47/24; A61K 47/28; A61K 48/00; A61P 31/12; A61P 31/14; C07K 14/165; C12N 15/88**

(86) International application number:
**PCT/JP2021/022057**

(87) International publication number:
**WO 2021/251453 (16.12.2021 Gazette 2021/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.06.2020 JP 2020101420**
**03.03.2021 JP 2021033278**

(71) Applicants:
- **Daiichi Sankyo Company, Limited**
  **Tokyo 103-8426 (JP)**
- **The University of Tokyo**
  **Bunkyo-ku, Tokyo 113-8654 (JP)**

(72) Inventors:
- **KAWAOKA, Yoshihiro**
  **Tokyo 113-8654 (JP)**
- **IMAI, Masaki**
  **Tokyo 113-8654 (JP)**
- **YAMAYOSHI, Seiya**
  **Tokyo 113-8654 (JP)**
- **ISHII, Ken**
  **Tokyo 113-8654 (JP)**
- **KOBIYAMA, Kouji**
  **Tokyo 113-8654 (JP)**
- **NAMBA, Eiko**
  **Tokyo 103-8426 (JP)**
- **OKA, Tatsuya**
  **Tokyo 103-8426 (JP)**
- **TOZUKA, Miyuki**
  **Tokyo 103-8426 (JP)**
- **JONAI, Nao**
  **Tokyo 103-8426 (JP)**
- **ONODERA, Yoshikuni**
  **Tokyo 103-8426 (JP)**
- **TAKESHITA, Fumihiko**
  **Tokyo 103-8426 (JP)**
- **SUZUKI, Takashi**
  **Tokyo 103-8426 (JP)**
- **NIWA, Takako**
  **Tokyo 103-8426 (JP)**
- **KOIZUMI, Makoto**
  **Tokyo 103-8426 (JP)**
- **NAKAMURA, Kensuke**
  **Tokyo 103-8426 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

(54) **NUCLEIC ACID LIPID PARTICLE VACCINE**

(54) **NUCLEIC ACID LIPID PARTICLE VACCINE**

(57) The present invention provides a vaccine for preventing and/or treating infections with severe acute respiratory syndrome coronavirus 2 (SARS-CoV-2).

The present invention relates to a lipid particle encapsulating a nucleic acid molecule capable of expressing the S protein and/or a fragment thereof of severe acute respiratory syndrome coronavirus 2 (SARS-CoV-2), wherein the lipid comprises a cationic lipid represented by general formula (Ia) or a pharmaceutically acceptable salt thereof

[Formula 1]

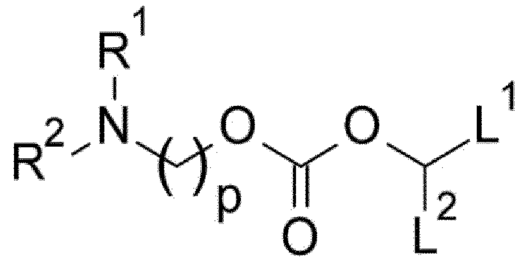

(Ia)

wherein $R^1$ and $R^2$ each independently represent a $C_1$-$C_3$ alkyl group;
$L^1$ represents a $C_{17}$-$C_{19}$ alkenyl group which may have one or a plurality of $C_2$-$C_4$ alkanoyloxy groups;
$L^2$ represents a $C_{10}$-$C_{19}$ alkyl group which may have one or a plurality of $C_2$-$C_4$ alkanoyloxy groups or a $C_{10}$-$C_{19}$ alkenyl group which may have one or a plurality of $C_2$-$C_4$ alkanoyloxy groups; and
p is 3 or 4.

[Fig. 7]

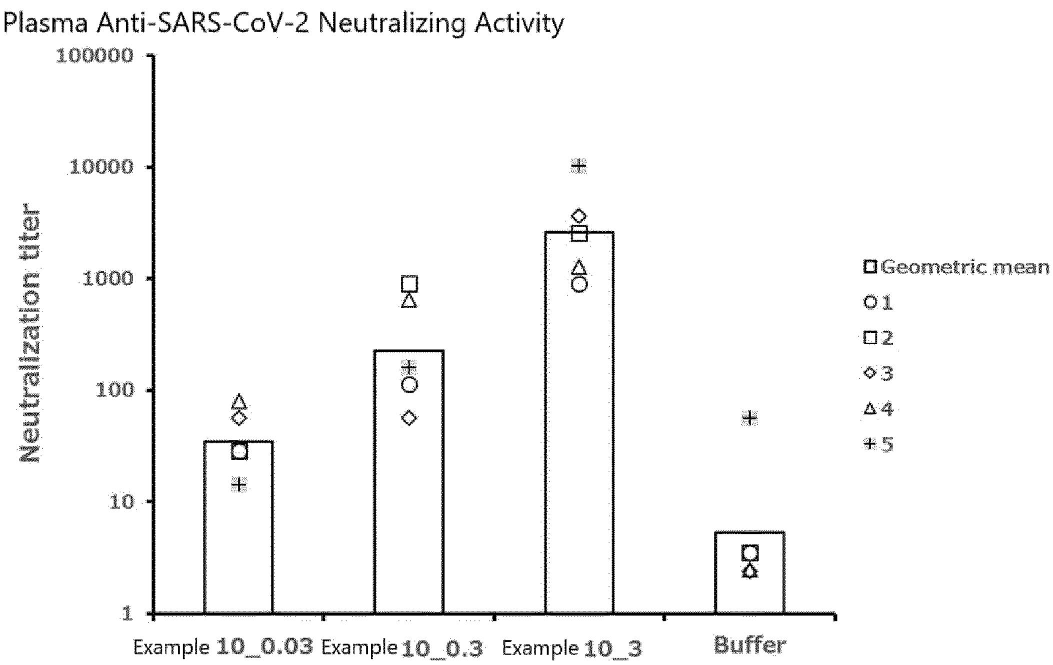

**Description**

TECHNICAL FIELD

The present invention relates to a nucleic acid lipid particle vaccine encapsulating SARS-CoV-2 mRNA.

BACKGROUND ART

The coronavirus disease 2019 (COVID-19) is an infectious disease caused by a novel coronavirus designated as severe acute respiratory syndrome coronavirus 2 (SARS-CoV-2). This disease presents a pathology mainly characterized by acute inflammation in the respiratory tract. In particular, a pathology mainly characterized by inflammations in the lower airway such as invasive neumonia and acute respiratory distress syndrome in high risk patients is the disease burden (Non-Patent Document No. 1). More than six types of coronavirus (CoV) are known to infect humans and present primarily respiratory symptoms. SARS-CoV-2 is classified in the genus Betacoronavirus and virologically resembles SARS-CoV and Middle East respiratory syndrome coronavirus (MERS-CoV) both of which caused outbreak in the past.
Spike protein (S) expressed on the surfaces of viral particles plays a key role in the mechanism of initial infection. S is a type I membrane protein composed of two subunits S1 and S2, and forms a trimer (approximately 500 kDa, 20 nm). A receptor-binding domain (RBD) existing in S1 interacts with angiotensin-converting enzyme 2 (ACE2) expressed on the surfaces of host cells. It is suggested that the S in SARS-CoV-2 has a 10 to 20-fold higher affinity to ACE2 and a higher thermodynamic stability than the S in SARS-CoV, and that these are involved in the high transmissibility of SARS-CoV-2 (Non-Patent Documents Nos. 2 and 3).
IgG to RBD remains in convalescent serum samples of SARS patients for at least 3 years or more. Once the serum has been treated with RBD protein for adsorption of anti-RBD antibody, neutralizing activity is attenuated to 50% or less. These suggest that anti-RBD antibody is responsible for the major neutralizing activity (Non-Patent Documents Nos. 4 and 5). Indeed, isolated anti-SARS-CoV-2 RBD monoclonal antibody is reported to have a neutralizing activity against SARS-CoV-2 (Non-Patent Documents Nos. 6 and 7).
Analysis using convalescent peripheral blood samples from COVID-19 patients who have spent about 3 weeks after becoming asymptomatic indicated that induction of specific $CD4^+$ and $CD8^+$ T cells is important for defense against SARS-CoV-2 infection (Non-Patent Document No. 8). Specifically, as a result of analysis of 10 to 20 cases of COVID-19 patients-derived blood samples, plasma anti-SARS-CoV-2 RBD antibody responses and SARS-CoV-2-specific $CD4^+$ T cell responses were confirmed in all cases; and SARS-CoV-2-specific $CD8^+$ T cell responses were confirmed in about 70% of the cases. Since plasma anti-SARS-CoV-2 RBD IgG titer and the frequency of S-specific $CD4^+$ T cells are correlated (R = 0.8109), it was suggested that S contains T cell epitopes and that S-specific $CD4^+$ T cells potentially play an important role in the induction of antibody responses (Non-Patent Document No. 8). Further, correlation between plasma anti-SARS-CoV-2 neutralizing activity and plasma anti-SARS-CoV-2 S IgG titer (R = 0.9279) has also been reported (Non-Patent Document No. 9).
As a mechanism of "immune enhancement" which exacerbates COVID-19 symptoms, it is assumed that cellular immunopathology and antibody-dependent enhancement (ADE) may possibly be involved therein (Non-Patent Document No. 10). In SARS, it is suggested that plasma cytokine profile becomes T helper (Th) 2-dominant in lethal patients, compared to patients recovered from mild illness (Non-Patent Document No. 11). In the mouse SARS-CoV infection model, it is suggested that Th2 dominant immune responses to S induce pulmonary immunopathology associated with inflammatory responses mainly in eosinophils (Non-Patent Document No. 12). On the other hand, as regards ADE, reports have been published in relation to other viruses such as Dengue virus, respiratory syncytial virus, etc., there has been no report that specific antibody to SARS-CoV induces ADE in SARS patients. As regards vaccine antigen candidates against SARS-CoV, the published data suggest that an antigen encoding not full-length S but RBD alone may be able to avoid the risk of pulmonary disorder (Non-Patent Document No. 13). As regards SARS-CoV-2, there are also no direct clinical evidences demonstrating that antibody to S is involved in ADE but it has been pointed out that appropriate cellular immune responses are necessary in order to avoid the risk (Non-Patent Document No. 14).

PRIOR ART LITERATURE

Non-Patent Documents

Non-Patent Document No. 1: Virology 12:372 2020
Non-Patent Document No. 2: Science 367:1260 2020
Non-Patent Document No. 3: Viruses 12:428 2020

Non-Patent Document No. 4: Virol J 7:299 2010
Non-Patent Document No. 5: Virology 334:74 2005
Non-Patent Document No. 6: Nat Commun 11:2251 2020
Non-Patent Document No. 7: Nature 583:290 2020
Non-Patent Document No. 8: Cell 181:1 2020
Non-Patent Document No. 9: Nat Med 26:1033 2020
Non-Patent Document No. 10: Nat Rev Immunol 20:347 2020
Non-Patent Document No. 11: J Immunol 181:5490 2008
Non-Patent Document No. 12: PLoS One 7:e35421 2012
Non-Patent Document No. 13: Vaccine 25:2832 2007
Non-Patent Document No. 14: PNAS 117:8218 2020

DISCLOSURE OF THE INVENTION

PROBLEM FOR SOLUTION BY THE INVENTION

It is an object of the present invention to provide a vaccine for preventing and/or treating infections with severe acute respiratory syndrome coronavirus 2 (SARS-CoV-2).

MEANS TO SOLVE THE PROBLEM

The present inventors administered a lipid particle encapsulating an mRNA molecule encoding the RBD of SARS-CoV-2 to mice, and found that induction of blood SARS-CoV-2 S protein IgG was observed and that this immune response was Th1-biased. The present invention has been achieved based on these findings.

A summary of the present invention is described as below.

(1) A lipid particle encapsulating a nucleic acid molecule capable of expressing the S protein and/or a fragment thereof of severe acute respiratory syndrome coronavirus 2 (SARS-CoV-2), wherein the lipid comprises a cationic lipid represented by general formula (Ia) or a pharmaceutically acceptable salt thereof:

[Formula 1]

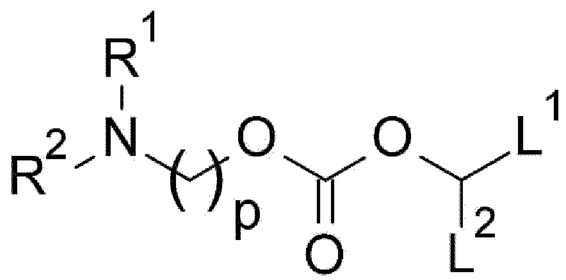

(Ia)

wherein $R^1$ and $R^2$ each independently represent a $C_1$-$C_3$ alkyl group;
$L^1$ represents a $C_{17}$-$C_{19}$ alkenyl group which may have one or a plurality of $C_2$-$C_4$ alkanoyloxy groups;
$L^2$ represents a $C_{10}$-$C_{19}$ alkyl group which may have one or a plurality of $C_2$-$C_4$ alkanoyloxy groups or a $C_{10}$-$C_{19}$ alkenyl group which may have one or a plurality of $C_2$-$C_4$ alkanoyloxy groups; and
p is 3 or 4.

(2) The particle of (1) above, wherein both $R^1$ and $R^2$ in general formula (Ia) are a methyl group.
(3) The particle of (1) or (2) above, wherein p in general formula (Ia) is 3.
(4) The particle of any one of (1) to (3) above, wherein $L^1$ in general formula (Ia) is a $C_{17}$-$C_{19}$ alkenyl group which may have one or a plurality of acetoxy groups.
(5) The particle of any one of (1) to (4) above, wherein $L^2$ in general formula (Ia) is a $C_{10}$-$C_{12}$ alkyl group which may have one or a plurality of acetoxy groups or a $C_{10}$-$C_{19}$ alkenyl group which may have one or a plurality of acetoxy groups.

(6) The particle of any one of (1) to (4) above, wherein $L^2$ in general formula (Ia) is a $C_{10}$-$C_{12}$ alkyl group which may have one or a plurality of acetoxy groups or a $C_{17}$-$C_{19}$ alkenyl group which may have one or a plurality of acetoxy groups.

(7) The particle of any one of (1) to (6) above, wherein $L^1$ in general formula (Ia) is an (R)-11-acetyloxy-cis-8-heptadecenyl group, a cis-8-heptadecenyl group or a (8Z,11Z)-heptadecadienyl group.

(8) The particle of any one of (1) to (7) above, wherein $L^2$ in general formula (Ia) is a decyl group, a cis-7-decenyl group, a dodecyl group or an (R)-11-acetyloxy-cis-8-heptadecenyl group.

(9) The particle of (1), wherein the cationic lipid is represented by the following structural formula:

[Formula 2]

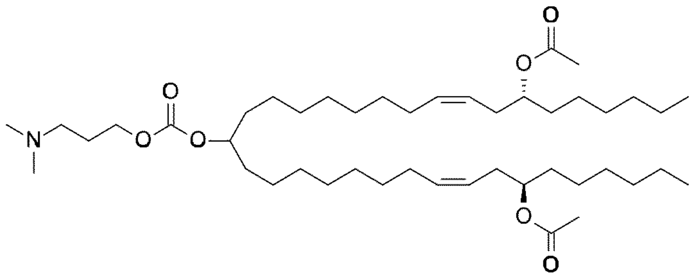

(10) The particle of (1), wherein the cationic lipid is represented by the following structural formula:

[Formula 3]

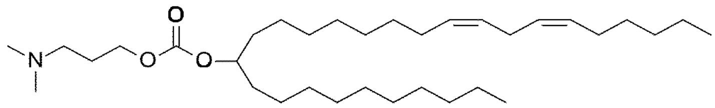

(11) The particle of (1), wherein the cationic lipid is represented by the following structural formula:

[Formula 4]

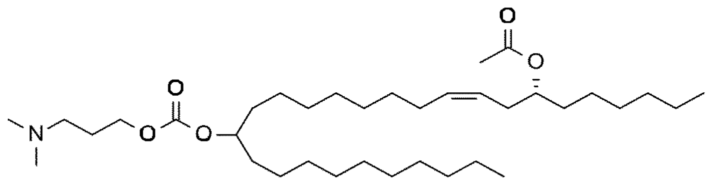

(12) The particle of any one of (1) to (11) above, wherein the lipid further comprises amphipathic lipids, sterols and PEG lipids.

(13) The particle of (12) above, wherein the amphipathic lipid is at least one selected from the group consisting of distearoyl phosphatidylcholine, dioleoyl phosphatidylcholine and dioleoyl phosphatidylethanolamine.

(14) The particle of (12) or (13) above, wherein the sterol is cholesterol.

(15) The particle of any one of (12) to (14) above, wherein the PEG lipid is 1,2-dimyristoyl-sn-glycerol methoxypolyethylene glycol and/or N-[methoxy poly(ethyleneglycol) 2000]carbamoyl]-1,2-dimyristyloxypropyl-3-amine.

(16) The particle of any one of (12) to (15) above, wherein the lipid composition of the amphipathic lipid, the sterol, the cationic lipid and the PEG lipid is 15% or less of the amphipathic lipid, 20 to 55% of the sterol, 40 to 65% of the cationic lipid and 1 to 5% of the PEG lipid in terms of molar quantity; and the ratio of the total lipid weight to the weight of nucleic acid is 15 to 30.

(17) The particle of (16) above, wherein the lipid composition of the amphipathic lipid, the sterol, the cationic lipid and the PEG lipid is 5 to 15% of the amphipathic lipid, 35 to 50% of the sterol, 40 to 55% of the cationic lipid and 1

to 3% of the PEG lipid in terms of molar quantity; and the ratio of the total lipid weight to the weight of nucleic acid is 15 to 25.

(18) The particle of (17) above, wherein the lipid composition of the amphipathic lipid, the sterol, the cationic lipid and the PEG lipid is 10 to 15% of the amphipathic lipid, 35 to 45% of the sterol, 40 to 50% of the cationic lipid and 1 to 2% of the PEG lipid in terms of molar quantity; and the ratio of the total lipid weight to the weight of nucleic acid is 17.5 to 22.5.

(19) The particle of (18) above, wherein the lipid composition of the amphipathic lipid, the sterol, the cationic lipid and the PEG lipid is 10 to 15% of the amphipathic lipid, 35 to 45% of the sterol, 45 to 50% of the cationic lipid and 1.5 to 2% of the PEG lipid in terms of molar quantity; and the ratio of the total lipid weight to the weight of nucleic acid is 17.5 to 22.5.

(20) The particle of any one of (1) to (19) above, wherein the fragment of the S protein of severe acute respiratory syndrome coronavirus 2 (SARS-CoV-2) comprises a receptor-binding domain.

(21) The particle of (20) above, wherein the receptor-binding domain in the fragment of the S protein of severe acute respiratory syndrome coronavirus 2 (SARS-CoV-2) consists of an amino acid sequence having at least 95% identity with the amino acid sequence as shown in SEQ ID NO: 11.

(22) The particle of (20) above, wherein the receptor-binding domain in the fragment of the S protein of severe acute respiratory syndrome coronavirus 2 (SARS-CoV-2) consists of an amino acid sequence having at least 95% identity with the amino acid sequence as shown in any one of SEQ ID NOS: 25, 29, 33, 37 and 94 to 107.

(23) The particle of (20) above, wherein the fragment of the S protein of severe acute respiratory syndrome coronavirus 2 (SARS-CoV-2) consists of an amino acid sequence having at least 95% identity with the amino acid sequence as shown in SEQ ID NO: 10.

(24) The particle of (20) above, wherein the fragment of the S protein of severe acute respiratory syndrome coronavirus 2 (SARS-CoV-2) consists of an amino acid sequence having at least 95% identity with the amino acid sequence as shown in any one of SEQ ID NOS: 24, 28, 32, 36 and 80 to 93.

(25) The particle of any one of (1) to (19) above, wherein the S protein of severe acute respiratory syndrome coronavirus 2 (SARS-CoV-2) consists of an amino acid sequence having at least 95% identity with the amino acid sequence as shown in SEQ ID NO: 6.

(26) The particle of (25) above, wherein the receptor-binding domain in the S protein of severe acute respiratory syndrome coronavirus 2 (SARS-CoV-2) consists of an amino acid sequence having at least 95% identity with the amino acid sequence as shown in SEQ ID NO: 11.

(27) The particle of (25) or (26) above, wherein the nucleic acid molecule capable of expressing the S protein of severe acute respiratory syndrome coronavirus 2 (SARS-CoV-2) is an mRNA molecule comprising a cap structure (Cap), 5' untranslated region (5'-UTR), S protein coding region, 3' untranslated region (3'-UTR) and a polyA tail (polyA).

(28) The particle of any one of (20) to (24) above, wherein the nucleic acid molecule capable of expressing the fragment of the S protein of severe acute respiratory syndrome coronavirus 2 (SARS-CoV-2) is an mRNA molecule comprising a cap structure (Cap), 5' untranslated region (5'-UTR), a leader sequence, the coding region of the receptor-binding domain in the S protein, 3' untranslated region (3'-UTR) and a polyA tail (polyA).

(29) The particle of (27) above, wherein the sequence of S protein coding region consists of a nucleotide sequence having at least 90% identity with the sequence of S protein coding region in the sequence as shown in SEQ ID NO: 5.

(30) The particle of (27) above, wherein the sequence of S protein coding region consists of a nucleotide sequence having at least 90% identity with the sequence of S protein coding region in the sequence as shown in SEQ ID NO: 16.

(31) The particle of (27) above, wherein the nucleic acid molecule capable of expressing the S protein of severe acute respiratory syndrome coronavirus 2 (SARS-CoV-2) consists of the nucleotide sequence as shown in SEQ ID NO: 5.

(32) The particle of (27) above, wherein the nucleic acid molecule capable of expressing the S protein of severe acute respiratory syndrome coronavirus 2 (SARS-CoV-2) consists of the nucleotide sequence as shown in SEQ ID NO: 16.

(33) The particle of (27) above, wherein the sequence of the coding region of the receptor-binding domain in the S protein consists of a nucleotide sequence having at least 90% identity with the sequence of the coding region of the receptor-binding domain in the S protein in the sequence as shown in SEQ ID NO: 9.

(34) The particle of (27) above, wherein the sequence of the coding region of the receptor-binding domain in the S protein consists of a nucleotide sequence having at least 90% identity with the sequence of the coding region of the receptor-binding domain in the S protein in the sequence as shown in SEQ ID NO: 19.

(35) The particle of (27) above, wherein the sequence of the coding region of the receptor-binding domain in the S protein consists of a nucleotide sequence having at least 90% identity with the sequence of the coding region of the receptor-binding domain in the S protein in the sequence as shown in any one of SEQ ID NOS: 21, 23, 27, 31, 35 and 66 to 79.

(36) The particle of (28) above, wherein the nucleic acid molecule capable of expressing the fragment of the S protein of severe acute respiratory syndrome coronavirus 2 (SARS-CoV-2) consists of the nucleotide sequence as shown in SEQ ID NO: 9.

(37) The particle of (28) above, wherein the nucleic acid molecule capable of expressing the fragment of the S protein of severe acute respiratory syndrome coronavirus 2 (SARS-CoV-2) consists of the nucleotide sequence as shown in SEQ ID NO: 19.

(38) The particle of any one of (1) to (37) above, wherein the nucleic acid molecule comprises at least one modified nucleotide.

(39) The particle of (38) above, wherein the modified nucleotide comprises at least one of 5-substituted pyrimidine nucleotide and/or pseudouridine optionally substituted at position 1.

(40) The particle of (38) above, wherein the modified nucleotide comprises at least one selected from the group consisting of 5-methylcytidine, 5-methoxyuridine, 5-methyluridine, pseudouridine and 1-alkylpseudouridine.

(41) The particle of any one of (1) to (40) above, wherein the mean particle size is 30 nm to 300 nm.

(42) Use of the particle of any one of (1) to (41) above for manufacturing a composition for preventing and/or treating infections with severe acute respiratory syndrome coronavirus 2 (SARS-CoV-2).

(43) A composition comprising the particle of any one of (1) to (41) above.

(44) The composition of (43) above for allowing the expression of the S protein and/or a fragment thereof of severe acute respiratory syndrome coronavirus 2 (SARS-CoV-2) *in vivo* or *in vitro.*

(45) The composition of (43) or (44) above for use as a pharmaceutical drug.

(46) The composition of (45) above for inducing immune responses to severe acute respiratory syndrome coronavirus 2 (SARS-CoV-2).

(47) The composition of (45) or (46) above for preventing and/or treating infections with severe acute respiratory syndrome coronavirus 2 (SARS-CoV-2).

(48) A method of expressing the S protein and/or a fragment thereof of severe acute respiratory syndrome coronavirus 2 (SARS-CoV-2) *in vitro,* comprising introducing into cells the composition of (43) or (44) above.

(49) A method of expressing the S protein and/or a fragment thereof of severe acute respiratory syndrome coronavirus 2 (SARS-CoV-2) *in vivo,* comprising administering to a mammal the composition of any one of (43) to (47) above.

(50) A method of inducing immune response to severe acute respiratory syndrome coronavirus 2 (SARS-CoV-2), comprising administering to a mammal the composition of (45) or (46) above.

(51) A method of preventing and/or treating infections with severe acute respiratory syndrome coronavirus 2 (SARS-CoV-2), comprising administering to a mammal the composition of any one of (45) to (47) above.

(52) The method of any one of (49) to (51) above, wherein the mammal is human.

## EFFECT OF THE INVENTION

According to the present invention, it becomes possible to prevent and/or treat infections with SARS-CoV-2.

The present specification encompasses the contents disclosed in the specification and/or the drawings of Japanese Patent Applications Nos. 2020-101420 and 2021-33278 based on which the present patent application claims priority.

## BRIEF DESCRIPTION OF THE DRAWINGS

[Fig. 1] Expression levels of RBD protein when the particles of Example 3 or Example 4 was used. Buffer: 10 mM histidine buffer containing 300 mM sucrose (pH 6.5).

[Fig. 2] Anti-RBD antibody responses induced by the particles of Example 3 and Example 4. Buffer: 10 mM histidine buffer containing 300 mM sucrose (pH 6.5). Vertical bars indicate the geometric mean, and symbols 1 to 5 indicate individual antibody levels.

[Fig. 3] Serum inhibitory activity against RBD-hACE binding in Example 3 and Example 4 groups. Buffer: 10 mM histidine buffer containing 300 mM sucrose (pH 6.5). Horizontal bars indicate the geometric mean, and circle symbols indicate individual inhibitory activity levels.

[Fig. 4] RBD-specific cellular immunity induced in Example 3 and Example 4 groups. Buffer: 10 mM histidine buffer containing 300 mM sucrose (pH 6.5). Vertical bars indicate the mean, and error bars indicate SEM. In all the treated groups, DMSO concentration was adjusted to 0.1% (v/v). No peptides: no peptide group.

[Fig. 5] Plasma anti-RBD antibody responses induced by administration of the particles from Example 4, Example 7 or Example 8. Buffer: 10 mM histidine buffer containing 300 mM sucrose (pH 7.0). Vertical bars indicate the geometric mean, and symbols 1 to 4 indicate individual antibody levels.

[Fig. 6] Plasma anti-RBD antibody responses induced by administration of the particles of Example 8 or Example 10. Buffer: 10 mM histidine buffer containing 300 mM sucrose (pH 7.0). Vertical bars indicate the geometric mean, and symbols 1 to 5 indicate individual antibody levels.

[Fig. 7] Plasma anti-SARS-CoV-2 neutralizing activity induced by administration of the particles of Example 10. Buffer: 10 mM histidine buffer containing 300 mM sucrose (pH 7.0). Vertical bars indicate the geometric mean, and symbols 1 to 5 indicate individual neutralizing activities.

[Fig. 8] Plasma anti-SARS-CoV-2 neutralizing activity induced by administration of the particles from Example 8 or Example 10. Buffer: 10 mM histidine buffer containing 300 mM sucrose (pH 7.0). Vertical bars indicate the geometric mean, and symbols 1 to 5 indicate individual neutralizing activities.

[Fig. 9] Plasma anti-RBD antibody responses induced by administration of the particles from Example 8 or Example 10. Buffer: 10 mM histidine buffer containing 300 mM sucrose (pH 7.0). Vertical bars indicate the geometric mean, and error bars indicate S.D.

[Fig. 10] RBD-specific cellular immunity induced by administration of the particles from Example 10. Buffer: 10 mM histidine buffer containing 300 mM sucrose (pH 7.0). Vertical bars indicate the mean, and symbols 1 to 7 indicate individual cytokine induction levels. In all the treated groups, DMSO concentration was adjusted to 0.1% (v/v). No peptides: no peptide group.

[Fig. 11] Mouse strain-specific immunogenicity of mRNA vaccine against SARS-CoV-2 RBD.

(a-e, g, and h) Six-week-old C57BL/6 and BALB/c mice were intramuscularly immunized with mock or LNP-mRNA-RBD (3 $\mu$g mRNA) twice in total with an interval of two weeks. (a) Two weeks after the second immunization, plasma anti-RBD antibody titers were measured using ELISA. (b-e) Lymphocytes were prepared from popliteal lymph nodes of immunized mice and subjected to flow cytometry. (b-d) Germinal center (GC) B cells were gated as $GL7^+CD38^-CD19^+$ cells. (e) $T_{FH}$ cells were gated as $CD185^+PD\text{-}1^+CD3\varepsilon^+CD4^+$ T cells. (f) Overlapping peptides of SARS-CoV-2 spike protein. Overlapping peptides were divided into eight pools, and each pool contained 16 peptides. (g and h) Splenocytes were prepared from the spleen of mice and re-stimulated with pooled peptides for 24 h. IFN-$\gamma$ levels in the culture supernatant were measured using ELISA. (g-h) Percentages of cytokine-producing $CD8^+$ and $CD4^+$ T cells after stimulation of pools 2, 3, and 4 for 6 h with protein transport inhibitor are shown in pie charts. $3^+$: IFN-$\gamma^+$IL-$2^+$TNF-$\alpha^+$, $2^+$: IFN-$\gamma^+$IL-$2^+$, IFN-$\gamma^+$TNF-$\alpha^+$, and IL-$2^+$TNF-$\alpha^+$, $1^+$: IFN-$\gamma^+$, IL-$2^+$, and TNF-$\alpha^+$. N = 4-5 mice per group. Vertical bars indicate the mean and error bars indicate SEM. *$p < 0.05$ by Mann-Whitney test.

[Fig. 12] Immunogenicity of HPLC-purified mRNA-encapsulating LNP-mRNA-RBD (mRNA-RBD (HPLC)). (a) Human peripheral blood mononuclear cells (PBMCs) from non-infected individuals were stimulated with LNP-mRNA-Full (0.4, 2, and 10 $\mu$g/mL in terms of mRNA), LNP-mRNA-RBD (0.4, 2, and 10 $\mu$g/mL in terms of mRNA), or mRNA-RBD (HPLC) (0.4, 2, and 10 $\mu$g/mL in terms of mRNA) for 24 h. IFN-$\alpha$ level in the culture supernatant was measured using ELISA. (b) Bone-marrow-derived dendritic cells (BM-DCs) from C57BL/6 and BALB/c mice were stimulated with LNP-mRNA-Full (0.4, 2, and 10 $\mu$g/mL in terms of mRNA), LNP-mRNA-RBD (0.4, 2, and 10 $\mu$g/mL in terms of mRNA), or mRNA-RBD (HPLC) (0.4, 2, and 10 $\mu$g/mL in terms of mRNA) for 24 h. IFN-$\alpha$ level in the culture supernatant was measured using ELISA. (c-i) C57BL/6 mice were intramuscularly immunized with mock, LNP-mRNA-RBD (3 $\mu$g mRNA), or mRNA-RBD (HPLC) (3 $\mu$g mRNA) at days 0 and 14. (c) Two weeks after the second immunization, plasma anti-RBD antibody titers were measured using ELISA. (d and e) Popliteal lymph nodes were collected from immunized mice, (d) GC B cells were gated as $GL7^+CD38^-CD19^+$ cells. (e) $T_{FH}$ cells were gated as $CD185^+PD\text{-}1^+CD3\varepsilon^+CD4^+$ T cells, (f and g) Splenocytes were prepared from the spleen of immunized mice and re-stimulated with pooled peptides for 24 h. IFN-$\gamma$ level in the culture supernatant was measured using ELISA. Percentages of cytokine-producing $CD8^+$ and $CD4^+$ T cells after stimulation of peptide pools 3 and 4 for 6 h with protein transport inhibitors are shown in pie charts. $3^+$: IFN-$\gamma^+$IL-$2^+$TNF-$\alpha^+$, $2^+$: IFN-$\gamma^+$IL-$2^+$, IFN-$\gamma^+$TNF-$\alpha^+$, and IL-$2^+$TNF-$\alpha^+$, $1^+$: IFN-$\gamma^+$, IL-$2^+$, and TNF-$\alpha^+$. (h and i) Representative data from Fig. 12f, g, Fig. 21 and Fig. 22 are shown. IFN-$\gamma^+$IL-$2^+$TNF-$\alpha^+$ and IFN-$\gamma^+$TNF-$\alpha^+CD8^+$ T cells are shown as a scatter dot plot. N = 4-5 mice. Vertical bars indicate the mean and error bars indicate SEM. * $p < 0.05$ by ANOVA followed by Dunn's multiple comparisons test.

[Fig. 13] Plasma anti-RBD antibody responses in cynomolgus macaques immunized with +HPLC-purified mRNA-encapsulating LNP-mRNA-RBD. (a) Schedule of immunization, infection, and sample collection, (b-c) Cynomolgus macaques were intramuscularly immunized with Mock or LNP-mRNA-RBD (HPLC) (100 $\mu$g) at days 0 and 21. (b) Plasma anti-RBD antibody titer at days 0, 7, 14, 21 and 28, and 7 days post infection (dpi) were measured using ELISA. (c) Neutralizing antibody responses were measured by neutralization assay. (d) Anti-RBD IgG antibody titers in the swab samples (conjunctiva, oral cavity, nasal cavity, tracheal, and rectum) were measured using ELISA. Black arrows indicate date of vaccination, and red arrows indicate infection date.

[Fig. 14] Defensive responses against SARS-CoV-2 infection in cynomolgus macaques immunized with mRNA-RBD (HPLC). One week after the second immunization, SARS-CoV-2 ($2 \times 10^7$ PFU) was inoculated into conjunctiva, nasal cavity, oral cavity, and trachea of cynomolgus. (a) Viral RNA and (b) viral titers in swab samples were measured by RT-PCR and a cell culture method, (c-d) Viral RNA in lung tissues was measured by RT-PCR. RU: right upper lobe, RM: right middle lobe, RL: right lower lobe, LU: left upper lobe, LM: left middle lobe, LL: left lower lobe.

[Fig. 15] Plasma anti-spike protein ectodomain (ECD) antibody responses in mice immunized with LNP-mRNA-RBD. C57BL/6 and BALB/c mice were intramuscularly immunized with mock or LNP-mRNA-RBD (3 μg mRNA) at days 0 and 14. Two weeks after the second immunization, plasma anti-ECD antibody titers were measured using ELISA. N = 4-5. Horizontal bars indicate the mean and symbols indicate individual data. $^{*}p < 0.05$ by Mann-Whitney test.

[Fig. 16] Gating strategy for GC B and $T_{FH}$ cells. Lymphocytes were prepared from popliteal lymph nodes of immunized mice and immunostained for GC B and $T_{FH}$ cells before flow cytometric analysis was conducted. Cells were gated for lymphocyte size, singlets, live, T or B cells, and $T_{FH}$ or GC B cells.

[Fig. 17] RBD-specific T cell responses. Splenocytes were prepared from the spleen of mRNA-immunized mice, re-stimulated with the spike protein peptide pool, ECD, or RBD for 24 h. IFN-γ and IL-13 levels in the culture supernatant were measured using ELISA. N = 4-5. Vertical bars the mean and error bars indicate SEM. $^{*}p < 0.05$ by ANOVA followed by Sidak's multiple comparisons test.

[Fig. 18] RBD-specific CD8 T cell responses. Splenocytes were prepared from the spleen of immunized mice and re-stimulated with pooled peptides for 6 h with a protein transport inhibitor. The percentage of cytokine-producing $CD8^+$ T cells was analyzed by flow cytometry. N = 4-5. Vertical bars indicate the mean and error bars indicate SEM. $^{*}p < 0.05$ by Mann-Whitney test.

[Fig. 19] RBD-specific CD4 T cell responses. Splenocytes were prepared from the spleen of immunized mice and re-stimulated with pooled peptides for 6 h with a protein transport inhibitor. The percentage of cytokine-producing $CD4^+$ T cells was analyzed by flow cytometry. N = 4-5. Vertical bars indicate the mean and error bars indicate SEM. $^{*}p < 0.05$ by Mann-Whitney test.

[Fig. 20] Spike protein-specific immune responses in mice immunized with mRNA-RBD (HPLC) (a) C57BL6 and BALB/c mice were intramuscularly immunized with mock, mRNA-RBD, or RBD (HPLC) (3 μg mRNA) at days 0 and 14. Two weeks after the second immunization, serum anti-ECD antibody titers were measured using ELISA. (b-e) Lymphocytes were prepared from the spleen of mRNA-immunized mice, re-stimulated with the peptide pool of spike protein, ECD, or RBD for 24 h. Vertical bars indicate the mean and error bars indicate SEM. * $p < 0.05$ by ANOVA followed by Dunn's or Sidak's multiple comparisons test.

[Fig. 21] RBD-specific T cell responses in C57/BL6 mice immunized with mRNA-RBD (HPLC). Lymphocytes were prepared from the spleen of immunized mice and re-stimulated by pooled peptides for 6 h with a protein transport inhibitor. The percentages of cytokine-producing $CD8^+$ and $CD4^+$ T cells were analyzed by flow cytometry. N = 4. Vertical bars indicate the mean and error bars indicate SEM. * $p < 0.05$ by ANOVA followed by Dunn's multiple comparisons test.

[Fig. 22] RBD-specific T cell responses in BALB/c mice immunized with mRNA-RBD (HPLC). Lymphocytes were prepared from the spleen of immunized mice and re-stimulated by pooled peptides for 6 h with a protein transport inhibitor. The percentages of cytokine-producing $CD8^+$ and $CD4^+$ T cells were analyzed by flow cytometry. N = 4. Vertical bars indicate the mean and error bars indicate SEM. * $p < 0.05$ by ANOVA followed by Dunn's multiple comparisons test.

[Fig. 23] Changes in body temperature before and after SARS-CoV-2 infection. One week after the second immunization with mRNA-RBD (HPLC), cynomolgus macaques were orally, intranasally, and intratracheally infected with SARS-CoV-2 ($2.2 \times 10^6$ PFU). Body temperature was recorded from two days before infection using telemetry transmitters and a computer.

[Fig. 24] Chest radiographs of cynomolgus macaques immunized with mRNA-RBD (HPLC) and infected with SARS-CoV-2.

[Fig. 25] Plasma anti-RBD antibody responses induced by the particles from Examples 10, 12, 14, 16, 18 and 20. Buffer: 10 mM histidine buffer containing 300 mM sucrose (pH 7.0). N = 4. Vertical bars indicate the geometric mean and symbols 1 to 4 indicate individual anti-RBD antibody levels.

[Fig. 26] Plasma anti-RBD antibody responses induced by the particles from Examples 10 and 21 to 30. Buffer: 10 mM histidine buffer containing 300 mM sucrose (pH 7.0). N = 4. Vertical bars indicate the geometric mean, and symbols 1 to 4 indicate individual anti-RBD antibody levels.

[Fig. 27] Plasma anti-RBD antibody responses induced by the particles from Examples 8, 32a, 32b, 32c, 32d, 32f and 33. Buffer: 10 mM histidine buffer containing 300 mM sucrose (pH 7.0). N = 4-5. Vertical bars indicate the geometric mean and symbols 1 to 5 indicate individual anti-RBD antibody levels. RBD antigens used for immobilization in ELISA are derived from Wuhan strain (Original) and B.1.351 strain (351).

[Fig. 28] Serum inhibitory activity against RBD-hACE2 binding in BALB/c mice immunized with the particles of Example 10. Buffer: 10 mM histidine buffer containing 300 mM sucrose (pH 7.0). As RBD antigens, SARS-CoV-1-derived antigen (Control), Wuhan strain-derived antigen (Original), and point mutants from Original (K417N, E484K, N501Y, and K417N/E484K/N501Y) were used. N = 4. Horizontal bars indicate the geometric mean, and symbols 1 to 4 indicate individual inhibitory activity levels.

[Fig. 29] Anti-SARS-CoV-2 neutralizing activity in the plasma of cynomolgus macaques immunized with the particle

of Example 10. N = 4. "Pre" indicates the activity before immunization with the particles of Example 10; "Post" indicates the activity after immunization with the same particles. Vertical bars indicate the geometric mean, and circle symbols indicate individual neutralizing activities.

BEST MODES FOR CARRYING OUT THE INVENTION

Hereinbelow, embodiments of the present invention will be described in detail.

The present invention provides lipid particles encapsulating a nucleic acid molecule capable of expressing the S protein and/or a fragment thereof of severe acute respiratory syndrome coronavirus 2 (SARS-CoV-2), wherein the lipid comprises a cationic lipid represented by general formula (Ia) or a pharmaceutically acceptable salt thereof

[Formula 5]

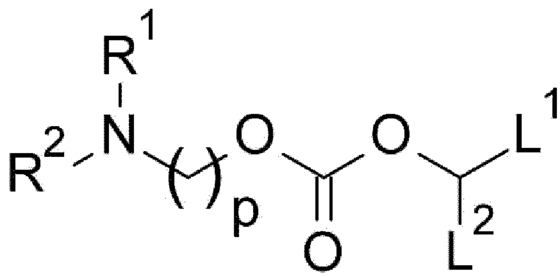

(Ia)

wherein $R^1$ and $R^2$ each independently represent a $C_1$-$C_3$ alkyl group;
$L^1$ represents a $C_{17}$-$C_{19}$ alkenyl group which may have one or a plurality of $C_2$-$C_4$ alkanoyloxy groups;
$L^2$ represents a $C_{10}$-$C_{19}$ alkyl group which may have one or a plurality of $C_2$-$C_4$ alkanoyloxy groups or a $C_{10}$-$C_{19}$ alkenyl group which may have one or a plurality of $C_2$-$C_4$ alkanoyloxy groups; and
p is 3 or 4.

$R^1$ and $R^2$ in general formula (Ia) each independently represent a $C_1$-$C_3$ alkyl group. Preferably, both $R^1$ and $R^2$ are a methyl group.
p in general formula (Ia) is 3 or 4, preferably 3.

$L^1$ in general formula (Ia) represents a $C_{17}$-$C_{19}$ alkenyl group which may have one or a plurality of $C_2$-$C_4$ alkanoyloxy groups. Preferably, $L^1$ is a $C_{17}$-$C_{19}$ alkenyl group which may have one or a plurality of acetoxy groups. Specific examples of $L^1$ include, but are not limited to, (R)-11-acetyloxy-cis-8-heptadecenyl group, cis-8-heptadecenyl group and (8Z,11Z)-heptadecadienyl group.

$L^2$ in general formula (Ia) represents a $C_{10}$-$C_{19}$ alkyl group which may have one or a plurality of $C_2$-$C_4$ alkanoyloxy groups, or a $C_{10}$-$C_{19}$ alkenyl group which may have one or a plurality of $C_2$-$C_4$ alkanoyloxy groups. Preferably, $L^2$ is a $C_{10}$-$C_{12}$ alkyl group which may have one or a plurality of acetoxy groups, or a $C_{10}$-$C_{19}$ alkenyl group which may have one or a plurality of acetoxy groups. Alternatively, it is also preferable that $L^2$ in general formula (Ia) is a $C_{10}$-$C_{12}$ alkyl group which may have one or a plurality of acetoxy groups, or a $C_{17}$-$C_{19}$ alkenyl group which may have one or a plurality of acetoxy groups. Specific examples of $L^2$ include, but are not limited to, decyl group, cis-7-decenyl group, dodecyl group and (R)-11-acetyloxy-cis-8-heptadecenyl group.

With respect to cationic lipid (a component which constitutes the particle of the present invention), those lipids which are represented by the following structural formulas may be enumerated as specific examples:

[Formula 6]

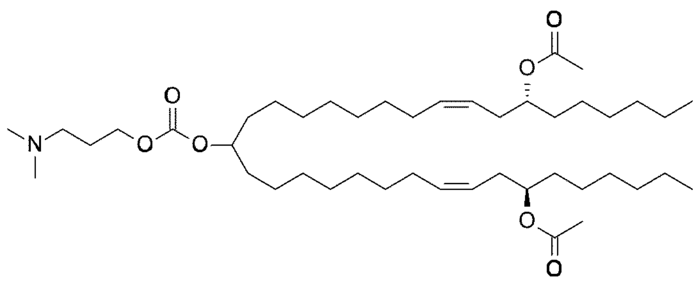

[Formula 7]

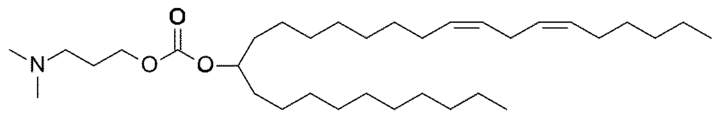

[Formula 8]

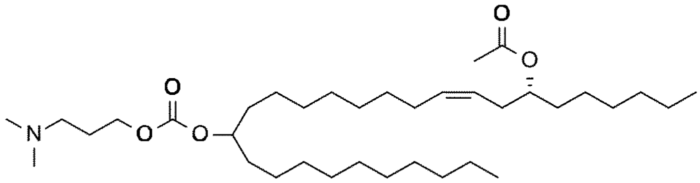

The term "pharmaceutically acceptable salt" as used herein means salts which may be used in pharmaceutical drug. Cationic lipid, a component which constitutes the particle of the present invention, may be a pharmaceutically acceptable salt. Examples of such salts include, but are not limited to, alkaline metal salts such as sodium salts, potassium salts or lithium salts; alkaline earth metal salts such as calcium salts or magnesium salts; metal salts such as aluminum salts, iron salts, zinc salts, copper salts, nickel salts or cobalt salts; amine salts including inorganic salts such as ammonium salts and organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts or tris(hydroxymethyl)aminomethane salts; inorganic acid salts including hydrohalogenic acid salts such as hydrofluorides, hydrochlorides, hydrobromides or hydroiodides, nitrates, perchlorates, sulfates or phosphates; organic acid salts including lower alkane sulfonic acid salts such as methanesulfonates, trifluoromethanesulfonates or ethanesulfonates, arylsulfonic acid salts such as benzenesulfonates or p-toluenesulfonates, acetates, malates, fumarates, succinates, citrates, tartrates, oxalates or maleates; and amino acid salts such as glycine salts, lysine salts, arginine salts, ornithine salts, glutamic acid salts or aspartic acid salts.

The cationic lipid represented by general formula (Ia) may be either a single compound or a combination of two or more compounds.

A method for preparing the cationic lipid represented by general formula (Ia) is disclosed in International Publication WO 2015/005253.

The lipid of the present invention may further comprise amphipathic lipids, sterols and PEG lipids.

The amphipathic lipid is a lipid which has affinity to both polar and non-polar solvents. Specific examples of the amphipathic lipid include, but are not limited to, distearoyl phosphatidylcholine, dioleoyl phosphatidylcholine, dioleoyl phosphatidylethanolamine and combinations thereof.

The sterol is a sterol which has a hydroxy group. Specific examples of the sterol include, but are not limited to, cholesterol.

The PEG lipid is a lipid modified with PEG. Specific examples of the PEG lipid include, but are not limited to, 1,2-dimyristoyl-sn-glycerol methoxypolyethylene glycol and/or N-[methoxy poly(ethyleneglycol) 2000]carbamoyl]-1,2-dimyristyloxypropyl-3-amine, or a combination thereof.

The lipid composition of the amphipathic lipid, the sterol, the cationic lipid and the PEG lipid is not particularly limited. Preferably, the lipid composition of the amphipathic lipid, the sterol, the cationic lipid and the PEG lipid is 15% or less of the amphipathic lipid, 20 to 55% of the sterol, 40 to 65% of the cationic lipid and 1 to 5% of the PEG lipid in terms of molar quantity; and the ratio of the total lipid weight to the weight of nucleic acid is 15 to 30. More preferably, the lipid composition of the amphipathic lipid, the sterol, the cationic lipid and the PEG lipid is 5 to 15% of the amphipathic lipid, 35 to 50% of the sterol, 40 to 55% of the cationic lipid and 1 to 3% of the PEG lipid in terms of molar quantity; and the ratio of the total lipid weight to the weight of nucleic acid is 15 to 25. Still more preferably, the lipid composition of the amphipathic lipid, the sterol, the cationic lipid and the PEG lipid is 10 to 15% of the amphipathic lipid, 35 to 45% of the sterol, 40 to 50% of the cationic lipid and 1 to 2% of the PEG lipid in terms of molar quantity; and the ratio of the total lipid weight to the weight of nucleic acid is 17.5 to 22.5. Further, still more preferably, the lipid composition of the amphipathic lipid, the sterol, the cationic lipid and the PEG lipid is 10 to 15% of the amphipathic lipid, 35 to 45% of the sterol, 45 to 50% of the cationic lipid and 1.5 to 2% of the PEG lipid in terms of molar quantity; and the ratio of the total

lipid weight to the weight of nucleic acid is 17.5 to 22.5.

The nucleic acid molecule to be encapsulated in the lipid particle in the present invention is one capable of expressing the S protein and/or a fragment thereof of severe acute respiratory syndrome coronavirus 2 (SARS-CoV-2). The sequence of SARS-CoV-2 Wuhan strain has been publicly disclosed (NCBI ID NC_045512) (https://www.ncbi.nlm.nih.gov/nuccore/NC_045512).

A fragment of the S protein of SARS-CoV-2 may suitably comprise a receptor-binding domain (RBD) existing in the S protein.

The receptor-binding domain may have a secretory peptide (a peptide encoded by a leader sequence) added thereto. As a leader sequence, S protein signal sequence may be given.

The amino acid sequence of the S protein of SARS-CoV-2 is shown in SEQ ID NO: 6. The nucleic acid molecule to be encapsulated in lipid particles may be one capable of expressing the S protein of SARS-CoV-2 consisting of an amino acid sequence having at least 95%, preferably 96%, and more preferably 97% identity with the amino acid sequence shown in SEQ ID NO: 6.

The amino acid sequence of the receptor-binding domain existing in the S protein of SARS-CoV-2 is shown in SEQ ID NO: 11. The receptor-binding domain existing in the S protein of SARS-CoV-2 may have a secretory peptide (e.g., S protein signal sequence) added thereto. The amino acid sequence of the S protein signal sequence-added receptor-binding domain is shown in SEQ ID NO: 10. The nucleic acid molecule to be encapsulated in lipid particles may be one capable of expressing the receptor-binding domain in the S protein of SARS-CoV-2 consisting of an amino acid sequence having at least 95%, preferably 96%, and more preferably 97% identity with the amino acid sequence shown in SEQ ID NO: 11 or 10.

The term "identity" used herein refers to the relationship between two or more nucleotide or amino acid sequences determined by comparison of the sequences, as known in the art. The term "identity" in the art also means, in some cases, the degree of relatedness in sequence between nucleic acid molecules or polypeptides as determined by the matching between rows of two or more nucleotide or amino acid sequences. Identity may be evaluated by calculating the percentage of exact match between a minor sequence in two or more sequences and a gapped alignment (if any) addressed by a specific mathematical model or computer program (i.e., "algorithm"). Specifically, identity may be evaluated using a software such as ClustalW2 supplied by European Molecular Biology Laboratory-European Bioinformatics Institute (EMBL-EBI). Alternatively, other software used by one of ordinary skill in the art may also be used.

The identity of sequence in the present invention is calculated with a sequence analysis software GENETYX-SV/RC (Genetyx Corporation). This algorism is commonly used in the art. The amino acid encoded by the nucleic acid molecule encapsulated in the lipid particle of the present invention may have mutations (substitutions), deletions, insertions and/or additions of amino acids, as long as the encoded amino acid retains at least a certain degree of identity with the amino acid sequence and/or a fragment thereof of the S protein of SARS-CoV-2 which is a target.

The amino acid encoded by the nucleic acid molecule encapsulated in the lipid particle of the present invention retains the sequence identity as described above and may yet have substitutions, deletions, insertions and/or additions of several amino acids (preferably 10 or less, more preferably 7 or less, still more preferably 5, 4, 3, 2 or 1) per position at several positions (preferably 5 or less, more preferably 3, 2 or 1) in the amino acid sequence of the target S protein of SARS-CoV-2 and/or the amino acid sequence of a fragment of the S protein.

The amino acid sequence of the receptor-binding domain existing in the S protein of SARS-CoV-2 may have deletions, substitutions or additions. For example, a sequence in which cysteine at position 538 (the number is counted from the N-terminus of S protein) is substituted with serine (SEQ ID NO: 25) (hereinafter, sometimes referred to as "C538S variant"); a sequence in which amino acids are deleted at the N-terminus and the C-terminus of the full length RBD sequence (R319-F541) (SEQ ID NO: 29); a sequence in which amino acids are added at the N-terminus and the C-terminus of the full length RBD sequence (R319-F541) (SEQ ID NO: 33); or a sequence in which a mutation (substitution) of a plurality of amino acid residues has been introduced (SEQ ID NO: 37) may be enumerated. These sequences may have a secretory peptide (e.g., S protein signal sequence) added thereto. Amino acid sequences in which S protein signal sequence is added to SEQ ID NOS: 25, 29, 33 and 37 are shown in SEQ ID NOS: 24, 28, 32 and 36, respectively.

The receptor-binding domain existing in the S protein of SARS-CoV-2 may be derived from variants. Amino acid sequences of the receptor binding protein of South African variant, UK variant, Brazilian variant, Californian variant, Indian variant, South African C538S variant, UK C538S variant, Brazilian C538S variant, Californian C538S variant, Indian C538S variant, combination variant (1) (see Example 33 described later), combination variant (2) (see Example 33 described later), combination variant (3) (see Example 33 described later), and combination variant (4) (see Example 33 described later) are shown in SEQ ID NOS: 94 to 107, respectively. Amino acid sequences consisting of an S protein signal sequence added to the amino acid sequences of SEQ ID NOS: 94 to 107 are shown in SEQ ID NOS: 80 to 93, respectively.

The nucleic acid molecule to be encapsulated in lipid particles may be one capable of expressing the receptor-binding domain in the S protein of SARS-CoV-2 consisting of an amino acid sequence having at least 95%, preferably 96% and more preferably 97% identity with the amino acid sequence (not comprising S protein signal sequence) as

shown in any one of SEQ ID NOS: 25, 29, 33, 37, and 94 to 107. Alternatively, the nucleic acid molecule to be encapsulated in lipid particles may also be one capable of expressing the receptor-binding domain in the S protein of SARS-CoV-2 consisting of an amino acid sequence having at least 95%, preferably 96% and more preferably 97% identity with the amino acid sequence (comprising S protein signal sequence) as shown in any one of SEQ ID NOS: 24, 28, 32, 36, and 80 to 93.

The nucleic acid molecule capable of expressing the S protein of SARS-CoV-2 may be an mRNA molecule comprising a cap structure (Cap), 5' untranslated region (5'-UTR), S protein coding region, 3' untranslated region (3'-UTR) and a polyA tail (polyA). A cap structure (Cap) is found at the 5' end of mRNA of many eukaryotes. This is a moiety having a 7-methylguanosine structure. Specific examples of the cap structure include, but are not limited to, cap0, cap1, cap2, ARCA or CleanCap™. As a cap structure of the mRNA of the present invention, cap1 or CleanCap is preferable, with CleanCap being more preferable. As an exemplary example of the sequence of 5' untranslated region (5'-UTR), a sequence represented by nucleotide numbers 19 to 88 in SEQ ID NO: 4 may be given. The sequence of S protein coding region is a sequence which is capable of expressing the whole or part of the amino acid sequence of the S protein, and may comprise a start codon and/or a stop codon. As an exemplary example of such sequence, a sequence represented by nucleotide numbers 89 to 3910 in SEQ ID NO: 4 may be given. Alternatively, the sequence of S protein coding region may also be a nucleotide sequence having at least 90% identity with the sequence of S protein coding region in SEQ ID NO: 5. As an exemplary example of the sequence of 3' untranslated region (3'-UTR), a sequence represented by nucleotide numbers 3911 to 4042 in SEQ ID NO: 4 may be given. As an exemplary example of the sequence of polyA tail (polyA), a sequence represented by nucleotide numbers 4043 to 4142 in SEQ ID NO: 4 may be given. Sequences of the cap structure (Cap), 5' untranslated region (5'-UTR), S protein coding region, 3' untranslated region (3'-UTR) and polyA tail (polyA) may be modified; and the sequence of a nucleic acid molecule capable of expressing the S protein of SARS-CoV-2 may consist of a nucleotide sequence having at least 90%, preferably 95% and more preferably 97% identity with the sequence as shown in SEQ ID NO: 5. Most preferably, the sequence of a nucleic acid molecule capable of expressing the S protein of SARS-CoV-2 consists of the nucleotide sequence as shown in SEQ ID NO: 5. Codons in the nucleic acid molecule may be suitably optimized. By optimizing codons, it may be possible to improve the efficacy as a vaccine and to reduce adverse effects. Codons may be optimized based on the codon usage frequency in the target organism. Optimization of codons may be performed, for example, in coding sequences. In the sequence as shown in SEQ ID NO: 16, codons in the sequence of S protein coding region are optimized. The sequence of a nucleic acid molecule capable of expressing the S protein of SARS-CoV-2 may conveniently consist of a nucleotide sequence having at least 90%, preferably 95% and more preferably 97% identity with the sequence as shown in SEQ ID NO: 16.

The nucleic acid molecule capable of expressing a fragment of the S protein of SARS-CoV-2 may be an mRNA molecule comprising a cap structure (Cap), 5' untranslated region (5'-UTR), a leader sequence, the coding region of the receptor-binding domain in the S protein, 3' untranslated region (3'-UTR) and a polyA tail (polyA). A cap structure (Cap) is found at the 5' end of mRNA of many eukaryotes. This is a moiety having a 7-methylguanosine structure. Specific examples of the cap structure include, but are not limited to, cap0, cap1, cap2, ARCA or CleanCap™. As a cap structure of the mRNA of the present invention, cap 1 or CleanCap is preferable, with CleanCap being more preferable. As an exemplary example of the sequence of 5' untranslated region (5'-UTR), a sequence represented by nucleotide numbers 19 to 88 in SEQ ID NO: 8 may be given. As an exemplary example of the leader sequence, a sequence represented by nucleotide numbers 89 to 127 in SEQ ID NO: 8 may be given. The sequence of the coding region of the receptor-binding domain in the S protein is a sequence which is capable of expressing the whole or part of the amino acid sequence of receptor-binding domain in the S protein, and may comprise a start codon and/or a stop codon. As an exemplary example of such sequence, a sequence represented by nucleotide numbers 128 to 799 in SEQ ID NO: 8 may be given. Alternatively, the sequence of the coding region of the receptor-binding domain in the S protein may also be a nucleotide sequence having at least 90% identity with the sequence of the coding region of the receptor-binding domain in S protein in SEQ ID NO: 9. As an exemplary example of the sequence of 3' untranslated region (3'-UTR), a sequence represented by nucleotide numbers 800 to 931 in SEQ ID NO: 8 may be given. As an exemplary example of the sequence of polyA tail (polyA), a sequence represented by nucleotide numbers 932 to 1031 in SEQ ID NO: 8 may be given. Sequences of the cap structure (Cap), 5' untranslated region (5'-UTR), the leader sequence, the coding region of the receptor-binding domain in the S protein, 3' untranslated region (3'-UTR) and polyA tail (polyA) may be modified; and the sequence of a nucleic acid molecule capable of expressing the receptor-binding domain in the S protein of SARS-CoV-2 may consist of a nucleotide sequence having at least 90%, preferably 95% and more preferably 97% identity with the sequence as shown in SEQ ID NO: 9. Most preferably, the sequence of a nucleic acid molecule capable of expressing the receptor-binding domain in the S protein of SARS-CoV-2 consists of the nucleotide sequence as shown in SEQ ID NO: 9. Codons in the nucleic acid molecule may be suitably optimized. By optimizing codons, it may be possible to improve the efficacy as a vaccine and to reduce adverse effects. Codons may be optimized based on the codon usage frequency in the target organism. Optimization of codons may be performed, for example, in coding sequences. In the sequence as shown in SEQ ID NO: 19, codons in the sequence of the coding region of the receptor-binding domain in the S protein are optimized. The sequence of a nucleic acid molecule capable of expressing the receptor-binding domain in the S protein of SARS-CoV-2 may conveniently consist of a nucleotide sequence having at least 90%, preferably 95% and more preferably 97% identity with the sequence as shown in SEQ ID NO: 19. Further, the sequence of the coding region of the receptor-binding domain in the S protein may be a nucleotide sequence having at least 90%, preferably 95% and more preferably 97% identity with the sequence of the coding region of the receptor-binding domain in the S protein in any one of SEQ ID NOS: 21, 23, 27, 31, 35, and 66 to 79.

SEQ ID NO: 21 shows the nucleotide sequence of the mRNA of Example 11, which is identical in sequence with the mRNA of Example 6 except for the sequence of polyA. While polyA in the sequence of the mRNA of Example 6 has 110 adenine nucleotides, polyA in the mRNA of Example 11 has 50 adenine nucleotides. The nucleic acid molecule encapsulated in the lipid particle of the present invention may be an mRNA molecule with a relatively short polyA moiety. The number of adenine nucleotide may be preferably 30 or more, more preferably 40 or more, and still more preferably 50 or more. The upper limit of polyA is not particularly limited. Preferably, the upper limit is 500 or less, 400 or less, 300 or less, 200 or less, or 110 or less.

SEQ ID NO: 23 shows the nucleotide sequence of the mRNA molecule of Example 13, which is an mRNA molecule capable of expressing a sequence in which cysteine at position 538 (the number is counted from the N-terminus of S protein) is substituted with serine.

SEQ ID NO: 27 shows the nucleotide sequence of the mRNA molecule of Example 15, which is an mRNA molecule capable of expressing the full length RBD sequence (R319-F541) with deletions of amino acids at both N- and C-terminus.

SEQ ID NO: 31 shows the nucleotide sequence of the mRNA molecule of Example 17, which is an mRNA molecule capable of expressing the full length RBD sequence (R319-F541) with additions of amino acids at both N- and C-terminus.

SEQ ID NO: 35 shows the nucleotide sequence of the mRNA molecule of Example 19, which is an mRNA molecule capable of expressing a sequence in which substitution of amino acid residue has occurred at a plurality of sites in the sequence as shown in SEQ ID NO: 6.

SEQ ID NOS: 66 to 79 show the nucleotide sequences of mRNA molecules which, respectively, are capable of expressing the amino acid sequences of the receptor-binding domains of South African variant, UK variant, Brazilian variant, Californian variant, Indian variant, South African C538S variant, UK C538S variant, Brazilian C538S variant, Californian C538S variant, Indian C538S variant, combination variant (1) (see Example 33 described later), combination variant (2) (see Example 33 described later), combination variant (3) (see Example 33 described later) and combination variant (4) (see Example 33 described later).

The nucleic acid molecule to be encapsulated in the lipid particle may be in any form, as long as it is a nucleic acid molecule capable of expressing the S protein and/or a fragment thereof of SARS-CoV-2. Examples that may be enumerated include single-stranded DNA, single-stranded RNA (e.g., mRNA), single-stranded polynucleotide in which DNA and RNA are mixed, double-stranded DNA, double-stranded RNA, hybrid polynucleotide of DNA-RNA, and double-stranded polynucleotide consisting of two types of polynucleotides in which DNA and RNA are mixed. Preferably, mRNA is used.

Nucleotides constituting the nucleic acid molecule to be encapsulated in the lipid particle may be either natural or modified nucleotides. Preferably, at least one of the nucleotides is a modified nucleotide.

Modified nucleotides may be modified in any moiety, i.e., base, sugar or phosphodiester bond. The modification may be at either one or two or more sites.

Examples of modified bases include, but are not limited to, cytosine as 5-methylated, 5-fluorinated or N4-methylated; uracil as 5-methylated (thymine) or 5-fluorinated; adenine as N6-methylated; and guanine as N2-methylated.

Examples of modified sugars include, but are not limited to, D-ribofuranose as 2'-O-methylated.

Examples of the modification of phosphodiester bond include, but are not limited to, phosphorothioate bond.

Preferably, modified nucleotides are those in which the base is modified. For example, 5-substituted pyrimidine nucleotide or pseudouridine optionally substituted at position 1 may be given. Specific examples of such modified nucleotide include, but are not limited to, 5-methylcytidine, 5-methoxyuridine, 5-methyluridine, pseudouridine and 1-alkylpseudouridine. As 1-alkylpseudouridine, 1-($C_1$-$C_6$ alkyl)pseudouridine may be given; and preferably, 1-methylpseudouridine or 1-ethylpseudouridine may be enumerated. Modified nucleotides in which the base is modified may be used alone or in combination of plurality of such modified nucleotides, instead of natural nucleotides. As examples of combinations of modified nucleotides in which the base is modified, a combination of 5-methylcytidine and 5-methyluridine, a combination of 5-methylcytidine and pseudouridine, or a combination of 5-methylcytidine and 1-methylpseudouridine may be given. Preferably, a combination of 5-methylcytidine and 5-methyluridine is used.

The nucleic acid molecule of the present invention capable of expressing the S protein and/or a fragment thereof of SARS-CoV-2 may be prepared from a DNA having a desired nucleotide sequence by *in vitro* transcription reaction. Enzymes, buffers and nucleoside-5'-triphosphate mixture [adenosine-5'-triphosphate (ATP), guanosine-5'-triphosphate (GTP), cytidine-5'-tripphosphate (CTP) and uridine-5'-triphosphate (UTP)] that are necessary for *in vitro* transcription are commercially available (AmpliScribeT7 High Yield Transcription Kit (Epicentre), mMESSAGE mMACHINE T7 Ultra

Kit (Life Technologies), and so forth). As regards the DNA to be used for preparing a single-stranded RNA, a cloned DNA (such as plasmid DNA or DNA fragment) is used. As regards plasmid DNA or DNA fragment, commercial products may be used. Alternatively, such DNA may be prepared by methods well known in the art (for example, see those methods described in Sambrook, J. et al., Molecular Cloning a Laboratory Manual second edition (1989); Rashtchian, A., Current Opinion in Biotechnology, 1995, 6(1), 30-36; and Gibson D. G. et al., Science, 2008, 319(5867), 1215-1220).

For the purpose of obtaining an mRNA with improved stability and/or safety, it is also possible to substitute the whole or part of unmodified nucleoside-5'-triphosphate with modified nucleoside-5'-triphosphate in *in vitro* transcription reaction to thereby substitute the whole or part of unmodified nucleotides in mRNA with modified nucleotides (Kormann, M., Nature Biotechnology, 2011, 29, 154-157).

For the purpose of obtaining an mRNA with improved stability and/or safety, it is also possible to introduce a cap structure (CapO structure as defined above) at the 5' end of mRNA after *in vitro* transcription reaction by a method using a capping enzyme. Further, it is possible to convert Cap0 to Cap1 by acting 2'-O-methyltransferase on mRNA having Cap0. As regards capping enzyme and 2'-O-methyltransferase, commercial products may be used (for example, Vaccinia Capping System, M2080 and mRNA Cap 2'-O-Methyltransferase, M0366, both of which are manufactured by New England Biolab). When commercial products are used, mRNA with a cap structure may be prepared according to the protocols attached to the products.

A cap structure at the 5' end of mRNA may also be introduced by a method different from the one using enzymes. For example, it is possible to introduce into mRNA the structure of a cap analogue which ARCA has or a Cap1 structure derived from CleanCap™ by adding ARCA or CleanCap™ to *in vitro* transcription reaction. As regards ARCA and CleanCap™, commercial products may be used (ARCA, N-7003 and CleanCap Reagent AG, N-7113, both of which are manufactured by TriLink BioTechnologies). When commercial products are used, mRNA with a cap structure may be prepared according to the protocols attached to the products.

The nucleic acid particle to be encapsulated in lipid particles in the present invention may suitably be purified by methods such as desalting, reversed phase column chromatography, gel filtration, HPLC, PAGE, or the like. Removal of impurities by purification treatment may potentially reduce the production of inflammatory cytokines in the living body which received the nucleic acid molecule.

As an exemplary example of the above impurities, double-stranded RNA (dsRNA) may be given. The amount of dsRNA contained in the nucleic acid molecule to be encapsulated in lipid particles is preferably 10% of less, more preferably 7.5% or less, still more preferably 5% or less, and especially preferably 3% or less in terms of mass percentage.

The lipid particle encapsulating a nucleic acid molecule according to the present invention may be prepared by various methods, such as a thin film method, a reverse phase evaporation method, an ethanol injection method, an ether injection method, a dehydration-rehydration method, a detergent dialysis method, a hydration method, a freezing-thawing method, and so forth. For example, the lipid particle encapsulating a nucleic acid molecule may be prepared by the methods described in WO2015/005253.

The mean particle size of the particle of the present invention may be 30 nm to 300 nm, preferably 30 nm to 200 nm, more preferably 30 nm to 150 nm, and still more preferably 30 nm to 100 nm. Mean particle size may be obtained by measuring volume mean particle size based on the principle of dynamic light scattering or the like using instruments such as Zeta Potential/Particle Sizer NICOMP™ 380ZLS (Particle Sizing Systems).

The particle of the present invention may be used for preparing a composition for preventing and/or treating infections with severe acute respiratory syndrome coronavirus 2 (SARS-CoV-2). The strain of SARS-CoV-2 is not particularly limited, with Wuhan strain being preferable.

It is possible to express the S protein and/or a fragment thereof of SARS-CoV-2 *in vivo* or *in vitro* using the particle of the present invention. Therefore, the present invention provides a method of expressing the S protein and/or a fragment thereof of SARS-CoV-2 *in vitro,* comprising introducing into cells a composition containing the above-described lipid particle. Further, the present invention also provides a method of expressing the S protein and/or a fragment thereof of SARS-CoV-2 *in vivo,* comprising administering to a mammal a composition containing the above-described lipid particle. By expressing the S protein and/or a fragment thereof of SARS-CoV-2 *in vivo,* it is possible to induce immune response to SARS-CoV-2. As a result, it becomes possible to prevent and/or treat infections with SARS-CoV-2. Therefore, the present invention provides a method of inducing immune response to SARS-CoV-2, comprising administering to a mammal a composition containing the above-described lipid particle. Further, the present invention provides a method of preventing and/or treating infections with SARS-CoV-2, comprising administering to a mammal a composition containing the above-described lipid particle.

The particle of the present invention may be used as a pharmaceutical drug or an experimental reagent. The particle of the present invention is usually added to a carrier (such as water, buffer, saline, etc.), and the resultant formulation (composition) may be introduced into a cell (*in vitro*) or administered to a mammal (*in vivo*). When the composition is administered to a mammal, the carrier may be a pharmacologically acceptable carrier (e.g., saline). Further, the particle of the present invention may also be prepared into such formulations as cream, paste, ointment, gel, lotion or the like that comprise fat, fatty oil, lanolin, vaseline, paraffin, wax, resin, plastic, glycols, higher alcohol,

glycerol, water, emulsifier, suspending agent, and the like as base materials.

The particle of the present invention may be administered to a mammal such as human, mouse, rat, hamster, guinea pig, rabbit, pig, monkey, cat, dog, horse, goat, sheep, cattle, etc. orally or parenterally through various routes such as intramuscular, intravenous, rectal, transdermal, transmucosal, subcutaneous or intradermal administration.

When the particle of the present invention is administered to a human, the particle may be administered, for example, at an approximate dose of 0.001-1 mg, preferably 0.01-0.2 mg (in terms of mRNA) per adult per administration either once or several times by intramuscular injection, subcutaneous injection, intradermal injection, intravenous infusion or intravenous injection. The dose and the number of times of administration may be changed appropriately depending on the type and symptoms of the disease, the age of the patient, administration route, etc.

When the particle of the present invention is used as an experimental reagent, it is possible to express the S protein and/or a fragment thereof of SARS-CoV-2 *in vitro* by introducing the particle into a cell in which expression of the S protein and/or a fragment thereof of SARS-CoV-2 is desired [e.g., HEK293 cells and cells derived therefrom (HEK293T cells, FreeStyle 293 cells, Expi293 cells, etc.), CHO cells, C2C12 mouse myoblast cells, immortalized mouse dendritic cells (MutuDC1940), or the like]. The expression of the S protein and/or a fragment thereof of SARS-CoV-2 may be analyzed by detecting the S protein and/or a fragment thereof of SARS-CoV-2 in samples based on Western blotting or by detecting peptide fragments specific to the S protein and/or a fragment thereof of SARS-CoV-2 based on mass spectrometry.

As used herein, the term "treat" refers to recovery, amelioration, relaxation and/or delaying the progression of clinical symptoms of diseases in patients who are developing infections with viruses or bacteria or diseases caused by such infections (e.g., pneumonia).

As used herein, the term "prevent" refers to reducing the incidence rate of diseases caused by infections with viruses or bacteria. "Prevent" encompasses lowering the risk of progression of diseases caused by infections with viruses or bacteria, or reducing exacerbation of such diseases. Since the particle of the present invention induces protective immune response, the particle of the present invention shows effectiveness on prevention and/or treatment of the above-described diseases.

[EXAMPLES]

Hereinbelow, the present invention will be described specifically with reference to the following examples. These examples are given only for explanation and are not intended to limit the scope of the present invention.

[Example 11 Preparation of SARS-CoV-2 S full mRNA-001

(1) Preparation of a template DNA for *in vitro* transcription (IVT) of SARS-CoV-2 S full

SARS-CoV-2 S full DNA was amplified by PCR and then purified in order to prepare a template DNA for *in vitro* transcription (IVT). Briefly, a DNA fragment (SEQ ID NO: 1) containing T7 promoter sequence, 5'-UTR sequence of human β-globin, KOZAK sequence, coding region of SARS-CoV-2 S full, 3'-UTR sequence of human β-globin was prepared by ligation in this order and then introduced into a plasmid to generate a plasmid of interest (pUC57mini-S full). This plasmid (6 ng) was dissolved in Nuclease-Free Water (849.6 μl). To this solution, 10x Buffer for KOD-Plus-Ver.2 (120 μl, Toyobo catalog #KOD-211), 2 mM dNTP mix (120 μl, Toyobo catalog #KOD-211), 25 mM $MgSO_4$ (72 μl, Toyobo catalog #KOD-211), 50 μM sense primer (7.2 μl, SEQ ID NO: 2), 50 μM antisense primer (7.2 μl, SEQ ID NO: 3) and KOD Plus polymerase (24 μl, Toyobo catalog #KOD-211) were added. The resultant mixture was incubated at 98°C for 1 minute, then subjected to 20 cycles of 98°C for 5 seconds, 55°C for 15 seconds, 68°C for 4 minutes, and finally incubated at 68°C for 1 minute, to thereby amplify S full DNA. After reaction, a template DNA (SEQ ID NO: 4) was purified with Wizard SV Gel and PCR Clean-Up System (Promega catalog #A9281).

(2) Preparation of SARS-CoV-2 S full mRNA-001 by *in vitro* transcription

The 360.5 μg/ml template DNA solution from Example 1-(1) (70 μl), 100 mM CleanCap AG (50 μl, TriLink catalog #T-7113), 100 mM ATP (50 μl, Hongene catalog #R1331), 100 mM GTP (50 μl, Hongene catalog #R2331),100 mM 5-Me-CTP (50 μl, Hongene catalog #R3-029), 100 mM 5-methyluridine triphosphate (50 μl), Nuclease-Free Water (380 μl, Thermo Fisher catalog #AM9937), T7 Transcription 5x buffer (200 μl, Promega catalog #P140X), Enzyme mix, and T7 RNA Polymerase (100 μl, Promega catalog #P137X) were mixed, and incubated at 37°C for 4 hours. RQ1 RNase-Free DNase (25 μl, Promega catalog #M6101) was added, and the resultant mixture was incubated at 37°C for 15 minutes. 8 M LiCl solution (500 μl, Sigma-Aldrich catalog #L7026) was also added, and the mixture was left to stand overnight at -20°C. After centrifugation (4°C, 4000 x g, 30 minutes), the supernatant was discarded and 70% ethanol was added to the precipitate. After centrifugation (4°C, 4000 x g, 30 minutes), the supernatant was discarded and 70%

ethanol was added to the precipitate. After centrifugation (4°C, 4000 x g, 10 minutes), the supernatant was discarded and the precipitate obtained was air-dried. The air-dried precipitate was dissolved in Nuclease-Free Water, followed by purification using RNeasy Maxi kit (Qiagen catalog #75162) according to the attached manual. The eluate obtained (5.8 ml; corresponding to 4906 μg DNA on the basis of UV absorbance), Nuclease-Free Water (419 μl), and rApid Alkaline Phosphatase (981 μl) and the buffer (800 μl) for this enzyme (Roche catalog #04 898 141 001) were mixed, incubated at 37°C for 30 minutes and then at 75°C for 3 minutes. 8M LiCl solution (8000 μl) was added, and the resultant mixture was left to stand overnight at -20°C. After centrifugation (4°C, 4000 x g, 30minutes), the supernatant was discarded and 70% ethanol was added to the precipitate. After centrifugation (4°C, 4000 x g, 10 minutes), the supernatant was discarded and the precipitate obtained was air-dried. The air-dried precipitate was dissolved in Nuclease-Free Water and purified with RNeasy Maxi kit according to the attached manual to thereby obtain the mRNA of interest.

The resultant mRNA has the sequence as shown in SEQ ID NO: 5. The mRNA was analyzed with LabChip GX Touch Standard RNA Reagent Kit (PerkinEhner catalog #CLS960010) to thereby confirm that the mRNA has an anticipated nucleotide length.

[Example 21 Preparation of SARS-CoV-2 RBD mRNA-002

(1) Preparation of a template DNA for *in vitro* transcription (IVT) of SARS-CoV-2 RBD

SARS-CoV-2 RBD DNA was amplified by PCR and then purified in order to prepare a template DNA for *in vitro* transcription (IVT). Briefly, a DNA fragment (SEQ ID NO: 7) containing T7 promoter sequence, 5'-UTR sequence of human β-globin, KOZAK sequence, signal sequence of SARS-CoV-2 S protein, coding region of SARS-CoV-2 RBD, and 3'-UTR sequence of human β-globin was prepared by ligation in this order and then introduced into a plasmid to generate a plasmid of interest (pUC57mini-RBD). This plasmid (6 ng) was dissolved in Nuclease-Free Water (849.6 μl). To this solution, 10x Buffer for KOD-Plus-Ver.2 (120 μl, Toyobo catalog #KOD-211), 2 mM dNTP mix (120 μl, Toyobo catalog #KOD-211), 25 mM $MgSO_4$ (72 μl, Toyobo catalog #KOD-211), 50 μM sense primer (7.2 μl, SEQ ID NO: 2), 50 μM antisense primer (7.2 μl, SEQ ID NO: 3) and KOD Plus polymerase (24 μl, Toyobo catalog #KOD-211) were added. The resultant mixture was incubated at 98°C for 1 minute, then subjected to 20 cycles of 98°C for 5 seconds, 55°C for 15 seconds, 68°C for 1 minute, and finally incubated at 68°C for 1 minute, to thereby amplify RBD DNA. After reaction, a template DNA (SEQ ID NO: 8) was purified with Wizard SV Gel and PCR Clean-Up System (Promega catalog #A9281).

(2) Preparation of SARS-CoV-2 RBD mRNA-002 by *in vitro* transcription

Using the template DNA from Example 2-(1) instead of the template DNA from Example 1-(1), the mRNA was obtained in the same manner as described in Example 1-(2).

The resultant mRNA has the sequence as shown in SEQ ID NO: 9. The mRNA was analyzed with LabChip GX Touch Standard RNA Reagent Kit to thereby confirm that the mRNA has an anticipated nucleotide length.

[Example 31 Preparation of nucleic acid lipid particles encapsulating the SARS-CoV-2 S full mRNA of Example 1

(1) Preparation of nucleic acid lipid particles encapsulating mRNA

Distearoyl phosphatidylcholine (1,2-Distearoyl-sn-glycero-3-phosphocholine; hereinafter, designated as DSPC; NOF CORPORATION), cholesterol (hereinafter, designated as Chol; Sigma-Aldrich, Inc.), (7R,9Z,26Z,29R)-18-({[3-(dimethylamino)propoxy]carbonyl}oxy)pentatriaconta-9,26-diene-7,29-diyl diacetate (a compound disclosed in Example 23 in WO2015/005253) (hereinafter, designated as LP) and 1,2-dimyristoyl-sn-glycerol-3-methoxypolyethylene glycol in which the polyethylene glycol part has the molecular weight of about 2000 (hereinafter, designated as PEG-DMG; NOF CORPORATION) were dissolved in ethanol so that a molar ratio of DSPC : Chol : LP : PEG-DMG is 12.5 : 41 : 45 : 1.5 to give a total lipid concentration of 5 mM.

On the other hand, SARS-CoV-2 S-full mRNA-001 obtained in Example 1 was diluted with 20 mM citrate buffer (pH 4.0) to prepare a solution of 52.7 μg/ml.

The lipid solution and the mRNA solution described above were mixed to give a volume ratio of 1:3 in a micro flow channel using NanoAssemblr BenchTop (Precision Nanosystems Inc.) to thereby obtain a crude dispersion of nucleic acid lipid particles. This dispersion was dialyzed against-about 25 to 50 volumes of 300 mM sucrose, 10 mM histidine buffer (pH 6.5) for 12 to 18 hours (Float-A-Lyzer G2, MWCO: 1,000 kD, Spectra/Por) to thereby remove ethanol and obtain a purified dispersion of nucleic acid lipid particles encapsulating mRNA.

LP was synthesized according to the method described in Example 23 of WO2015/005253.

(2) Characterization of nucleic acid lipid particles encapsulating mRNA

The dispersion containing the nucleic acid lipid particles prepared in (1) above was characterized. Methods of characterization of each property will be described below.

(2-1) Encapsulation rate of mRNA

Encapsulation rate of mRNA was measured with Quant-iT RiboGreen RNA Assay kit (Invitrogen) according to the attached protocol with necessary modifications.

Briefly, mRNA in the dispersion of nucleic acid lipid particles was quantified in the presence or absence of 0.015% Triton X-100 surfactant, and then encapsulation rate was calculated by the following formula.

{([amount of mRNA in the presence of surfactant] - [amount of mRNA in the absence of surfactant]) / [amount of mRNA in the presence of surfactant]} x 100(%).

(2-2) Ratio of mRNA and lipids

The amount of mRNA in the dispersion of nucleic acid lipid particles was measured by reversed phase chromatography (System: Agilent 1100 series; Column: Bioshell A400 Protein C4 (10 cm × 4.6 mm, 3.4 μm) (SUPELCO); Buffer A: 0.1 M triethylamine acetate (pH 7.0); Buffer B: acetonitrile; (B%): 5-50% (0-15 min); Flow Rate: 1 ml/min; Temperature: 70°C; Detection: 260 nm).

The amount of each lipid in the dispersion of nucleic acid lipid particles was measured by reversed phase chromatography (System: DIONEX UltiMate 3000; Column: XSelect CSH C18 (150 mm × 3 mm, 3.5 μm, 130 Å) (Waters catalog #186005263); Buffer A: 0.2% formic acid; Buffer B: 0.2% formic acid, methanol; (B%): 75-100% (0-6 min), 100% (6-15 min); Flow Rate: 0.45 ml/min; Temperature: 50°C; Detection: Corona CAD (Charged Aerosol Detector)).

The ratio of the total lipid to mRNA was calculated by the following formula.

[Total lipid concentration] / [mRNA concentration] (wt/wt)

(2-3) Mean particle size

The particle size of nucleic acid lipid particles in a dispersion was measured with Zeta Potential/Particle Sizer NICOMP™ 380ZLS (Particle Sizing Systems). The mean particle size in the Table below represents the volume mean particle sizes together with its deviation.

The results are shown in Table 1.

[Example 4] Preparation of nucleic acid lipid particles encapsulating the SARS-CoV-2 RBD mRNA of Example 2

Nucleic acid lipid particles encapsulating the mRNA described in Example 2 were prepared and characterized in the same manner as described in Example 3. The results are shown in Table 1.

(Table 1)

| Example | mRNA | DSPC/Chol/LP/PEG-DMG (mol %) | mRNA Encapsulation Rate | Lipid/mRNA (wt/wt) | Mean particle size (nm) |
|---|---|---|---|---|---|
| 3 | Example 1 | 12.5/41/45/1.5 | 97% | 17 | 112±29 |
| 4 | Example 2 | 12.5/41/45/1.5 | 96% | 19 | 118±34 |

The results shown in Table 1 clearly reveal that more than 90% of mRNA is encapsulated in lipid particles with mean particle sizes of approximately 100 to 130 nm.

[Example 5] Preparation of SARS-CoV-2 S full optimized mRNA-003

(1) Preparation of a template DNA for *in vitro* transcription of SARS-CoV-2 S full optimized

A DNA fragment (SEQ ID NO: 12) containing T7 promoter sequence, 5'-UTR sequence of human β-globin, KOZAK sequence, coding region of SARS-CoV-2 S full optimized, and 3'-UTR sequence of human β-globin ligated in this order was artificially synthesized and then introduced into a plasmid to generate a plasmid of interest (S_opt2 EcoRI). This plasmid (1 ng) was dissolved in Nuclease-Free Water (69 μl). To this solution, 5x SuperFi Green Buffer (20 μl, Thermo Fisher Scientific catalog #12357-010), 2.5 mM dNTP mix (8 μl, Takara Bio catalog #4030), 50 μM sense primer 2 (1 μl, SEQ ID NO: 13), 50 μM antisense primer 2 (1 μl, SEQ ID NO: 14) and Platinum SuperFi DNA Polymerase (1 μl, Thermo Fisher Scientific catalog #12357-010) were added. The resultant mixture was incubated at 98°C for 30 seconds, then subjected to 20 cycles of 98°C for 5 seconds, 60°C for 10 seconds, 72°C for 2 minutes, and finally incubated at 72°C for 1 minute, to thereby amplify a template DNA for SARS-CoV-2 S full optimized (SEQ ID NO: 15). This template DNA was digested with restriction enzymes NheI and HindIII and then introduced into a plasmid predigested with the same enzymes, to thereby prepare a template plasmid (pUCKIVT1 S full optimized). This plasmid was digested with a restriction enzyme BspQI and subjected to isopropanol precipitation to purify DNA. Thus, a linear plasmid DNA was prepared.

(2) Preparation of SARS-CoV-2 S full optimized mRNA-003 by *in vitro* transcription

Using the linear plasmid DNA from Example 5-(1) instead of the template DNA from Example 1-(1), the mRNA was obtained in the same manner as described in Example 1-(2).

The resultant mRNA has the sequence as shown in SEQ ID NO: 16. The mRNA was analyzed with LabChip GX Touch Standard RNA Reagent Kit to thereby confirm that the mRNA has an anticipated nucleotide length.

[Example 6] Preparation of SARS-CoV-2 RBD optimized mRNA-004

(1) Preparation of a template DNA for *in vitro* transcription (IVT) of SARS-CoV-2 RBD optimized

A DNA fragment (SEQ ID NO: 17) containing T7 promoter sequence, 5'-UTR sequence of human β-globin, KOZAK sequence, coding region of SARS-CoV-2 RBD optimized, and 3'-UTR sequence of human β-globin ligated in this order was artificially synthesized and then introduced into a plasmid to generate a plasmid of interest (S_RBD_opt2 EcoRI). This plasmid (1 ng) was dissolved in Nuclease-Free Water (69 μl). To this solution, 5x SuperFi Green Buffer (20 μl, Thermo Fisher Scientific catalog #12357-010), 2.5 mM dNTP mix (8 μl, Takara Bio catalog #4030), 50 μM sense primer 2 (1 μl, SEQ ID NO: 13), 50 μM antisense primer 2 (1 μl, SEQ ID NO: 14) and Platinum SuperFi DNA Polymerase (1 μl, Thermo Fisher Scientific catalog #12357-010) were added. The resultant mixture was incubated at 98°C for 30 seconds, then subjected to 20 cycles of 98°C for 5 seconds, 60°C for 10 seconds, 72°C for 1 minute, and finally incubated at 72°C for 1 minute, to thereby amplify SARS-CoV-2 S RBD optimized DNA (SEQ ID NO: 18). This template DNA was digested with restriction enzymes NheI and HindIII and then introduced into a plasmid predigested with the same enzymes, to thereby prepare a template plasmid (pUCKIVT1-RBD optimized). This plasmid was digested with a restriction enzyme BspQI and subjected to isopropanol precipitation to purify DNA. Thus, a linear plasmid DNA was prepared.

(2) Preparation of SARS-CoV-2 RBD optimized mRNA-004 by *in vitro* transcription

Using the linear plasmid DNA from Example 6-(1) instead of the template DNA from Example 1-(1), the mRNA was obtained in the same manner as described in Example 1-(2).

The resultant mRNA has the sequence as shown in SEQ ID NO: 19. The mRNA was analyzed with LabChip GX Touch Standard RNA Reagent Kit to thereby confirm that the mRNA has an anticipated nucleotide length.

[Example 71 Preparation of nucleic acid lipid particles encapsulating the SARS-CoV-2 S full optimized mRNA of Example 5

Nucleic acid lipid particles encapsulating the mRNA described in Example 5 were prepared and characterized in the same manner as described in Example 3, except that dialysis was performed using 300 mM sucrose, 10 mM histidine buffer (pH 7.0) instead of 300 mM sucrose, 10 mM histidine buffer (pH 6.5) to obtain a dispersion of nucleic acid particles encapsulating the mRNA. The results are shown in Table 2.

[Example 8] Preparation of nucleic acid lipid particles encapsulating the SARS-CoV-2 RBD optimized mRNA of Example 6

Nucleic acid lipid particles encapsulating the mRNA described in Example 6 were prepared and characterized in the same manner as described in Example 3, except that dialysis was performed using 300 mM sucrose, 10 mM histidine buffer (pH 7.0) instead of 300 mM sucrose, 10 mM histidine buffer (pH 6.5) to obtain a dispersion of nucleic acid particles encapsulating the mRNA. The results are shown in Table 2.

[Example 9] HPLC purification of SARS CoV-2 RBD optimized mRNA-004

The mRNA obtained as described in Example 6-(2) was fractionated/purified by reversed phase column chromatography (YMC-Triart Bio C4 (YMC catalog #TB30S05-1510WT), 5% acetonitrile, 400 mM triethylamine acetate (pH 7.0)/25% acetonitrile, 400 mM triethylamine acetate (pH 7.0), 75°C).

[Example 101 Preparation of nucleic acid lipid particles encapsulating the SARS-CoV-2 RBD optimized mRNA of Example 6

Nucleic acid lipid particles encapsulating the mRNA described in Example 9 were prepared and characterized in the same manner as described in Example 8. The results are shown in Table 2.

(Table 2)

| Example | mRNA | DSPC/Chol/LP/PEG-DMG (mol %) | mRNA Encapsulation Rate | Lipid/mRNA (wt/wt) | Mean particle size (nm) |
|---|---|---|---|---|---|
| 7 | Example 3 | 12.5/41/45/1.5 | 99% | 20 | 117±25 |
| 8 | Example 4 | 12.5/41/45/1.5 | 98% | 19 | 116±21 |
| 10 | Example 9 | 12.5/41/45/1.5 | 99% | 19 | 96±9 |

The results shown in Table 2 clearly reveal that more than 90% of mRNA is encapsulated in lipid particles with mean particle sizes of approximately 90 to 130 nm.

[Example 111 Preparation of SARS-CoV-2 RBD S2000 mRNA

(1) Preparation of a template DNA for *in vitro* transcription (IVT) of SARS-CoV-2 RBD S2000

A plasmid was constructed in order to prepare a template DNA for *in vitro* transcription (IVT) of SARS-CoV-2 RBD S2000. Briefly, a DNA fragment (SEQ ID NO: 20) containing GCTAGC (NheI site), T7 promoter sequence, 5'-UTR sequence of human β-globin, KOZAK sequence, signal sequence of SARS-CoV-2 S protein, coding region of SARS-CoV-2 RBD, 3'-UTR sequence of human β-globin, polyA tail, and GAAGAGC (BspQI site) was prepared by ligation in this order and then introduced into a plasmid to generate a plasmid of interest (pUC57-S2000). This plasmid (100 μg) was dissolved in Nuclease-Free Water (860 μl, Thermo Fisher catalog #AM9937). To this solution, 10x NEB Buffer 3.1 (100 μl, New England Biolabs, catalog #R7203S) and BspQI (40 μl, New England Biolabs, catalog #R0712) were added, and the resultant mixture was incubated at 50°C for 1 hour. Isopropanol (1400 μl) was added to the incubated solution, which was then left to stand overnight at -80°C. After centrifugation (-8°C, 15,000 rpm, 10 minutes), the supernatant was discarded and the precipitate obtained was suspended in 70% ethanol. After centrifugation (-8°C, 15,000 rpm, 10 minutes), the supernatant was discarded and the resultant precipitate was air-dried. TE-Buffer (pH 8.0) was added to the dried precipitate to prepare a template DNA solution of 500 μg/ml.

(2) Preparation of SARS-CoV-2 RBD S2000 mRNA by *in vitro* transcription

Using the template DNA from Example 11-(1) instead of the template DNA from Example 1-(1), the mRNA was obtained in the same manner as described in Example 1-(2).

The resultant mRNA has the sequence as shown in SEQ ID NO: 21. The mRNA was analyzed with LabChip GX Touch Standard RNA Reagent Kit to thereby confirm that the mRNA has an anticipated nucleotide length.

[Example 12] Preparation of nucleic acid lipid particles encapsulating the SARS-CoV-2 RBD S2000 mRNA of Example 11

Nucleic acid lipid particles encapsulating the mRNA described in Example 11 were prepared and characterized in the same manner as described in Example 8, except that the amount of mRNA was measured by the method described below.

Briefly, a dispersion of nucleic acid lipid particles was diluted/dissolved in 90% methanol, and the amount of mRNA in the nucleic acid lipid particles was measured with a Perkin Elmer UV-visible spectrophotometer (LAMBDA™ 465). Then, the mRNA concentration was calculated by the following formula.

$$\{[\text{absorbance at 260 nm}] - [\text{absorbance at 350 nm}]\} \times 40 \times \text{dilution rate } (\mu g/ml)$$

The results are shown in Table 3. The results of characterization clearly reveal that more than 95% of mRNA is encapsulated in lipid particles with mean particle size of approximately 150 nm.

[Example 13] Preparation of SARS-CoV-2 RBD S2001 mRNA

(1) Preparation of a template DNA for *in vitro* transcription (IVT) of SARS-CoV-2 RBD S2001

A plasmid was constructed in order to prepare a template DNA for *in vitro* transcription (IVT) of SARS-CoV-2 RBD S2001. Briefly, a DNA fragment (SEQ ID NO: 22) containing GCTAGC (NheI site), T7 promoter sequence, 5'-UTR sequence of human β-globin, KOZAK sequence, signal sequence of SARS-CoV-2 S protein, coding region of SARS-CoV-2 RBD, 3'-UTR sequence of human β-globin, polyA tail, and GAAGAGC (BspQI site) was prepared by ligation in this order and then introduced into a plasmid to generate a plasmid of interest (pUC57-S2001). This plasmid (100 μg) was dissolved in Nuclease-Free Water (860 μl, Thermo Fisher catalog #AM9937). To this solution, 10x NEB Buffer 3.1 (100 μl, New England Biolabs, catalog #R7203S) and BspQI (40 μl, New England Biolabs, catalog #R0712) were added, and the resultant mixture was incubated at 50°C for 1 hour. Isopropanol (1400 μl) was added to the incubated solution, which was then left to stand overnight at -80°C. After centrifugation (-8°C, 15,000 rpm, 10 minutes), the supernatant was discarded and the precipitate obtained was suspended in 70% ethanol. After centrifugation (-8°C, 15,000 rpm, 10 minutes), the supernatant was discarded and the resultant precipitate was air-dried. TE-Buffer (pH 8.0) was added to the dried precipitate to prepare a template DNA solution of 500 μg/ml.

(2) Preparation of SARS-CoV-2 RBD S2001 mRNA by *in vitro* transcription

Using the template DNA from Example 13-(1) instead of the template DNA from Example 1-(1), the mRNA was obtained in the same manner as described in Example 1-(2).

The resultant mRNA has the sequence as shown in SEQ ID NO: 23. The mRNA was analyzed with LabChip GX Touch Standard RNA Reagent Kit to thereby confirm that the mRNA has an anticipated nucleotide length.

[Example 14] Preparation of nucleic acid lipid particles encapsulating the SARS-CoV-2 RBD S2001 mRNA of Example 13

Nucleic acid lipid particles encapsulating the mRNA described in Example 13 were prepared and characterized in the same manner as described in Example 12. The results are shown in Table 3. The results of characterization clearly reveal that more than 95% of mRNA is encapsulated in lipid particles with mean particle size of approximately 140 nm.

[Example 15] Preparation of SARS-CoV-2 RBD S2002 mRNA

(1) Preparation of a template DNA for *in vitro* transcription (IVT) of SARS-CoV-2 RBD S2002

A plasmid was constructed in order to prepare a template DNA for *in vitro* transcription (IVT) of SARS-CoV-2 RBD S2002. Briefly, a DNA fragment (SEQ ID NO: 26) containing GCTAGC (NheI site), T7 promoter sequence, 5'-UTR sequence of human β-globin, KOZAK sequence, signal sequence of SARS-CoV-2 S protein, coding region of SARS-CoV-2 RBD, 3'-UTR sequence of human β-globin, polyA tail, and GAAGAGC (BspQI site) was prepared by ligation in this order and then introduced into a plasmid to generate a plasmid of interest (pUC57-S2002). This plasmid (100 μg) was dissolved in Nuclease-Free Water (860 μl, Thermo Fisher catalog #AM9937). To this solution, 10x NEB Buffer 3.1 (100 μl, New England Biolabs, catalog #R7203S) and BspQI (40 μl, New England Biolabs, catalog #R0712) were added, and the resultant mixture was incubated at 50°C for 1 hour. Isopropanol (1400 μl) was added to the incubated solution, which was then left to stand overnight at -80°C. After centrifugation (-8°C, 15,000 rpm, 10 minutes), the supernatant

was discarded and the precipitate obtained was suspended in 70% ethanol. After centrifugation (-8°C, 15,000 rpm, 10 minutes), the supernatant was discarded and the resultant precipitate was air-dried. TE-Buffer (pH 8.0) was added to the dried precipitate to prepare a template DNA solution of 500 μg/ml.

(2) Preparation of SARS-CoV-2 RBD S2002 mRNA by *in vitro* transcription

Using the template DNA from Example 15-(1) instead of the template DNA from Example 1-(1), the mRNA was obtained in the same manner as described in Example 1-(2).

The resultant mRNA has the sequence as shown in SEQ ID NO: 27. The mRNA was analyzed with LabChip GX Touch Standard RNA Reagent Kit to thereby confirm that the mRNA has an anticipated nucleotide length.

[Example 16] Preparation of nucleic acid lipid particles encapsulating the SARS CoV-2 RBD S2002 mRNA of Example 15

Nucleic acid lipid particles encapsulating the mRNA described in Example 15 were prepared and characterized in the same manner as described in Example 12. The results are shown in Table 3. The results of characterization clearly reveal that more than 95% of mRNA is encapsulated in lipid particles with mean particle size of approximately 140 nm.

[Example 17] Preparation of SARS-CoV-2 RBD S2003 mRNA

(1) Preparation of a template DNA for *in vitro* transcription (IVT) of SARS-CoV-2 RBD S2003

A plasmid was constructed in order to prepare a template DNA for *in vitro* transcription (IVT) of SARS-CoV-2 RBD S2003. Briefly, a DNA fragment (SEQ ID NO: 30) containing GCTAGC (NheI site), T7 promoter sequence, 5'-UTR sequence of human β-globin, KOZAK sequence, signal sequence of SARS-CoV-2 S protein, coding region of SARS-CoV-2 RBD, 3'-UTR sequence of human β-globin, polyA tail, and GAAGAGC (BspQI site) was prepared by ligation in this order and then introduced into a plasmid to generate a plasmid of interest (pCC1-S2003). This plasmid (100 μg) was dissolved in Nuclease-Free Water (860 μl, Thermo Fisher catalog #AM9937). To this solution, 10x NEB Buffer 3.1 (100 μl, New England Biolabs, catalog #R7203S) and BspQI (40 μl, New England Biolabs, catalog #R0712) were added, and the resultant mixture was incubated at 50°C for 1 hour. Isopropanol (1400 μl) was added to the incubated solution, which was then left to stand overnight at -80°C. After centrifugation (-8°C, 15,000 rpm, 10 minutes), the supernatant was discarded and the precipitate obtained was suspended in 70% ethanol. After centrifugation (-8°C, 15,000 rpm, 10 minutes), the supernatant was discarded and the resultant precipitate was air-dried. TE-Buffer (pH 8.0) was added to the dried precipitate to prepare a template DNA solution of 500 μg/ml.

(2) Preparation of SARS-CoV-2 RBD S2003 mRNA by *in vitro* transcription

Using the template DNA from Example 17-(1) instead of the template DNA from Example 1-(1), the mRNA was obtained in the same manner as described in Example 1-(2).

The resultant mRNA has the sequence as shown in SEQ ID NO: 31. The mRNA was analyzed with LabChip GX Touch Standard RNA Reagent Kit to thereby confirm that the mRNA has an anticipated nucleotide length.

[Example 18] Preparation of nucleic acid lipid particles encapsulating the SARS-CoV-2 RBD S2003 mRNA of Example 17

Nucleic acid lipid particles encapsulating the mRNA described in Example 17 were prepared and characterized in the same manner as described in Example 12. The results are shown in Table 3. The results of characterization clearly reveal that more than 95% of mRNA is encapsulated in lipid particles with mean particle size of approximately 140 nm.

[Example 19] Preparation of SARS-CoV-2 RBD S2004 mRNA

(1) Preparation of a template DNA for *in vitro* transcription (IVT) of SARS-CoV-2 RBD S2004

A plasmid was constructed in order to prepare a template DNA for *in vitro* transcription (IVT) of SARS-CoV-2 RBD S2004. Briefly, a DNA fragment (SEQ ID NO: 34) containing GCTAGC (NheI site), T7 promoter sequence, 5'-UTR sequence of human β-globin, KOZAK sequence, signal sequence of SARS-CoV-2 S protein, coding region of SARS-CoV-2 RBD, 3'-UTR sequence of human β-globin, polyA tail, and GAAGAGC (BspQI site) was prepared by ligation in this order and then introduced into a plasmid to generate a plasmid of interest (pCC1-S2004). This plasmid (100 μg) was dissolved in Nuclease-Free Water (860 μl, Thermo Fisher catalog #AM9937). To this solution, 10x NEB Buffer 3.1 (100 μl, New England Biolabs, catalog #R7203S) and BspQI (40 μl, New England Biolabs, catalog #R0712) were added,

and the resultant mixture was incubated at 50°C for 1 hour. Isopropanol (1400 μl) was added to the incubated solution, which was then left to stand overnight at -80°C. After centrifugation (-8°C, 15,000 rpm, 10 minutes), the supernatant was discarded and the precipitate obtained was suspended in 70% ethanol. After centrifugation (-8°C, 15,000 rpm, 10 minutes), the supernatant was discarded and the resultant precipitate was air-dried. TE-Buffer (pH 8.0) was added to the dried precipitate to prepare a template DNA solution of 500 μg/ml.

(2) Preparation of SARS-CoV-2 RBD S2004 mRNA by *in vitro* transcription

Using the template DNA from Example 19-(1) instead of the template DNA from Example 1-(1), the mRNA was obtained in the same manner as described in Example 1-(2).

The resultant mRNA has the sequence as shown in SEQ ID NO: 35. The mRNA was analyzed with LabChip GX Touch Standard RNA Reagent Kit to thereby confirm that the mRNA has an anticipated nucleotide length.

[Example 20] Preparation of nucleic acid lipid particles encapsulating the SARS-CoV-2 RBD S2004 mRNA of Example 19

Nucleic acid lipid particles encapsulating the mRNA described in Example 19 were prepared and characterized in the same manner as described in Example 12. The results are shown in Table 3. The results of characterization clearly reveal that more than 95% of mRNA is encapsulated in lipid particles with mean particle size of approximately 180 nm.

[Examples 21 to 301 Preparation of nucleic acid lipid particles encapsulating the mRNA of Example 6

(1) Preparation of nucleic acid lipid particles encapsulating mRNA

Distearoyl phosphatidylcholine (DSPC), cholesterol, (7R,9Z,26Z,29R)-18-({[3-(dimethylamino)propoxy]carbonyl}oxy)pentatriaconta-9,26-diene-7,29-diyl diacetate (LP) and 1,2-dimyristoyl-sn-glycerol methoxypolyethylene glycol in which the polyethylene glycol part has the molecular weight of about 2000 (PEG-DMG) were dissolved in ethanol at the molar ratios indicated in Table 4 to give a total lipid concentration of 5 mM.

On the other hand, the mRNA obtained in Example 6 was diluted with 20 mM citrate buffer (pH 4.0) to prepare mRNA solutions.

The lipid solutions and the mRNA solutions described above were mixed in a micro flow channel using NanoAssemblr BenchTop (Precision Nanosystems Inc.) so that the weight ratio of the total lipid to mRNA (Lipids/mRNA) would assume the values indicated in Table 4 and yet their volume ratio would be 1:3, whereby crude dispersions of nucleic acid lipid particles were obtained. These dispersions were dialyzed against-about 25 to 50 volumes of buffer for 12 to 18 hours (Float-A-Lyzer G2, MWCO: 1,000 kD, Spectra/Por) to thereby remove ethanol and obtain purified dispersions of nucleic acid lipid particles encapsulating mRNA.

(2) Characterization of nucleic acid lipid particles encapsulating mRNA

The dispersions containing the nucleic acid lipid particles as prepared in (1) above were characterized. Methods of characterization of each property are described below.

(2-1) Encapsulation rate of mRNA

Encapsulation rate of mRNA was measured with Quant-iT RiboGreen RNA Assay kit (Invitrogen) according to the attached protocol with necessary modifications.

Briefly, mRNA in the dispersions of nucleic acid lipid particles was quantified in the presence or absence of 0.015% Triton X-100 surfactant, and then encapsulation rate was calculated by the following formula.

$${([\text{amount of mRNA in the presence of surfactant}] - [\text{amount of mRNA in the absence of surfactant}]) / [\text{amount of mRNA in the presence of surfactant}]} \times 100(\%).$$

(2-2) Ratio of mRNA and lipids

The amount of mRNA in the dispersions of nucleic acid lipid particles was measured with a UV-visible spectrophotometer. Briefly, the dispersions of nucleic acid lipid particles were diluted/dissolved in 90% methanol, and the amount of mRNA in the nucleic acid lipid particles was measured with a Perkin Elmer UV-visible spectrophotometer

(LAMBDA™ 465). Then, the mRNA concentration was calculated by the following formula.

$$\{[\text{absorbance at 260 nm}] - [\text{absorbance at 350 nm}]\} \times 40 \times \text{dilution rate } (\mu g/ml)$$

The amount of each lipid in the dispersions of nucleic acid lipid particles was measured by reversed phase chromatography (System: DIONEX UltiMate 3000; Column: XSelect CSH C18 (130 Å, 3.5 μm, 3.0 mm × 150 mm) (Waters catalog #186005263); Buffer A: 0.2% formic acid; Buffer B: 0.2% formic acid, methanol; (B%): 75-100% (0-6 min), 100% (6-15 min); Flow Rate: 0.45 ml/min; Temperature: 50°C; Detection: Corona CAD (Charged Aerosol Detector)).

The ratio of the total lipid to mRNA was calculated by the following formula.

$$[\text{Total lipid concentration}] / [\text{mRNA concentration}] \text{ (wt/wt)}$$

(2-3) Mean particle size

The particle size of nucleic acid lipid particles was measured with Zeta Potential/Particle Sizer NICOMP™ 380ZLS (Particle Sizing Systems). The tabulated mean particle size represents the volume mean particle size, with the numerals after ± representing the deviation.

The results of characterization are shown in Table 5. These results clearly show that more than 95% of mRNA is encapsulated in lipid particles with mean particle sizes of approximately 90 nm to 140 nm.

[Example 31] Preparation of SARS-CoV-2 variant RBD mRNA

With respect to RBDs having mutations as indicated in Table 6, SARS-CoV-2 RBD mRNA was prepared. Those alphabetical marks which come after Example numbers in Table 7 correspond to respective variants as shown in Table 6. For example, "Example 32-a" represents the nucleic acid lipid particles obtained in Example 32 encapsulating mRNA having mutations of South African variant.

(1) Preparation of a template DNA for *in vitro* transcription (IVT) of SARS-CoV-2 variant RBD

SARS-CoV-2 variant RBD DNA was amplified by PCR and then purified in order to prepare a template DNA for *in vitro* transcription (IVT). Briefly, a DNA fragment (SEQ ID NO: 38) containing T7 promoter sequence, 5'-UTR sequence of human β-globin, KOZAK sequence, signal sequence of SARS-CoV-2 S protein, coding region of SARS-CoV-2 variant RBD, and 3'-UTR sequence of human β-globin was prepared by ligation in this order and then introduced into a plasmid to generate a plasmid of interest (pUC57mini-variant RBD). This plasmid (10 ng) was dissolved in Nuclease-Free Water (566.4 μl). To this solution, 10x Buffer for KOD-Plus-Ver.2 (80 μl, Toyobo catalog #KOD-211), 2 mM dNTP mix (80 μl, Toyobo catalog #KOD-211), 25 mM MgSOa (48 μl, Toyobo catalog #KOD-211), 50 μM sense primer (4.8 μl, SEQ ID NO: 2), 50 μM antisense primer (4.8 μl, SEQ ID NO: 3) and KOD Plus polymerase (16 μl, Toyobo catalog #KOD-211) were added. The resultant mixture was incubated at 98°C for 15 seconds, then subjected to 20 cycles of 98°C for 5 seconds, 55°C for 15 seconds, 68°C for 1 minute, and finally incubated at 68°C for 1 minute, to thereby amplify RBD DNA. After reaction, a template DNA (SEQ ID NO: 52) was purified with Wizard SV Gel and PCR Clean-Up System (Promega catalog #A9281).

Using DNA fragments of SEQ ID NOS: 39 to 41, 43 and 48 to 51 instead of the DNA fragment of SEQ ID NO: 38, template DNAs of SEQ ID NOS: 53 to 55, 57, and 62 to 65 were obtained, respectively, in the same manner.

(2) Preparation of SARS-CoV-2 variant RBD mRNA by *in vitro* transcription

Using the template DNA (SEQ ID NO: 52) from Example 31-(1) instead of the template DNA from Example 1-(1), the mRNA was obtained in the same manner as described in Example 1-(2).

The resultant mRNA has the sequence as shown in SEQ ID NO: 66. The mRNA was analyzed with LabChip GX Touch Standard RNA Reagent Kit to thereby confirm that the mRNA has an anticipated nucleotide length.

Using template DNAs of SEQ ID NOS: 53 to 55, 57 and 62 to 65 instead of the template DNA (SEQ ID NO: 52), mRNA molecules of SEQ ID NOS: 67 to 69, 71 and 76 to 79 were obtained, respectively, in the same manner.

[Example 32] Preparation of nucleic acid lipid particles encapsulating the SARS-CoV-2 RBD mRNA of Example 31

Nucleic acid lipid particles encapsulating the mRNA described in Example 31 were prepared and characterized

in the same manner as described in Example 8. The results are shown in Table 7.

The results of characterization clearly show that more than 95% of mRNA is encapsulated in lipid particles with mean particle sizes of approximately 110 nm to 130 nm.

[Example 33] Preparation of nucleic acid lipid particles encapsulating the SARS-CoV-2 RBD mRNA of Example 6

Nucleic acid lipid particles encapsulating the mRNA described in Example 6 were prepared and characterized in the same manner as described in Example 8. The results are shown in Table 7.

The results of characterization clearly show that more than 95% of mRNA is encapsulated in lipid particles with mean particle size of approximately 110 nm.

[Table 3]

| Example | mRNA | mRNA Encapsulation Rate | Lipid/mRNA (wt/wt) | Mean Particle Size (nm) |
|---|---|---|---|---|
| Example 12 | Example 11 | 98% | 17 | 146±19 |
| Example 14 | Example 13 | 99% | 18 | 142±24 |
| Example 16 | Example 15 | 97% | 18 | 144±25 |
| Example 18 | Example 17 | 98% | 19 | 136±19 |
| Example 20 | Example 19 | 99% | 17 | 178±59 |

[Table 4]

| Example No. | mRNA | DSPC | Chol | LP | PEG-DMG | Lipids/mRNA (wt/wt) |
|---|---|---|---|---|---|---|
| Example 21 | Example 6 | 10.0% | 43.5% | 45.0% | 1.50% | 20 |
| Example 22 | Example 6 | 15.0% | 38.5% | 45.0% | 1.50% | 20 |
| Example 23 | Example 6 | 13.5% | 35.0% | 50.0% | 1.50% | 20 |
| Example 24 | Example 6 | 10.0% | 45.0% | 43.5% | 1.50% | 20 |
| Example 25 | Example 6 | 13.5% | 45.0% | 40.0% | 1.50% | 20 |
| Example 26 | Example 6 | 10.0% | 38.5% | 50.0% | 1.50% | 20 |
| Example 27 | Example 6 | 12.5% | 41.25% | 45.0% | 1.25% | 20 |
| Example 28 | Example 6 | 12.5% | 40.5% | 45.0% | 2.00% | 20 |
| Example 29 | Example 6 | 12.5% | 41.0% | 45.0% | 1.50% | 17.5 |
| Example 30 | Example 6 | 12.5% | 41.0% | 45.0% | 1.50% | 22.5 |

[Table 5]

| Example | mRNA | mRNA Encapsulation Rate | Lipid/mRNA (wt/wt) | Mean Particle Size (nm) |
|---|---|---|---|---|
| Example 21 | Example 6 | 99% | 20 | 127±53 |
| Example 22 | Example 6 | 99% | 20 | 110±44 |
| Example 23 | Example 6 | 99% | 20 | 110±24 |
| Example 24 | Example 6 | 99% | 21 | 130±26 |
| Example 25 | Example 6 | 99% | 20 | 130±57 |
| Example 26 | Example 6 | 99% | 20 | 108±44 |
| Example 27 | Example 6 | 99% | 20 | 139±51 |
| Example 28 | Example 6 | 99% | 23 | 92±20 |
| Example 29 | Example 6 | 99% | 17 | 105±28 |

(continued)

| Example | mRNA | mRNA Encapsulation Rate | Lipid/mRNA (wt/wt) | Mean Particle Size (nm) |
|---|---|---|---|---|
| Example 30 | Example 6 | 99% | 21 | 109±44 |

[Table 6]

| Designation of Variant | Site of Mutation | Sequence Identification Mark |
|---|---|---|
| South African variant | K417N, E484K, N501Y | a |
| UK variant | N501Y | b |
| Brazilian variant | K417T,E484K, N501Y | c |
| Californian variant | L452R | d |
| Indian variant | L452R, E484Q | e |
| South African C538S variant | K417N, E484K, N501Y, C538S | f |
| UK C538S variant | N501Y, C538S | g |
| Brazilian C538S variant | K417T, E484K, N501Y, C538S | h |
| Californian C538S variant | L452R, C538S | i |
| Indian C538S variant | L452R, E484Q, C538S | j |
| Combination variant (1) | N439K, L452R, A475V, V483A | k |
| Combination variant (2) | S477R, F490L | 1 |
| Combination variant (3) | K417N, S477R, E484K, N501Y | m |
| Combination variant (4) | F486S, F490L | n |

Table 7

| Example | Designation of Variant | SEQ ID NO | mRNA | mRNA Encapsulation Rate | Lipid/mRNA (wt/wt) | Mean Particle Size (nm) |
|---|---|---|---|---|---|---|
| Example 32-a | South African variant | 66 | Example 31-a | 99% | 20 | 121±25 |
| Example 32-f | South African C538S variant | 71 | Example 31-f | 99% | 20 | 111±38 |
| Example 32-b | UK variant | 67 | Example 31-b | 99% | 21 | 119±33 |
| Example 32-c | Brazilian variant | 68 | Example 31-c | 99% | 21 | 111±37 |
| Example 32-d | Californian variant | 69 | Example 31-d | 99% | 21 | 114±40 |
| Example 32-k | Combination variant (1) | 76 | Example 31-k | 99% | 21 | 114±33 |
| Example 32-l | Combination variant (2) | 77 | Example 31-l | 99% | 21 | 115±34 |
| Example 32-m | Combination variant (3) | 78 | Example 31-m | 99% | 21 | 115±17 |

(continued)

| Example | Designation of Variant | SEQ ID NO | mRNA | mRNA Encapsulation Rate | Lipid/mRNA (wt/wt) | Mean Particle Size (nm) |
|---|---|---|---|---|---|---|
| Example 32-n | Combination variant (4) | 79 | Example 31-n | 99% | 21 | 111±36 |
| Example 33 | - | - | Example 6 | 99% | 20 | 111±36 |

[Test Example 1]

Administration (Fig. 2 to Fig. 4)

A test substance was administered to mice in the calf of the hind limb under anesthesia with vaporized 1-4% (v/v) isoflurane. In 3-dose test, booster administration was performed 7 and 21 days after the first administration (1st time: right hind limb; 2nd time: left hind limb; 3rd time: right hind limb). In 2-dose test, booster administration was performed 13 days after the first administration (1st time: right hind limb; 2nd time: left hind limb). A test substance was administered at a dose of 3 μg mRNA/20 μl/body or 1 μg mRNA/20 μl/body per administration (these doses are indicated as "Example No._3" and "Example No._1", respectively, in Figs. 2 to 4. For example, the expression "Example 3_3" in these Figures means a group which received the lipid particle from Example 3 at a dose of 3 μg mRNA/20 μl/body). Injection solutions were prepared with 300 mM sucrose-containing 10 mM histidine buffer (pH 6.5). A commercial saponin adjuvant (Quil-A Adjuvant, Invivogen, Cat #vac-quil)-added S1 protein (Sino Biological, Cat #40591-V08H) was provided as a positive control group for anti-RBD antibody responses (S1/Quil-A group). S1 protein and Quil-A were administered at a dose of 1 μg S1 and 10 μg Quil-A /20 μl/body per administration.

Preparation of Sera and Splenocytes

Blood samples obtained from the tail vein at the time of administration of the test substance were collected in serum separator (BD, Cat #365967)-containing tubes. After centrifugation (15,000 rpm, 4°C, 5 minutes, centrifuge: TOMY, MX-205), serum was collected. Blood samples obtained from the heart 14 days after the final administration in 3-dose test were collected in tubes, left to stand for 3 hours at room temperature, and then left to stand in a refrigerator set at 4°C for 22 hours. After centrifugation, (1700×g, 4°C, 5 minutes), serum was collected. Further, the spleen was collected from mice exsanguinated under isoflurane anesthesia, and a cell suspension was prepared with a cell strainer (CORNING, Cat #352350). The resultant cell suspension was subjected to hemolysis treatment using ACK solution (Lysing Buffer, BD, Cat #555899) to prepare splenocytes.

Analysis of Protein Expression

The lipid particles of Example 3 or 4 were added to Expi293F cells (Thermo Fisher Scientific, Cat #A14527) so that the mRNA concentration in the medium would become 10 μg/ml. As a negative control, buffer equivalent to the volume of the lipid particles was added to the cells. Three days after the addition, the culture supernatant and the cell pellet were collected. The cell pellet was dissolved in 1×Protease/Phosphatase inhibitor (Thermo Fisher Scientific, Cat #78443)-added M-PER (Thermo Fisher Scientific, Cat #78501) and centrifuged (9100 ×g, 4°C, 10 minutes). After centrifugation, the cell lysate was collected. The culture supernatant diluted to 810-fold or 2430-fold or the cell lysate diluted to 10-fold or 30-fold in D-PBS were immobilized in 96 half well plates (Coaster, Cat #3690), and Enzyme-Linked Immunosorbent Assay (ELISA) was performed using anti-RBD antibody (Sino Biological, Cat #40592-T62) to thereby detect proteins expressed by the lipid particles of Example 3 or 4.

Plasma Anti-RBD Antibody Titer (Fig. 2 to Fig. 4)

Recombinant RBD protein (Sino Biological, Cat #40592-V08H) was diluted to a 0.25 μg/ml solution with a blocking solution (PBS containing 1% BSA and 10.05% Tween 20), added to Ni plates (QIAGEN, Cat#35061) (50 μl/well) and left to stand at room temperature for 2 hours. Then, plates were washed 3 times with a washing solution (0.05% Tween 20-contaning PBS) (300 μl/well). Sample dilution series were prepared with the blocking solution as 4-fold serial dilutions with 8 steps from the highest concentration (100-fold dilution of serum). Standard serum dilution series were

prepared with the blocking solution as 3-fold serial dilutions with 8 steps from the highest concentration of 2 DS UNIT/ml. The sample dilution series or the standard serum dilution series were added to the plates (50 μl/well), which were then left to stand at room temperature for 1 hour. Then, the plates were washed with the washing solution 3 times. As a detection antibody, HRP-labeled anti-mouse IgG antibody (Southern Biotech, Cat #1030-05) was diluted 4000-fold with the blocking solution and added to the plates (50 μl/well), which were then left to stand at room temperature for 1 hour. After washing 3 times with the washing solution, TMB Microwell Peroxidase Substrate System (SERACARE Life Sciences, Cat #5120-0047) was added to the plates (50 μl/well), which were then left to stand for 10 minutes. As a reaction stop solution, TMB Stop Solution (SERACARE Life Sciences, Cat #5150-0021, 50 μl/well) was used. Absorbance at wavelength 450 nm (control wavelength: 540 nm) was measured with a plate reader, and corrected absorbance (Delta) was obtained by subtracting the absorbance at 540 nm from the absorbance at 450 nm and used for analysis. From the anti-RBD antibody concentration of standard serum and Delta values, calibration curves were prepared using Nonlinear Regression: 4 Parameter. Anti-RBD antibody concentrations in test samples were calculated from the calibration curves, dilution rates of test samples, and Delta. The mean value of antibody concentrations in the wells presenting Delta values of 0.5 to 1.5 was calculated as the anti-RBD antibody concentration of test sample. When the well with a highest sample concentration presented a Delta value of less than 0.5, data were obtained by substituting 20 DS LTNIT/ml.

Inhibitory Activity against RBD-hACE2 Binding

Ten micrograms per milliliter Streptavidin (Thermo Fisher Scientific, Cat #21125, dissolved in PBS) was added to 96 half well plates (Coaster, Cat #3690), which were then left to stand at 4°C overnight and washed 3 times with a washing solution (0.05 % Tween 20-containing PBS). A blocking solution (1% BSA and 0.05% Tween 20-containing PBS) was added to the plates, which were then left to stand at room temperature for 1 hour and washed 3 times with the washing solution. Subsequently, a 0.2 μg/ml solution of recombinant RBD protein (Aero Biosystems, Cat #SPD-C82E9) prepared with the blocking solution was added to the plates, which were then left to stand at room temperature for 1 hour and washed 3 times with the washing solution. Mouse serum diluted 20-fold with the blocking solution was added to the plates, which were then left to stand at room temperature for 1 hour and washed 3 times with the washing solution. A 1 μg/ml solution of recombinant hACE2 (Aero Biosystems, Cat #AC2-H5257) prepared with the blocking solution was added to the plates, which were then left to stand at room temperature for 1 hour and washed 3 times with the washing solution. As a detection antibody, HRP-labeled anti-human IgG1 antibody (CYGNUS TECHNOLOGIES, Cat #IM50) was diluted 500-fold with the blocking solution and added to the plates, which were then left to stand at room temperature for 1 hour. After washing 3 times with the washing solution, TMB Microwell Peroxidase Substrate System (SERACARE Life Sciences, Cat #5120-0047) was added to the plates, which were then left to stand for 10 minutes. As a reaction stop solution, TMB Stop Solution (SERACARE Life Sciences, Cat #5150-0021) was used. Absorbance at wavelength 450 nm was measured and analyzed with a plate reader.

SARS-CoV-2 Epitope Peptide Pool

Synthesis of 253 overlapping peptides (#1 to #253) was entrusted (Eurofins) so that the full length of the S protein of SARS-CoV-2 would be covered. Peptides were dissolved in dimethyl sulfoxide (DMSO, Nacalai Tesque, Cat #13408-64) in an amount of 200 μl per peptide. In order to cover RBD and flanking regions thereof, peptides #1 to #62, peptides #63 to #107, and peptides #108 to #253 were individually mixed in equivalent volumes to thereby prepare 3 epitope peptide pools (Euro1, Euro2 and Euro3 in this order). On the other hand, commercial epitope peptide pools covering the full length of the S protein of SARS-CoV-2 (JPT, Cat #PM-WCPV-S-1; 2 vials; peptide pool covering the N-terminal region is JPT-N; and peptide pool covering the C-terminal region is JPT-C) were dissolved in DMSO in an amount of 40 μl/vial.

RBD-Specific Cellular Immune Responses

Splenocytes were diluted with RPMI Complete medium (containing 10% FBS [Sigma-Aldrich,Cat #172012-500ML] and 1% PS [Penicillin-Streptomycin Mixed Solution, Nacalai Tesque, Cat #26253-84]; 1 mM Sodium Pyruvate [Thermo Fisher Scientific, Cat #11360-070], 10 mM HEPES [Thermo Fisher Scientific, Cat #15630080], 1×StemSure [FUJIFILM Wako Pure Chemical Corporation, Cat #195-15791], 1×MEM Non-Essential Amino Acids Solution [Thermo Fisher Scientific, Cat #11140-050]) to a density of $1\times10^7$ cells/ml, and seeded in U-bottom 96-well plates. Epitope peptide pools Euro 1 to 3 adjusted with RPMI Complete medium to give a final concentration of 0.1% (v/v) and commercial epitope peptide pools JPT-N and JPT-C adjusted with RPMI Complete medium to give a final concentration of 0.025% (v/v) were added to the Splenocytes, which were then cultured for 48 hours under conditions of 37°C and 5% $CO_2$. The amounts of IFN-γ and IL-13 in the culture supernatant were measured with Mouse IFN-γ DuoSet ELISA (R&D Systems, Cat #DY485) and Mouse IL-13 Duoset ELISA (R&D systems, Cat #DY413). Absorbance

at wavelength 450 nm (control wavelength: 540 nm) was measured with a plate reader, and corrected absorbance (Delta) was obtained by subtracting the absorbance at 540 nm from the absorbance at 450 nm and used for analysis. From the cytokine concentrations of standard solution and Delta values, calibration curves were prepared using Nonlinear Regression: 4 Parameter, and cytokine concentrations of test samples were calculated from the calibration curves. When IL-13 concentration turned out to be less than 0.000 (<0.000), data was obtained by substituting a cut-off value 0.005.

Statistical Analysis

With respect to comparison of plasma anti-RBD antibody responses and that of RBD-hACE2 binding inhibitory activities, t-test was performed for 3-dose tests, and Dunnet test was performed for 2-dose tests using Buffer group as control. With respect to comparison of RBD-specific cellular immune responses, Dunnett test was performed for each peptide treatment using S1/Quil-A group as control. For all analyses, SAS ver. 9.2 was used.

Administration to Mice (Figs. 5 to 9, and Figs. 25 to 28)

A test substance was administered to BALB/c mice (Figs. 5 to 8, Fig. 25, Fig. 26 and Fig. 28) or C57BL/6 mice (Fig. 9) in the calf of the hind limb under anesthesia with vaporized 1-4% (v/v) isoflurane twice with an interval of 2 weeks (Fig. 5) or twice with an interval of 3 weeks (Figs. 6 to 9, Fig. 25, Fig. 26 and Fig. 28). In Fig. 27, a test substance was administered to BALB/c mice in the calf of the hind limb only once. A test substance was administered at a dose of 0.03, 0.3 or 3 μg mRNA/20 μl/body/administration (for example, the expression "Example 8_0.03" appearing in Fig. 5 means a group which received the lipid particle of Example 8 at a dose of 0.03 μg mRNA/20 μl/body). In Fig. 25 and Fig. 27, a test substance was administered at a dose of 2 μg mRNA/20 μl/body/administration. In Fig. 26 and Fig. 28, a test substance was administered at a dose of 3 μg mRNA/20 μl/body/administration. For preparation of test substance solutions to be administered, 10 mM histidine buffer containing 300 mM sucrose (pH 7.0) was used.

Administration to Monkeys (Fig. 29)

The lipid particle of Example 10 was administered to cynomolgus macaques in the deltoid muscle of the upper arm 3 times with an interval of 2 weeks. The lipid particle of Example 10 was administered at a dose of 50 μg mRNA/200 μl/body/administration. For preparation of test substance solution to be administered, 10 mM histidine buffer containing 300 mM sucrose (pH 7.0) was used.

Plasma Anti-RBD Antibody Titer (Fig. 5, Fig. 6, Fig. 9, and Figs. 25 to 27)

Immobilizer Streptavidin (Thermo Fisher Scientific Inc.) was added to ELISA plates at 25 μl/well, which were then left to stand overnight in a refrigerator set at 4°C. The plates were washed 3 times with Wash Buffer (180 μl/well) using a plate washer (AMW-96SX, BioTec Co., Ltd.). Subsequently, 1% BSA/PBST was added to the plates (150 μl/well), which were then left to stand for more than 1 hour to perform blocking. After washing 3 times with Wash Buffer (180 μl/well) using a plate washer, RBD solution (Original strain RBD: Aero Biosystems, Cat #SPD-C82E9; or 351 strain RBD: Sino Biological, Cat #40592-V08H85-B) was added to the plates (25 μl/well), which were then left to stand at room temperature for more than 1 hour. After washing 3 times with Wash Buffer (180 μl/well) using a plate washer, test sample serial dilutions or standard serum serial dilutions (Fig. 5, Fig. 6, Fig. 9, Fig. 25, and Fig. 26) were added to the plates at 25 μl/well, which were then left to stand at room temperature for more than 1 hour. As standard sample in Fig. 27, serial dilutions of anti-RBD antibody (clone #3) that binds to Original strain-derived RBD and B.1.351strain-derived RBD equally were used. After washing 3 times with Wash Buffer (180 μl/well) using a plate washer, dilutions of HRP-labeled anti-mouse IgG antibody (Southern Biotech, Cat #1030-05) (detection antibody) were added to the plates (25 μl/well), which were then left to stand at room temperature for 1 hour. After washing 3 times with a washing solution, TMB Microwell Peroxidase Substrate System (SERACARE Life Sciences, Cat #5120-0047) was added to the plates (30 μl/well), which were then left to stand for 10 minutes. As a reaction stop solution, TMB Stop Solution (SERACARE Life Sciences, Cat #5150-0021, 30 μl/well) was used. Absorbance at wavelength 450 nm (control wavelength: 540 nm) was measured with a plate reader, and corrected absorbance (Delta) was obtained by subtracting the absorbance at 540 nm from the absorbance at 450 nm and used for analysis. From the anti-RBD antibody concentration of standard serum and Delta values, calibration curves were prepared using Nonlinear Regression: 4 Parameter. Anti-RBD antibody concentrations in test samples were calculated from the calibration curves, dilution rates of test samples, and Delta.

Plasma Anti-SARS-CoV-2 Neutralizing Activity (Fig. 7 and Fig. 8)

VeroE6 cells were seeded in plates and cultured overnight in an incubator set at 37±2°C with an atmosphere

of 5±1% $CO_2$. Serial dilutions of mouse serum and SARS-CoV-2 WA1/2020 strain were mixed and left to stand in an incubator set at 37±2°C with an atmosphere of 5±1% $CO_2$ for 2 to 2.5 hours. Subsequently, the mixed solution of mouse serum and SARS-CoV-2 WA1/2020 strain was added to the VeroE6 cells, which were then cultured in an incubator set at 37±2°C with an atmosphere of 5±1% $CO_2$ for 72±8 hours. Subsequently, viable cell count was measured with CellTiter-Glo (Promega), and anti-SARS-CoV-2 neutralizing activity titer of mouse serum was calculated.

RBD-Specific Cellular Immune Responses (Fig. 10)

Splenocytes were diluted with RPMI Complete medium to a density of 1×$10^7$ cells/ml and seeded in U-bottom 96-well plates. MHC class II epitope peptide pool of RBD prepared with RPMI Complete medium to give a final concentration of 0.1% (v/v) was added to the Splenocytes, which were then cultured under conditions of 37°C, 5% $CO_2$ for 48 hours. The amounts of IFN-γ and IL-13 in the culture supernatant were measured with Mouse IFN-γ DuoSet ELISA and Mouse IL-13 Duoset ELISA. Absorbance at wavelength 450 nm (control wavelength: 540 nm) was measured with a plate reader, and the value obtained by subtracting the absorbance at 540 nm from the absorbance at 450 nm was used for analysis. From the cytokine concentrations of standard solution and measured values, calibration curves were prepared using Nonlinear Regression: 4 Parameter. Then, cytokine concentrations of test samples were calculated from the calibration curves.

Statistical Analysis

With respect to the plasma anti-RBD antibody responses shown in Fig. 5, comparison between two groups of different doses (0.03 μg mRNA/body and 0.3 μg mRNA/body) was analyzed by the Wilcoxon test. For comparison of three groups which received the same dose of 3 μg mRNA/body), the Steel test was performed using Example 8 as a comparison subject.

With respect to the plasma anti-RBD antibody responses shown in Fig. 6, comparison between two groups at each dose of 0.03 μg mRNA/body, 0.3 μg mRNA/body, and 3 μg mRNA/body was analyzed by the Wilcoxon test.

With respect to the plasma anti-SARS-CoV-2 neutralizing activity shown in Fig. 7, the Steel test was performed using Buffer group as a comparison subject. For comparison between the two groups in Fig. 8, the Wilcoxon test was performed.

With respect to the plasma anti-RBD antibody responses shown in Fig. 9, the Steel test was performed using Buffer group as a comparison subject.

With respect to the RBD-specific antibody responses shown in Fig. 10, the Steel-Dwass test was performed.

For all analyses, SAS ver. 9.2 was used.

Inhibitory Activity against RBD-hACE2 Binding (Fig. 28)

Anti-His-Tag antibody (Wako Pure Chemical Industries, Cat #017-23211) was added to 96-well plates, which were then left to stand at 4°C overnight. The plates were washed 3 times with a washing solution (0.05 % Tween 20-containing PBS). Subsequently, a blocking solution (1% BSA, 0.05% Tween 20-containing PBS) was added to the plates, which were then left to stand at room temperature for 1 hour and washed 3 times with the washing solution. Subsequently, recombinant RBD proteins (Control: Aero Biosystems, Cat #SPD-S52H6; Original: Sino Biological, Cat #40592-V08H; K417N: Sino Biological, Cat #40592-V08H59; E484K: ACRO Biosystems, Cat #SRD-C52H3; N501Y: Sino Biological, Cat #40592-V08H82; and K417N/E484K/N501Y: ACRO Biosystems, Cat #SPD-C52Hp) individually diluted to 0.2 μg/ml with the blocking solution were added to the plates, which were then left to stand at room temperature for 1 hour and washed 3 times with the washing solution. Mouse serum serial dilutions with the blocking solution were added to the plates, which were then left to stand at room temperature for 1 hour and washed 3 times with the washing solution. A recombinant hACE2 protein (Acro Biosystems, Cat #AC2-H5257) diluted to 1 μg/ml with the blocking solution was added to the plates, which were then left to stand at room temperature for 1 hour and washed 3 times with the washing solution. As a detection antibody, HRP-labeled anti-human IgG1 antibody (CYGNUS TECHNOLOGIES, Cat #IM50) was diluted 500-fold with the blocking solution and added to the plates, which were then left to stand at room temperature for 1 hour. After washing 3 times with the washing solution, TMB Microwell Peroxidase Substrate System (SERACARE Life Sciences, Cat #5120-0047) was added to the plates, which were then left to stand for 10 minutes. As a reaction stop solution, TMB Stop Solution (SERACARE Life Sciences, Cat #5150-0021) was used. Absorbance at wavelength 450 nm (control wavelength: 540 nm) was measured with a plate reader, and corrected absorbance (Delta) was obtained by subtracting the absorbance at 540 nm from the absorbance at 450 nm and used for analysis. Data indicate the dilution rate of mouse serum showing 50% inhibition ($IC_{50}$).

Plasma Anti-SARS-CoV-2 Neutralizing Activity (Fig. 29)

Vero-TMPRSS2 cells were seeded in plates. Serial dilutions of monkey plasma were mixed with 100 $TCID_{50}$ of respective SARS-CoV-2 strains (D614G: HP095; B.1.1.7 variant: QHN001; P.1 variant: TY7-501; and B.1.351 variant: TY8-612). The resultant mixtures were left to stand in a $CO_2$ incubator. Then, the mixture of monkey plasma and SARS-CoV-2 was added to Vero-TMPRSS2 cells, which were then cultured in a $CO_2$ incubator for 3 days. Subsequently, the highest dilution rate at which cytopathic effect (CPE) is no longer recognized was calculated as neutralizing antibody titer.

Results

RBD Protein Expression Inducing Capacity of the Particle of Example 4

With respect to the mechanism of action of the nucleic acid lipid particle vaccine of the present invention, it is suggested that upon administration into the living body, an antigen protein is produced from the mRNA encoding the antigen gene to thereby induce specific immune responses to the antigen. It is presumed that the efficacy of the nucleic acid lipid particle vaccine of the present invention depends on two major elements; i.e. delivery of the active ingredient mRNA to tissues and cells and translation from the mRNA. For the purpose of evaluating this series of elements comprehensively, titers were evaluated using, as an indicator, expression inducing capacity for antigen protein in cultured cells. Briefly, the particle from Example 3, the particle from Example 4, or buffer was added to Expi293F cells. After three days, the amounts of RBD protein expressed in the culture supernatant and within cells were quantified by ELISA. The results are shown in Fig. 1. The RBD protein expressed by the particle from Example 4 was observed both in the culture supernatant and within cells. The full-length S protein expressed by the particle from Example 3 was only observed within cells.

Plasma Anti-RBD Antibody Responses

Plasma anti-RBD antibody responses induced by administration of the particles of Example 3 or Example 4 were evaluated. The results are shown in Fig. 2. Compared to the 3-dose group and 2-dose groups of Example 3, the 3-dose group of Example 4 showed a high plasma anti-RBD antibody titer (P = 0.0346). Further, compared to buffer group, the 2-dose groups of Example 4 showed a high plasma anti-RBD antibody titer (Example 4_3: P = 0.0019; Example 4_1: P = 0.0313).

Inhibitory Activity against RBD-hACE2 Binding

Serum inhibitory activity against RBD-hACE2 binding induced by administration of the particles from Example 3 or Example 4 was evaluated. The results are shown in Fig. 3. Compared to the 2-dose groups and 3-dose group of Example 3, the serum of the 3-dose group of Example 4 showed a high RBD-hACE2 binding inhibitory activity (P = 0.0005). Further, compared to buffer group, the sera of the 2-dose groups of Example 4 showed a high inhibitory activity against RBD-hACE2 binding (Example 4_3: P < 0.0001; Example 4_1: P = 0.0006).

RBD-Specific Cellular Immune Responses

Splenocytes were prepared, and RBD-specific cellular immune responses from cultured splenocytes were evaluated. The results are shown in Fig. 4. Compared to S1/Quil-A group, Example 4 groups showed a higher IFN-$\gamma$ production level when treated with Euro2 or JPT-N epitope peptide pool each of which cover RBD (P < 0.001). On the other hand, compared to S1/Quil-A group, Example 4 groups showed a low IL-13 production level when treated with Euro2 or JPT-N epitope peptide pool (P <0.005). These results revealed that the nucleic acid lipid particle vaccine of the present invention induces Th1 type-dominant immune responses.

Plasma Anti-RBD Antibody Responses in BALB/c Mice

Plasma anti-RBD antibody responses induced by administration of the particles from Example 8 or Example 4 were evaluated. The results are shown in Fig. 5. At both doses of 0.03 $\mu$g mRNA/body and 0.3 $\mu$g mRNA/body, Example 8 showed a higher plasma anti-RBD antibody titer compared to Example 4 (P = 0.0286 for both doses). Further, at a dose of 3 $\mu$g mRNA/body, significant difference was not recognized in the plasma anti-RBD antibody titers of Example 4, Example 7 and Example 8 (P = 0.061 for all groups).

Plasma anti-RBD antibody responses induced by administration of the particles from Example 10 or Example 8 were evaluated. The results are shown in Fig. 6. At any of the doses used, no significant difference was recognized

in the plasma anti-RBD antibody titers of Example 10 and Example 8 (0.03 μg mRNA/body: P = 0.8413; 0.3 μg mRNA/body: P = 0.0952; and 3 μg mRNA/body: P = 0.6905).

Plasma Anti-SARS-CoV-2 Neutralizing Activity

Plasma anti-SARS-CoV-2 neutralizing activity induced by administration of the particles from Example 10 was evaluated. The results are shown in Fig. 7. Compared to buffer group, the group of Example 10 at 3 μg mRNA/body showed a high plasma anti-SARS-CoV-2 neutralizing activity (P = 0.0374). Further, as a result of comparison of the plasma anti-SARS-CoV-2 neutralizing activities induced by administration of the particles from Example 8 and Example 10, respectively, no significant difference was recognized (Fig. 8, P = 1),

Plasma Anti-RBD Antibody Responses in C57BL/6 Mouse

Plasma anti-RBD antibody responses induced by administration of the particle from Example 8 or Example 10 were evaluated. The results are shown in Fig. 9. At both doses of 3 μg mRNA/body and 10 μg mRNA/body, Example 8 group and Example 10 group showed a high plasma anti-RBD antibody titer, compared to Buffer group ($P < 0.05$ for both doses in Example 10 group).

RBD-Specific Cellular Immune Responses

Splenocytes were prepared, and RBD-specific cellular immune responses from cultured splenocytes were evaluated. The results are shown in Fig. 10A. Compared to the group which received 0.1 μg/body RBD protein plus 100 μg/body alum adjuvant, the group which received the particle from Example 10 at 3 μg/body showed a high IFN-γ induction ($P < 0.05$). Further, compared to the group which received 1.0 μg/body RBD protein plus 100 μg/body alum adjuvant, the groups which received the particle from Example 10 at 0.03 μg/body and 3 μg/body, respectively, showed a high IFN-γ induction ($P < 0.05$ for both groups).

In order to evaluate the Th cell profiles of the particle from Example 10, IFN-γ level/IL-5 level ratio and IFN-γ level/IL-13 level ratio were analyzed. The results are shown in Fig. 10B. Compared to the alum adjuvant-added RBD protein groups, Example 10 groups showed a high IFN-γ level/IL-13 level ratio ($P < 0.05$ in any of the 3 groups of Example 10, compared to the two, alum adjuvant-added RBD protein groups). These results revealed that the nucleic acid lipid particle vaccine of the present invention induces Th1 type-dominant immune responses.

Plasma Anti-RBD Antibody Responses in BALB/c Mouse (Fig. 25 to Fig. 27)

Plasma anti-RBD antibody responses induced by administration of the particle from Example 10, 12, 14, 16, 18, or 20 were evaluated. The results are shown in Fig 25. Compared to Buffer group, any of the Example groups showed a high plasma anti-RBD antibody level.

Plasma anti-RBD antibody responses induced by administration of the particle from Example 10 or any one of Examples 21 to 30 were evaluated. The results are shown in Fig 26. Compared to Buffer group, any of the particle used showed a high plasma anti-RBD antibody titer.

Plasma anti-RBD antibody responses induced by administration of the particle from Example 10, 32a, 32b, 32c, 32d, 32f, or 33 were evaluated. The results are shown in Fig 27. Compared to the particle from Example 32a, the particles from Examples 10, 32b, 32c, 32d, 32f, and 33 showed a higher plasma anti-RBD antibody level.

RBD-hACE2 Binding Inhibitory Activity (Fig. 28)

RBD-hACE2 binding inhibitory activity induced by administration of the particle from Example 10 was evaluated. The results are shown in Fig. 28. Compared to Control RBD, the bindings of Original RBD and RBD variants of K417N, E484K, N501Y and K417N/E484K/N501Y to hACE2 were inhibited to an equivalent degree by the sera of Example 10 groups.

Plasma SARS-CoV-2 Neutralizing Activity (Fig. 29)

Plasma SARS-CoV-2 neutralizing activity induced by administration of the particle from Example 10 was evaluated. The results are shown in Fig. 29. Infections of Vero-TMPRSS2 cells with D614G strain, B.1.1.7 variant, P.1 variant, and B.1.351 variant were neutralized to an equivalent degree by the sera of Example 10 groups.

[Test Example 2]

Optimization of LNP-mRNA Vaccine Candidates Encoding SARS-CoV-2 RBD

The coronavirus disease (COVID-19) pandemic caused by severe acute respiratory syndrome coronavirus 2 (SARS-CoV-2) led to the successful development and commercialization of two mRNA-based vaccines, encoding the full length of the viral surface spike protein[1,2]. However, these vaccines need be improved with respect to reactogenicity such as fever.

The present inventors developed a lipid nanoparticle (LNP)-based mRNA vaccine encoding the receptor-binding domain (RBD) in SARS-CoV-2 spike protein (LNP-mRNA-RBD), and optimized LNP-mRNA-RBD candidates using immunogenicity as an indicator.

First, the present inventors immunized 6-8-week-old mice of either C57BL/6 or BALB/c strain intramuscularly with 3 $\mu$g (in terms of mRNA) of LNP-mRNA-RBD twice at an interval of 2 weeks, and evaluated plasma anti-RBD antibody responses. As a result, compared to C57BL/6 mice, BALB/c mice showed high plasma anti-RBD antibody responses (Fig. 11a, Fig. 15). In order to compare RBD-specific B cell responses induced by LNP-mRNA-RBD between mouse lineages, the present inventors analyzed $T_{FH}$ and GC B cells collected from mouse draining popliteal lymph nodes (pLN) by flow cytometry (Fig. 16). The results revealed that, in correlation with serum antibody responses, the frequency (%) of both $T_{FH}$($CD4^+CD185^+PD\text{-}1^+$ cells) and GC B cells ($CD38^-GL7^+CD19^+$ cells) in the pLN was significantly higher in BALB/c mice immunized with LNP-mRNA-RBD than in C57BL/6 mice immunized with the same (Fig. 11b-e).

To further analyze antigen-specific $CD8^+$ and $CD4^+$ T cells induced by LNP-mRNA-RBD, the present inventors designed a peptide library of the spike protein. This library consists of 128 peptides, each consisting of a 20-amino acid sequence of spike protein with 10 overlapping amino acids. This peptide library was divided into eight pools each containing 16 peptides (Fig. 11f). When splenocytes prepared from LNP-mRNA-RBD-immunized mice were stimulated with peptide pools 3 and 4, splenocytes from C57BL/6 mice induced IFN-$\gamma$ production, while in BALB/c mice this was achieved with peptide pool 3 (Fig. 11g and h; Fig. 17a and b). IL-13 production was not found in the supernatant of the splenocyte culture with peptides in either C57BL/6 or BALB/c mice (Fig. 17c and d). To further characterize LNP-mRNA-RBD-induced antigen-specific T cells, the present inventors stimulated splenocytes with peptide pool 2, 3 or 4, and conducted a flow cytometric analysis of those T cells which produced three cytokines (IL-2, IFN-$\gamma$, and TNF-$\alpha$). As a result, spike antigen-specific polyfunctional $CD8^+$ and $CD4^+$ T cells were significantly upregulated in LNP-mRNA-RBD-immunized BALB/c mice after re-stimulating the splenocytes with peptide pools 3 and 4 (Fig. 11h, Fig. 18b and Fig. 19b). However, splenocytes in the immunized C57BL/6 mice showed substantial polyfunctional $CD8^+$ T cells and weak $CD4^+$ T cell responses (Fig. 11g, Fig. 18a and Fig. 19a). These data suggest that LNP-mRNA-RBD induces higher B and T cell responses in BALB/c mice, than in C57BL/6 mice.

Nucleic acid-based vaccines are known to utilize their backbone DNA or RNA as built-in adjuvants[14-16]. In LNP-mRNA vaccines, it has been shown that mRNA itself acts as an endogenous adjuvant sensed by toll-like receptors (TLR) 3, 7, 8, RIG-I or MDA5[17]. Kariko et al. reported that modification of RNA by methylation or other alternative bases (e.g., pseudouridine) is used for regulation of innate immune activation and improvement of expression efficiency of antigen protein[18, 19]. Several studies have revealed that type IIFN elicited by LNP-mRNA interferes with the $CD^8$ T cell responses and the translation efficiency of the encoded antigen protein[20, 21, 22]. With respect to SARS-CoV-2 vaccines, LNP-mRNA-RBD showed higher reactogenicity than LNP-mRNA-Full encoding the full length of the S protein; therefore, only the LNP-mRNA-Full has been evaluated in a Phase III clinical trial and commercialized[13]. The reason for the difference in reactogenicity remains unclear, but the present inventors considered that innate immunostimulatory activity of the mRNA in LNP formulation might be attributed to its reactogenicity[13].

In order to analyze the innate immunostimulatory activity by LNP-mRNA, the present inventors conducted an ELISA-based measurement of the type I IFN production level from human PBMCs treated with LNP-mRNA-RBD. As a result, PBMCs from three healthy humans produced a higher amount of IFN-$\alpha$ than that induced by LNP-mRNA-Full (Fig. 12a). The present inventors then performed a similar experiment using mouse bone marrow-derived dendritic cells (BM-DCs) from either C57BL/6 or BALB/c mice. Surprisingly, a high level of IFN-$\alpha$ was observed upon culture with LNP-mRNA-full or LNP-mRNA-RBD in BM-DCs of C57BL/6 mice, but very low or no IFN-$\alpha$ production was observed in BM-DCs of BALB/c mice (Fig. 12b). During the manufacturing process of mRNA to be encapsulated in LNP-mRNA, undesirable RNA, such as dsRNA as TLR3 ligand, is contained therein and this might affect innate immune activation[22]. Then, in order to remove such RNA and other contaminants, the present inventors purified mRNA by HPLC, and prepared LNP-mRNA (mRNA-RBD (HPLC)) encapsulating HPLC-purified mRNA. As a result, production of type IIFN from both human PBMCs and mouse BM-DCs treated with mRNA-RBD (HPLC) decreased remarkably, compared to those cells treated with LNP-mRNA-RBD (Fig. 12a and b).

To evaluate the immunogenicity of the HPLC-purified LNP-mRNA vaccine, the present inventors immunized C57BL/6 or BALB/c mice with mRNA-RBD (HPLC). As a result, mRNA-RBD (HPLC) group enhanced plasma anti-RBD IgG1 titer, IgG2 titer, and total IgG titer in both BALB/c and C57BL/6 mice (Fig. 12C and Fig. 20a). Further, LNP-mRNA-RBD (HPLC) induced significantly higher production of GC B cells in the draining lymph nodes (pLN) of C57BL/6 mice than without HPLC (Fig. 12d and e). In addition to antibody responses, HPLC purification improved T cell responses. HPLC-purified LNP-mRNA-RBD improved the production of RBD-specific polyfunctional $CD8^+$ and $CD4^+$ T cells that produce IFN-$\gamma$ and other type-I cytokines (Fig. 12f-i, Fig. 20b-e, Fig. 21 and Fig. 22).

The present inventors evaluated the protective efficacy of mRNA-RBD (HPLC) vaccine against SARS-CoV-2 in non-human primates (NHPs), cynomolgus macaques. In this study, the present inventors immunized four macaques intramuscularly with mRNA-RBD (HPLC) with two macaques as mock controls. As a result, mRNA-RBD (HPLC) group showed higher anti-RBD antibody responses than the mock control group (Fig. 13b). mRNA-RBD (HPLC) group also showed plasma anti-SARS-CoV-2 neutralizing activity (Fig. 13c). Further, mRNA-RBD (HPLC) group also showed higher anti-RBD IgG responses in mucosal tissues in the conjunctiva, nasal cavity, oral cavity, trachea, and rectum than in the mock group (Fig. 13d).

mRNA-RBD (HPLC) immunization drastically decreased the RNA levels of SARS-CoV-2 (Fig. 14a) and infectious virus (Fig. 14b) in the swab at day 1 post-infection. Viral RNA levels in the trachea, bronchus, and lung also decreased in mRNA-RBD (HPLC) group at day 7 post-infection (Fig 14c and Fig. 25). Further, the mock control group showed fever and pneumonia after infection with SARS-CoV-2 (Figs. 23-24).

Analysis of lung tissues after infection with SARS-CoV-2 was performed. Infiltration of lymphocytes and neutrophils was observed and thickening of the alveolar wall and viral antigen were also confirmed in the mock control group, whereas such phenomena were not confirmed in mRNA-RBD (HPLC) group (Fig. 14d, 14e). Formation of bronchus-associated lymphoid tissue (BALT) was confirmed in mRNA-RBD (HPLC) group (Fig. 14d). These results suggest the possibility that antibodies induced, mediated by BALT formation, in the mucosa of nasal cavity or trachea bind to SARS-CoV-2 to thereby neutralize the virus, which resulted in the decrease of the RNA levels of SARS-CoV-2 and infectious virus in the swab at day 1 post-infection.

Materials and Methods

Mice

Six to eight week-old C57BL/6 and BALB/c mice were purchased from CLEA, Japan. The mice were maintained under specific pathogen-free conditions. All mouse studies were approved by the Animal Experiment Committee of the Institute of Medical Science, University of Tokyo.

Cynomolgus macaque

Seven to ten-year-old female cynomolgus macaques born at Shiga University of Medical Science and originating from Philippines, Vietnam, and China were used. All procedures were performed under ketamine and xylazine anesthesia, and all efforts were made to minimize suffering. Food pellets of CMK-2 (CLEA Japan, Inc., Tokyo, Japan) were provided once a day after recovery from anesthesia and drinking water was available ad libitum. The animals were singly housed in cages under controlled conditions of light (12-h light/12-h dark cycle, lights on at 8:00 a.m.). The macaques were challenged with the SARS-CoV-2 ($2 \times 10^7$ PFU/7 ml HBSS), which was inoculated into the conjunctiva (0.05 ml $\times$ 2), nostrils (0.5 ml $\times$ 2), oral cavity (0.9 ml), and trachea (5 ml) with pipettes and catheters under ketamine/xylazine anesthesia. Under ketamine/xylazine anesthesia, two cotton swabs (Eiken Chemical, Ltd., Tokyo, Japan) were used to collect fluid samples from the conjunctivas, nasal cavities, oral cavities and tracheas, and the swabs were subsequently immersed in 1 ml of Dulbecco's modified Eagle medium (DMEM, Nacalai Tesque, Kyoto, Japan) containing 0.1% bovine serum albumin (BSA) and antibiotics. A bronchoscope (MEV-2560; Machida Endoscope Co. Ltd., Tokyo, Japan) and cytology brushes (BC-203D-2006; Olympus Co., Tokyo, Japan) were used to obtain bronchial samples.

LNP-mRNA vaccines

The nucleic acid lipid particle encapsulating mRNA prepared in Example 10 was used.

Reagents

Overlapping 20-aa peptides of the spike protein were synthesized and purchased from Eurofins Genomics (Ebersberg, Germany). The SARS-CoV-2 spike protein ectodomain (ECD) and RBD were purchased from GenScript (Piscataway, NJ, USA).

Virus

SARS-CoV-2 isolates were propagated in VeroE6 cells in Opti-MEM I (Invitrogen, Carlsbad, CA, USA) containing 0.3% bovine serum albumin (BSA) and 1 μg of L-1-tosylamide-2-phenylethyl chloromethyl ketone (TPCK)-treated trypsin/ml at 37°C.

Immunization

Six to eight week-old C57BL/6 and BALB/c mice were intramuscularly immunized with mock, LNP-mRNA-RBD (3 μg), or LNP-mRNA-RBD (HPLC) (3 μg) on days 0 and 14. Two weeks after the second immunization, the popliteal lymph nodes, spleen, and blood were collected. Cynomolgus macaques were intramuscularly immunized with mock or LNP-mRNA-RBD (HPLC) (100 μg) on days 0 and 21. Blood samples were taken on days 0, 7, 14, 21, and 28.

ELISA

ECD and RBD-specific antibody titers were measured using ELISA. In brief, half-area 96-well plates were coated with ECD (1 μg/ml) or RBD (1 μg/ml) in bicarbonate buffer at 4°C. Plates were blocked with PBS containing 1% BSA for 60 min at room temperature. Plates were washed with PBST three times and incubated with diluted plasma or swab samples at room temperature for 120 min. Plates were washed with PBST three times and incubated with HRP-labeled goat anti-mouse IgG, IgG1, IgG2a, IgG2c, or mouse anti-monkey IgG at room temperature for 120 min. After washing with PBST three times, TMB substrate buffer was added, followed by incubation at room temperature for 10 min. Then, 1 N $H_2SO_4$ was added to stop the reaction. OD values at 450 and 540 or 560 nm were measured using a spectrophotometer. The reciprocal value of the plasma dilution with $OD_{450}$-$OD_{540}$ or $OD_{450}$-$OD_{560}$ of 0.2 was defined as the antibody titer.

Single-cell suspensions of splenocytes from immunized mice were stimulated with peptide pools 1 to 8, ECD, and RBD protein for 24 hours. IFN-γ and IL-13 levels in the supernatant were measured using ELISA (R&D).

GC B cell and $T_{FH}$ staining

Single-cell suspensions of popliteal lymph nodes were stained with LIVE/DEAD Aqua, anti-CD279 (29F.1A12), anti-CD8a (53-6.7), anti-CD3e (145-2C11), anti-GL7 (GL7), anti-CD4 (RM4-5), anti-CD185 (L138D7), anti-CD38 (90), and anti-CD19 (6D5) antibodies. All antibodies were purchased from BioLegend, San Diego, CA, USA. The percentages of GC B cells and $T_{FH}$ cells were analyzed by flow cytometry.

Intracellular staining assay for cytokines

Single-cell suspensions of splenocytes were stimulated with peptide pools 2, 3, and 4 together with protein transport inhibitors (eBioscience, San Diego, CA, USA) for 6 h. After stimulation, the cells were stained with LIVE/DEAD Aqua for dead cells. After washing, the cells were stained with anti-CD8a (53-6.7), anti-CD4 (RM4-5: Invitrogen), anti-TCRβ (H57-597), anti-F4/80 (RM8), anti-TER-119 (TER-119), anti-CD11b (M1/70), anti-CD19 (6D5), anti-CD1 1c (N418), anti-NK-1.1 (PK136), and anti-CD45R/B220 (RA3-6B2) antibodies. All antibodies were purchased from BioLegend unless otherwise stated. After fixation and permeabilization by IC Fixation Buffer (eBioscience), intracellular cytokines and CD3 were stained with anti-IFN-γ (XMG1.2), anti-IL-2 (JES6-5H4), anti-TNF-α (MP6-XT22), and anti-CD3 (17A2) antibodies. All antibodies were purchased from BioLegend. The percentages of cytokine-producing $CD8^+$ and $CD4^+$ T cells were determined by flow cytometry.

Preparation and stimulation of human peripheral blood mononuclear cells

Peripheral blood mononuclear cells (PBMCs) were obtained from three SARS-CoV-2-uninfected healthy adult volunteers after obtaining informed consent. All experiments using human PBMCs were approved by the Institutional Review Board of the Institute of Medical Science, University of Tokyo. After preparation of PBMCs using Ficoll Histopaque, the cells were stimulated with LNP-mRNA-Full (0.4, 2, and 10 μg/ml), LNP-mRNA-RBD (0.4, 2, and 10 μg/ml), or LNP-mRNA-RBD (HPLC) (0.4, 2, and 10 μg/ml) for 24 h. IFN-α level in the culture supernatant was measured using ELISA (Mabtech, Stockholm, Sweden).

Bone marrow-derived dendritic cells and stimulation

Bone marrow-derived dendritic cells (BM-DCs) were differentiated by culturing for seven days with murine GM-CSF. Cells were stimulated with LNP-mRNA-Full (0.4, 2, and 10 μg/ml), LNP-mRNA-RBD (0.4, 2, and 10 μg/ml), or LNP-mRNA-RBD (HPLC) (0.4, 2, and 10 μg/ml) for 24 h. IFN-α in the culture supernatant was measured using ELISA (Invitrogen).

Neutralization antibody titer

Thirty-five microliters of virus (140 tissue culture infectious dose 50) was incubated with 35 μl of two-fold serial dilutions of sera for 1 h at room temperature, and 50 μl of the mixture was added to confluent VeroE6/TMPRSS2 cells in 96-well plates and incubated for 1 h at 37°C. After addition of 50 μl of DMEM containing 5% FCS, the cells were further incubated for three days at 37°C. Viral cytopathic effects (CPE) were observed under an inverted microscope, and virus neutralization titers were determined as the reciprocal of the highest serum dilution that completely prevented CPE (24).

Virus titration using VeroE6/TMPRSS2 against SARS-CoV-2

Confluent TMPRSS2-expressing Vero E6 cell line (JCRB Cell Bank, Japan) was incubated with diluted swab samples and 10% w/v tissue homogenate samples for 1 h. The cells were washed with HBSS and incubated with DMEM containing 0.1% BSA for three days (25). Virus titers were monitored using a microscope and calculated using the Reed-Muench methods.

Real-time RT-PCR of viral RNA

Viral RNA from swab samples and tissues (20 mg) was collected using a QIAamp Viral RNA Mini kit and RNeasy Mini Kit, respectively. Viral RNA was measured by real-time RT-PCR (2019-nCoV_N1-F, 2019-nCoV_N1-R, 2019-nCoV_N1-P, and TaqMan Fast Virus 1-step Master Mix) using CFX-96 (Bio-Rad, Hercules, CA, USA).

Body temperature

Two weeks before virus inoculation, two temperature data loggers (iButton, Maxim Integrated, San Jose, CA) were implanted in the peritoneal cavity or subcutaneous tissue of each macaque under ketamine/xylazine anesthesia, followed by isoflurane inhalation to monitor body temperature.

X-ray

Chest X-ray radiographs were taken with I-PACS system (Konica Minolta, Inc.) and PX-20BT (Kenko Tokina Corporation).

References

1 Baden, L. R. et al. Efficacy and Safety of the mRNA-1273 SARS-CoV-2 Vaccine. N Engl J Med, doi:10.1056/NEJMoa2035389 (2020).

2 Polack, F. P. et al. Safety and Efficacy of the BNT162b2 mRNA Covid-19 Vaccine. N Engl J Med 383, 2603-2615, doi:10.1056/NEJMoa2034577 (2020).

3 Lan, J. et al. Structure of the SARS-CoV-2 spike receptor-binding domain bound to the ACE2 receptor. Nature 581, 215-220, doi:10.1038/s41586-020-2180-5 (2020).

4 Rydyznski Moderbacher, C. et al. Antigen-Specific Adaptive Immunity to SARS-CoV-2 in Acute COVID-19 and Associations with Age and Disease Severity. Cell 183, 996-1012 e1019, doi:10.1016/j.cell.2020.09.038 (2020).

5 McMahan, K. et al. Correlates of protection against SARS-CoV-2 in rhesus macaques. Nature, doi:10.1038/s41586-020-03041-6 (2020).

6 Vogel, A. B. et al. BNT162b vaccines are immunogenic and protect non-human primates against SARS-CoV-2. bioRxiv, 2020.2012.2011.421008, doi:10.1101/2020.12.11.421008 (2020).

7 Elia, U. et al. Design of SARS-CoV-2 RBD mRNA Vaccine Using Novel Ionizable Lipids. bioRxiv, 2020.2010.2015.341537, doi:10.1101/2020.10.15.341537 (2020).

8 Tai, W. et al. A novel receptor-binding domain (RBD)-based mRNA vaccine against SARS-CoV-2. Cell Res 30, 932-935, doi:10.1038/s41422-020-0387-5 (2020).

9 Lederer, K. et al. SARS-CoV-2 mRNA Vaccines Foster Potent Antigen-Specific Germinal Center Responses

Associated with Neutralizing Antibody Generation. Immunity 53, 1281-1295 e1285, doi:10.1016/j.immuni.2020.11.009 (2020).
10 Zhou, P. et al. A pneumonia outbreak associated with a new coronavirus of probable bat origin. Nature 579, 270-273, doi:10.1038/s41586-020-2012-7 (2020).
11 Jackson, L. A. et al. An mRNA Vaccine against SARS-CoV-2 - Preliminary Report. N Engl J Med 383, 1920-1931, doi:10.1056/NEJMoa2022483 (2020).
12 Anderson, E. J. et al. Safety and Immunogenicity of SARS-CoV-2 mRNA-1273 Vaccine in Older Adults. N Engl J Med 383, 2427-2438, doi:10.1056/NEJMoa2028436 (2020).
13 Walsh, E. E. et al. Safety and Immunogenicity of Two RNA-Based Covid-19 Vaccine Candidates. N Engl J Med 383, 2439-2450, doi:10.1056/NEJMoa2027906 (2020).
14 Desmet, C. J. & Ishii, K. J. Nucleic acid sensing at the interface between innate and adaptive immunity in vaccination. Nat Rev Immunol 12, 479-491, doi:10.1038/nri3247 (2012).
15 Coban, C. et al. Novel strategies to improve DNA vaccine immunogenicity. Curr Gene Ther 11, 479-484, doi:10.2174/156652311798192815 (2011).
16 Pardi, N., Hogan, M. J., Porter, F. W. & Weissman, D. mRNA vaccines - a new era in vaccinology. Nat Rev Drug Discov 17, 261-279, doi:10.1038/nrd.2017.243 (2018).
17 Iavarone, C., O'Hagan D, T., Yu, D., Delahaye, N. F. & Ulmer, J. B. Mechanism of action of mRNA-based vaccines. Expert Rev Vaccines 16, 871-881, doi:10.1080/14760584.2017.1355245 (2017).
18 Kariko, K., Buckstein, M., Ni, H. & Weissman, D. Suppression of RNA recognition by Toll-like receptors: the impact of nucleoside modification and the evolutionary origin of RNA. Immunity 23, 165-175, doi:10.1016/j.immuni.2005.06.008 (2005).
19 Kariko, K. et al. Incorporation of pseudouridine into mRNA yields superior nonimmunogenic vector with increased translational capacity and biological stability. Mol Ther 16, 1833-1840, doi:10.1038/mt.2008.200 (2008).
20 Kariko, K., Muramatsu, H., Ludwig, J. & Weissman, D. Generating the optimal mRNA for therapy: HPLC purification eliminates immune activation and improves translation of nucleoside-modified, protein-encoding mRNA. Nucleic Acids Res 39, e142, doi:10.1093/nar/gkr695 (2011).
21 De Beuckelaer, A. et al. Type I Interferons Interfere with the Capacity of mRNA Lipoplex Vaccines to Elicit Cytolytic T Cell Responses. Mol Ther 24, 2012-2020, doi:10.1038/mt.2016.161 (2016).
22 Linares-Fernandez, S., Lacroix, C., Exposito, J. Y. & Verrier, B. Tailoring mRNA Vaccine to Balance Innate/Adaptive Immune Response. Trends Mol Med 26, 311-323, doi:10.1016/j.molmed.2019.10.002 (2020).
23 Corbett, K. S. et al. Evaluation of the mRNA-1273 Vaccine against SARS-CoV-2 in Nonhuman Primates. N Engl J Med 383, 1544-1555, doi:10.1056/NEJMoa2024671 (2020).
24 Imai, M. et al. Syrian hamsters as a small animal model for SARS-CoV-2 infection and countermeasure development. Proc Natl Acad Sci U S A 117, 16587-16595, doi:10.1073/pnas.2009799117 (2020).
25 Ishigaki, H. et al. Neutralizing antibody-dependent and -independent immune responses against SARS-CoV-2 in cynomolgus macaques. Virology 554, 97-105, doi:10.1016/j.virol.2020.12.013 (2021).

All publications, patents and patent applications cited herein are incorporated herein by reference in their entirety.

INDUSTRIAL APPLICABILITY

The present invention is applicable to prevention and/or treatment of infections with SARS-CoV-2.

SEQUENCE LISTING FREE TEXT

<SEQ ID NO: 1> (DNA fragment comprising SARS-CoV-2 S full.)

GTAATACGACTCACTATAAGGAGACCCAAGCTACATTTGCTTCTGACACAAC

TGTGTTCACTAGCAACCTCAAACAGACACCGCCACCATGTTTGTTTTTCTTG

TTTTATTGCCACTAGTCTCTAGTCAGTGTGTTAATCTTACAACCAGAACTCA

ATTACCCCCTGCATACACTAATTCTTTCACACGTGGTGTTTATTACCCTGAC

AAAGTTTTCAGATCCTCAGTTTTACATTCAACTCAGGACTTGTTCTTACCTT

TCTTTTCCAATGTTACTTGGTTCCATGCTATACATGTCTCTGGGACCAATGG

TACTAAGAGGTTTGATAACCCTGTCCTACCATTTAATGATGGTGTTTATTTT

GCTTCCACTGAGAAGTCTAACATAATAAGAGGCTGGATTTTTGGTACTACTT

TAGATTCGAAGACCCAGTCCCTACTTATTGTTAATAACGCTACTAATGTTGT

TATTAAAGTCTGTGAATTTCAATTTTGTAATGATCCATTTTTGGGTGTTTAT

TACCACAAAAACAACAAAAGTTGGATGGAAAGTGAGTTCAGAGTTTATTCTA

GTGCGAATAATTGCACTTTTGAATATGTCTCTCAGCCTTTTCTTATGGACCT

TGAAGGAAAACAGGGTAATTTCAAAAATCTTAGGGAATTTGTGTTTAAGAAT

ATTGATGGTTATTTTAAAATATATTCTAAGCACACGCCTATTAATTTAGTGC

GTGATCTCCCTCAGGGTTTTTCGGCTTTAGAACCATTGGTAGATTTGCCAAT

AGGTATTAACATCACTAGGTTTCAAACTTTACTTGCTTTACATAGAAGTTAT

TTGACTCCTGGTGATTCTTCTTCAGGTTGGACAGCTGGTGCTGCAGCTTAT

TATGTGGGTTATCTTCAACCTAGGACTTTTCTATTAAAATATAATGAAAATG

GAACCATTACAGATGCTGTAGACTGTGCACTTGACCCTCTCTCAGAAACAAA

GTGTACGTTGAAATCCTTCACTGTAGAAAAAGGAATCTATCAAACTTCTAAC

TTTAGAGTCCAACCAACAGAATCTATTGTTAGATTTCCTAATATTACAAACTT

GTGCCCTTTTGGTGAAGTTTTTAACGCCACCAGATTTGCATCTGTTTATGCT

TGGAACAGGAAGAGAATCAGCAACTGTGTTGCTGATTATTCTGTCCTATATA

ATTCCGCATCATTTTCCACTTTTAAGTGTTATGGAGTGTCTCCTACTAAATT

AAATGATCTCTGCTTTACTAATGTCTATGCAGATTCATTTGTAATTAGAGGT

GATGAAGTCAGACAAATCGCTCCAGGGCAAACTGGAAAGATTGCTGATTATA

ATTATAAATTACCAGATGATTTTACAGGCTGCGTTATAGCTTGGAATTCTAA

CAATCTTGATTCTAAGGTTGGTGGTAATTATAATTACCTGTATAGATTGTTT

AGGAAGTCTAATCTCAAACCTTTTGAGAGAGATATTTCAACTGAAATCTATC

AGGCCGGTAGCACACCTTGTAATGGTGTTGAAGGTTTTAATTGTTACTTTCC

TTTACAATCATATGGTTTCCAACCCACTAATGGTGTTGGTTACCAACCATAC

AGAGTAGTAGTACTTTCTTTTGAACTTCTACATGCACCAGCAACTGTTTGTG

GACCTAAAAAGTCTACTAATTTGGTTAAAAACAAATGTGTCAATTTCAACTTC

AATGGTTTAACAGGCACAGGTGTTCTTACTGAGTCTAACAAAAAGTTTCTGC

CTTTCCAACAATTTGGCAGAGACATTGCTGACACTACTGATGCTGTCCGTGA

TCCACAGACACTTGAGATTCTTGACATTACACCATGTTCTTTTGGTGGTGTC

AGTGTTATAACACCAGGAACAAATACTTCTAACCAGGTTGCTGTTCTTTATC

AGGATGTTAACTGCACAGAAGTCCCTGTTGCTATTCATGCAGATCAACTTAC

TCCTACTTGGCGTGTTTATTCTACAGGTTCTAATGTTTTTCAAACACGTGCA

GGCTGTTTAATAGGGGCTGAACATGTCAACAACTCATATGAGTGTGACATAC

CCATTGGTGCAGGTATATGCGCTAGTTATCAGACTCAGACTAATTCTCCTCG

GCGGGCACGTAGTGTAGCTAGTCAATCCATCATTGCCTACACTATGTCACTT

GGTGCAGAAAATTCAGTTGCTTACTCTAATAACTCTATTGCCATACCCACAA

ATTTTACTATTAGTGTTACCACAGAAATTCTACCAGTGTCTATGACCAAGAC

ATCAGTAGATTGTACAATGTACATTTGTGGTGATTCAACTGAATGCAGCAAT

CTTTTGTTGCAATATGGCAGTTTTTGTACACAATTAAACCGTGCTTTAACTG

GAATAGCTGTTGAACAAGACAAAAACACCCAAGAAGTTTTTGCACAAGTCAA

ACAAATTTACAAAACACCACCAATTAAAGATTTTGGTGGTTTTAATTTTTCAC
AAATATTACCAGATCCATCAAAACCAAGCAAGAGGTCATTTATTGAAGATCTA
CTTTTCAACAAAGTGACACTTGCAGATGCTGGCTTCATCAAACAATATGGTG
ATTGCCTTGGTGATATTGCTGCTAGAGACCTCATTTGTGCACAAAAGTTTAA
CGGCCTTACTGTTTTGCCACCTTTGCTCACAGATGAAATGATTGCTCAATAC
ACTTCTGCACTGTTAGCGGGTACAATCACTTCTGGTTGGACCTTTGGTGCA
GGTGCTGCATTACAAATACCATTTGCTATGCAAATGGCTTATAGGTTTAATG
GTATTGGAGTTACACAGAATGTTCTCTATGAGAACCAAAAATTGATTGCCAA
CCAATTTAATAGTGCTATTGGCAAAATTCAAGACTCACTTTCTTCCACAGCA
AGTGCACTTGGAAAACTTCAAGATGTGGTCAACCAAAATGCACAAGCTTTAA
ACACGCTTGTTAAACAACTTAGCTCCAATTTTGGTGCAATTTCAAGTGTTTT
AAATGATATCCTTTCACGTCTTGACAAAGTTGAGGCTGAAGTGCAAATTGAT
AGGTTGATCACAGGCAGACTTCAAAGTTTGCAGACATATGTGACTCAACAAT
TAATTAGAGCTGCAGAAATCAGAGCTTCTGCTAATCTTGCTGCTACTAAAAT
GTCAGAGTGTGTACTTGGACAATCAAAAAGAGTTGATTTTTGTGGAAAGGG
CTATCATCTTATGTCCTTCCCTCAGTCAGCACCTCATGGTGTAGTCTTCTTG
CATGTGACTTATGTCCCTGCACAAGAAAAGAACTTCACAACTGCTCCTGCCA
TTTGTCATGATGGAAAAGCACACTTTCCTCGTGAAGGTGTCTTTGTTTCAAA

TGGCACACACTGGTTTGTAACACAAAGGAATTTTTATGAACCACAAATCATT
ACTACAGACAACACATTTGTGTCTGGTAACTGTGATGTTGTAATAGGAATTG
TCAACAACACAGTTTATGATCCTTTGCAACCTGAATTAGACTCATTCAAGGA
GGAGTTAGATAAATATTTTAAGAATCATACATCACCAGATGTTGATTTAGGT
GACATCTCTGGCATTAATGCTTCAGTTGTAAACATTCAAAAAGAAATTGACC
GCCTCAATGAGGTTGCCAAGAATTTAAATGAATCTCTCATCGATCTCCAAGA
ACTTGGAAAGTATGAGCAGTATATAAAATGGCCATGGTACATTTGGCTAGGT
TTTATAGCTGGCTTGATTGCCATAGTAATGGTGACAATTATGCTTTGCTGTA
TGACCAGTTGCTGTAGTTGTCTCAAGGGCTGTTGTTCTTGTGGATCCTGCT
GCAAATTTGATGAAGACGACTCTGAGCCAGTGCTCAAAGGAGTCAAATTACA
TTACACATGAGCTCGCTTTCTTGCTGTCCAATTTCTATTAAAGGTTCCTTTG
TTCCCTAAGTCCAACTACTAAACTGGGGGATATTATGAAGGGCCTTGAGCAT
CTGGATTCTGCCTAATAAAAAACATTTATTTTCATTGC

<SEQ ID NO: 2> (Sense primer)
GTAATACGACTCACTATAA
<SEQ ID NO: 3> (Antisense primer)

TTTTTTTTTTTTTTTTTTTTTTTTTTTTTTTTTTTTTTTTTTTTTTTTTTTTT
TTTTTTTTTTTTTTTTTTTTTTTTTTTTTTTTTTTTTTTTTTTTTTTGCAATG
AAAATAAATGTTTTTTATTAGGC

<SEQ ID NO: 4> (Template DNA for SARS-CoV-2 S-full)

GTAATACGACTCACTATAAGGAGACCCAAGCTACATTTGCTTCTGACACAAC
TGTGTTCACTAGCAACCTCAAACAGACACCGCCACCATGTTTGTTTTTCTTG
TTTTATTGCCACTAGTCTCTAGTCAGTGTGTTAATCTTACAACCAGAACTCA
ATTACCCCCTGCATACACTAATTCTTTCACACGTGGTGTTTATTACCCTGAC
AAAGTTTTCAGATCCTCAGTTTTACATTCAACTCAGGACTTGTTCTTACCTT
TCTTTTCCAATGTTACTTGGTTCCATGCTATACATGTCTCTGGGACCAATGG
TACTAAGAGGTTTGATAACCCTGTCCTACCATTTAATGATGGTGTTTATTTT
GCTTCCACTGAGAAGTCTAACATAATAAGAGGCTGGATTTTTGGTACTACTT
TAGATTCGAAGACCCAGTCCCTACTTATTGTTAATAACGCTACTAATGTTGT
TATTAAAGTCTGTGAATTTCAATTTTGTAATGATCCATTTTTGGGTGTTTAT
TACCACAAAAACAACAAAAGTTGGATGGAAAGTGAGTTCAGAGTTTATTCTA
GTGCGAATAATTGCACTTTTGAATATGTCTCTCAGCCTTTTCTTATGGACCT
TGAAGGAAAACAGGGTAATTTCAAAAATCTTAGGGAATTTGTGTTTAAGAAT

ATTGATGGTTATTTTAAAATATATTCTAAGCACACGCCTATTAATTTAGTGC GTGATCTCCCTCAGGGTTTTTCGGCTTTAGAACCATTGGTAGATTTGCCAAT AGGTATTAACATCACTAGGTTTCAAACTTTACTTGCTTTACATAGAAGTTAT TTGACTCCTGGTGATTCTTCTTCAGGTTGGACAGCTGGTGCTGCAGCTTAT TATGTGGGTTATCTTCAACCTAGGACTTTTCTATTAAAATATAATGAAAATG GAACCATTACAGATGCTGTAGACTGTGCACTTGACCCTCTCTCAGAAACAAA GTGTACGTTGAAATCCTTCACTGTAGAAAAAGGAATCTATCAAACTTCTAAC TTTAGAGTCCAACCAACAGAATCTATTGTTAGATTTCCTAATATTACAAACTT GTGCCCTTTTGGTGAAGTTTTTAACGCCACCAGATTTGCATCTGTTTATGCT TGGAACAGGAAGAGAATCAGCAACTGTGTTGCTGATTATTCTGTCCTATATA ATTCCGCATCATTTTCCACTTTTAAGTGTTATGGAGTGTCTCCTACTAAATT AAATGATCTCTGCTTTACTAATGTCTATGCAGATTCATTTGTAATTAGAGGT GATGAAGTCAGACAAATCGCTCCAGGGCAAACTGGAAAGATTGCTGATTATA ATTATAAATTACCAGATGATTTTACAGGCTGCGTTATAGCTTGGAATTCTAA CAATCTTGATTCTAAGGTTGGTGGTAATTATAATTACCTGTATAGATTGTTT AGGAAGTCTAATCTCAAACCTTTTGAGAGAGATATTTCAACTGAAATCTATC AGGCCGGTAGCACACCTTGTAATGGTGTTGAAGGTTTTAATTGTTACTTTCC TTTACAATCATATGGTTTCCAACCCACTAATGGTGTTGGTTACCAACCATAC

AGAGTAGTAGTACTTTCTTTTGAACTTCTACATGCACCAGCAACTGTTTGTG

GACCTAAAAAGTCTACTAATTTGGTTAAAAACAAATGTGTCAATTTCAACTTC

AATGGTTTAACAGGCACAGGTGTTCTTACTGAGTCTAACAAAAAGTTTCTGC

CTTTCCAACAATTTGGCAGAGACATTGCTGACACTACTGATGCTGTCCGTGA

TCCACAGACACTTGAGATTCTTGACATTACACCATGTTCTTTTGGTGGTGTC

AGTGTTATAACACCAGGAACAAATACTTCTAACCAGGTTGCTGTTCTTTATC

AGGATGTTAACTGCACAGAAGTCCCTGTTGCTATTCATGCAGATCAACTTAC

TCCTACTTGGCGTGTTTATTCTACAGGTTCTAATGTTTTTCAAACACGTGCA

GGCTGTTTAATAGGGGCTGAACATGTCAACAACTCATATGAGTGTGACATAC

CCATTGGTGCAGGTATATGCGCTAGTTATCAGACTCAGACTAATTCTCCTCG

GCGGGCACGTAGTGTAGCTAGTCAATCCATCATTGCCTACACTATGTCACTT

GGTGCAGAAAATTCAGTTGCTTACTCTAATAACTCTATTGCCATACCCACAA

ATTTTACTATTAGTGTTACCACAGAAATTCTACCAGTGTCTATGACCAAGAC

ATCAGTAGATTGTACAATGTACATTTGTGGTGATTCAACTGAATGCAGCAAT

CTTTTGTTGCAATATGGCAGTTTTTGTACACAATTAAACCGTGCTTTAACTG

GAATAGCTGTTGAACAAGACAAAAACACCCAAGAAGTTTTTGCACAAGTCAA

ACAAATTTACAAAACACCACCAATTAAAGATTTTGGTGGTTTTAATTTTTCAC

AAATATTACCAGATCCATCAAAACCAAGCAAGAGGTCATTTATTGAAGATCTA

CTTTTCAACAAAGTGACACTTGCAGATGCTGGCTTCATCAAACAATATGGTG

ATTGCCTTGGTGATATTGCTGCTAGAGACCTCATTTGTGCACAAAAGTTTAA

CGGCCTTACTGTTTTGCCACCTTTGCTCACAGATGAAATGATTGCTCAATAC

ACTTCTGCACTGTTAGCGGGTACAATCACTTCTGGTTGGACCTTTGGTGCA

GGTGCTGCATTACAAATACCATTTGCTATGCAAATGGCTTATAGGTTTAATG

GTATTGGAGTTACACAGAATGTTCTCTATGAGAACCAAAAATTGATTGCCAA

CCAATTTAATAGTGCTATTGGCAAAATTCAAGACTCACTTTCTTCCACAGCA

AGTGCACTTGGAAAACTTCAAGATGTGGTCAACCAAAATGCACAAGCTTTAA

ACACGCTTGTTAAACAACTTAGCTCCAATTTTGGTGCAATTTCAAGTGTTTT

AAATGATATCCTTTCACGTCTTGACAAAGTTGAGGCTGAAGTGCAAATTGAT

AGGTTGATCACAGGCAGACTTCAAAGTTTGCAGACATATGTGACTCAACAAT

TAATTAGAGCTGCAGAAATCAGAGCTTCTGCTAATCTTGCTGCTACTAAAAT

GTCAGAGTGTGTACTTGGACAATCAAAAAGAGTTGATTTTTGTGGAAAGGG

CTATCATCTTATGTCCTTCCCTCAGTCAGCACCTCATGGTGTAGTCTTCTTG

CATGTGACTTATGTCCCTGCACAAGAAAAGAACTTCACAACTGCTCCTGCCA

TTTGTCATGATGGAAAAGCACACTTTCCTCGTGAAGGTGTCTTTGTTTCAAA

TGGCACACACTGGTTTGTAACACAAAGGAATTTTTATGAACCACAAATCATT

ACTACAGACAACACATTTGTGTCTGGTAACTGTGATGTTGTAATAGGAATTG

TCAACAACACAGTTTATGATCCTTTGCAACCTGAATTAGACTCATTCAAGGA

GGAGTTAGATAAATATTTTAAGAATCATACATCACCAGATGTTGATTTAGGT

GACATCTCTGGCATTAATGCTTCAGTTGTAAACATTCAAAAAGAAATTGACC

GCCTCAATGAGGTTGCCAAGAATTTAAATGAATCTCTCATCGATCTCCAAGA

ACTTGGAAAGTATGAGCAGTATATAAAATGGCCATGGTACATTTGGCTAGGT

TTTATAGCTGGCTTGATTGCCATAGTAATGGTGACAATTATGCTTTGCTGTA

TGACCAGTTGCTGTAGTTGTCTCAAGGGCTGTTGTTCTTGTGGATCCTGCT

GCAAATTTGATGAAGACGACTCTGAGCCAGTGCTCAAAGGAGTCAAATTACA

TTACACATGAGCTCGCTTTCTTGCTGTCCAATTTCTATTAAAGGTTCCTTTG

TTCCCTAAGTCCAACTACTAAACTGGGGATATTATGAAGGGCCTTGAGCAT

CTGGATTCTGCCTAATAAAAACATTTATTTTCATTGCAAAAAAAAAAAAAAA

AAAAAAAAAAAAAAAAAAAAAAAAAAAAAAAAAAAAAAAAAAAAAAAAAAAAAA

AAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 89-94): nucleotide numbers 19-88
Spike protein full-length sequence: nucleotide numbers 89-3910
3'-UTR sequence: nucleotide numbers 3911-4042
polyA sequence (A100): nucleotide numbers 4043-4142

<SEQ ID NO: 5> (SARS-CoV-2 S-full mRNA-001)

GUAAUACGACUCACUAUAAGGAGACCCAAGCUACAUUUGCUUCUGACACAA
CUGUGUUCACUAGCAACCUCAAACAGACACCGCCACCAUGUUUGUUUUUCU
UGUUUUAUUGCCACUAGUCUCUAGUCAGUGUGUUAAUCUUACAACCAGAA
CUCAAUUACCCCCUGCAUACACUAAUUCUUUCACACGUGGUGUUUAUUACC
CUGACAAAGUUUUCAGAUCCUCAGUUUUACAUUCAACUCAGGACUUGUUC
UUACCUUUCUUUUCCAAUGUUACUUGGUUCCAUGCUAUACAUGUCUCUGG
GACCAAUGGUACUAAGAGGUUUGAUAACCCUGUCCUACCAUUUAAUGAUG
GUGUUUAUUUUGCUUCCACUGAGAAGUCUAACAUAAUAAGAGGCUGGAUU
UUUGGUACUACUUUAGAUUCGAAGACCCAGUCCCUACUUAUUGUUAAUAA
CGCUACUAAUGUUGUUAUUAAAGUCUGUGAAUUUCAAUUUUGUAAUGAUC
CAUUUUUGGGUGUUUAUUACCACAAAAACAACAAAAGUUGGAUGGAAAGU
GAGUUCAGAGUUUAUUCUAGUGCGAAUAAUUGCACUUUUGAAUAUGUCUC

UCAGCCUUUUCUUAUGGACCUUGAAGGAAAACAGGGUAAUUUCAAAAAUC

UUAGGGAAUUUGUGUUUAAGAAUAUUGAUGGUUAUUUUAAAAUAUAUUCU

AAGCACACGCCUAUUAAUUUAGUGCGUGAUCUCCCUCAGGGUUUUUCGGC

UUUAGAACCAUUGGUAGAUUUGCCAAUAGGUAUUAACAUCACUAGGUUUC

AAACUUUACUUGCUUUACAUAGAAGUUAUUUGACUCCUGGUGAUUCUUCU

UCAGGUUGGACAGCUGGUGCUGCAGCUUAUUAUGUGGGUUAUCUUCAACC

UAGGACUUUUCUAUUAAAAUAUAAUGAAAAUGGAACCAUUACAGAUGCUGU

AGACUGUGCACUUGACCCUCUCUCAGAAACAAAGUGUACGUUGAAAUCCUU

CACUGUAGAAAAAGGAAUCUAUCAAACUUCUAACUUUAGAGUCCAACCAAC

AGAAUCUAUUGUUAGAUUUCCUAAUAUUACAAACUUGUGCCCUUUUGGUG

AAGUUUUUAACGCCACCAGAUUUGCAUCUGUUUAUGCUUGGAACAGGAAG

AGAAUCAGCAACUGUGUUGCUGAUUAUUCUGUCCUAUAUAAUUCCGCAUC

AUUUUCCACUUUUAAGUGUUAUGGAGUGUCUCCUACUAAAUUAAAUGAUC

UCUGCUUUACUAAUGUCUAUGCAGAUUCAUUUGUAAUUAGAGGUGAUGAA

GUCAGACAAAUCGCUCCAGGGCAAACUGGAAAGAUUGCUGAUUAUAAUUAU

AAAUUACCAGAUGAUUUUACAGGCUGCGUUAUAGCUUGGAAUUCUAACAA

UCUUGAUUCUAAGGUUGGUGGUAAUUAUAAUUACCUGUAUAGAUUGUUUA

GGAAGUCUAAUCUCAAACCUUUUGAGAGAGAUAUUUCAACUGAAAUCUAUC

AGGCCGGUAGCACACCUUGUAAUGGUGUUGAAGGUUUUAAUUGUUACUUU

CCUUUACAAUCAUAUGGUUUCCAACCCACUAAUGGUGUUGGUUACCAACCA

UACAGAGUAGUAGUACUUUCUUUUGAACUUCUACAUGCACCAGCAACUGU

UUGUGGACCUAAAAAGUCUACUAAUUUGGUUAAAAACAAAUGUGUCAAUU

UCAACUUCAAUGGUUUAACAGGCACAGGUGUUCUUACUGAGUCUAACAAAA

AGUUUCUGCCUUUCCAACAAUUUGGCAGAGACAUUGCUGACACUACUGAU

GCUGUCCGUGAUCCACAGACACUUGAGAUUCUUGACAUUACACCAUGUUC

UUUUGGUGGUGUCAGUGUUAUAACACCAGGAACAAAUACUUCUAACCAGG

UUGCUGUUCUUUAUCAGGAUGUUAACUGCACAGAAGUCCCUGUUGCUAUU

CAUGCAGAUCAACUUACUCCUACUUGGCGUGUUUAUUCUACAGGUUCUAA

UGUUUUUCAAACACGUGCAGGCUGUUUAAUAGGGGCUGAACAUGUCAACA

ACUCAUAUGAGUGUGACAUACCCAUUGGUGCAGGUAUAUGCGCUAGUUAU

CAGACUCAGACUAAUUCUCCUCGGCGGGCACGUAGUGUAGCUAGUCAAUC

CAUCAUUGCCUACACUAUGUCACUUGGUGCAGAAAAUUCAGUUGCUUACU

CUAAUAACUCUAUUGCCAUACCCACAAAUUUUACUAUUAGUGUUACCACAG

AAAUUCUACCAGUGUCUAUGACCAAGACAUCAGUAGAUUGUACAAUGUACA

UUUGUGGUGAUUCAACUGAAUGCAGCAAUCUUUUGUUGCAAUAUGGCAGU

UUUUGUACACAAUUAAACCGUGCUUUAACUGGAAUAGCUGUUGAACAAGA

CAAAAACACCCAAGAAGUUUUUGCACAAGUCAAACAAAUUUACAAAACACCA
CCAAUUAAAGAUUUUGGUGGUUUUAAUUUUUCACAAAUAUUACCAGAUCC
AUCAAAACCAAGCAAGAGGUCAUUUAUUGAAGAUCUACUUUUCAACAAAGU
GACACUUGCAGAUGCUGGCUUCAUCAAACAAUAUGGUGAUUGCCUUGGUG
AUAUUGCUGCUAGAGACCUCAUUUGUGCACAAAAGUUUAACGGCCUUACU
GUUUUGCCACCUUUGCUCACAGAUGAAAUGAUUGCUCAAUACACUUCUGC
ACUGUUAGCGGGUACAAUCACUUCUGGUUGGACCUUUGGUGCAGGUGCU
GCAUUACAAAUACCAUUUGCUAUGCAAAUGGCUUAUAGGUUUAAUGGUAU
UGGAGUUACACAGAAUGUUCUCUAUGAGAACCAAAAAUUGAUUGCCAACCA
AUUUAAUAGUGCUAUUGGCAAAAUUCAAGACUCACUUUCUUCCACAGCAAG
UGCACUUGGAAAACUUCAAGAUGUGGUCAACCAAAAUGCACAAGCUUUAAA
CACGCUUGUUAAACAACUUAGCUCCAAUUUUGGUGCAAUUUCAAGUGUUU
UAAAUGAUAUCCUUUCACGUCUUGACAAAGUUGAGGCUGAAGUGCAAAUU
GAUAGGUUGAUCACAGGCAGACUUCAAAGUUUGCAGACAUAUGUGACUCA
ACAAUUAAUUAGAGCUGCAGAAAUCAGAGCUUCUGCUAAUCUUGCUGCUAC
UAAAAUGUCAGAGUGUGUACUUGGACAAUCAAAAAGAGUUGAUUUUUGUG
GAAAGGGCUAUCAUCUUAUGUCCUUCCCUCAGUCAGCACCUCAUGGUGUA
GUCUUCUUGCAUGUGACUUAUGUCCCUGCACAAGAAAAGAACUUCACAACU

GCUCCUGCCAUUUGUCAUGAUGGAAAAGCACACUUUCCUCGUGAAGGUGU

CUUUGUUUCAAAUGGCACACACUGGUUUGUAACACAAAGGAAUUUUUAUG

AACCACAAAUCAUUACUACAGACAACACAUUUGUGUCUGGUAACUGUGAUG

UUGUAAUAGGAAUUGUCAACAACACAGUUUAUGAUCCUUUGCAACCUGAA

UUAGACUCAUUCAAGGAGGAGUUAGAUAAAUAUUUUAAGAAUCAUACAUCA

CCAGAUGUUGAUUUAGGUGACAUCUCUGGCAUUAAUGCUUCAGUUGUAAA

CAUUCAAAAAGAAAUUGACCGCCUCAAUGAGGUUGCCAAGAAUUUAAAUGA

AUCUCUCAUCGAUCUCCAAGAACUUGGAAAGUAUGAGCAGUAUAUAAAAUG

GCCAUGGUACAUUUGGCUAGGUUUUAUAGCUGGCUUGAUUGCCAUAGUAA

UGGUGACAAUUAUGCUUUGCUGUAUGACCAGUUGCUGUAGUUGUCUCAAG

GGCUGUUGUUCUUGUGGAUCCUGCUGCAAAUUUGAUGAAGACGACUCUGA

GCCAGUGCUCAAAGGAGUCAAAUUACAUUACACAUGAGCUCGCUUUCUUG

CUGUCCAAUUUCUAUUAAAGGUUCCUUUGUUCCCUAAGUCCAACUACUAAA

CUGGGGGAUAUUAUGAAGGGCCUUGAGCAUCUGGAUUCUGCCUAAUAAAA

AACAUUUAUUUUCAUUGCAAAAAAAAAAAAAAAAAAAAAAAAAAAAAAAA

AAAAAAAAAAAAAAAAAAAAAAAAAAAAAAAAAAAAAAAAAAAAAAAAAA

AAAAAAAAAA

<SEQ ID NO: 6> (Amino acid sequence of SARS-CoV-2 S-full)

MFVFLVLLPLVSSQCVNLTTRTQLPPAYTNSFTRGVYYPDKVFRSSVLHSTQDLF LPFFSNVTWFHAIHVSGTNGTKRFDNPVLPFNDGVYFASTEKSNIIRGWIFGTTL DSKTQSLLIVNNATNVVIKVCEFQFCNDPFLGVYYHKNNKSWMESEFRVYSSANN CTFEYVSQPFLMDLEGKQGNFKNLREFVFKNIDGYFKIYSKHTPINLVRDLPQGFS ALEPLVDLPIGINITRFQTLLALHRSYLTPGDSSSGWTAGAAAYYVGYLQPRTFLL KYNENGTITDAVDCALDPLSETKCTLKSFTVEKGIYQTSNFRVQPTESIVRFPNIT NLCPFGEVFNATRFASVYAWNRKRISNCVADYSVLYNSASFSTFKCYGVSPTKLND LCFTNVYADSFVIRGDEVRQIAPGQTGKIADYNYKLPDDFTGCVIAWNSNNLDSK VGGNYNYLYRLFRKSNLKPFERDISTEIYQAGSTPCNGVEGFNCYFPLQSYGFQPT NGVGYQPYRVVVLSFELLHAPATVCGPKKSTNLVKNKCVNFNFNGLTGTGVLTE SNKKFLPFQQFGRDIADTTDAVRDPQTLEILDITPCSFGGVSVITPGTNTSNQVA VLYQDVNCTEVPVAIHADQLTPTWRVYSTGSNVFQTRAGCLIGAEHVNNSYECD IPIGAGICASYQTQTNSPRRARSVASQSIIAYTMSLGAENSVAYSNNSIAIPTNFTISV TTEILPVSMTKTSVDCTMYICGDSTECSNLLLQYGSFCTQLNRALTGIAVEQDKN TQEVFAQVKQIYKTPPIKDFGGFNFSQILPDPSKPSKRSFIEDLLFNKVTLADAGFI KQYGDCLGDIAARDLICAQKFNGLTVLPPLLTDEMIAQYTSALLAGTITSGWTFG AGAALQIPFAMQMAYRFNGIGVTQNVLYENQKLIANQFNSAIGKIQDSLSSTASAL

GKLQDVVNQNAQALNTLVKQLSSNFGAISSVLNDILSRLDKVEAEVQIDRLITGR

LQSLQTYVTQQLIRAAEIRASANLAATKMSECVLGQSKRVDFCGKGYHLMSFPQS

APHGVVFLHVTYVPAQEKNFTTAPAICHDGKAHFPREGVFVSNGTHWFVTQRN

FYEPQIITTDNTFVSGNCDVVIGIVNNTVYDPLQPELDSFKEELDKYFKNHTSPDV

DLGDISGINASVVNIQKEIDRLNEVAKNLNESLIDLQELGKYEQYIKWPWYIWLGFI

AGLIAIVMVTIMLCCMTSCCSCLKGCCSCGSCCKFDEDDSEPVLKGVKLHYT

RBD sequence: amino acid numbers 319-541
<SEQ ID NO: 7> (DNA fragment comprising SARS-CoV-2 RBD)

GTAATACGACTCACTATAAGGAGACCCAAGCTACATTTGCTTCTGACACAAC

TGTGTTCACTAGCAACCTCAAACAGACACCGCCACCATGTTTGTTTTTCTTG

TTTTATTGCCACTAGTCTCTAGTAGAGTCCAACCAACAGAATCTATTGTTAG

ATTTCCTAATATTACAAACTTGTGCCCTTTTGGTGAAGTTTTTAACGCCACC

AGATTTGCATCTGTTTATGCTTGGAACAGGAAGAGAATCAGCAACTGTGTTG

CTGATTATTCTGTCCTATATAATTCCGCATCATTTTCCACTTTTAAGTGTTA

TGGAGTGTCTCCTACTAAATTAAATGATCTCTGCTTTACTAATGTCTATGCA

GATTCATTTGTAATTAGAGGTGATGAAGTCAGACAAATCGCTCCAGGGCAAA

CTGGAAAGATTGCTGATTATAATTATAAATTACCAGATGATTTTACAGGCTG
CGTTATAGCTTGGAATTCTAACAATCTTGATTCTAAGGTTGGTGGTAATTAT
AATTACCTGTATAGATTGTTTAGGAAGTCTAATCTCAAACCTTTTGAGAGAG
ATATTTCAACTGAAATCTATCAGGCCGGTAGCACACCTTGTAATGGTGTTGA
AGGTTTTAATTGTTACTTTCCTTTACAATCATATGGTTTCCAACCCACTAAT
GGTGTTGGTTACCAACCATACAGAGTAGTAGTACTTTCTTTTGAACTTCTAC
ATGCACCAGCAACTGTTTGTGGACCTAAAAAGTCTACTAATTTGGTTAAAAA
CAAATGTGTCAATTTCTGAGCTCGCTTTCTTGCTGTCCAATTTCTATTAAAG
GTTCCTTTGTTCCCTAAGTCCAACTACTAAACTGGGGGATATTATGAAGGGC
CTTGAGCATCTGGATTCTGCCTAATAAAAAACATTTATTTTCATTGC

<SEQ ID NO: 8> (Template DNA for SARS-CoV-2 RBD)

GTAATACGACTCACTATAAGGAGACCCAAGCTACATTTGCTTCTGACACAAC
TGTGTTCACTAGCAACCTCAAACAGACACCGCCACCATGTTTGTTTTTCTTG
TTTTATTGCCACTAGTCTCTAGTAGAGTCCAACCAACAGAATCTATTGTTAG
ATTTCCTAATATTACAAACTTGTGCCCTTTTGGTGAAGTTTTAACGCCACC
AGATTTGCATCTGTTTATGCTTGGAACAGGAAGAGAATCAGCAACTGTGTTG
CTGATTATTCTGTCCTATATAATTCCGCATCATTTTCCACTTTTAAGTGTTA

TGGAGTGTCTCCTACTAAATTAAATGATCTCTGCTTTACTAATGTCTATGCA

GATTCATTTGTAATTAGAGGTGATGAAGTCAGACAAATCGCTCCAGGGCAAA

CTGGAAAGATTGCTGATTATAATTATAAATTACCAGATGATTTTACAGGCTG

CGTTATAGCTTGGAATTCTAACAATCTTGATTCTAAGGTTGGTGGTAATTAT

AATTACCTGTATAGATTGTTTAGGAAGTCTAATCTCAAACCTTTTGAGAGAG

ATATTTCAACTGAAATCTATCAGGCCGGTAGCACACCTTGTAATGGTGTTGA

AGGTTTTAATTGTTACTTTCCTTTACAATCATATGGTTTCCAACCCACTAAT

GGTGTTGGTTACCAACCATACAGAGTAGTAGTACTTTCTTTTGAACTTCTAC

ATGCACCAGCAACTGTTTGTGGACCTAAAAAGTCTACTAATTTGGTTAAAAA

CAAATGTGTCAATTTCTGAGCTCGCTTTCTTGCTGTCCAATTTCTATTAAAG

GTTCCTTTGTTCCCTAAGTCCAACTACTAAACTGGGGGATATTATGAAGGGC

CTTGAGCATCTGGATTCTGCCTAATAAAAACATTTATTTTCATTGCAAAAAA

AAAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 89-94): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 9> (SARS-CoV-2 RBD mRNA-002)

AGGAGACCCAAGCUACAUUUGCUUCUGACACAACUGUGUUCACUAGCAACC

UCAAACAGACACCGCCACCAUGUUUGUUUUUCUUGUUUUAUUGCCACUAG

UCUCUAGUAGAGUCCAACCAACAGAAUCUAUUGUUAGAUUUCCUAAUAUUA

CAAACUUGUGCCCUUUUGGUGAAGUUUUUAACGCCACCAGAUUUGCAUCU

GUUUAUGCUUGGAACAGGAAGAGAAUCAGCAACUGUGUUGCUGAUUAUUC

UGUCCUAUAUAAUUCCGCAUCAUUUUCCACUUUUAAGUGUUAUGGAGUGU

CUCCUACUAAAUUAAAUGAUCUCUGCUUUACUAAUGUCUAUGCAGAUUCA

UUUGUAAUUAGAGGUGAUGAAGUCAGACAAAUCGCUCCAGGGCAAACUGG

AAAGAUUGCUGAUUAUAAUUAUAAAUUACCAGAUGAUUUUACAGGCUGCG

UUAUAGCUUGGAAUUCUAACAAUCUUGAUUCUAAGGUUGGUGGUAAUUAU

AAUUACCUGUAUAGAUUGUUUAGGAAGUCUAAUCUCAAACCUUUUGAGAG
AGAUAUUUCAACUGAAAUCUAUCAGGCCGGUAGCACACCUUGUAAUGGUG
UUGAAGGUUUUAAUUGUUACUUUCCUUUACAAUCAUAUGGUUUCCAACCC
ACUAAUGGUGUUGGUUACCAACCAUACAGAGUAGUAGUACUUUCUUUUGA
ACUUCUACAUGCACCAGCAACUGUUUGUGGACCUAAAAAGUCUACUAAUUU
GGUUAAAAACAAAUGUGUCAAUUUCUGAGCUCGCUUUCUUGCUGUCCAAU
UUCUAUUAAAGGUUCCUUUGUUCCCUAAGUCCAACUACUAAACUGGGGGA
UAUUAUGAAGGGCCUUGAGCAUCUGGAUUCUGCCUAAUAAAAAACAUUUA
UUUUCAUUGCAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAA
AAA

<SEQ ID NO: 10> (Amino acid sequence of SARS-CoV-2 RBD (including S protein signal sequence))

MFVFLVLLPLVSSRVQPTESIVRFPNITNLCPFGEVFNATRFASVYAWNRKRISNCV
ADYSVLYNSASFSTFKCYGVSPTKLNDLCFTNVYADSFVIRGDEVRQIAPGQTGKI
ADYNYKLPDDFTGCVIAWNSNNLDSKVGGNYNYLYRLFRKSNLKPFERDISTEIY
QAGSTPCNGVEGFNCYFPLQSYGFQPTNGVGYQPYRVVVLSFELLHAPATVCGP
KKSTNLVKNKCVNF

S protein signal sequence: amino acid numbers 1-13
RBD sequence: amino acid numbers 14-236

<SEQ ID NO: 11> (Amino acid sequence of SARS-CoV-2 RBD (not including S protein signal sequence))

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYLYRLFRKSNLKPFERDISTEIYQAGSTPCNGVEG

FNCYFPLQSYGFQPTNGVGYQPYRVVVLSFELLHAPATVCGPKKSTNLVKNKCV

NF

<SEQ ID NO: 12> (S_opt2 EcoRI)

GCTAGCGTAATACGACTCACTATAAGGAGACCCAAGCTACATTTGCTTCTGA

CACAACTGTGTTCACTAGCAACCTCAAACAGACACCGCCACCATGTTCGTGT

TCCTGGTGCTGCTGCCCCTGGTGAGCAGCCAGTGCGTGAACCTGACCACCA

GAACACAGCTGCCCCCAGCCTACACCAACAGCTTCACCAGAGGCGTGTACTA
CCCCGACAAGGTGTTCAGAAGCAGCGTGCTGCACAGCACCCAGGACCTGTT
CCTGCCCTTCTTCAGCAACGTGACCTGGTTCCACGCCATCCACGTGAGCGG
CACCAACGGCACCAAGAGATTCGACAACCCCGTGCTGCCCTTCAACGACGGG
GTGTACTTCGCCAGCACCGAGAAGTCCAACATCATCAGAGGCTGGATCTTCG
GCACCACACTGGACAGCAAGACCCAGAGCCTGCTGATCGTGAACAACGCCAC
CAACGTGGTCATCAAAGTGTGCGAGTTCCAGTTCTGCAACGACCCCTTCCTG
GGCGTCTACTACCACAAGAACAACAAGAGCTGGATGGAAAGCGAGTTCCGG
GTGTACAGCAGCGCCAACAACTGCACCTTCGAGTACGTGAGCCAGCCCTTCC
TGATGGACCTGGAAGGCAAGCAGGGCAACTTCAAGAACCTGCGCGAGTTCG
TGTTCAAGAACATCGACGGCTACTTCAAGATCTACAGCAAGCACACCCCCAT
CAACCTCGTGCGGGACCTGCCCCAGGGCTTCAGCGCCCTGGAACCCCTGGT
GGACCTGCCCATCGGCATCAACATCACCCGGTTCCAGACACTGCTGGCCCTG
CACAGAAGCTACCTGACACCCGGCGACAGCAGCAGCGGATGGACAGCCGGC
GCCGCCGCCTACTACGTGGGCTACCTGCAGCCCAGAACCTTCCTGCTGAAG
TACAACGAGAACGGCACCATCACCGACGCCGTGGACTGCGCCCTGGACCCC
CTGAGCGAGACAAAGTGCACCCTGAAGTCCTTCACCGTGGAAAAGGGCATC
TACCAGACCAGCAACTTCCGGGTGCAGCCCACCGAAAGCATCGTGCGGTTC

CCCAACATCACCAACCTGTGCCCCTTCGGCGAGGTGTTCAACGCCACCAGAT
TCGCCAGCGTGTACGCCTGGAACCGGAAGCGGATCAGCAACTGCGTGGCCG
ACTACAGCGTGCTGTACAACAGCGCCAGCTTCAGCACCTTCAAGTGCTACGG
CGTGAGCCCCACCAAGCTGAACGACCTGTGCTTCACAAACGTGTACGCCGAC
AGCTTCGTGATCCGGGGAGACGAAGTGCGGCAGATCGCCCCCGGACAGACA
GGCAAGATCGCCGACTACAACTACAAGCTGCCCGACGACTTCACCGGCTGCG
TGATCGCCTGGAACAGCAACAACCTGGACAGCAAAGTCGGCGGCAACTACAA
CTACCTGTACCGGCTGTTCCGGAAGTCCAACCTGAAGCCCTTCGAGCGGGA
CATCAGCACCGAGATCTACCAGGCCGGCAGCACCCCCTGCAACGGCGTGGA
AGGCTTCAACTGCTACTTCCCACTGCAGAGCTACGGCTTCCAGCCCACAAAC
GGCGTGGGCTACCAGCCCTACAGAGTGGTGGTGCTGAGCTTCGAACTGCTG
CACGCCCCCGCCACAGTGTGCGGCCCCAAGAAAAGCACCAACCTCGTGAAGA
ACAAATGCGTGAACTTCAACTTCAACGGCCTGACCGGCACCGGCGTGCTGAC
AGAGAGCAACAAGAAGTTCCTGCCATTCCAGCAGTTCGGCCGGGACATCGC
CGACACCACAGACGCCGTCAGAGACCCCCAGACACTGGAAATCCTGGACATC
ACCCCCTGCAGCTTCGGCGGAGTGAGCGTGATCACCCCCGGCACCAACACC
AGCAACCAGGTGGCAGTGCTGTACCAGGACGTGAACTGCACCGAAGTGCCC
GTGGCCATCCACGCCGACCAGCTGACACCCACATGGCGGGTGTACAGCACC

GGCAGCAACGTGTTCCAGACCAGAGCCGGCTGCCTGATCGGAGCCGAGCAC

GTGAACAACAGCTACGAGTGCGACATCCCCATCGGCGCCGGCATCTGCGCC

AGCTACCAGACACAGACAAACAGCCCCAGACGGGCCAGAAGCGTGGCCAGCC

AGAGCATCATCGCCTACACAATGAGCCTGGGCGCCGAGAACAGCGTGGCCT

ACAGCAACAACAGCATCGCCATCCCCACCAACTTCACCATCAGCGTGACCAC

AGAGATCCTGCCCGTGAGCATGACCAAGACCAGCGTGGACTGCACCATGTA

CATCTGCGGCGACAGCACCGAGTGCAGCAACCTGCTGCTGCAGTACGGCAG

CTTCTGCACCCAGCTGAACAGAGCCCTGACAGGGATCGCCGTGGAACAGGA

CAAGAACACCCAAGAGGTGTTCGCCCAAGTGAAGCAGATCTACAAGACCCCC

CCCATCAAGGACTTCGGCGGCTTCAACTTCAGCCAGATCCTGCCCGACCCCA

GCAAGCCCAGCAAGCGGAGCTTCATCGAGGACCTGCTGTTCAACAAAGTGA

CACTGGCCGACGCCGGCTTCATCAAGCAGTACGGCGACTGCCTGGGCGACA

TCGCCGCCAGGGACCTGATCTGCGCCCAGAAGTTCAACGGACTGACAGTGC

TGCCCCCCCTGCTGACCGACGAGATGATCGCCCAGTACACAAGCGCCCTGCT

GGCCGGCACAATCACAAGCGGCTGGACATTCGGAGCCGGCGCCGCCCTGCA

GATCCCCTTCGCCATGCAGATGGCCTACCGGTTCAACGGCATCGGAGTGAC

CCAGAACGTGCTGTACGAGAACCAGAAGCTGATCGCCAACCAGTTCAACAGC

GCCATCGGCAAGATCCAGGACAGCCTGAGCAGCACAGCAAGCGCCCTGGGA

AAGCTGCAGGACGTGGTCAACCAGAACGCCCAGGCACTGAACACCCTGGTC
AAGCAGCTGAGCAGCAACTTCGGCGCCATCAGCAGCGTGCTGAACGACATC
CTGAGCAGACTGGACAAGGTGGAAGCCGAGGTGCAGATCGACAGACTGATC
ACCGGAAGGCTGCAGAGCCTGCAGACCTACGTCACCCAGCAGCTGATCAGA
GCCGCCGAGATCAGAGCCAGCGCCAACCTGGCCGCCACCAAGATGAGCGAG
TGCGTGCTGGGCCAGAGCAAGAGAGTGGACTTCTGCGGCAAGGGCTACCAC
CTGATGAGCTTCCCCCAGAGCGCCCCCCACGGCGTGGTGTTCCTGCACGTG
ACATACGTGCCCGCCCAAGAGAAGAACTTCACCACCGCCCCAGCCATCTGCC
ACGACGGCAAAGCCCACTTCCCCAGAGAAGGCGTGTTCGTGAGCAACGGCA
CCCACTGGTTCGTGACCCAGCGGAACTTCTACGAGCCCCAGATCATCACCAC
CGACAACACCTTCGTGAGCGGCAACTGCGACGTCGTGATCGGCATCGTGAA
CAACACCGTGTACGACCCCCTGCAGCCCGAGCTGGACAGCTTCAAAGAGGAA
CTGGACAAGTACTTCAAGAACCACACAAGCCCCGACGTGGACCTGGGCGACA
TCAGCGGAATCAACGCCAGCGTCGTGAACATCCAGAAAGAGATCGACCGGCT
GAACGAGGTGGCCAAGAACCTGAACGAGAGCCTGATCGACCTGCAAGAACT
GGGGAAGTACGAGCAGTACATCAAGTGGCCCTGGTACATCTGGCTGGGCTT
CATCGCCGGACTGATCGCCATCGTGATGGTCACAATCATGCTGTGCTGCAT
GACCAGCTGCTGCAGCTGCCTGAAGGGCTGCTGCAGCTGCGGCAGCTGCTG

CAAGTTCGACGAGGACGACAGCGAGCCCGTGCTGAAGGGCGTGAAACTGCA

CTACACATGAGCTCGCTTTCTTGCTGTCCAATTTCTATTAAAGGTTCCTTTG

TTCCCTAAGTCCAACTACTAAACTGGGGGATATTATGAAGGGCCTTGAGCAT

CTGGATTCTGCCTAATAAAAAACATTTATTTTCATTGCGAATTC

NheI sequence: nucleotide numbers 1-6
T7 promoter sequence: nucleotide numbers 7-24
A: transcription start site: nucleotide number 25
5'-UTR sequence (including transcription start site and KOZAK sequence of nucleotide numbers 89-94): nucleotide numbers 25-94
Spike protein full-length sequence: nucleotide numbers 95-3916
3'-UTR sequence: nucleotide numbers 3917-4048
EcoRI sequence: nucleotide numbers 4049-4054

<SEQ ID NO: 13> (sense primer 2)
TGATGCTAGCGTAATACGACTCACTATAAG
NheI sequence: nucleotide numbers 5-10
<SEQ ID NO: 14> (antisense primer 2)

GCCAAAGCTTGCTCTTCGTTTTTTTTTTTTTTTTTTTTTTTTTTTTTTTTTT

TTTTTTTTTTTTTTTTTTTTTTTTTTTTTTTTTTTTTTTTTTTTTTTTTTTTT

TTTTTTTTTTTTTTTTTTTTTTTGCAATGAAAATAAATGTTTTTT

HindIII sequence: nucleotide numbers 5-10
BspQI sequence: nucleotide numbers 11-17

<SEQ ID NO: 15> (Template DNA for SARS-CoV-2 S full optimized)

TGATGCTAGCGTAATACGACTCACTATAAGGAGACCCAAGCTACATTTGCTT
CTGACACAACTGTGTTCACTAGCAACCTCAAACAGACACCGCCACCATGTTC
GTGTTCCTGGTGCTGCTGCCCCTGGTGAGCAGCCAGTGCGTGAACCTGACC
ACCAGAACACAGCTGCCCCCAGCCTACACCAACAGCTTCACCAGAGGCGTGT
ACTACCCCGACAAGGTGTTCAGAAGCAGCGTGCTGCACAGCACCCAGGACCT
GTTCCTGCCCTTCTTCAGCAACGTGACCTGGTTCCACGCCATCCACGTGAGC
GGCACCAACGGCACCAAGAGATTCGACAACCCCGTGCTGCCCTTCAACGACG
GGGTGTACTTCGCCAGCACCGAGAAGTCCAACATCATCAGAGGCTGGATCT
TCGGCACCACACTGGACAGCAAGACCCAGAGCCTGCTGATCGTGAACAACGC

CACCAACGTGGTCATCAAAGTGTGCGAGTTCCAGTTCTGCAACGACCCCTTC
CTGGGCGTCTACTACCACAAGAACAACAAGAGCTGGATGGAAAGCGAGTTCC
GGGTGTACAGCAGCGCCAACAACTGCACCTTCGAGTACGTGAGCCAGCCCT
TCCTGATGGACCTGGAAGGCAAGCAGGGCAACTTCAAGAACCTGCGCGAGT
TCGTGTTCAAGAACATCGACGGCTACTTCAAGATCTACAGCAAGCACACCCC
CATCAACCTCGTGCGGGACCTGCCCCAGGGCTTCAGCGCCCTGGAACCCCT
GGTGGACCTGCCCATCGGCATCAACATCACCCGGTTCCAGACACTGCTGGC
CCTGCACAGAAGCTACCTGACACCCGGCGACAGCAGCAGCGGATGGACAGC
CGGCGCCGCCGCCTACTACGTGGGCTACCTGCAGCCCAGAACCTTCCTGCT
GAAGTACAACGAGAACGGCACCATCACCGACGCCGTGGACTGCGCCCTGGA
CCCCCTGAGCGAGACAAAGTGCACCCTGAAGTCCTTCACCGTGGAAAAGGG
CATCTACCAGACCAGCAACTTCCGGGTGCAGCCCACCGAAAGCATCGTGCGG
TTCCCCAACATCACCAACCTGTGCCCCTTCGGCGAGGTGTTCAACGCCACCA
GATTCGCCAGCGTGTACGCCTGGAACCGGAAGCGGATCAGCAACTGCGTGG
CCGACTACAGCGTGCTGTACAACAGCGCCAGCTTCAGCACCTTCAAGTGCTA
CGGCGTGAGCCCCACCAAGCTGAACGACCTGTGCTTCACAAACGTGTACGC
CGACAGCTTCGTGATCCGGGGAGACGAAGTGCGGCAGATCGCCCCCGGACA
GACAGGCAAGATCGCCGACTACAACTACAAGCTGCCCGACGACTTCACCGGC

TGCGTGATCGCCTGGAACAGCAACAACCTGGACAGCAAAGTCGGCGGCAAC
TACAACTACCTGTACCGGCTGTTCCGGAAGTCCAACCTGAAGCCCTTCGAGC
GGGACATCAGCACCGAGATCTACCAGGCCGGCAGCACCCCCTGCAACGGCG
TGGAAGGCTTCAACTGCTACTTCCCACTGCAGAGCTACGGCTTCCAGCCCAC
AAACGGCGTGGGCTACCAGCCCTACAGAGTGGTGGTGCTGAGCTTCGAACT
GCTGCACGCCCCCGCCACAGTGTGCGGCCCCAAGAAAAGCACCAACCTCGT
GAAGAACAAATGCGTGAACTTCAACTTCAACGGCCTGACCGGCACCGGCGT
GCTGACAGAGAGCAACAAGAAGTTCCTGCCATTCCAGCAGTTCGGCCGGGA
CATCGCCGACACCACAGACGCCGTCAGAGACCCCCAGACACTGGAAATCCTG
GACATCACCCCCTGCAGCTTCGGCGGAGTGAGCGTGATCACCCCCGGCACC
AACACCAGCAACCAGGTGGCAGTGCTGTACCAGGACGTGAACTGCACCGAA
GTGCCCGTGGCCATCCACGCCGACCAGCTGACACCCACATGGCGGGTGTAC
AGCACCGGCAGCAACGTGTTCCAGACCAGAGCCGGCTGCCTGATCGGAGCC
GAGCACGTGAACAACAGCTACGAGTGCGACATCCCCATCGGCGCCGGCATC
TGCGCCAGCTACCAGACACAGACAAACAGCCCCAGACGGGCCAGAAGCGTG
GCCAGCCAGAGCATCATCGCCTACACAATGAGCCTGGGCGCCGAGAACAGC
GTGGCCTACAGCAACAACAGCATCGCCATCCCCACCAACTTCACCATCAGCG
TGACCACAGAGATCCTGCCCGTGAGCATGACCAAGACCAGCGTGGACTGCA

CCATGTACATCTGCGGCGACAGCACCGAGTGCAGCAACCTGCTGCTGCAGT
ACGGCAGCTTCTGCACCCAGCTGAACAGAGCCCTGACAGGGATCGCCGTGG
AACAGGACAAGAACACCCAAGAGGTGTTCGCCCAAGTGAAGCAGATCTACAA
GACCCCCCCCATCAAGGACTTCGGCGGCTTCAACTTCAGCCAGATCCTGCCC
GACCCCAGCAAGCCCAGCAAGCGGAGCTTCATCGAGGACCTGCTGTTCAACA
AAGTGACACTGGCCGACGCCGGCTTCATCAAGCAGTACGGCGACTGCCTGG
GCGACATCGCCGCCAGGGACCTGATCTGCGCCCAGAAGTTCAACGGACTGA
CAGTGCTGCCCCCCCTGCTGACCGACGAGATGATCGCCCAGTACACAAGCG
CCCTGCTGGCCGGCACAATCACAAGCGGCTGGACATTCGGAGCCGGCGCCG
CCCTGCAGATCCCCTTCGCCATGCAGATGGCCTACCGGTTCAACGGCATCG
GAGTGACCCAGAACGTGCTGTACGAGAACCAGAAGCTGATCGCCAACCAGTT
CAACAGCGCCATCGGCAAGATCCAGGACAGCCTGAGCAGCACAGCAAGCGC
CCTGGGAAAGCTGCAGGACGTGGTCAACCAGAACGCCCAGGCACTGAACAC
CCTGGTCAAGCAGCTGAGCAGCAACTTCGGCGCCATCAGCAGCGTGCTGAA
CGACATCCTGAGCAGACTGGACAAGGTGGAAGCCGAGGTGCAGATCGACAG
ACTGATCACCGGAAGGCTGCAGAGCCTGCAGACCTACGTCACCCAGCAGCT
GATCAGAGCCGCCGAGATCAGAGCCAGCGCCAACCTGGCCGCCACCAAGAT
GAGCGAGTGCGTGCTGGGCCAGAGCAAGAGAGTGGACTTCTGCGGCAAGG

GCTACCACCTGATGAGCTTCCCCCAGAGCGCCCCCCACGGCGTGGTGTTCC

TGCACGTGACATACGTGCCCGCCCAAGAGAAGAACTTCACCACCGCCCCAGC

CATCTGCCACGACGGCAAAGCCCACTTCCCCAGAGAAGGCGTGTTCGTGAG

CAACGGCACCCACTGGTTCGTGACCCAGCGGAACTTCTACGAGCCCCAGATC

ATCACCACCGACAACACCTTCGTGAGCGGCAACTGCGACGTCGTGATCGGCA

TCGTGAACAACACCGTGTACGACCCCCTGCAGCCCGAGCTGGACAGCTTCAA

AGAGGAACTGGACAAGTACTTCAAGAACCACACAAGCCCCGACGTGGACCTG

GGCGACATCAGCGGAATCAACGCCAGCGTCGTGAACATCCAGAAAGAGATC

GACCGGCTGAACGAGGTGGCCAAGAACCTGAACGAGAGCCTGATCGACCTG

CAAGAACTGGGGAAGTACGAGCAGTACATCAAGTGGCCCTGGTACATCTGG

CTGGGCTTCATCGCCGGACTGATCGCCATCGTGATGGTCACAATCATGCTG

TGCTGCATGACCAGCTGCTGCAGCTGCCTGAAGGGCTGCTGCAGCTGCGGC

AGCTGCTGCAAGTTCGACGAGGACGACAGCGAGCCCGTGCTGAAGGGCGTG

AAACTGCACTACACATGAGCTCGCTTTCTTGCTGTCCAATTTCTATTAAAGG

TTCCTTTGTTCCCTAAGTCCAACTACTAAACTGGGGGATATTATGAAGGGCC

TTGAGCATCTGGATTCTGCCTAATAAAAAACATTTATTTTCATTGCAAAAAAA

AAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAACGAAG

AGCAAGCTTTGGC

NheI sequence: nucleotide numbers 5-10

T7 promoter sequence: nucleotide numbers 11-28
A: transcription start site: nucleotide number 29
5'-UTR sequence (including transcription start site and KOZAK sequence of nucleotide numbers 89-94): nucleotide numbers 29-98
Spike protein full-length sequence: nucleotide numbers 99-3920
3'-UTR sequence: nucleotide numbers 3921-4052
PolyA sequence (A110): nucleotide numbers 4053-4162
BspQI sequence: nucleotide numbers 4164-4170
HindIII sequence: nucleotide numbers 4171-4176

<SEQ ID NO: 16> (SARS-CoV-2 S full optimized mRNA-003)

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCCAGUGCGUGAACCUGACCACCAGAACACAGCUGCCCCCAGCCU

ACACCAACAGCUUCACCAGAGGCGUGUACUACCCCGACAAGGUGUUCAGAA
GCAGCGUGCUGCACAGCACCCAGGACCUGUUCCUGCCCUUCUUCAGCAAC
GUGACCUGGUUCCACGCCAUCCACGUGAGCGGCACCAACGGCACCAAGAGA
UUCGACAACCCCGUGCUGCCCUUCAACGACGGGGUGUACUUCGCCAGCAC
CGAGAAGUCCAACAUCAUCAGAGGCUGGAUCUUCGGCACCACACUGGACAG
CAAGACCCAGAGCCUGCUGAUCGUGAACAACGCCACCAACGUGGUCAUCAA
AGUGUGCGAGUUCCAGUUCUGCAACGACCCCUUCCUGGGCGUCUACUACC
ACAAGAACAACAAGAGCUGGAUGGAAAGCGAGUUCCGGGUGUACAGCAGCG
CCAACAACUGCACCUUCGAGUACGUGAGCCAGCCCUUCCUGAUGGACCUGG
AAGGCAAGCAGGGCAACUUCAAGAACCUGCGCGAGUUCGUGUUCAAGAACA
UCGACGGCUACUUCAAGAUCUACAGCAAGCACACCCCCAUCAACCUCGUGC
GGGACCUGCCCCAGGGCUUCAGCGCCCUGGAACCCCUGGUGGACCUGCCC
AUCGGCAUCAACAUCACCCGGUUCCAGACACUGCUGGCCCUGCACAGAAGC
UACCUGACACCCGGCGACAGCAGCAGCGGAUGGACAGCCGGCGCCGCCGCC
UACUACGUGGGCUACCUGCAGCCCAGAACCUUCCUGCUGAAGUACAACGAG
AACGGCACCAUCACCGACGCCGUGGACUGCGCCCUGGACCCCCUGAGCGAG
ACAAAGUGCACCCUGAAGUCCUUCACCGUGGAAAAGGGCAUCUACCAGACC
AGCAACUUCCGGGUGCAGCCCACCGAAAGCAUCGUGCGGUUCCCCAACAUC

ACCAACCUGUGCCCCUUCGGCGAGGUGUUCAACGCCACCAGAUUCGCCAGC
GUGUACGCCUGGAACCGGAAGCGGAUCAGCAACUGCGUGGCCGACUACAG
CGUGCUGUACAACAGCGCCAGCUUCAGCACCUUCAAGUGCUACGGCGUGA
GCCCCACCAAGCUGAACGACCUGUGCUUCACAAACGUGUACGCCGACAGCU
UCGUGAUCCGGGGAGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCUGUACCGGCUGUUCCGGAAGUCCAACCUGAAGCCCUUCGAGCGGGA
CAUCAGCACCGAGAUCUACCAGGCCGGCAGCACCCCCUGCAACGGCGUGGA
AGGCUUCAACUGCUACUUCCCACUGCAGAGCUACGGCUUCCAGCCCACAAA
CGGCGUGGGCUACCAGCCCUACAGAGUGGUGGUGCUGAGCUUCGAACUGC
UGCACGCCCCCGCCACAGUGUGCGGCCCCAAGAAAGCACCAACCUCGUGA
AGAACAAAUGCGUGAACUUCAACUUCAACGGCCUGACCGGCACCGGCGUGC
UGACAGAGAGCAACAAGAAGUUCCUGCCAUUCCAGCAGUUCGGCCGGGACA
UCGCCGACACCACAGACGCCGUCAGAGACCCCCAGACACUGGAAAUCCUGG
ACAUCACCCCCUGCAGCUUCGGCGGAGUGAGCGUGAUCACCCCCGGCACCA
ACACCAGCAACCAGGUGGCAGUGCUGUACCAGGACGUGAACUGCACCGAAG
UGCCCGUGGCCAUCCACGCCGACCAGCUGACACCCACAUGGCGGGUGUACA

GCACCGGCAGCAACGUGUUCCAGACCAGAGCCGGCUGCCUGAUCGGAGCC
GAGCACGUGAACAACAGCUACGAGUGCGACAUCCCCAUCGGCGCCGGCAUC
UGCGCCAGCUACCAGACACAGACAAACAGCCCCAGACGGGCCAGAAGCGUG
GCCAGCCAGAGCAUCAUCGCCUACACAAUGAGCCUGGGCGCCGAGAACAGC
GUGGCCUACAGCAACAACAGCAUCGCCAUCCCCACCAACUUCACCAUCAGC
GUGACCACAGAGAUCCUGCCCGUGAGCAUGACCAAGACCAGCGUGGACUGC
ACCAUGUACAUCUGCGGCGACAGCACCGAGUGCAGCAACCUGCUGCUGCAG
UACGGCAGCUUCUGCACCCAGCUGAACAGAGCCCUGACAGGGAUCGCCGU
GGAACAGGACAAGAACACCCAAGAGGUGUUCGCCCAAGUGAAGCAGAUCUA
CAAGACCCCCCCCAUCAAGGACUUCGGCGGCUUCAACUUCAGCCAGAUCCU
GCCCGACCCCAGCAAGCCCAGCAAGCGGAGCUUCAUCGAGGACCUGCUGUU
CAACAAAGUGACACUGGCCGACGCCGGCUUCAUCAAGCAGUACGGCGACUG
CCUGGGCGACAUCGCCGCCAGGGACCUGAUCUGCGCCCAGAAGUUCAACG
GACUGACAGUGCUGCCCCCCCUGCUGACCGACGAGAUGAUCGCCCAGUACA
CAAGCGCCCUGCUGGCCGGCACAAUCACAAGCGGCUGGACAUUCGGAGCCG
GCGCCGCCCUGCAGAUCCCCUUCGCCAUGCAGAUGGCCUACCGGUUCAAC
GGCAUCGGAGUGACCCAGAACGUGCUGUACGAGAACCAGAAGCUGAUCGCC
AACCAGUUCAACAGCGCCAUCGGCAAGAUCCAGGACAGCCUGAGCAGCACA

GCAAGCGCCCUGGGAAAGCUGCAGGACGUGGUCAACCAGAACGCCCAGGCA CUGAACACCCUGGUCAAGCAGCUGAGCAGCAACUUCGGCGCCAUCAGCAGC GUGCUGAACGACAUCCUGAGCAGACUGGACAAGGUGGAAGCCGAGGUGCA GAUCGACAGACUGAUCACCGGAAGGCUGCAGAGCCUGCAGACCUACGUCAC CCAGCAGCUGAUCAGAGCCGCCGAGAUCAGAGCCAGCGCCAACCUGGCCGC CACCAAGAUGAGCGAGUGCGUGCUGGGCCAGAGCAAGAGAGUGGACUUCU GCGGCAAGGGCUACCACCUGAUGAGCUUCCCCCAGAGCGCCCCCACGGCG UGGUGUUCCUGCACGUGACAUACGUGCCCGCCCAAGAGAAGAACUUCACCA CCGCCCCAGCCAUCUGCCACGACGGCAAAGCCCACUUCCCCAGAGAAGGCG UGUUCGUGAGCAACGGCACCCACUGGUUCGUGACCCAGCGGAACUUCUAC GAGCCCCAGAUCAUCACCACCGACAACACCUUCGUGAGCGGCAACUGCGAC GUCGUGAUCGGCAUCGUGAACAACACCGUGUACGACCCCCUGCAGCCCGAG CUGGACAGCUUCAAAGAGGAACUGGACAAGUACUUCAAGAACCACACAAGC CCCGACGUGGACCUGGGCGACAUCAGCGGAAUCAACGCCAGCGUCGUGAAC AUCCAGAAAGAGAUCGACCGGCUGAACGAGGUGGCCAAGAACCUGAACGAG AGCCUGAUCGACCUGCAAGAACUGGGGAAGUACGAGCAGUACAUCAAGUG GCCCUGGUACAUCUGGCUGGGCUUCAUCGCCGGACUGAUCGCCAUCGUGA UGGUCACAAUCAUGCUGUGCUGCAUGACCAGCUGCUGCAGCUGCCUGAAG

GGCUGCUGCAGCUGCGGCAGCUGCUGCAAGUUCGACGAGGACGACAGCGA

GCCCGUGCUGAAGGGCGUGAAACUGCACUACACAUGAGCUCGCUUUCUUG

CUGUCCAAUUUCUAUUAAAGGUUCCUUUGUUCCCUAAGUCCAACUACUAAA

CUGGGGGAUAUUAUGAAGGGCCUUGAGCAUCUGGAUUCUGCCUAAUAAAA

AACAUUUAUUUUCAUUGCAAAAAAAAAAAAAAAAAAAAAAAAAAAAAAAAAA

AAAAAAAAAAAAAAAAAAAAAAAAAAAAAAAAAAAAAAAAAAAAAAAAAAAA

AAAAAAAAAAAAAAAAAAAAA

<SEQ ID NO: 17> (S_RBD_opt2 EcoRI)

GCTAGCGTAATACGACTCACTATAAGGAGACCCAAGCTACATTTGCTTCTGA

CACAACTGTGTTCACTAGCAACCTCAAACAGACACCGCCACCATGTTCGTGT

TCCTGGTGCTGCTGCCCCTGGTGAGCAGCAGAGTGCAGCCCACCGAGAGCA

TCGTGCGGTTCCCCAACATCACCAACCTGTGCCCCTTCGGCGAGGTGTTCAA

CGCCACCAGATTCGCCAGCGTGTACGCCTGGAACCGGAAGCGGATCAGCAA

CTGCGTGGCCGACTACAGCGTGCTGTACAACAGCGCCAGCTTCAGCACCTT

CAAGTGCTACGGCGTGAGCCCCACCAAGCTGAACGACCTGTGCTTCACCAAC

GTGTACGCCGACAGCTTCGTGATCAGAGGCGACGAAGTGCGGCAGATCGCC

CCCGGACAGACAGGCAAGATCGCCGACTACAACTACAAGCTGCCCGACGACT

TCACCGGCTGCGTGATCGCCTGGAACAGCAACAACCTGGACAGCAAAGTCG

GCGGCAACTACAACTACCTGTACCGGCTGTTCCGGAAGTCCAACCTGAAGCC

CTTCGAGCGGGACATCAGCACCGAGATCTACCAGGCCGGCAGCACCCCCTG

CAACGGCGTGGAAGGCTTCAACTGCTACTTCCCACTGCAGAGCTACGGCTT

CCAGCCCACAAACGGCGTGGGCTACCAGCCCTACAGAGTGGTGGTGCTGAG

CTTCGAGCTGCTGCACGCCCCCGCCACAGTGTGCGGCCCCAAGAAAAGCAC

CAACCTGGTCAAGAACAAATGCGTGAACTTCTGAGCTCGCTTTCTTGCTGTC

CAATTTCTATTAAAGGTTCCTTTGTTCCCTAAGTCCAACTACTAAACTGGGG

GATATTATGAAGGGCCTTGAGCATCTGGATTCTGCCTAATAAAAAACATTTA

TTTTCATTGCGAATTC

NheI sequence: nucleotide numbers 1-6
T7 promoter sequence: nucleotide numbers 7-24
A: transcription start site: nucleotide number 25
5'-UTR sequence (including transcription start site and KOZAK sequence of nucleotide numbers 89-94): nucleotide numbers 25-94
RBD sequence: nucleotide numbers 95-805
3'-UTR sequence: nucleotide numbers 806-937
EcoRI sequence: nucleotide numbers 938-943

<SEQ ID NO: 18> (Template DNA for SARS-CoV-2 RBD optimized)

TGATGCTAGCGTAATACGACTCACTATAAGGAGACCCAAGCTACATTTGCTT

CTGACACAACTGTGTTCACTAGCAACCTCAAACAGACACCGCCACCATGTTC

GTGTTCCTGGTGCTGCTGCCCCTGGTGAGCAGCAGAGTGCAGCCCACCGAG

AGCATCGTGCGGTTCCCCAACATCACCAACCTGTGCCCCTTCGGCGAGGTG

TTCAACGCCACCAGATTCGCCAGCGTGTACGCCTGGAACCGGAAGCGGATC

AGCAACTGCGTGGCCGACTACAGCGTGCTGTACAACAGCGCCAGCTTCAGC

ACCTTCAAGTGCTACGGCGTGAGCCCCACCAAGCTGAACGACCTGTGCTTCA

CCAACGTGTACGCCGACAGCTTCGTGATCAGAGGCGACGAAGTGCGGCAGA

TCGCCCCCGGACAGACAGGCAAGATCGCCGACTACAACTACAAGCTGCCCGA

CGACTTCACCGGCTGCGTGATCGCCTGGAACAGCAACAACCTGGACAGCAAA

GTCGGCGGCAACTACAACTACCTGTACCGGCTGTTCCGGAAGTCCAACCTGA

AGCCCTTCGAGCGGGACATCAGCACCGAGATCTACCAGGCCGGCAGCACCC

CCTGCAACGGCGTGGAAGGCTTCAACTGCTACTTCCCACTGCAGAGCTACG

GCTTCCAGCCCACAAACGGCGTGGGCTACCAGCCCTACAGAGTGGTGGTGC

TGAGCTTCGAGCTGCTGCACGCCCCCGCCACAGTGTGCGGCCCCAAGAAAA

GCACCAACCTGGTCAAGAACAAATGCGTGAACTTCTGAGCTCGCTTTCTTGC

TGTCCAATTTCTATTAAAGGTTCCTTTGTTCCCTAAGTCCAACTACTAAACT

GGGGGATATTATGAAGGGCCTTGAGCATCTGGATTCTGCCTAATAAAAAACA

TTTATTTTCATTGCAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAAAAAA

AAAAAAAAAAAAAAAACGAAGAGCAAGCTTTGGC

NheI sequence: nucleotide numbers 5-10
T7 promoter sequence: nucleotide numbers 11-28
A: transcription start site: nucleotide number 29
5'-UTR sequence (including transcription start site and KOZAK sequence of nucleotide numbers 89-94): nucleotide numbers 29-98
Spike protein signal sequence: nucleotide numbers 99-137
RBD sequence: nucleotide numbers 138-809
3'-UTR sequence: nucleotide numbers 810-941
PolyA sequence (A110): nucleotide numbers 942-1051
BspQI sequence: nucleotide numbers 1053-1059
HindIII sequence: nucleotide numbers 1060-1065

<SEQ ID NO: 19> (SARS-CoV-2 RBD optimized mRNA-004)

AGGAGACCCAAGCUACAUUUGCUUCUGACACAACUGUGUUCACUAGCAACCUCAAACAGACACCGCCACCAUGUUCGUGUUCCUGGUGCUGCUGCCCCUGGUGAGCAGCAGAGUGCAGCCCACCGAGAGCAUCGUGCGGUUCCCCAACAUCACCAACCUGUGCCCCUUCGGCGAGGUGUUCAACGCCACCAGAUUCGCCAGCGUGUACGCCUGGAACCGGAAGCGGAUCAGCAACUGCGUGGCCGACUACAGCGUGCUGUACAACAGCGCCAGCUUCAGCACCUUCAAGUGCUACGGCGUGAGCCCCACCAAGCUGAACGACCUGUGCUUCACCAACGUGUACGCCGACAGCUUCGUGAUCAGAGGCGACGAAGUGCGGCAGAUCGCCCCCGGACAGACAGGCAAGAUCGCCGACUACAACUACAAGCUGCCCGACGACUUCACCGGCUGCGUGAUCGCCUGGAACAGCAACAACCUGGACAGCAAAGUCGGCGGCAACUACAACUACCUGUACCGGCUGUUCCGGAAGUCCAACCUGAAGCCCUUCGAGCGGGACAUCAGCACCGAGAUCUACCAGGCCGGCAGCACCCCCUGCAACGGCGUGGAAGGCUUCAACUGCUACUUCCCACUGCAGAGCUACGGCUUCCAGCCCACAAACGGCGUGGGCUACCAGCCCUACAGAGUGGUGGUGCUGAGCUUCGAGCUGCUGCACGCCCCCGCCACAGUGUGCGGCCCCAAGAAAGCACCAACCUGGUCAAGAACAAAUGCGUGAACUUCUGAGCUCGCUUUCUUGCUGUCCAAUUUCUA

UUAAAGGUUCCUUUGUUCCCUAAGUCCAACUACUAAACUGGGGGAUAUUA

UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAA

AAAAAAA

<SEQ ID NO: 20> Template DNA for SARS-CoV-2 RBD S2000

GCTAGCGTAATACGACTCACTATAAGGAGACCCAAGCTACATTTGCTTCTGA

CACAACTGTGTTCACTAGCAACCTCAAACAGACACCGCCACCATGTTCGTGT

TCCTGGTGCTGCTGCCCCTGGTGAGCAGCAGAGTGCAGCCCACCGAGAGCA

TCGTGCGGTTCCCCAACATCACCAACCTGTGCCCCTTCGGCGAGGTGTTCAA

CGCCACCAGATTCGCCAGCGTGTACGCCTGGAACCGGAAGCGGATCAGCAA

CTGCGTGGCCGACTACAGCGTGCTGTACAACAGCGCCAGCTTCAGCACCTT

CAAGTGCTACGGCGTGAGCCCCACCAAGCTGAACGACCTGTGCTTCACCAAC

GTGTACGCCGACAGCTTCGTGATCAGAGGCGACGAAGTGCGGCAGATCGCC

CCCGGACAGACAGGCAAGATCGCCGACTACAACTACAAGCTGCCCGACGACT

TCACCGGCTGCGTGATCGCCTGGAACAGCAACAACCTGGACAGCAAAGTCG

GCGGCAACTACAACTACCTGTACCGGCTGTTCCGGAAAAGCAACCTGAAGCC

CTTCGAGCGGGACATCAGCACCGAGATCTACCAGGCCGGCAGCACCCCCTG

CAACGGCGTGGAAGGCTTCAACTGCTACTTCCCACTGCAGAGCTACGGCTT

CCAGCCCACAAACGGCGTGGGCTACCAGCCCTACAGAGTGGTGGTGCTGAG

CTTCGAGCTGCTGCACGCCCCCGCCACAGTGTGCGGCCCCAAGAAAAGCAC

CAACCTGGTCAAGAACAAATGCGTGAACTTCTGAGCTCGCTTTCTTGCTGTC

CAATTTCTATTAAAGGTTCCTTTGTTCCCTAAGTCCAACTACTAAACTGGGG

GATATTATGAAGGGCCTTGAGCATCTGGATTCTGCCTAATAAAAAACATTTA

TTTTCATTGCAAAAAAAAAAAAAAAAAAAAAAAAAAAAAAAAAAAAAAAA

AAAAAACGAAGAGC

NheI sequence: nucleotide numbers 1-6
T7 promoter sequence: nucleotide numbers 7-24
A: transcription start site: nucleotide number 25
5'-UTR sequence (including transcription start site and KOZAK sequence of nucleotide numbers 89-94): nucleotide numbers 25-94
Spike protein signal sequence: nucleotide numbers 95-133
RBD sequence: nucleotide numbers 134-805
3'-UTR sequence: nucleotide numbers 806-937
PolyA sequence (A50): nucleotide numbers 938-987
BspQI sequence: nucleotide numbers 989-995

<SEQ ID NO: 21> mRNA sequence of SARS-CoV-2 RBD S2000

AGGAGACCCAAGCUACAUUUGCUUCUGACACAACUGUGUUCACUAGCAACC
UCAAACAGACACCGCCACCAUGUUCGUGUUCCUGGUGCUGCUGCCCCUGG
UGAGCAGCAGAGUGCAGCCCACCGAGAGCAUCGUGCGGUUCCCCAACAUCA
CCAACCUGUGCCCCUUCGGCGAGGUGUUCAACGCCACCAGAUUCGCCAGC
GUGUACGCCUGGAACCGGAAGCGGAUCAGCAACUGCGUGGCCGACUACAG
CGUGCUGUACAACAGCGCCAGCUUCAGCACCUUCAAGUGCUACGGCGUGA
GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCUGUACCGGCUGUUCCGGAAAAGCAACCUGAAGCCCUUCGAGCGGGA
CAUCAGCACCGAGAUCUACCAGGCCGGCAGCACCCCCUGCAACGGCGUGGA
AGGCUUCAACUGCUACUUCCCACUGCAGAGCUACGGCUUCCAGCCCACAAA
CGGCGUGGGCUACCAGCCCUACAGAGUGGUGGUGCUGAGCUUCGAGCUGC

UGCACGCCCCCGCCACAGUGUGCGGCCCCAAGAAAAGCACCAACCUGGUCA
AGAACAAAUGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC
AUUGCAAAAAAAAAAAAAAAAAAAAAAAAAAAAAAAAAAAAAAAAAAAAAAA
A

<SEQ ID NO: 22> Template DNA for SARS-CoV-2 RBD S2001

GCTAGCGTAATACGACTCACTATAAGGAGACCCAAGCTACATTTGCTTCTGA
CACAACTGTGTTCACTAGCAACCTCAAACAGACACCGCCACCATGTTCGTGT
TCCTGGTGCTGCTGCCCCTGGTGAGCAGCAGAGTGCAGCCCACCGAGAGCA
TCGTGCGGTTCCCCAACATCACCAACCTGTGCCCCTTCGGCGAGGTGTTCAA
CGCCACCAGATTCGCCAGCGTGTACGCCTGGAACCGGAAGCGGATCAGCAA
CTGCGTGGCCGACTACAGCGTGCTGTACAACAGCGCCAGCTTCAGCACCTT
CAAGTGCTACGGCGTGAGCCCCACCAAGCTGAACGACCTGTGCTTCACCAAC
GTGTACGCCGACAGCTTCGTGATCAGAGGCGACGAAGTGCGGCAGATCGCC
CCCGGACAGACAGGCAAGATCGCCGACTACAACTACAAGCTGCCCGACGACT
TCACCGGCTGCGTGATCGCCTGGAACAGCAACAACCTGGACAGCAAAGTCG

GCGGCAACTACAACTACCTGTACCGGCTGTTCCGGAAAAGCAACCTGAAGCC

CTTCGAGCGGGACATCAGCACCGAGATCTACCAGGCCGGCAGCACCCCCTG

CAACGGCGTGGAAGGCTTCAACTGCTACTTCCCACTGCAGAGCTACGGCTT

CCAGCCCACAAACGGCGTGGGCTACCAGCCCTACAGAGTGGTGGTGCTGAG

CTTCGAGCTGCTGCACGCCCCCGCCACAGTGTGCGGCCCCAAGAAAAGCAC

CAACCTGGTCAAGAACAAGAGCGTGAACTTCTGAGCTCGCTTTCTTGCTGTC

CAATTTCTATTAAAGGTTCCTTTGTTCCCTAAGTCCAACTACTAAACTGGGG

GATATTATGAAGGGCCTTGAGCATCTGGATTCTGCCTAATAAAAAACATTTA

TTTTCATTGCAAAAAAAAAAAAAAAAAAAAAAAAAAAAAAAAAAAAAAAAAAAA

AAAAAACGAAGAGC

NheI sequence: nucleotide numbers 1-6
T7 promoter sequence: nucleotide numbers 7-24
A: transcription start site: nucleotide number 25
5'-UTR sequence (including transcription start site and KOZAK sequence of nucleotide numbers 89-94): nucleotide numbers 25-94
Spike protein signal sequence: nucleotide numbers 95-133
RBD sequence: nucleotide numbers 134-805
3'-UTR sequence: nucleotide numbers 806-937
PolyA sequence (A50): nucleotide numbers 938-987
BspQI sequence: nucleotide numbers 989-995

<SEQ ID NO: 23> mRNA sequence of SARS-CoV-2 RBD S2001

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCAGAGUGCAGCCCACCGAGAGCAUCGUGCGGUUCCCCAACAUCA

CCAACCUGUGCCCCUUCGGCGAGGUGUUCAACGCCACCAGAUUCGCCAGC

GUGUACGCCUGGAACCGGAAGCGGAUCAGCAACUGCGUGGCCGACUACAG

CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU

UCGUGAUCAGAGGCGACGAAGUGCGGCAGAUCGCCCCCGGACAGACAGGC

AAGAUCGCCGACUACAACUACAAGCUGCCCGACGACUUCACCGGCUGCGUG

AUCGCCUGGAACAGCAACAACCUGGACAGCAAAGUCGGCGGCAACUACAAC

UACCUGUACCGGCUGUUCCGGAAAAGCAACCUGAAGCCCUUCGAGCGGGA

CAUCAGCACCGAGAUCUACCAGGCCGGCAGCACCCCCUGCAACGGCGUGGA

AGGCUUCAACUGCUACUUCCCACUGCAGAGCUACGGCUUCCAGCCCACAAA

CGGCGUGGGCUACCAGCCCUACAGAGUGGUGGUGCUGAGCUUCGAGCUGC

UGCACGCCCCCGCCACAGUGUGCGGCCCCAAGAAAAGCACCAACCUGGUCA

AGAACAAGAGCGUGAACUUCUGAGCUCGCUUUCUUGCUGUCCAAUUUCUA

UUAAAGGUUCCUUUGUUCCCUAAGUCCAACUACUAAACUGGGGGAUAUUA

UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAAA

A

<SEQ ID NO: 24> Amino acid sequence of SARS-CoV-2 RBD S2001 (including S protein signal sequence)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYLYRLFRKSNLKPFERDISTEIY

QAGSTPCNGVEGFNCYFPLQSYGFQPTNGVGYQPYRVVVLSFELLHAPATVCGP

KKSTNLVKNKSVNF

S protein signal sequence: amino acid numbers 1-13
RBD sequence: amino acid numbers 14-236

<SEQ ID NO: 25> Amino acid sequence of SARS-CoV-2 RBD S2001 (not including S protein signal sequence)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYLYRLFRKSNLKPFERDISTEIYQAGSTPCNGVEG

FNCYFPLQSYGFQPTNGVGYQPYRVVVLSFELLHAPATVCGPKKSTNLVKNKSV

NF

<SEQ ID NO: 26> Template DNA for SARS-CoV-2 RBD S2002

GCTAGCGTAATACGACTCACTATAAGGAGACCCAAGCTACATTTGCTTCTGA
CACAACTGTGTTCACTAGCAACCTCAAACAGACACCGCCACCATGTTCGTGT
TCCTGGTGCTGCTGCCCCTGGTGAGCAGCTTCCCCAACATCACCAACCTGTG
CCCCTTCGGCGAGGTGTTCAACGCCACAAGATTCGCCAGCGTGTACGCCTG
GAACCGGAAGCGGATCAGCAACTGCGTGGCCGACTACAGCGTGCTGTACAA
CAGCGCCAGCTTCAGCACCTTCAAGTGCTACGGCGTGAGCCCCACCAAGCT
GAACGACCTGTGCTTCACCAACGTGTACGCCGACAGCTTCGTGATCAGAGG
CGACGAAGTGCGGCAGATCGCCCCCGGACAGACAGGCAAGATCGCCGACTA
CAACTACAAGCTGCCCGACGACTTCACCGGCTGCGTGATCGCCTGGAACAG
CAACAACCTGGACAGCAAAGTCGGCGGCAACTACAACTACCTGTACCGGCTG
TTCCGGAAAAGCAACCTGAAGCCCTTCGAGCGGGACATCAGCACCGAGATCT
ACCAGGCCGGCAGCACCCCCTGCAACGGCGTGGAAGGCTTCAACTGCTACT
TCCCACTGCAGAGCTACGGCTTCCAGCCCACAAACGGCGTGGGCTACCAGC
CCTACAGAGTGGTGGTGCTGAGCTTCGAGCTGCTGCACGCCCCCGCCACAG
TGTGCGGACCCAAATGAGCTCGCTTTCTTGCTGTCCAATTTCTATTAAAGGT
TCCTTTGTTCCCTAAGTCCAACTACTAAACTGGGGGATATTATGAAGGGCCT
TGAGCATCTGGATTCTGCCTAATAAAAAACATTTATTTTCATTGCAAAAAAAA
AAAAAAAAAAAAAAAAAAAAAAAAAAAAAAAAAAAAAACGAAGAGC

NheI sequence: nucleotide numbers1-6
T7promoter sequence: nucleotide numbers 7-24

A: transcription start site: nucleotide number 25
5'-UTR sequence (including transcription start site and KOZAK sequence of nucleotide numbers 89-94): nucleotide numbers 25-94
Spike protein signal sequence: nucleotide numbers 95-133
RBD sequence: nucleotide numbers 134-736
3'-UTR sequence: nucleotide numbers 737-868
PolyA sequence (A50): nucleotide numbers 869-918
BspQI sequence: nucleotide numbers 920-926

<SEQ ID NO: 27> mRNA sequence of SARS-CoV-2 RBD S2002

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCUUCCCCAACAUCACCAACCUGUGCCCCUUCGGCGAGGUGUUCA

ACGCCACAAGAUUCGCCAGCGUGUACGCCUGGAACCGGAAGCGGAUCAGCA

ACUGCGUGGCCGACUACAGCGUGCUGUACAACAGCGCCAGCUUCAGCACCU

UCAAGUGCUACGGCGUGAGCCCCACCAAGCUGAACGACCUGUGCUUCACCA

ACGUGUACGCCGACAGCUUCGUGAUCAGAGGCGACGAAGUGCGGCAGAUC

GCCCCCGGACAGACAGGCAAGAUCGCCGACUACAACUACAAGCUGCCCGAC

GACUUCACCGGCUGCGUGAUCGCCUGGAACAGCAACAACCUGGACAGCAAA

GUCGGCGGCAACUACAACUACCUGUACCGGCUGUUCCGGAAAAGCAACCUG

AAGCCCUUCGAGCGGGACAUCAGCACCGAGAUCUACCAGGCCGGCAGCACC

CCCUGCAACGGCGUGGAAGGCUUCAACUGCUACUUCCCACUGCAGAGCUAC

GGCUUCCAGCCCACAAACGGCGUGGGCUACCAGCCCUACAGAGUGGUGGU

GCUGAGCUUCGAGCUGCUGCACGCCCCCGCCACAGUGUGCGGACCCAAAU

GAGCUCGCUUUCUUGCUGUCCAAUUUCUAUUAAAGGUUCCUUUGUUCCCU

AAGUCCAACUACUAAACUGGGGGAUAUUAUGAAGGGCCUUGAGCAUCUGG

AUUCUGCCUAAUAAAAAACAUUUAUUUUCAUUGCAAAAAAAAAAAAAAAAA

AAAAAAAAAAAAAAAAAAAAAAAAAAAAAAAAA

<SEQ ID NO: 28> Amino acid sequence of SARS-CoV-2 RBD S2002 (including S protein signal sequence)

MFVFLVLLPLVSSFPNITNLCPFGEVFNATRFASVYAWNRKRISNCVADYSVLYNS

ASFSTFKCYGVSPTKLNDLCFTNVYADSFVIRGDEVRQIAPGQTGKIADYNYKLP

DDFTGCVIAWNSNNLDSKVGGNYNYLYRLFRKSNLKPFERDISTEIYQAGSTPCN

GVEGFNCYFPLQSYGFQPTNGVGYQPYRVVVLSFELLHAPATVCGPK

S protein signal sequence: amino acid numbers 1-13
RBD sequence: amino acid numbers 14-213

<SEQ ID NO: 29> Amino acid sequence of SARS-CoV-2 RBD S2002 (not including S protein signal sequence)

FPNITNLCPFGEVFNATRFASVYAWNRKRISNCVADYSVLYNSASFSTFKCYGVSP

TKLNDLCFTNVYADSFVIRGDEVRQIAPGQTGKIADYNYKLPDDFTGCVIAWNS

NNLDSKVGGNYNYLYRLFRKSNLKPFERDISTEIYQAGSTPCNGVEGFNCYFPLQS

YGFQPTNGVGYQPYRVVVLSFELLHAPATVCGPK

<SEQ ID NO: 30> Template DNA for SARS-CoV-2 RBD S2003

GCTAGCGTAATACGACTCACTATAAGGAGACCCAAGCTACATTTGCTTCTGA
CACAACTGTGTTCACTAGCAACCTCAAACAGACACCGCCACCATGTTCGTGT
TCCTGGTGCTGCTGCCCCTGGTGAGCAGCGAGAAGGGCATCTACCAGACCA
GCAACTTCAGAGTGCAGCCCACCGAGAGCATCGTGCGGTTCCCCAACATCAC
CAACCTGTGCCCCTTCGGCGAGGTGTTCAACGCCACCAGATTCGCCAGCGT
GTACGCCTGGAACCGGAAGCGGATCAGCAACTGCGTGGCCGACTACAGCGT
GCTGTACAACAGCGCCAGCTTCAGCACCTTCAAGTGCTACGGCGTGAGCCC
CACCAAGCTGAACGACCTGTGCTTCACCAACGTGTACGCCGACAGCTTCGTG
ATCAGAGGCGACGAAGTGCGGCAGATCGCCCCCGGACAGACAGGCAAGATC
GCCGACTACAACTACAAGCTGCCCGACGACTTCACCGGCTGCGTGATCGCCT
GGAACAGCAACAACCTGGACAGCAAAGTCGGCGGCAACTACAACTACCTGTA
CCGGCTGTTCCGGAAAAGCAACCTGAAGCCCTTCGAGCGGGACATCAGCAC

CGAGATCTACCAGGCCGGCAGCACCCCCTGCAACGGCGTGGAAGGCTTCAA
CTGCTACTTCCCACTGCAGAGCTACGGCTTCCAGCCCACAAACGGCGTGGG
CTACCAGCCCTACAGAGTGGTGGTGCTGAGCTTCGAGCTGCTGCACGCCCC
CGCCACAGTGTGCGGCCCCAAGAAAAGCACCAACCTGGTCAAGAACAAGAGC
GTGAACTTCAACTTCAACGGCCTGACCGGCACCGGCGTGTTGACAGAATGA
GCTCGCTTTCTTGCTGTCCAATTTCTATTAAAGGTTCCTTTGTTCCCTAAGT
CCAACTACTAAACTGGGGGATATTATGAAGGGCCTTGAGCATCTGGATTCT
GCCTAATAAAAAACATTTATTTTCATTGCAAAAAAAAAAAAAAAAAAAAAAAA
AAAAAAAAAAAAAAAAAAAAAAAAAACGAAGAGC

NheI sequence: nucleotide numbers 1-6
T7 promoter sequence: nucleotide numbers 7-24
A: transcription start site: nucleotide number 25
5'-UTR sequence (including transcription start site and KOZAK sequence of nucleotide numbers 89-94): nucleotide numbers 25-94
Spike protein signal sequence: nucleotide numbers 95-133
RBD sequence: nucleotide numbers 134-874
3'-UTR sequence: nucleotide numbers 875-1006
PolyA sequence (A50): nucleotide numbers 1007-1056
BspQI sequence: nucleotide numbers 1058-1064

<SEQ ID NO: 31> mRNA sequence of SARS-CoV-2 RBD S2003

AGGAGACCCAAGCUACAUUUGCUUCUGACACAACUGUGUUCACUAGCAACC UCAAACAGACACCGCCACCAUGUUCGUGUUCCUGGUGCUGCUGCCCCUGG UGAGCAGCGAGAAGGGCAUCUACCAGACCAGCAACUUCAGAGUGCAGCCCA CCGAGAGCAUCGUGCGGUUCCCCAACAUCACCAACCUGUGCCCCUUCGGCG AGGUGUUCAACGCCACCAGAUUCGCCAGCGUGUACGCCUGGAACCGGAAG CGGAUCAGCAACUGCGUGGCCGACUACAGCGUGCUGUACAACAGCGCCAGC UUCAGCACCUUCAAGUGCUACGGCGUGAGCCCCACCAAGCUGAACGACCUG UGCUUCACCAACGUGUACGCCGACAGCUUCGUGAUCAGAGGCGACGAAGU GCGGCAGAUCGCCCCCGGACAGACAGGCAAGAUCGCCGACUACAACUACAA GCUGCCCGACGACUUCACCGGCUGCGUGAUCGCCUGGAACAGCAACAACCU GGACAGCAAAGUCGGCGGCAACUACAACUACCUGUACCGGCUGUUCCGGAA AAGCAACCUGAAGCCCUUCGAGCGGGACAUCAGCACCGAGAUCUACCAGGC CGGCAGCACCCCCUGCAACGGCGUGGAAGGCUUCAACUGCUACUUCCCACU GCAGAGCUACGGCUUCCAGCCCACAAACGGCGUGGGCUACCAGCCCUACAG

AGUGGUGGUGCUGAGCUUCGAGCUGCUGCACGCCCCCGCCACAGUGUGCG
GCCCCAAGAAAAGCACCAACCUGGUCAAGAACAAGAGCGUGAACUUCAACU
UCAACGGCCUGACCGGCACCGGCGUGUUGACAGAAUGAGCUCGCUUUCUU
GCUGUCCAAUUUCUAUUAAAGGUUCCUUUGUUCCCUAAGUCCAACUACUA
AACUGGGGGAUAUUAUGAAGGGCCUUGAGCAUCUGGAUUCUGCCUAAUAA
AAAACAUUUAUUUUCAUUGCAAAAAAAAAAAAAAAAAAAAAAAAAAAAAAAA
AAAAAAAAAAAAAAAAAA

<SEQ ID NO: 32> Amino acid sequence of SARS-CoV-2 RBD S2003 (including S protein signal sequence)

MFVFLVLLPLVSSEKGIYQTSNFRVQPTESIVRFPNITNLCPFGEVFNATRFASVYA
WNRKRISNCVADYSVLYNSASFSTFKCYGVSPTKLNDLCFTNVYADSFVIRGDEVR
QIAPGQTGKIADYNYKLPDDFTGCVIAWNSNNLDSKVGGNYNYLYRLFRKSNLK
PFERDISTEIYQAGSTPCNGVEGFNCYFPLQSYGFQPTNGVGYQPYRVVVLSFELL
HAPATVCGPKKSTNLVKNKSVNFNFNGLTGTGVLTE

S protein signal sequence: amino acid numbers 1-13
RBD sequence: amino acid numbers 14-259

<SEQ ID NO: 33> Amino acid sequence of SARS-CoV-2 RBD S2003 (not including S protein signal sequence)

EKGIYQTSNFRVQPTESIVRFPNITNLCPFGEVFNATRFASVYAWNRKRISNCVAD
YSVLYNSASFSTFKCYGVSPTKLNDLCFTNVYADSFVIRGDEVRQIAPGQTGKIAD
YNYKLPDDFTGCVIAWNSNNLDSKVGGNYNYLYRLFRKSNLKPFERDISTEIYQA
GSTPCNGVEGFNCYFPLQSYGFQPTNGVGYQPYRVVVLSFELLHAPATVCGPKK
STNLVKNKSVNFNFNGLTGTGVLTE

<SEQ ID NO: 34> Template DNA for SARS-CoV-2 RBD S2004

GCTAGCGTAATACGACTCACTATAAGGAGACCCAAGCTACATTTGCTTCTGA

CACAACTGTGTTCACTAGCAACCTCAAACAGACACCGCCACCATGTTCGTGT

TCCTGGTGCTGCTGCCCCTGGTGAGCAGCAGAGTGCAGCCCACCGAGAGCA

TCGTGCGGTTCCCCAACATCACCAACCTGTGCCCCTTCGGCGAGGTGTTCAA

CGCCACCAGATTCGCCAGCGTGTACGCCTGGAACCGGAAGCGGATCAACAA

CTGCGTGGCCGACTACAGCGTGCTGTACAACAGCACCAGCTTCAGCACCTTC

AAGTGCTACGGCGTGAGCCCCACCAAGCTGAACGACCTGTGCTTCACCAACG

TGTACGCCGACAGCTTCGTGATCAGAGGCGACGAAGTGCGGCAGATCGCCC
CCGGACAGACAGGCAAGATCGCCGACTACAACTACAAGCTGCCCGACGACTT
CACCGGCTGCGTGATCGCCTGGAACAGCAACACCCTGGACAGCAAAGTCGG
CGGCAACTACACCTACCTGTACCGGCTGTTCAGAAAGAGCAACCTGAAGCCC
TTCGAGCGGGACATCAGCACCGAGATCTACCAGGCCGGCAGCACCCCCTGC
AACGGCGTGGAAGGCTTCAACTGCTACTTCCCACTGCAGAGCTACGGCTTC
CAGCCCACAAACGGCGTGGGCTACCAGCCCTACAGAGTGGTGGTGCTGAGC
TTCGAGCTGCTGCACGCCCCCGCCACAGTGTGCGGCCCCAAGAAAGCACC
AACCTGGTCAAGAACAAGAGCGTGAACTTCTGAGCTCGCTTTCTTGCTGTCC
AATTTCTATTAAAGGTTCCTTTGTTCCCTAAGTCCAACTACTAAACTGGGGG
ATATTATGAAGGGCCTTGAGCATCTGGATTCTGCCTAATAAAAAACATTTAT
TTTCATTGCAAAAAAAAAAAAAAAAAAAAAAAAAAAAAAAAAAAAAAAAAAA
AAAAACGAAGAGC

NheI sequence: nucleotide numbers 1-6
T7 promoter sequence: nucleotide numbers 7-24
A: transcription start site: nucleotide number 25
5'-UTR sequence (including transcription start site ad KOZAK sequence of nucleotide numbers 89-94): nucleotide numbers 25-94
Spike protein signal sequence: nucleotide numbers 95-133
RBD sequence: nucleotide numbers 134-805
3'-UTR sequence: nucleotide numbers 806-937
PolyA sequence (A50): nucleotide numbers 938-987
BspQI sequence: nucleotide numbers 989-995

<SEQ ID NO: 35> mRNA sequence of SARS-CoV-2 RBD S2004

AGGAGACCCAAGCUACAUUUGCUUCUGACACAACUGUGUUCACUAGCAACC
UCAAACAGACACCGCCACCAUGUUCGUGUUCCUGGUGCUGCUGCCCCUGG
UGAGCAGCAGAGUGCAGCCCACCGAGAGCAUCGUGCGGUUCCCCAACAUCA
CCAACCUGUGCCCCUUCGGCGAGGUGUUCAACGCCACCAGAUUCGCCAGC
GUGUACGCCUGGAACCGGAAGCGGAUCAACAACUGCGUGGCCGACUACAG
CGUGCUGUACAACAGCACCAGCUUCAGCACCUUCAAGUGCUACGGCGUGA
GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAACACCCUGGACAGCAAAGUCGGCGGCAACUACACC
UACCUGUACCGGCUGUUCAGAAAGAGCAACCUGAAGCCCUUCGAGCGGGAC
AUCAGCACCGAGAUCUACCAGGCCGGCAGCACCCCCUGCAACGGCGUGGAA
GGCUUCAACUGCUACUUCCCACUGCAGAGCUACGGCUUCCAGCCCACAAAC
GGCGUGGGCUACCAGCCCUACAGAGUGGUGGUGCUGAGCUUCGAGCUGCU
GCACGCCCCCGCCACAGUGUGCGGCCCCAAGAAAGCACCAACCUGGUCAA
GAACAAGAGCGUGAACUUCUGAGCUCGCUUUCUUGCUGUCCAAUUUCUAU
UAAAGGUUCCUUUGUUCCCUAAGUCCAACUACUAAACUGGGGGAUAUUAU
GAAGGGCCUUGAGCAUCUGGAUUCUGCCUAAUAAAAAACAUUUAUUUUCA
UUGCAAAAAAAAAAAAAAAAAAAAAAAAAAAAAAAAAAAAAAAAAAAAAAA

<SEQ ID NO: 36> Amino acid sequence of SARS-CoV-2 RBD S2004 (including S protein signal sequence)

MFVFLVLLPLVSSRVQPTESIVRFPNITNLCPFGEVFNATRFASVYAWNRKRINNC
VADYSVLYNSTSFSTFKCYGVSPTKLNDLCFTNVYADSFVIRGDEVRQIAPGQTG
KIADYNYKLPDDFTGCVIAWNSNTLDSKVGGNYTYLYRLFRKSNLKPFERDISTEI
YQAGSTPCNGVEGFNCYFPLQSYGFQPTNGVGYQPYRVVVLSFELLHAPATVCG
PKKSTNLVKNKSVNF

S protein signal sequence: amino acid numbers 1-13
RBD sequence: amino acid numbers 14-236

<SEQ ID NO: 37> Amino acid sequence of SARS-CoV-2 RBD S2004 (not including S protein signal sequence)

RVQPTESIVRFPNITNLCPFGEVFNATRFASVYAWNRKRINNCVADYSVLYNSTSF
STFKCYGVSPTKLNDLCFTNVYADSFVIRGDEVRQIAPGQTGKIADYNYKLPDDF
TGCVIAWNSNTLDSKVGGNYTYLYRLFRKSNLKPFERDISTEIYQAGSTPCNGVE
GFNCYFPLQSYGFQPTNGVGYQPYRVVVLSFELLHAPATVCGPKKSTNLVKNKS
VNF

<SEQ ID NO: 38> DNA fragment comprising variant SARS-CoV-2 RBD (South African variant) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAACATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGCACCCCCTGCAACGGC
GTGAAAGGCTTCAACTGCTACTTCCCACTGCAGAGCTACGGCTTCCAGCCCA
CATACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAATGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 39> DNA fragment comprising variant SARS-CoV-2 RBD) (UK variant) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGCACCCCCTGCAACGGC
GTGGAAGGCTTCAACTGCTACTTCCCACTGCAGAGCTACGGCTTCCAGCCCA
CATACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAATGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 40> DNA fragment comprising variant SARS-CoV-2 RBD (Brazilian variant) (Mutated codons are

indicated with underline; and sites of mutation with bold letters.)

GTAATACGACTCACTATAAGGAGACCCAAGCTACATTTGCTTCTGACACAACTGTGTTCACTAGCAACCTCAAACAGACACCGCCACCATGTTCGTGTTCCTGGTGCTGCTGCCCCTGGTGAGCAGCAGAGTGCAGCCCACCGAGAGCATCGTGCGGTTCCCCAACATCACCAACCTGTGCCCCTTCGGCGAGGTGTTCAACGCCACCAGATTCGCCAGCGTGTACGCCTGGAACCGGAAGCGGATCAGCAACTGCGTGGCCGACTACAGCGTGCTGTACAACAGCGCCAGCTTCAGCACCTTCAAGTGCTACGGCGTGAGCCCCACCAAGCTGAACGACCTGTGCTTCACCAACGTGTACGCCGACAGCTTCGTGATCAGAGGCGACGAAGTGCGGCAGATCGCCCCCGGACAGACAGGCACGATCGCCGACTACAACTACAAGCTGCCCGACGACTTCACCGGCTGCGTGATCGCCTGGAACAGCAACAACCTGGACAGCAAAGTCGGCGGCAACTACAACTACCTGTACCGGCTGTTCCGGAAGTCCAACCTGAAGCCCTTCGAGCGGGACATCAGCACCGAGATCTACCAGGCCGGCAGCACCCCCTGCAACGGCGTGAAAGGCTTCAACTGCTACTTCCCACTGCAGAGCTACGGCTTCCAGCCCACATACGGCGTGGGCTACCAGCCCTACAGAGTGGTGGTGCTGAGCTTCGAGCTGCTGCACGCCCCCGCCACAGTGTGCGGCCCCAAGAAAAGCACCAACCTGGTCAAGAACAAATGCGTGAACTTCTGAGCTCGCTTTCTTGCTGTCCAATTTCTATTAAAGGTTCCTTTGTTCCCTAAGTCCAACTACTAAACTGGGGGATATTATGAAGGGCCTTGAGCATCTGGATTCTGCCTAATAAAAAACATTTATTTTCATTGC

<SEQ ID NO: 41> DNA fragment comprising variant SARS-CoV-2 RBD (Californian variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCGGTACCGGCTGTTCCGGAAGTCCAACCTGAAGCCCTTCGAG
CGGGACATCAGCACCGAGATCTACCAGGCCGGCAGCACCCCCTGCAACGGC
GTGGAAGGCTTCAACTGCTACTTCCCACTGCAGAGCTACGGCTTCCAGCCCA
CAAACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAATGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 42> DNA fragment comprising variant SARS-CoV-2 RBD (Indian variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCAAGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCGGTACCGGCTGTTCCGGAAGTCCAACCTGAAGCCCTTCGAG

CGGGACATCAGCACCGAGATCTACCAGGCCGGCAGCACCCCCTGCAACGGC

GTGCAAGGCTTCAACTGCTACTTCCCACTGCAGAGCTACGGCTTCCAGCCCA

CAAACGGCGTGGGCTACCAGCCCTACAGAGTGGTGGTGCTGAGCTTCGAGC

TGCTGCACGCCCCCGCCACAGTGTGCGGCCCCAAGAAAAGCACCAACCTGG

TCAAGAACAAATGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GC

<SEQ ID NO: 43> DNA fragment comprising variant SARS-CoV-2 RBD (South African C538S variant) (Mutated

codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAACATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGCACCCCCTGCAACGGC
GTGAAAGGCTTCAACTGCTACTTCCCACTGCAGAGCTACGGCTTCCAGCCCA
CATACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAAAGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 44> DNA fragment comprising variant SARS-CoV-2 RBD (UK C538S variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCAAGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCTGTACCGGCTGTTCCGGAAGTCCAACCTGAAGCCCTTCGAG

CGGGACATCAGCACCGAGATCTACCAGGCCGGCAGCACCCCCTGCAACGGC

GTGGAAGGCTTCAACTGCTACTTCCCACTGCAGAGCTACGGCTTCCAGCCCA

CATACGGCGTGGGCTACCAGCCCTACAGAGTGGTGGTGCTGAGCTTCGAGC

TGCTGCACGCCCCCGCCACAGTGTGCGGCCCCAAGAAAGCACCAACCTGG

TCAAGAACAAAAGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GC

<SEQ ID NO: 45> DNA fragment comprising variant SARS-CoV-2 RBD (Brazilian C538S variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCACGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGCACCCCCTGCAACGGC
GTGAAAGGCTTCAACTGCTACTTCCCACTGCAGAGCTACGGCTTCCAGCCCA
CATACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAAAGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 46> DNA fragment comprising variant SARS-CoV-2 RBD (Californian C538S variant) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCGGTACCGGCTGTTCCGGAAGTCCAACCTGAAGCCCTTCGAG
CGGGACATCAGCACCGAGATCTACCAGGCCGGCAGCACCCCCTGCAACGGC
GTGGAAGGCTTCAACTGCTACTTCCCACTGCAGAGCTACGGCTTCCAGCCCA
CAAACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAAAGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 47> DNA fragment comprising variant SARS-CoV-2 RBD (Indian C538S variant) (Mutated codons

are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCGGTACCGGCTGTTCCGGAAGTCCAACCTGAAGCCCTTCGAG
CGGGACATCAGCACCGAGATCTACCAGGCCGGCAGCACCCCCTGCAACGGC
GTGCAAGGCTTCAACTGCTACTTCCCACTGCAGAGCTACGGCTTCCAGCCCA
CAAACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAAAGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 48> DNA fragment comprising variant SARS-CoV-2 RBD (Combination variant (1)) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAAGAACCTGGACAGCAAAGTCGGCGGCAA
CTACAACTACCGGTACCGGCTGTTCCGGAAGTCCAACCTGAAGCCCTTCGAG
CGGGACATCAGCACCGAGATCTACCAGGTCGGCAGCACCCCCTGCAACGGC
GCGGAAGGCTTCAACTGCTACTTCCCACTGCAGAGCTACGGCTTCCAGCCCA
CAAACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAATGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 49> DNA fragment comprising variant SARS-CoV-2 RBD (Combination variant (2)) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGAACCCCCTGCAACGGC
GTGGAAGGCTTCAACTGCTACCTCCCACTGCAGAGCTACGGCTTCCAGCCCA
CAAACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAATGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT
GC

<SEQ ID NO: 50> DNA fragment comprising variant SARS-CoV-2 RBD (Combination variant (3)) (Mutated codons

are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAACATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGAACCCCCTGCAACGGC

GTGAAAGGCTTCAACTGCTACTTCCCACTGCAGAGCTACGGCTTCCAGCCCA

CATACGGCGTGGGCTACCAGCCCTACAGAGTGGTGGTGCTGAGCTTCGAGC

TGCTGCACGCCCCCGCCACAGTGTGCGGCCCCAAGAAAAGCACCAACCTGG

TCAAGAACAAATGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GC

<SEQ ID NO: 51> DNA fragment comprising variant SARS-CoV-2 RBD (Combination variant (4)) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGCACCCCCTGCAACGGC
GTGGAAGGCAGCAACTGCTACCTCCCACTGCAGAGCTACGGCTTCCAGCCC
ACAAACGGCGTGGGCTACCAGCCCTACAGAGTGGTGGTGCTGAGCTTCGAG
CTGCTGCACGCCCCCGCCACAGTGTGCGGCCCCAAGAAAGCACCAACCTG
GTCAAGAACAAATGCGTGAACTTCTGAGCTCGCTTTCTTGCTGTCCAATTTC
TATTAAAGGTTCCTTTGTTCCCTAAGTCCAACTACTAAACTGGGGGATATTA
TGAAGGGCCTTGAGCATCTGGATTCTGCCTAATAAAAAACATTTATTTTCAT
TGC

<SEQ ID NO: 52> Template DNA for variant SARS-CoV-2 RBD (South African variant) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAACATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGCACCCCCTGCAACGGC
GTGAAAGGCTTCAACTGCTACTTCCCACTGCAGAGCTACGGCTTCCAGCCCA
CATACGGCGTGGGCTACCAGCCCTACAGAGTGGTGGTGCTGAGCTTCGAGC
TGCTGCACGCCCCCGCCACAGTGTGCGGCCCCAAGAAAAGCACCAACCTGG
TCAAGAACAAATGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 53> Template DNA for variant SARS-CoV-2 RBD (UK variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCAAGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCTGTACCGGCTGTTCCGGAAGTCCAACCTGAAGCCCTTCGAG

CGGGACATCAGCACCGAGATCTACCAGGCCGGCAGCACCCCCTGCAACGGC

GTGGAAGGCTTCAACTGCTACTTCCCACTGCAGAGCTACGGCTTCCAGCCCA

CATACGGCGTGGGCTACCAGCCCTACAGAGTGGTGGTGCTGAGCTTCGAGC

TGCTGCACGCCCCCGCCACAGTGTGCGGCCCCAAGAAAGCACCAACCTGG

TCAAGAACAAATGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931

PolyA sequence (A100): nucleotide numbers 932-1031

<SEQ ID NO: 54> Template DNA for variant SARS-CoV-2 RBD (Brazilian variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCACGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCTGTACCGGCTGTTCCGGAAGTCCAACCTGAAGCCCTTCGAG

CGGGACATCAGCACCGAGATCTACCAGGCCGGCAGCACCCCCTGCAACGGC

GTGAAAGGCTTCAACTGCTACTTCCCACTGCAGAGCTACGGCTTCCAGCCCA

CATACGGCGTGGGCTACCAGCCCTACAGAGTGGTGGTGCTGAGCTTCGAGC

TGCTGCACGCCCCCGCCACAGTGTGCGGCCCCAAGAAAGCACCAACCTGG

TCAAGAACAAATGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAACATTTATTTTCATT

GCAAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 55> Template DNA for variant SARS-CoV-2 RBD (Californian variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCAAGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCGGTACCGGCTGTTCCGGAAGTCCAACCTGAAGCCCTTCGAG

CGGGACATCAGCACCGAGATCTACCAGGCCGGCAGCACCCCCTGCAACGGC

GTGGAAGGCTTCAACTGCTACTTCCCACTGCAGAGCTACGGCTTCCAGCCCA

CAAACGGCGTGGGCTACCAGCCCTACAGAGTGGTGGTGCTGAGCTTCGAGC

TGCTGCACGCCCCCGCCACAGTGTGCGGCCCCAAGAAAAGCACCAACCTGG

TCAAGAACAAATGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 56> Template DNA for variant SARS-CoV-2 RBD (Indian variant) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCGGTACCGGCTGTTCCGGAAGTCCAACCTGAAGCCCTTCGAG
CGGGACATCAGCACCGAGATCTACCAGGCCGGCAGCACCCCCTGCAACGGC
GTGCAAGGCTTCAACTGCTACTTCCCACTGCAGAGCTACGGCTTCCAGCCCA
CAAACGGCGTGGGCTACCAGCCCTACAGAGTGGTGGTGCTGAGCTTCGAGC
TGCTGCACGCCCCCGCCACAGTGTGCGGCCCCAAGAAAGCACCAACCTGG

TCAAGAACAAATGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 57> Template DNA for variant SARS-CoV-2 RBD (South African C538S variant) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAACATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGCACCCCCTGCAACGGC
GTGAAAGGCTTCAACTGCTACTTCCCACTGCAGAGCTACGGCTTCCAGCCCA
CATACGGCGTGGGCTACCAGCCCTACAGAGTGGTGGTGCTGAGCTTCGAGC
TGCTGCACGCCCCCGCCACAGTGTGCGGCCCCAAGAAAGCACCAACCTGG
TCAAGAACAAAAGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 58> Template DNA for variant SARS-CoV-2 RBD (UK C538S variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCAAGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCTGTACCGGCTGTTCCGGAAGTCCAACCTGAAGCCCTTCGAG

CGGGACATCAGCACCGAGATCTACCAGGCCGGCAGCACCCCCTGCAACGGC

GTGGAAGGCTTCAACTGCTACTTCCCACTGCAGAGCTACGGCTTCCAGCCCA

CATACGGCGTGGGCTACCAGCCCTACAGAGTGGTGGTGCTGAGCTTCGAGC

TGCTGCACGCCCCCGCCACAGTGTGCGGCCCCAAGAAAAGCACCAACCTGG

TCAAGAACAAAAGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931

PolyA sequence (A100): nucleotide numbers 932-1031

<SEQ ID NO: 59> Template DNA for variant SARS-CoV-2 RBD (Brazilian C538S variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCACGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCTGTACCGGCTGTTCCGGAAGTCCAACCTGAAGCCCTTCGAG

CGGGACATCAGCACCGAGATCTACCAGGCCGGCAGCACCCCCTGCAACGGC

GTGAAAGGCTTCAACTGCTACTTCCCACTGCAGAGCTACGGCTTCCAGCCCA

CATACGGCGTGGGCTACCAGCCCTACAGAGTGGTGGTGCTGAGCTTCGAGC

TGCTGCACGCCCCCGCCACAGTGTGCGGCCCCAAGAAAGCACCAACCTGG

TCAAGAACAAAAGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAACATTTATTTTCATT

GCAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 60> Template DNA for variant SARS-CoV-2 RBD (Californian C538S variant) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCAAGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCGGTACCGGCTGTTCCGGAAGTCCAACCTGAAGCCCTTCGAG

CGGGACATCAGCACCGAGATCTACCAGGCCGGCAGCACCCCCTGCAACGGC

GTGGAAGGCTTCAACTGCTACTTCCCACTGCAGAGCTACGGCTTCCAGCCCA

CAAACGGCGTGGGCTACCAGCCCTACAGAGTGGTGGTGCTGAGCTTCGAGC

TGCTGCACGCCCCCGCCACAGTGTGCGGCCCCAAGAAAGCACCAACCTGG

TCAAGAACAAAAGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 61> Template DNA for variant SARS-CoV-2 RBD (Indian C538S variant) (Mutated codons are indicated with underline; and sites of mutation with bold letters.)

GTAATACGACTCACTATAAGGAGACCCAAGCTACATTTGCTTCTGACACAAC TGTGTTCACTAGCAACCTCAAACAGACACCGCCACCATGTTCGTGTTCCTGG TGCTGCTGCCCCTGGTGAGCAGCAGAGTGCAGCCCACCGAGAGCATCGTGC GGTTCCCCAACATCACCAACCTGTGCCCCTTCGGCGAGGTGTTCAACGCCAC CAGATTCGCCAGCGTGTACGCCTGGAACCGGAAGCGGATCAGCAACTGCGT GGCCGACTACAGCGTGCTGTACAACAGCGCCAGCTTCAGCACCTTCAAGTGC TACGGCGTGAGCCCCACCAAGCTGAACGACCTGTGCTTCACCAACGTGTACG CCGACAGCTTCGTGATCAGAGGCGACGAAGTGCGGCAGATCGCCCCCGGAC AGACAGGCAAGATCGCCGACTACAACTACAAGCTGCCCGACGACTTCACCGG CTGCGTGATCGCCTGGAACAGCAACAACCTGGACAGCAAAGTCGGCGGCAA

CTACAACTACCGGTACCGGCTGTTCCGGAAGTCCAACCTGAAGCCCTTCGAG

CGGGACATCAGCACCGAGATCTACCAGGCCGGCAGCACCCCCTGCAACGGC

GTGCAAGGCTTCAACTGCTACTTCCCACTGCAGAGCTACGGCTTCCAGCCCA

CAAACGGCGTGGGCTACCAGCCCTACAGAGTGGTGGTGCTGAGCTTCGAGC

TGCTGCACGCCCCCGCCACAGTGTGCGGCCCCAAGAAAAGCACCAACCTGG

TCAAGAACAAAAGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAACATTTATTTTCATT

GCAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 62> Template DNA for variant SARS-CoV-2 RBD (combination variant (1)) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAAGAACCTGGACAGCAAAGTCGGCGGCAA
CTACAACTACCGGTACCGGCTGTTCCGGAAGTCCAACCTGAAGCCCTTCGAG
CGGGACATCAGCACCGAGATCTACCAGGTCGGCAGCACCCCCTGCAACGGC
GCGGAAGGCTTCAACTGCTACTTCCCACTGCAGAGCTACGGCTTCCAGCCCA

CAAACGGCGTGGGCTACCAGCCCTACAGAGTGGTGGTGCTGAGCTTCGAGC

TGCTGCACGCCCCCGCCACAGTGTGCGGCCCCAAGAAAAGCACCAACCTGG

TCAAGAACAAATGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAA

T7 promoter sequence: nucleotide numbers1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 63> Template DNA for variant SARS-CoV-2 RBD (combination variant (2)) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAAGATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGAACCCCCTGCAACGGC
GTGGAAGGCTTCAACTGCTACCTCCCACTGCAGAGCTACGGCTTCCAGCCCA
CAAACGGCGTGGGCTACCAGCCCTACAGAGTGGTGGTGCTGAGCTTCGAGC
TGCTGCACGCCCCCGCCACAGTGTGCGGCCCCAAGAAAGCACCAACCTGG

TCAAGAACAAATGCGTGAACTTCTGAGCTCGCTTTCTTGCTGTCCAATTTCT

ATTAAAGGTTCCTTTGTTCCCTAAGTCCAACTACTAAACTGGGGGATATTAT

GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931
PolyA sequence (A100): nucleotide numbers 932-1031

<SEQ ID NO: 64> Template DNA for variant SARS-CoV-2 RBD (combination variant (3)) (Mutated codons are indicated with underline; and sites of mutation with bold letters.)

GTAATACGACTCACTATAAGGAGACCCAAGCTACATTTGCTTCTGACACAAC
TGTGTTCACTAGCAACCTCAAACAGACACCGCCACCATGTTCGTGTTCCTGG
TGCTGCTGCCCCTGGTGAGCAGCAGAGTGCAGCCCACCGAGAGCATCGTGC
GGTTCCCCAACATCACCAACCTGTGCCCCTTCGGCGAGGTGTTCAACGCCAC
CAGATTCGCCAGCGTGTACGCCTGGAACCGGAAGCGGATCAGCAACTGCGT
GGCCGACTACAGCGTGCTGTACAACAGCGCCAGCTTCAGCACCTTCAAGTGC
TACGGCGTGAGCCCCACCAAGCTGAACGACCTGTGCTTCACCAACGTGTACG
CCGACAGCTTCGTGATCAGAGGCGACGAAGTGCGGCAGATCGCCCCCGGAC
AGACAGGCAACATCGCCGACTACAACTACAAGCTGCCCGACGACTTCACCGG
CTGCGTGATCGCCTGGAACAGCAACAACCTGGACAGCAAAGTCGGCGGCAA
CTACAACTACCTGTACCGGCTGTTCCGGAAGTCCAACCTGAAGCCCTTCGAG
CGGGACATCAGCACCGAGATCTACCAGGCCGGCAGAACCCCCTGCAACGGC
GTGAAAGGCTTCAACTGCTACTTCCCACTGCAGAGCTACGGCTTCCAGCCCA
CATACGGCGTGGGCTACCAGCCCTACAGAGTGGTGGTGCTGAGCTTCGAGC
TGCTGCACGCCCCCGCCACAGTGTGCGGCCCCAAGAAAGCACCAACCTGG
TCAAGAACAAATGCGTGAACTTCTGAGCTCGCTTTCTTGCTGTCCAATTTCT
ATTAAAGGTTCCTTTGTTCCCTAAGTCCAACTACTAAACTGGGGGATATTAT
GAAGGGCCTTGAGCATCTGGATTCTGCCTAATAAAAAACATTTATTTTCATT

GCAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBDs sequence: nucleotide numbers 128-799
3'-UTR sequence: nucleotide numbers 800-931
PolyA sequence (A100): nucleotide numbers 932-1031

<SEQ ID NO: 65> Template DNA for variant SARS-CoV-2 RBD (combination variant (4)) (Mutated codons are indicated with underline; and sites of mutation with bold letters.)

GTAATACGACTCACTATAAGGAGACCCAAGCTACATTTGCTTCTGACACAAC

TGTGTTCACTAGCAACCTCAAACAGACACCGCCACCATGTTCGTGTTCCTGG

TGCTGCTGCCCCTGGTGAGCAGCAGAGTGCAGCCCACCGAGAGCATCGTGC

GGTTCCCCAACATCACCAACCTGTGCCCCTTCGGCGAGGTGTTCAACGCCAC

CAGATTCGCCAGCGTGTACGCCTGGAACCGGAAGCGGATCAGCAACTGCGT

GGCCGACTACAGCGTGCTGTACAACAGCGCCAGCTTCAGCACCTTCAAGTGC

TACGGCGTGAGCCCCACCAAGCTGAACGACCTGTGCTTCACCAACGTGTACG

CCGACAGCTTCGTGATCAGAGGCGACGAAGTGCGGCAGATCGCCCCCGGAC

AGACAGGCAAGATCGCCGACTACAACTACAAGCTGCCCGACGACTTCACCGG

CTGCGTGATCGCCTGGAACAGCAACAACCTGGACAGCAAAGTCGGCGGCAA

CTACAACTACCTGTACCGGCTGTTCCGGAAGTCCAACCTGAAGCCCTTCGAG

CGGGACATCAGCACCGAGATCTACCAGGCCGGCAGCACCCCCTGCAACGGC

GTGGAAGGCAGCAACTGCTACCTCCCACTGCAGAGCTACGGCTTCCAGCCC

ACAAACGGCGTGGGCTACCAGCCCTACAGAGTGGTGGTGCTGAGCTTCGAG

CTGCTGCACGCCCCCGCCACAGTGTGCGGCCCCAAGAAAGCACCAACCTG

GTCAAGAACAAATGCGTGAACTTCTGAGCTCGCTTTCTTGCTGTCCAATTTC

TATTAAAGGTTCCTTTGTTCCCTAAGTCCAACTACTAAACTGGGGGATATTA

TGAAGGGCCTTGAGCATCTGGATTCTGCCTAATAAAAAACATTTATTTTCAT

TGCAAAAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAAAAA

T7 promoter sequence: nucleotide numbers 1-18
A: transcription start site: nucleotide number 19
5'-UTR sequence (including transcription start site and KOZAK sequence of nucleotide numbers 83-88): nucleotide numbers 19-88
Spike protein signal sequence: nucleotide numbers 89-127
RBD sequence: nucleotide numbers 128-799
3'-UTR sequence: nucleotide numbers 800-931

PolyA sequence (A100): nucleotide numbers 932-1031

<SEQ ID NO: 66> Variant SARS-CoV-2 RBD mRNA (South African variant) (Mutated codons are indicated with underline; and sites of mutation with bold letters.)

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCAGAGUGCAGCCCACCGAGAGCAUCGUGCGGUUCCCCAACAUCA

CCAACCUGUGCCCCUUCGGCGAGGUGUUCAACGCCACCAGAUUCGCCAGC

GUGUACGCCUGGAACCGGAAGCGGAUCAGCAACUGCGUGGCCGACUACAG

CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU

UCGUGAUCAGAGGCGACGAAGUGCGGCAGAUCGCCCCCGGACAGACAGGC

AACAUCGCCGACUACAACUACAAGCUGCCCGACGACUUCACCGGCUGCGUG

AUCGCCUGGAACAGCAACAACCUGGACAGCAAAGUCGGCGGCAACUACAAC

UACCUGUACCGGCUGUUCCGGAAGUCCAACCUGAAGCCCUUCGAGCGGGA

CAUCAGCACCGAGAUCUACCAGGCCGGCAGCACCCCCUGCAACGGCGUGAA

AGGCUUCAACUGCUACUUCCCACUGCAGAGCUACGGCUUCCAGCCCACAUA

CGGCGUGGGCUACCAGCCCUACAGAGUGGUGGUGCUGAGCUUCGAGCUGC

UGCACGCCCCCGCCACAGUGUGCGGCCCCAAGAAAGCACCAACCUGGUCA

AGAACAAAUGCGUGAACUUCUGAGCUCGCUUUCUUGCUGUCCAAUUUCUA

UUAAAGGUUCCUUUGUUCCCUAAGUCCAACUACUAAACUGGGGGAUAUUA

UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAA

<SEQ ID NO: 67> Variant SARS-CoV-2 RBD mRNA (UK variant) (Mutated codons are indicated with underline; and sites of mutation with bold letters.)

AGGAGACCCAAGCUACAUUUGCUUCUGACACAACUGUGUUCACUAGCAACC
UCAAACAGACACCGCCACCAUGUUCGUGUUCCUGGUGCUGCUGCCCCUGG
UGAGCAGCAGAGUGCAGCCCACCGAGAGCAUCGUGCGGUUCCCCAACAUCA
CCAACCUGUGCCCCUUCGGCGAGGUGUUCAACGCCACCAGAUUCGCCAGC
GUGUACGCCUGGAACCGGAAGCGGAUCAGCAACUGCGUGGCCGACUACAG
CGUGCUGUACAACAGCGCCAGCUUCAGCACCUUCAAGUGCUACGGCGUGA
GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCUGUACCGGCUGUUCCGGAAGUCCAACCUGAAGCCCUUCGAGCGGGA
CAUCAGCACCGAGAUCUACCAGGCCGGCAGCACCCCCUGCAACGGCGUGGA
AGGCUUCAACUGCUACUUCCCACUGCAGAGCUACGGCUUCCAGCCCACAUA
CGGCGUGGGCUACCAGCCCUACAGAGUGGUGGUGCUGAGCUUCGAGCUGC
UGCACGCCCCCGCCACAGUGUGCGGCCCCAAGAAAGCACCAACCUGGUCA
AGAACAAAUGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAAAAAAAA

<SEQ ID NO: 68> Variant SARS-CoV-2 RBD mRNA (Brazilian variant) (Mutated codons are indicated with underline; and sites of mutation with bold letters.)

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCAGAGUGCAGCCCACCGAGAGCAUCGUGCGGUUCCCCAACAUCA

CCAACCUGUGCCCCUUCGGCGAGGUGUUCAACGCCACCAGAUUCGCCAGC

GUGUACGCCUGGAACCGGAAGCGGAUCAGCAACUGCGUGGCCGACUACAG

CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU

UCGUGAUCAGAGGCGACGAAGUGCGGCAGAUCGCCCCCGGACAGACAGGC

<u>ACG</u>AUCGCCGACUACAACUACAAGCUGCCCGACGACUUCACCGGCUGCGUG

AUCGCCUGGAACAGCAACAACCUGGACAGCAAAGUCGGCGGCAACUACAAC

UACCUGUACCGGCUGUUCCGGAAGUCCAACCUGAAGCCCUUCGAGCGGGA

CAUCAGCACCGAGAUCUACCAGGCCGGCAGCACCCCCUGCAACGGCGUG<u>AA</u>

AGGCUUCAACUGCUACUUCCCACUGCAGAGCUACGGCUUCCAGCCCACAUA

CGGCGUGGGCUACCAGCCCUACAGAGUGGUGGUGCUGAGCUUCGAGCUGC

UGCACGCCCCCGCCACAGUGUGCGGCCCCAAGAAAAGCACCAACCUGGUCA

AGAACAAAUGCGUGAACUUCUGAGCUCGCUUUCUUGCUGUCCAAUUUCUA

UUAAAGGUUCCUUUGUUCCCUAAGUCCAACUACUAAACUGGGGGAUAUUA

UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAA

<SEQ ID NO: 69> Variant SARS-CoV-2 RBD mRNA (Californian variant) (Mutated codons are indicated with underline; and sites of mutation with bold letters.)

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCAGAGUGCAGCCCACCGAGAGCAUCGUGCGGUUCCCCAACAUCA

CCAACCUGUGCCCCUUCGGCGAGGUGUUCAACGCCACCAGAUUCGCCAGC

GUGUACGCCUGGAACCGGAAGCGGAUCAGCAACUGCGUGGCCGACUACAG

CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCGGUACCGGCUGUUCCGGAAGUCCAACCUGAAGCCCUUCGAGCGGGA
CAUCAGCACCGAGAUCUACCAGGCCGGCAGCACCCCCUGCAACGGCGUGGA
AGGCUUCAACUGCUACUUCCCACUGCAGAGCUACGGCUUCCAGCCCACAAA
CGGCGUGGGCUACCAGCCCUACAGAGUGGUGGUGCUGAGCUUCGAGCUGC
UGCACGCCCCCGCCACAGUGUGCGGCCCCAAGAAAGCACCAACCUGGUCA
AGAACAAAUGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC
AUUGCAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAA

<SEQ ID NO: 70> Variant SARS-CoV-2 RBD mRNA (Indian variant) (Mutated codons are indicated with underline; and sites of mutation with bold letters.)

AGGAGACCCAAGCUACAUUUGCUUCUGACACAACUGUGUUCACUAGCAACCUCAAACAGACACCGCCACCAUGUUCGUGUUCCUGGUGCUGCUGCCCCUGGUGAGCAGCAGAGUGCAGCCCACCGAGAGCAUCGUGCGGUUCCCCAACAUCACCAACCUGUGCCCCUUCGGCGAGGUGUUCAACGCCACCAGAUUCGCCAGCGUGUACGCCUGGAACCGGAAGCGGAUCAGCAACUGCGUGGCCGACUACAGCGUGCUGUACAACAGCGCCAGCUUCAGCACCUUCAAGUGCUACGGCGUGAGCCCCACCAAGCUGAACGACCUGUGCUUCACCAACGUGUACGCCGACAGCUUCGUGAUCAGAGGCGACGAAGUGCGGCAGAUCGCCCCCGGACAGACAGGCAAGAUCGCCGACUACAACUACAAGCUGCCCGACGACUUCACCGGCUGCGUGAUCGCCUGGAACAGCAACAACCUGGACAGCAAAGUCGGCGGCAACUACAACUACCGGUACCGGCUGUUCCGGAAGUCCAACCUGAAGCCCUUCGAGCGGGACAUCAGCACCGAGAUCUACCAGGCCGGCAGCACCCCCUGCAACGGCGUGCAAGGCUUCAACUGCUACUUCCCACUGCAGAGCUACGGCUUCCAGCCCACAAACGGCGUGGGCUACCAGCCCUACAGAGUGGUGGUGCUGAGCUUCGAGCUGCUGCACGCCCCCGCCACAGUGUGCGGCCCCAAGAAAAGCACCAACCUGGUCAAGAACAAAUGCGUGAACUUCUGAGCUCGCUUUCUUGCUGUCCAAUUUCUAUUAAAGGUUCCUUUGUUCCCUAAGUCCAACUACUAAACUGGGGGAUAUUAUGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAAAAAAAAA

<SEQ ID NO: 71> Variant SARS-CoV-2 RBD mRNA (South African C538S variant) (Mutated codons are indicated with underline; and sites of mutation with bold letters.)

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCAGAGUGCAGCCCACCGAGAGCAUCGUGCGGUUCCCCAACAUCA

CCAACCUGUGCCCCUUCGGCGAGGUGUUCAACGCCACCAGAUUCGCCAGC

GUGUACGCCUGGAACCGGAAGCGGAUCAGCAACUGCGUGGCCGACUACAG

CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU

UCGUGAUCAGAGGCGACGAAGUGCGGCAGAUCGCCCCCGGACAGACAGGC

AACAUCGCCGACUACAACUACAAGCUGCCCGACGACUUCACCGGCUGCGUG

AUCGCCUGGAACAGCAACAACCUGGACAGCAAAGUCGGCGGCAACUACAAC

UACCUGUACCGGCUGUUCCGGAAGUCCAACCUGAAGCCCUUCGAGCGGGA

CAUCAGCACCGAGAUCUACCAGGCCGGCAGCACCCCCUGCAACGGCGUGAA

AGGCUUCAACUGCUACUUCCCACUGCAGAGCUACGGCUUCCAGCCCACAUA

CGGCGUGGGCUACCAGCCCUACAGAGUGGUGGUGCUGAGCUUCGAGCUGC

UGCACGCCCCCGCCACAGUGUGCGGCCCCAAGAAAAGCACCAACCUGGUCA

AGAACAAAAGCGUGAACUUCUGAGCUCGCUUUCUUGCUGUCCAAUUUCUA

UUAAAGGUUCCUUUGUUCCCUAAGUCCAACUACUAAACUGGGGGAUAUUA

UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAA

<SEQ ID NO: 72> Variant SARS-CoV-2 RBD mRNA (UK C538S variant) (Mutated codons are indicated with underline; and sites of mutation with bold letters.)

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCAGAGUGCAGCCCACCGAGAGCAUCGUGCGGUUCCCCAACAUCA

CCAACCUGUGCCCCUUCGGCGAGGUGUUCAACGCCACCAGAUUCGCCAGC

GUGUACGCCUGGAACCGGAAGCGGAUCAGCAACUGCGUGGCCGACUACAG

CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCUGUACCGGCUGUUCCGGAAGUCCAACCUGAAGCCCUUCGAGCGGGA
CAUCAGCACCGAGAUCUACCAGGCCGGCAGCACCCCCUGCAACGGCGUGGA
AGGCUUCAACUGCUACUUCCCACUGCAGAGCUACGGCUUCCAGCCCACAUA
CGGCGUGGGCUACCAGCCCUACAGAGUGGUGGUGCUGAGCUUCGAGCUGC
UGCACGCCCCCGCCACAGUGUGCGGCCCCAAGAAAGCACCAACCUGGUCA
AGAACAAAAGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC
AUUGCAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA

<SEQ ID NO: 73> Variant SARS-CoV-2 RBD mRNA (Brazilian C538S variant) (Mutated codons are indicated with underline; and sites of mutation with bold letters.)

AGGAGACCCAAGCUACAUUUGCUUCUGACACAACUGUGUUCACUAGCAACC
UCAAACAGACACCGCCACCAUGUUCGUGUUCCUGGUGCUGCUGCCCCUGG
UGAGCAGCAGAGUGCAGCCCACCGAGAGCAUCGUGCGGUUCCCCAACAUCA
CCAACCUGUGCCCCUUCGGCGAGGUGUUCAACGCCACCAGAUUCGCCAGC
GUGUACGCCUGGAACCGGAAGCGGAUCAGCAACUGCGUGGCCGACUACAG
CGUGCUGUACAACAGCGCCAGCUUCAGCACCUUCAAGUGCUACGGCGUGA
GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
ACGAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCUGUACCGGCUGUUCCGGAAGUCCAACCUGAAGCCCUUCGAGCGGGA
CAUCAGCACCGAGAUCUACCAGGCCGGCAGCACCCCCUGCAACGGCGUGAA
AGGCUUCAACUGCUACUUCCCACUGCAGAGCUACGGCUUCCAGCCCACAUA
CGGCGUGGGCUACCAGCCCUACAGAGUGGUGGUGCUGAGCUUCGAGCUGC
UGCACGCCCCCGCCACAGUGUGCGGCCCCAAGAAAGCACCAACCUGGUCA
AGAACAAAAGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAA

<SEQ ID NO: 74> Variant SARS-CoV-2 RBD mRNA (Californian C538S variant) (Mutated codons are indicated with underline; and sites of mutation with bold letters.)

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCAGAGUGCAGCCCACCGAGAGCAUCGUGCGGUUCCCCAACAUCA

CCAACCUGUGCCCCUUCGGCGAGGUGUUCAACGCCACCAGAUUCGCCAGC

GUGUACGCCUGGAACCGGAAGCGGAUCAGCAACUGCGUGGCCGACUACAG

CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU

UCGUGAUCAGAGGCGACGAAGUGCGGCAGAUCGCCCCCGGACAGACAGGC

AAGAUCGCCGACUACAACUACAAGCUGCCCGACGACUUCACCGGCUGCGUG

AUCGCCUGGAACAGCAACAACCUGGACAGCAAAGUCGGCGGCAACUACAAC

UACCGGUACCGGCUGUUCCGGAAGUCCAACCUGAAGCCCUUCGAGCGGGA

CAUCAGCACCGAGAUCUACCAGGCCGGCAGCACCCCCUGCAACGGCGUGGA

AGGCUUCAACUGCUACUUCCCACUGCAGAGCUACGGCUUCCAGCCCACAAA
CGGCGUGGGCUACCAGCCCUACAGAGUGGUGGUGCUGAGCUUCGAGCUGC
UGCACGCCCCCGCCACAGUGUGCGGCCCCAAGAAAAGCACCAACCUGGUCA
AGAACAAAAGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC
AUUGCAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA

<SEQ ID NO: 75> Variant SARS-CoV-2 RBD mRNA (Indian C538S variant) (Mutated codons are indicated with underline; and sites of mutation with bold letters.)

AGGAGACCCAAGCUACAUUUGCUUCUGACACAACUGUGUUCACUAGCAACC
UCAAACAGACACCGCCACCAUGUUCGUGUUCCUGGUGCUGCUGCCCCUGG
UGAGCAGCAGAGUGCAGCCCACCGAGAGCAUCGUGCGGUUCCCCAACAUCA
CCAACCUGUGCCCCUUCGGCGAGGUGUUCAACGCCACCAGAUUCGCCAGC
GUGUACGCCUGGAACCGGAAGCGGAUCAGCAACUGCGUGGCCGACUACAG
CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCGGUACCGGCUGUUCCGGAAGUCCAACCUGAAGCCCUUCGAGCGGGA
CAUCAGCACCGAGAUCUACCAGGCCGGCAGCACCCCCUGCAACGGCGUGCA
AGGCUUCAACUGCUACUUCCCACUGCAGAGCUACGGCUUCCAGCCCACAAA
CGGCGUGGGCUACCAGCCCUACAGAGUGGUGGUGCUGAGCUUCGAGCUGC
UGCACGCCCCCGCCACAGUGUGCGGCCCCAAGAAAGCACCAACCUGGUCA
AGAACAAAAGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC
AUUGCAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAA

<SEQ ID NO: 76> Variant SARS-CoV-2 RBD mRNA (combination variant (1)) (Mutated codons are indicated with underline; and sites of mutation with bold letters.)

AGGAGACCCAAGCUACAUUUGCUUCUGACACAACUGUGUUCACUAGCAACC
UCAAACAGACACCGCCACCAUGUUCGUGUUCCUGGUGCUGCUGCCCCUGG
UGAGCAGCAGAGUGCAGCCCACCGAGAGCAUCGUGCGGUUCCCCAACAUCA
CCAACCUGUGCCCCUUCGGCGAGGUGUUCAACGCCACCAGAUUCGCCAGC
GUGUACGCCUGGAACCGGAAGCGGAUCAGCAACUGCGUGGCCGACUACAG
CGUGCUGUACAACAGCGCCAGCUUCAGCACCUUCAAGUGCUACGGCGUGA
GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAAGAACCUGGACAGCAAAGUCGGCGGCAACUACAAC
UACCGGUACCGGCUGUUCCGGAAGUCCAACCUGAAGCCCUUCGAGCGGGA
CAUCAGCACCGAGAUCUACCAGGUCGGCAGCACCCCCUGCAACGGCGCGGA
AGGCUUCAACUGCUACUUCCCACUGCAGAGCUACGGCUUCCAGCCCACAAA
CGGCGUGGGCUACCAGCCCUACAGAGUGGUGGUGCUGAGCUUCGAGCUGC
UGCACGCCCCCGCCACAGUGUGCGGCCCCAAGAAAGCACCAACCUGGUCA
AGAACAAAUGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAA

<SEQ ID NO: 77> Variant SARS-CoV-2 RBD mRNA (combination variant (2)) (Mutated codons are indicated with underline; and sites of mutation with bold letters.)

AGGAGACCCAAGCUACAUUUGCUUCUGACACAACUGUGUUCACUAGCAACC

UCAAACAGACACCGCCACCAUGUUCGUGUUCCUGGUGCUGCUGCCCCUGG

UGAGCAGCAGAGUGCAGCCCACCGAGAGCAUCGUGCGGUUCCCCAACAUCA

CCAACCUGUGCCCCUUCGGCGAGGUGUUCAACGCCACCAGAUUCGCCAGC

GUGUACGCCUGGAACCGGAAGCGGAUCAGCAACUGCGUGGCCGACUACAG

CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU

UCGUGAUCAGAGGCGACGAAGUGCGGCAGAUCGCCCCCGGACAGACAGGC

AAGAUCGCCGACUACAACUACAAGCUGCCCGACGACUUCACCGGCUGCGUG

AUCGCCUGGAACAGCAACAACCUGGACAGCAAAGUCGGCGGCAACUACAAC

UACCUGUACCGGCUGUUCCGGAAGUCCAACCUGAAGCCCUUCGAGCGGGA

CAUCAGCACCGAGAUCUACCAGGCCGGCAGAACCCCCUGCAACGGCGUGGA

AGGCUUCAACUGCUACCUCCCACUGCAGAGCUACGGCUUCCAGCCCACAAA
CGGCGUGGGCUACCAGCCCUACAGAGUGGUGGUGCUGAGCUUCGAGCUGC
UGCACGCCCCCGCCACAGUGUGCGGCCCCAAGAAAAGCACCAACCUGGUCA
AGAACAAAUGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC
AUUGCAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAA

<SEQ ID NO: 78> Variant SARS-CoV-2 RBD mRNA (combination variant (3)) (Mutated codons are indicated with underline; and sites of mutation with bold letters.)

AGGAGACCCAAGCUACAUUUGCUUCUGACACAACUGUGUUCACUAGCAACC
UCAAACAGACACCGCCACCAUGUUCGUGUUCCUGGUGCUGCUGCCCCUGG
UGAGCAGCAGAGUGCAGCCCACCGAGAGCAUCGUGCGGUUCCCCAACAUCA
CCAACCUGUGCCCCUUCGGCGAGGUGUUCAACGCCACCAGAUUCGCCAGC
GUGUACGCCUGGAACCGGAAGCGGAUCAGCAACUGCGUGGCCGACUACAG
CGUGCUGUACAACAGCGCCAGCUUCAGCACCUUCAAGUGCUACGGCGUGA

GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AACAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCUGUACCGGCUGUUCCGGAAGUCCAACCUGAAGCCCUUCGAGCGGGA
CAUCAGCACCGAGAUCUACCAGGCCGGCAGAACCCCCUGCAACGGCGUGAA
AGGCUUCAACUGCUACUUCCCACUGCAGAGCUACGGCUUCCAGCCCACAUA
CGGCGUGGGCUACCAGCCCUACAGAGUGGUGGUGCUGAGCUUCGAGCUGC
UGCACGCCCCCGCCACAGUGUGCGGCCCCAAGAAAGCACCAACCUGGUCA
AGAACAAAUGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAACAUUUAUUUC
AUUGCAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAA

<SEQ ID NO: 79> Variant SARS-CoV-2 RBD mRNA (combination variant (4)) (Mutated codons are indicated with underline; and sites of mutation with bold letters.)

AGGAGACCCAAGCUACAUUUGCUUCUGACACAACUGUGUUCACUAGCAACC
UCAAACAGACACCGCCACCAUGUUCGUGUUCCUGGUGCUGCUGCCCCUGG
UGAGCAGCAGAGUGCAGCCCACCGAGAGCAUCGUGCGGUUCCCCAACAUCA
CCAACCUGUGCCCCUUCGGCGAGGUGUUCAACGCCACCAGAUUCGCCAGC
GUGUACGCCUGGAACCGGAAGCGGAUCAGCAACUGCGUGGCCGACUACAG
CGUGCUGUACAACAGCGCCAGCUUCAGCACCUUCAAGUGCUACGGCGUGA
GCCCCACCAAGCUGAACGACCUGUGCUUCACCAACGUGUACGCCGACAGCU
UCGUGAUCAGAGGCGACGAAGUGCGGCAGAUCGCCCCCGGACAGACAGGC
AAGAUCGCCGACUACAACUACAAGCUGCCCGACGACUUCACCGGCUGCGUG
AUCGCCUGGAACAGCAACAACCUGGACAGCAAAGUCGGCGGCAACUACAAC
UACCUGUACCGGCUGUUCCGGAAGUCCAACCUGAAGCCCUUCGAGCGGGA
CAUCAGCACCGAGAUCUACCAGGCCGGCAGCACCCCCUGCAACGGCGUGGA
AGGCAGCAACUGCUACCUCCCACUGCAGAGCUACGGCUUCCAGCCCACAAA
CGGCGUGGGCUACCAGCCCUACAGAGUGGUGGUGCUGAGCUUCGAGCUGC
UGCACGCCCCCGCCACAGUGUGCGGCCCCAAGAAAGCACCAACCUGGUCA
AGAACAAAUGCGUGAACUUCUGAGCUCGCUUUCUUGCUGUCCAAUUUCUA
UUAAAGGUUCCUUUGUUCCCUAAGUCCAACUACUAAACUGGGGGAUAUUA
UGAAGGGCCUUGAGCAUCUGGAUUCUGCCUAAUAAAAAACAUUUAUUUUC

AUUGCAAAAAAAAAAAAAAAAAAAAAAAAAAAAAAAAAAAAAAAAAAAAAAAA

AAAAAAAAAAAAAAAAAAAAAAAAAAAAAAAAAAAAAAAAAAAAAAAAAA

<SEQ ID NO: 80> Amino acid sequence of variant SARS-CoV-2 RBD (South African variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGNI

ADYNYKLPDDFTGCVIAWNSNNLDSKVGGNYNYLYRLFRKSNLKPFERDISTEIY

QAGSTPCNGVKGFNCYFPLQSYGFQPTYGVGYQPYRVVVLSFELLHAPATVCGP

KKSTNLVKNKCVNF

S protein signal sequence: amino acid numbers 1-13
RBD sequence: amino acid numbers 14-236

<SEQ ID NO: 81> Amino acid sequence of variant SARS-CoV-2 RBD (UK variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYLYRLFRKSNLKPFERDISTEIY

QAGSTPCNGVEGFNCYFPLQSYGFQPTYGVGYQPYRVVVLSFELLHAPATVCGP

KKSTNLVKNKCVNF

S protein signal sequence: amino acid numbers 1-13
RBD sequence: amino acid numbers 14-236

<SEQ ID NO: 82> Amino acid sequence of variant SARS-CoV-2 RBD (Brazilian variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGTI

ADYNYKLPDDFTGCVIAWNSNNLDSKVGGNYNYLYRLFRKSNLKPFERDISTEIY

QAGSTPCNGVKGFNCYFPLQSYGFQPTYGVGYQPYRVVVLSFELLHAPATVCGP

KKSTNLVKNKCVNF

S protein signal sequence: amino acid numbers 1-13
RBDs sequence: amino acid numbers 14-236

<SEQ ID NO: 83> Amino acid sequence of variant SARS-CoV-2 RBD (Californian variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYRYRLFRKSNLKPFERDISTEIY

QAGSTPCNGVEGFNCYFPLQSYGFQPTNGVGYQPYRVVVLSFELLHAPATVCGP

KKSTNLVKNKCVNF

S protein signal sequence: amino acid numbers 1-13
RBD sequence: amino acid numbers 14-236

<SEQ ID NO: 84> Amino acid sequence of variant SARS-CoV-2 RBD (Indian variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYRYRLFRKSNLKPFERDISTEIY

QAGSTPCNGVQGFNCYFPLQSYGFQPTNGVGYQPYRVVVLSFELLHAPATVCGP

KKSTNLVKNKCVNF

S protein signal sequence: amino acid numbers 1-13

RBD sequence: amino acid numbers 14-236

<SEQ ID NO: 85> Amino acid sequence of variant SARS-CoV-2 RBD (South African C538S variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGNI

ADYNYKLPDDFTGCVIAWNSNNLDSKVGGNYNYLYRLFRKSNLKPFERDISTEIY

QAGSTPCNGVKGFNCYFPLQSYGFQPTYGVGYQPYRVVVLSFELLHAPATVCGP

KKSTNLVKNKSVNF

S protein signal sequence: amino acid numbers 1-13
RBD sequence: amino acid numbers 14-236

<SEQ ID NO: 86> Amino acid sequence of variant SARS-CoV-2 RBD (UK C538S variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYLYRLFRKSNLKPFERDISTEIY

QAGSTPCNGVEGFNCYFPLQSYGFQPTYGVGYQPYRVVVLSFELLHAPATVCGP

KKSTNLVKNKSVNF

S protein signal sequence: amino acid numbers 1-13
RBDs sequence: amino acid numbers 14-236

<SEQ ID NO: 87> Amino acid sequence of variant SARS-CoV-2 RBD (Brazilian C538S variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGTI

ADYNYKLPDDFTGCVIAWNSNNLDSKVGGNYNYLYRLFRKSNLKPFERDISTEIY

QAGSTPCNGVKGFNCYFPLQSYGFQPTYGVGYQPYRVVVLSFELLHAPATVCGP

KKSTNLVKNKSVNF

<SEQ ID NO: 88> Amino acid sequence of variant SARS-CoV-2 RBD (Californian C538S variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYRYRLFRKSNLKPFERDISTEIY

QAGSTPCNGVEGFNCYFPLQSYGFQPTNGVGYQPYRVVVLSFELLHAPATVCGP

KKSTNLVKNKSVNF

S protein signal sequence: amino acid numbers 1-13
RBD sequence: amino acid numbers 14-236

<SEQ ID NO: 89> Amino acid sequence of variant SARS-CoV-2 RBD (Indian C538S variant) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYRYRLFRKSNLKPFERDISTEIY

QAGSTPCNGVQGFNCYFPLQSYGFQPTNGVGYQPYRVVVLSFELLHAPATVCGP

KKSTNLVKNKSVNF

S protein signal sequence: amino acid numbers 1-13
RBD sequence: amino acid numbers 14-236

<SEQ ID NO: 90> Amino acid sequence of variant SARS-CoV-2 RBD (combination variant (1)) (including S protein

signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSKNLDSKVGGNYNYRYRLFRKSNLKPFERDISTEIY

QVGSTPCNGAEGFNCYFPLQSYGFQPTNGVGYQPYRVVVLSFELLHAPATVCGP

KKSTNLVKNKCVNF

S protein signal sequence: amino acid numbers 1-13
RBD sequence: amino acid numbers 14-236

<SEQ ID NO: 91> Amino acid sequence of variant SARS-CoV-2 RBD (combination variant (2)) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYLYRLFRKSNLKPFERDISTEIY

QAGRTPCNGVEGFNCYLPLQSYGFQPTNGVGYQPYRVVVLSFELLHAPATVCGP

KKSTNLVKNKCVNF

S protein signal sequence: amino acid numbers 1-13
RBD sequence: amino acid numbers 14-236

<SEQ ID NO: 92> Amino acid sequence of variant SARS-CoV-2 RBD (combination variant (3)) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGNI

ADYNYKLPDDFTGCVIAWNSNNLDSKVGGNYNYLYRLFRKSNLKPFERDISTEIY

QAGRTPCNGVKGFNCYFPLQSYGFQPTYGVGYQPYRVVVLSFELLHAPATVCGP

KKSTNLVKNKCVNF

S protein signal sequence: amino acid numbers 1-13
RBD sequence: amino acid numbers 14-236

<SEQ ID NO: 93> Amino acid sequence of variant SARS-CoV-2 RBD (combination variant (4)) (including S protein signal sequence. Mutated amino acids are indicated with underline.)

MFVFLVLLPLVSSRVQPTESIVRFPNITNLCPFGEVFNATRFASVYAWNRKRISNCV

ADYSVLYNSASFSTFKCYGVSPTKLNDLCFTNVYADSFVIRGDEVRQIAPGQTGKI

ADYNYKLPDDFTGCVIAWNSNNLDSKVGGNYNYLYRLFRKSNLKPFERDISTEIY

QAGSTPCNGVEGSNCYLPLQSYGFQPTNGVGYQPYRVVVLSFELLHAPATVCGP

KKSTNLVKNKCVNF

S protein signal sequence: amino acid numbers 1-13
RBD sequence: amino acid numbers 14-236

<SEQ ID NO: 94> Amino acid sequence of variant SARS-CoV-2 RBD (South African variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGNIADYNYKLPDDFT

GCVIAWNSNNLDSKVGGNYNYLYRLFRKSNLKPFERDISTEIYQAGSTPCNGVKG

FNCYFPLQSYGFQPTYGVGYQPYRVVVLSFELLHAPATVCGPKKSTNLVKNKCV

NF

<SEQ ID NO: 95> Amino acid sequence of variant SARS-CoV-2 RBD (UK variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYLYRLFRKSNLKPFERDISTEIYQAGSTPCNGVEG

FNCYFPLQSYGFQPTYGVGYQPYRVVVLSFELLHAPATVCGPKKSTNLVKNKCV

NF

<SEQ ID NO: 96> Amino acid sequence of variant SARS-CoV-2 RBD (Brazilian variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGTIADYNYKLPDDFT

GCVIAWNSNNLDSKVGGNYNYLYRLFRKSNLKPFERDISTEIYQAGSTPCNGVKG

FNCYFPLQSYGFQPTYGVGYQPYRVVVLSFELLHAPATVCGPKKSTNLVKNKCV

NF

<SEQ ID NO: 97> Amino acid sequence of variant SARS-CoV-2 RBD (Californian variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYRYRLFRKSNLKPFERDISTEIYQAGSTPCNGVEG

FNCYFPLQSYGFQPTNGVGYQPYRVVVLSFELLHAPATVCGPKKSTNLVKNKCV

NF

<SEQ ID NO: 98> Amino acid sequence of variant SARS-CoV-2 RBD (Indian variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYRYRLFRKSNLKPFERDISTEIYQAGSTPCNGVQG

FNCYFPLQSYGFQPTNGVGYQPYRVVVLSFELLHAPATVCGPKKSTNLVKNKCV

NF

<SEQ ID NO: 99> Amino acid sequence of variant SARS-CoV-2 RBD (South African C538S variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGNIADYNYKLPDDFT

GCVIAWNSNNLDSKVGGNYNYLYRLFRKSNLKPFERDISTEIYQAGSTPCNGVKG

FNCYFPLQSYGFQPTYGVGYQPYRVVVLSFELLHAPATVCGPKKSTNLVKNKSVN

F

<SEQ ID NO: 100> Amino acid sequence of variant SARS-CoV-2 RBD (UK C538S variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYLYRLFRKSNLKPFERDISTEIYQAGSTPCNGVEG

FNCYFPLQSYGFQPTYGVGYQPYRVVVLSFELLHAPATVCGPKKSTNLVKNKSVN

F

<SEQ ID NO: 101> Amino acid sequence of variant SARS-CoV-2 RBD (Brazilian C538S variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGTIADYNYKLPDDFT

GCVIAWNSNNLDSKVGGNYNYLYRLFRKSNLKPFERDISTEIYQAGSTPCNGVKG

FNCYFPLQSYGFQPTYGVGYQPYRVVVLSFELLHAPATVCGPKKSTNLVKNKSVN

F

<SEQ ID NO: 102> Amino acid sequence of variant SARS-CoV-2 RBD (Californian C538S variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYRYRLFRKSNLKPFERDISTEIYQAGSTPCNGVEG

FNCYFPLQSYGFQPTNGVGYQPYRVVVLSFELLHAPATVCGPKKSTNLVKNKSV

NF

<SEQ ID NO: 103> Amino acid sequence of variant SARS-CoV-2 RBD (Indian C538S variant) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYRYRLFRKSNLKPFERDISTEIYQAGSTPCNGVQG

FNCYFPLQSYGFQPTNGVGYQPYRVVVLSFELLHAPATVCGPKKSTNLVKNKSV

NF

<SEQ ID NO: 104> Amino acid sequence of variant SARS-CoV-2 RBD (combination variant (1)) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSKNLDSKVGGNYNYRYRLFRKSNLKPFERDISTEIYQVGSTPCNGAEG

FNCYFPLQSYGFQPTNGVGYQPYRVVVLSFELLHAPATVCGPKKSTNLVKNKCV

NF

<SEQ ID NO: 105> Amino acid sequence of variant SARS-CoV-2 RBD (combination variant (2)) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYLYRLFRKSNLKPFERDISTEIYQAGRTPCNGVEG

FNCYLPLQSYGFQPTNGVGYQPYRVVVLSFELLHAPATVCGPKKSTNLVKNKCV

NF

<SEQ ID NO: 106> Amino acid sequence of variant SARS-CoV-2 RBD (combination variant (3)) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGNIADYNYKLPDDFT

GCVIAWNSNNLDSKVGGNYNYLYRLFRKSNLKPFERDISTEIYQAGRTPCNGVKG

FNCYFPLQSYGFQPTYGVGYQPYRVVVLSFELLHAPATVCGPKKSTNLVKNKCV

NF

<SEQ ID NO: 107> Amino acid sequence of variant SARS-CoV-2 RBD (combination variant (4)) (not including S protein signal sequence. Mutated amino acids are indicated with underline.)

RVQPTESIVRFPNITNLCPFGEVFNATRFASVYAWNRKRISNCVADYSVLYNSASFS

TFKCYGVSPTKLNDLCFTNVYADSFVIRGDEVRQIAPGQTGKIADYNYKLPDDFT

GCVIAWNSNNLDSKVGGNYNYLYRLFRKSNLKPFERDISTEIYQAGSTPCNGVEG

SNCYLPLQSYGFQPTNGVGYQPYRVVVLSFELLHAPATVCGPKKSTNLVKNKCV

NF

## Claims

1. A lipid particle encapsulating a nucleic acid molecule capable of expressing the S protein and/or a fragment thereof of severe acute respiratory syndrome coronavirus 2 (SARS-CoV-2), wherein the lipid comprises a cationic lipid represented by general formula (Ia) or a pharmaceutically acceptable salt thereof:

[Formula 1]

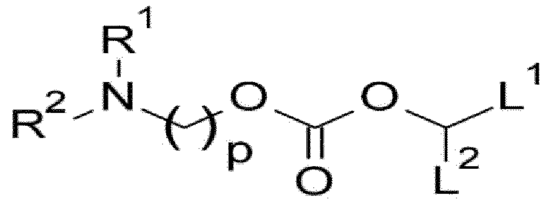

(Ia)

wherein $R^1$ and $R^2$ each independently represent a $C_1$-$C_3$ alkyl group;
L’ represents a $C_{17}$-$C_{19}$ alkenyl group which may have one or a plurality of $C_2$-$C_4$ alkanoyloxy groups;
$L^2$ represents a $C_{10}$-$C_{19}$ alkyl group which may have one or a plurality of $C_2$-$C_4$ alkanoyloxy groups or a $C_{10}$-$C_{19}$ alkenyl group which may have one or a plurality of $C_2$-$C_4$ alkanoyloxy groups; and
p is 3 or 4.

2. The particle of claim 1, wherein both $R^1$ and $R^2$ in general formula (Ia) are a methyl group.

3. The particle of claim 1 or 2, wherein p in general formula (Ia) is 3.

4. The particle of any one of claims 1 to 3, wherein $L^1$ in general formula (Ia) is a $C_{17}$-$C_{19}$ alkenyl group which may have one or a plurality of acetoxy groups.

5. The particle of any one of claims 1 to 4, wherein $L^2$ in general formula (Ia) is a $C_{10}$-$C_{12}$ alkyl group which may have one or a plurality of acetoxy groups or a $C_{10}$-$C_{19}$ alkenyl group which may have one or a plurality of acetoxy groups.

6. The particle of any one of claims 1 to 4, wherein $L^2$ in general formula (Ia) is a $C_{10}$-$C_{12}$ alkyl group which may have one or a plurality of acetoxy groups or a $C_{17}$-$C_{19}$ alkenyl group which may have one or a plurality of acetoxy groups.

7. The particle of any one of claims 1 to 6, wherein $L^1$ in general formula (Ia) is an (R)-11-acetyloxy-cis-8-heptadecenyl group, a cis-8-heptadecenyl group or a (8Z,11Z)-heptadecadienyl group.

8. The particle of any one of claims 1 to 7, wherein $L^2$ in general formula (Ia) is a decyl group, a cis-7-decenyl group,

a dodecyl group or an (R)-11-acetyloxy-cis-8-heptadecenyl group.

9. The particle of claim 1, wherein the cationic lipid is represented by the following structural formula:

[Formula 2]

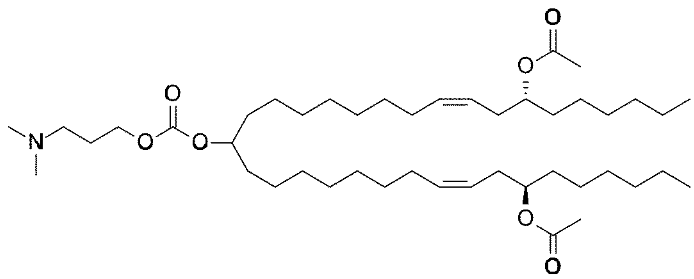

10. The particle of claim 1, wherein the cationic lipid is represented by the following structural formula:

[Formula 3]

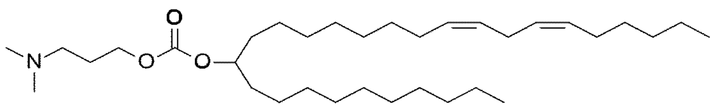

11. The particle of claim 1, wherein the cationic lipid is represented by the following structural formula:

[Formula 4]

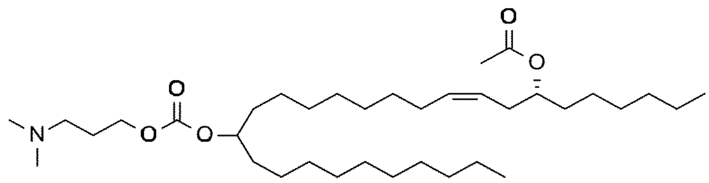

.

12. The particle of any one of claims 1 to 11, wherein the lipid further comprises amphipathic lipids, sterols and PEG lipids.

13. The particle of claim 12, wherein the amphipathic lipid is at least one selected from the group consisting of distearoyl phosphatidylcholine, dioleoyl phosphatidylcholine and dioleoyl phosphatidylethanolamine.

14. The particle of claim 12 or 13, wherein the sterol is cholesterol.

15. The particle of any one of claims 12 to 14, wherein the PEG lipid is 1,2-dimyristoyl-sn-glycerol methoxypolyethylene glycol and/or N-[methoxy poly(ethyleneglycol) 2000] carbamoyl]-1,2-dimyristyloxypropyl-3-amine.

16. The particle of any one of claims 12 to 15, wherein the lipid composition of the amphipathic lipid, the sterol, the cationic lipid and the PEG lipid is 15% or less of the amphipathic lipid, 20 to 55% of the sterol, 40 to 65% of the cationic lipid and 1 to 5% of the PEG lipid in terms of molar quantity; and the ratio of the total lipid weight to the weight of nucleic acid is 15 to 30.

17. The particle of claim 16, wherein the lipid composition of the amphipathic lipid, the sterol, the cationic lipid and the PEG lipid is 5 to 15% of the amphipathic lipid, 35 to 50% of the sterol, 40 to 55% of the cationic lipid and 1 to 3% of the PEG lipid in terms of molar quantity; and the ratio of the total lipid weight to the weight of nucleic acid is 15 to 25.

**18.** The particle of claim 17, wherein the lipid composition of the amphipathic lipid, the sterol, the cationic lipid and the PEG lipid is 10 to 15% of the amphipathic lipid, 35 to 45% of the sterol, 40 to 50% of the cationic lipid and 1 to 2% of the PEG lipid in terms of molar quantity; and the ratio of the total lipid weight to the weight of nucleic acid is 17.5 to 22.5.

**19.** The particle of claim 18, wherein the lipid composition of the amphipathic lipid, the sterol, the cationic lipid and the PEG lipid is 10 to 15% of the amphipathic lipid, 35 to 45% of the sterol, 45 to 50% of the cationic lipid and 1.5 to 2% of the PEG lipid in terms of molar quantity; and the ratio of the total lipid weight to the weight of nucleic acid is 17.5 to 22.5.

**20.** The particle of any one of claims 1 to 19, wherein the fragment of the S protein of severe acute respiratory syndrome coronavirus 2 (SARS-CoV-2) comprises a receptor-binding domain.

**21.** The particle of claim 20, wherein the receptor-binding domain in the fragment of the S protein of severe acute respiratory syndrome coronavirus 2 (SARS-CoV-2) consists of an amino acid sequence having at least 95% identity with the amino acid sequence as shown in SEQ ID NO: 11.

**22.** The particle of claim 20, wherein the receptor-binding domain in the fragment of the S protein of severe acute respiratory syndrome coronavirus 2 (SARS-CoV-2) consists of an amino acid sequence having at least 95% identity with the amino acid sequence as shown in any one of SEQ ID NOS: 25, 29, 33, 37 and 94 to 107.

**23.** The particle of claim 20, wherein the fragment of the S protein of severe acute respiratory syndrome coronavirus 2 (SARS-CoV-2) consists of an amino acid sequence having at least 95% identity with the amino acid sequence as shown in SEQ ID NO: 10.

**24.** The particle of claim 20, wherein the fragment of the S protein of severe acute respiratory syndrome coronavirus 2 (SARS-CoV-2) consists of an amino acid sequence having at least 95% identity with the amino acid sequence as shown in any one of SEQ ID NOS: 24, 28, 32, 36 and 80 to 93.

**25.** The particle of any one of claims 1 to 19, wherein the S protein of severe acute respiratory syndrome coronavirus 2 (SARS-CoV-2) consists of an amino acid sequence having at least 95% identity with the amino acid sequence as shown in SEQ ID NO: 6.

**26.** The particle of claim 25, wherein the receptor-binding domain in the S protein of severe acute respiratory syndrome coronavirus 2 (SARS-CoV-2) consists of an amino acid sequence having at least 95% identity with the amino acid sequence as shown in SEQ ID NO: 11.

**27.** The particle of claim 25 or 26, wherein the nucleic acid molecule capable of expressing the S protein of severe acute respiratory syndrome coronavirus 2 (SARS-CoV-2) is an mRNA molecule comprising a cap structure (Cap), 5' untranslated region (5'-UTR), S protein coding region, 3' untranslated region (3'-UTR) and a PolyA tail (PolyA).

**28.** The particle of any one of claims 20 to 24, wherein the nucleic acid molecule capable of expressing the fragment of the S protein of severe acute respiratory syndrome coronavirus 2 (SARS-CoV-2) is an mRNA molecule comprising a cap structure (Cap), 5' untranslated region (5'-UTR), a leader sequence, the coding region of the receptor-binding domain in the S protein, 3' untranslated region (3'-UTR) and a PolyA tail (PolyA).

**29.** The particle of claim 27, wherein the sequence of S protein coding region consists of a nucleotide sequence having at least 90% identity with the sequence of S protein coding region in the sequence as shown in SEQ ID NO: 5.

**30.** The particle of claim 27, wherein the sequence of S protein coding region consists of a nucleotide sequence having at least 90% identity with the sequence of S protein coding region in the sequence as shown in SEQ ID NO: 16.

**31.** The particle of claim 27, wherein the nucleic acid molecule capable of expressing the S protein of severe acute respiratory syndrome coronavirus 2 (SARS-CoV-2) consists of the nucleotide sequence as shown in SEQ ID NO: 5.

**32.** The particle of claim 27, wherein the nucleic acid molecule capable of expressing the S protein of severe acute respiratory syndrome coronavirus 2 (SARS-CoV-2) consists of the nucleotide sequence as shown in SEQ ID NO: 16.

**33.** The particle of claim 27, wherein the sequence of the coding region of the receptor-binding domain in the S protein consists of a nucleotide sequence having at least 90% identity with the sequence of the coding region of the receptor-binding domain in the S protein in the sequence as shown in SEQ ID NO: 9.

**34.** The particle of claim 27, wherein the sequence of the coding region of the receptor-binding domain in the S protein consists of a nucleotide sequence having at least 90% identity with the sequence of the coding region of the receptor-binding domain in the S protein in the sequence as shown in SEQ ID NO: 19.

**35.** The particle of claim 27, wherein the sequence of the coding region of the receptor-binding domain in the S protein consists of a nucleotide sequence having at least 90% identity with the sequence of the coding region of the receptor-binding domain in the S protein in the sequence as shown in any one of SEQ ID NOS: 21, 23, 27, 31, 35 and 66 to 79.

**36.** The particle of claim 28, wherein the nucleic acid molecule capable of expressing the fragment of the S protein of severe acute respiratory syndrome coronavirus 2 (SARS-CoV-2) consists of the nucleotide sequence as shown in SEQ ID NO: 9.

**37.** The particle of claim 28, wherein the nucleic acid molecule capable of expressing the fragment of the S protein of severe acute respiratory syndrome coronavirus 2 (SARS-CoV-2) consists of the nucleotide sequence as shown in SEQ ID NO: 19.

**38.** The particle of any one of claims 1 to 37, wherein the nucleic acid molecule comprises at least one modified nucleotide.

**39.** The particle of claim 38, wherein the modified nucleotide comprises at least one of 5-substituted pyrimidine nucleotide and/or pseudouridine optionally substituted at position 1.

**40.** The particle of claim 38, wherein the modified nucleotide comprises at least one selected from the group consisting of 5-methylcytidine, 5-methoxyuridine, 5-methyluridine, pseudouridine and 1-alkylpseudouridine.

**41.** The particle of any one of claims 1 to 40, wherein the mean particle size is 30 nm to 300 nm.

**42.** Use of the particle of any one of claims 1 to 41 for manufacturing a composition for preventing and/or treating infections with severe acute respiratory syndrome coronavirus 2 (SARS-CoV-2).

**43.** A composition comprising the particle of any one of claims 1 to 41.

**44.** The composition of claim 43 for allowing the expression of the S protein and/or a fragment thereof of severe acute respiratory syndrome coronavirus 2 (SARS-CoV-2) *in vivo* or *in vitro.*

**45.** The composition of claim 43 or 44 for use as a pharmaceutical drug.

**46.** The composition of claim 45 for inducing immune responses to severe acute respiratory syndrome coronavirus 2 (SARS-CoV-2).

**47.** The composition of claim 45 or 46 for preventing and/or treating infections with severe acute respiratory syndrome coronavirus 2 (SARS-CoV-2).

**48.** A method of expressing the S protein and/or a fragment thereof of severe acute respiratory syndrome coronavirus 2 (SARS-CoV-2) *in vitro,* comprising introducing into cells the composition of claim 43 or 44.

**49.** A method of expressing the S protein and/or a fragment thereof of severe acute respiratory syndrome coronavirus 2 (SARS-CoV-2) *in vivo,* comprising administering to a mammal the composition of any one of claims 43 to 47.

**50.** A method of inducing immune response to severe acute respiratory syndrome coronavirus 2 (SARS-CoV-2), comprising administering to a mammal the composition of claim 45 or 46.

**51.** A method of preventing and/or treating infections with severe acute respiratory syndrome coronavirus 2 (SARS-CoV-2), comprising administering to a mammal the composition of any one of claims 45 to 47.

**52.** The method of any one of claims 49 to 51, wherein the mammal is human.

[Fig. 1]

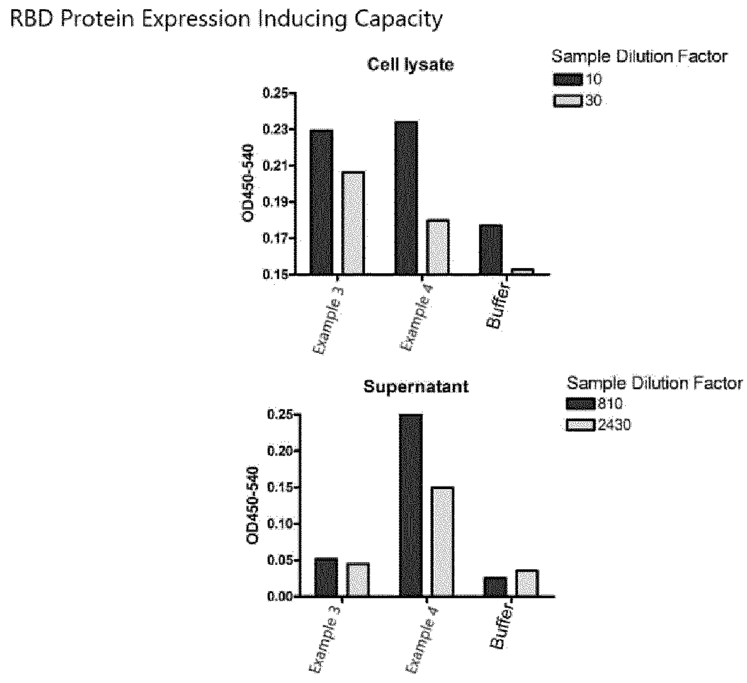
RBD Protein Expression Inducing Capacity
Cell lysate
Sample Dilution Factor
10
30
OD450-540
0.25
0.23
0.21
0.19
0.17
0.15
Example 3
Example 4
Buffer
Supernatant
Sample Dilution Factor
810
2430
OD450-540
0.25
0.20
0.15
0.10
0.05
0.00
Example 3
Example 4
Buffer

[Fig. 2]

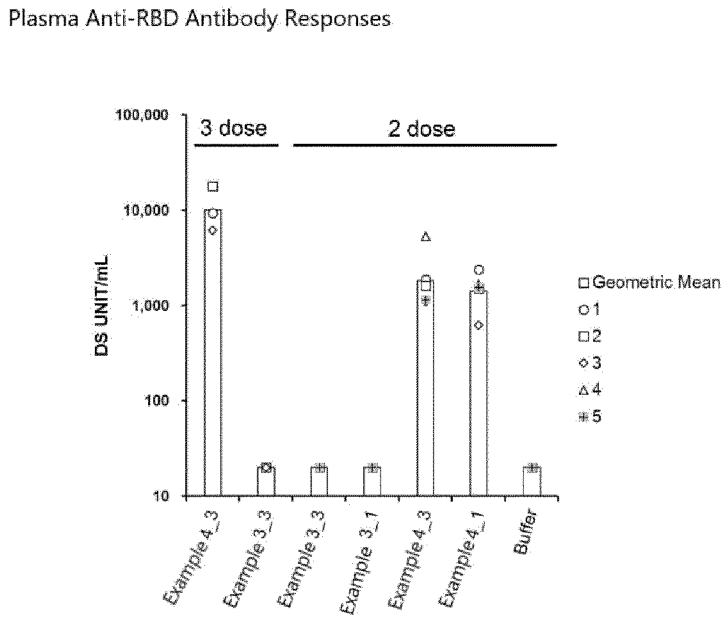
Plasma Anti-RBD Antibody Responses
3 dose
2 dose
DS UNIT/mL
100,000
10,000
1,000
100
10
Geometric Mean
1
2
3
4
5
Example4_3
Example 3_3
Example 3_3
Example 3_1
Example 4_3
Example4_1
Buffer

[Fig. 3]

RBD-hACE2 Binding Inhibitory Activity

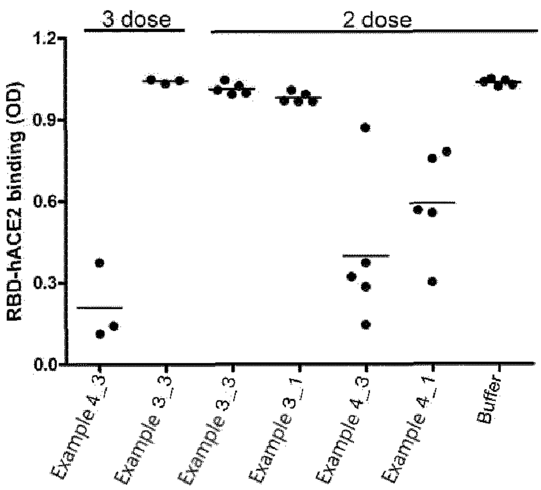

[Fig. 4]

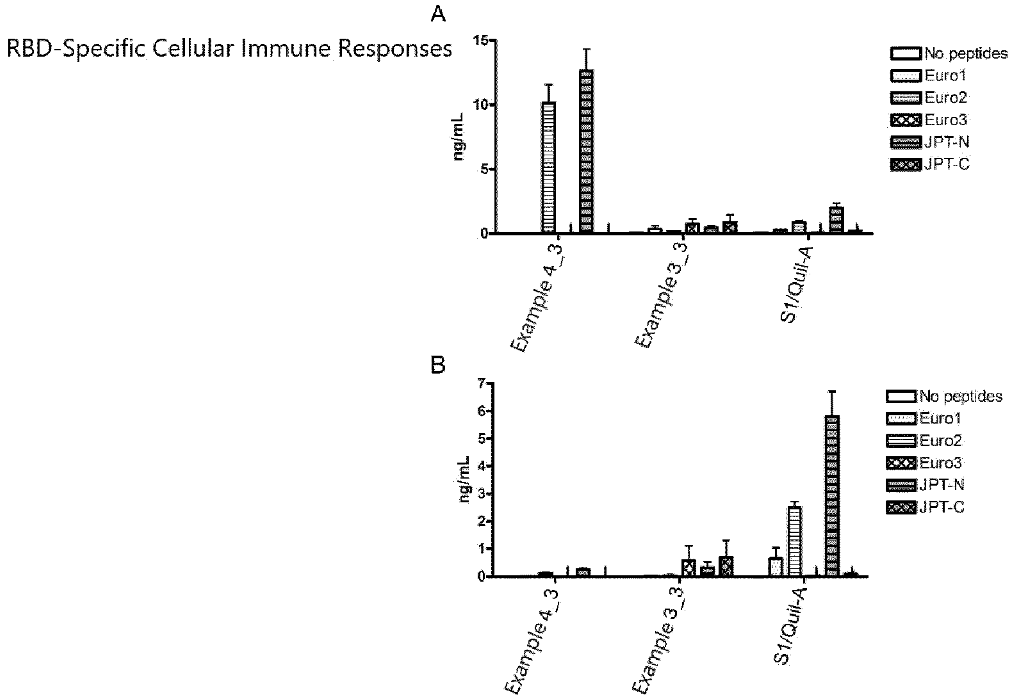
A
RBD-Specific Cellular Immune Responses
ng/mL
15
10
5
0
Example4_3
Example3_3
S1/Quil-A
No peptides
Euro1
Euro2
Euro3
JPT-N
JPT-C
B
ng/mL
7
6
5
4
3
2
1
0
Example4_3
Example3_3
S1/Quil-A
No peptides
Euro1
Euro2
Euro3
JPT-N
JPT-C

[Fig. 5]

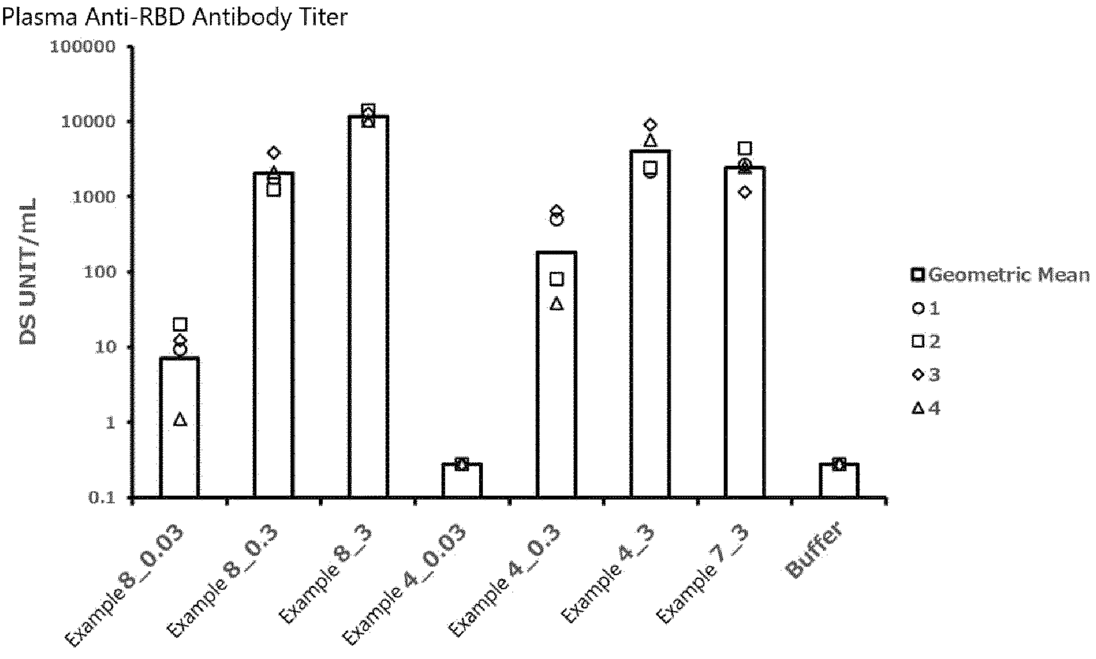
Plasma Anti-RBD Antibody Titer
DS UNIT/mL
100000
10000
1000
100
10
1
0.1
Example8_0.03
Example8_0.3
Example8_3
Example4_0.03
Example4_0.3
Example4_3
Example7_3
Buffer
Geometric Mean
1
2
3
4

[Fig. 6]

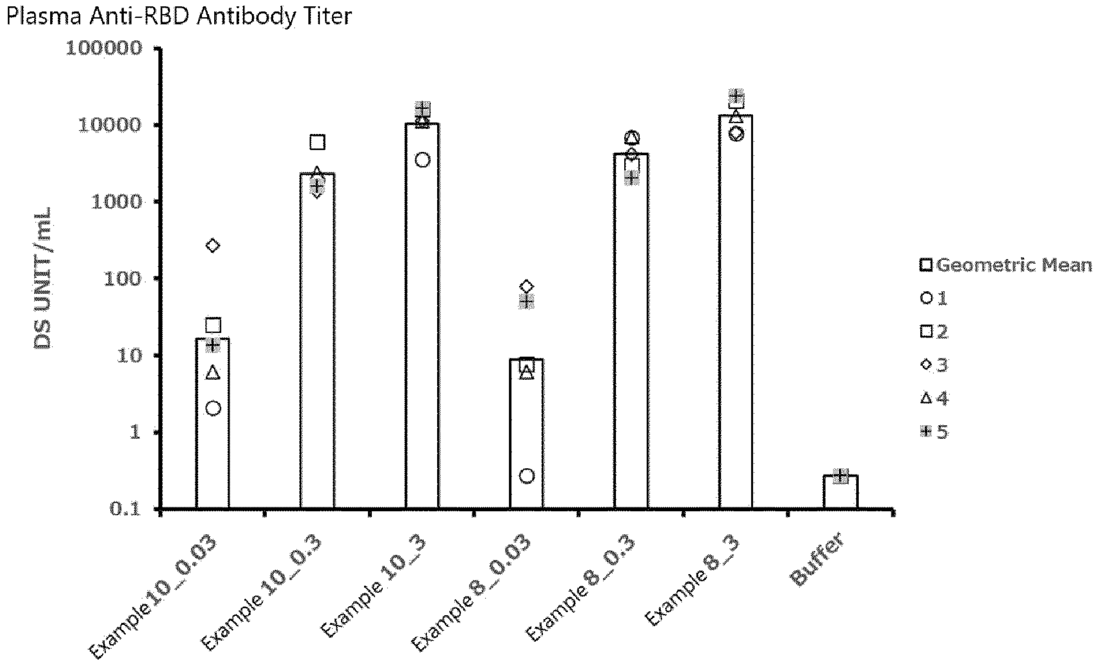
Plasma Anti-RBD Antibody Titer
100000
10000
1000
100
10
1
0.1
DS UNIT/mL
Example10_0.03
Example10_0.3
Example10_3
Example8_0.03
Example8_0.3
Example8_3
Buffer
Geometric Mean
1
2
3
4
5

[Fig. 7]

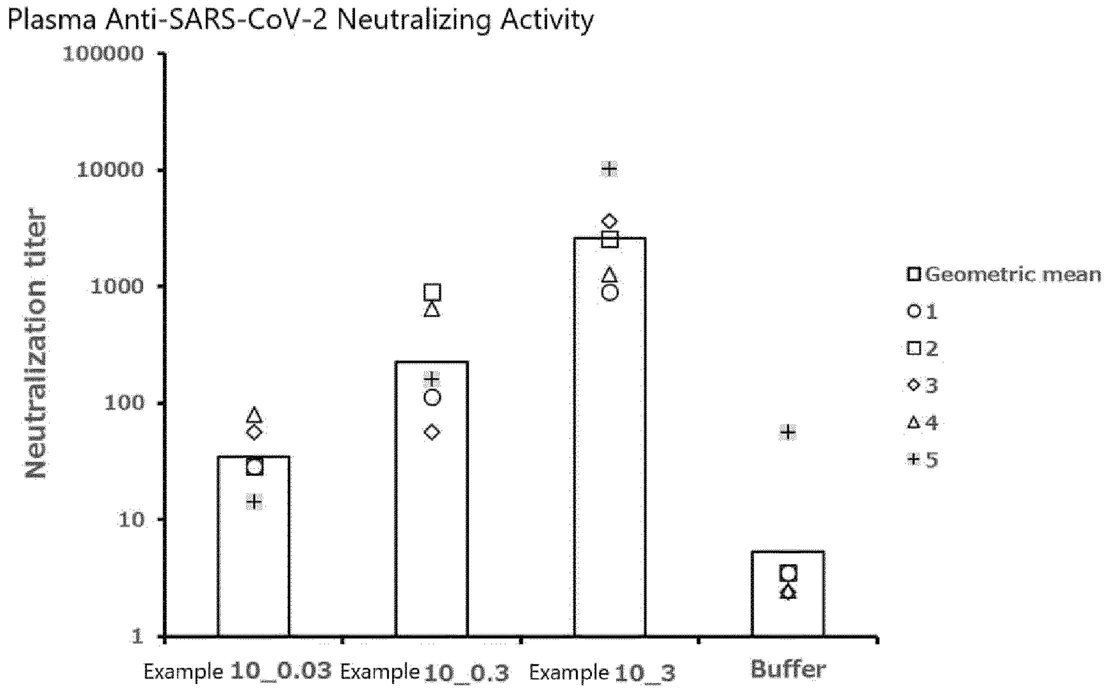
Plasma Anti-SARS-CoV-2 Neutralizing Activity
Neutralization titer
100000
10000
1000
100
10
1
Example 10_0.03
Example 10_0.3
Example 10_3
Buffer
Geometric mean
1
2
3
4
5

[Fig. 8]

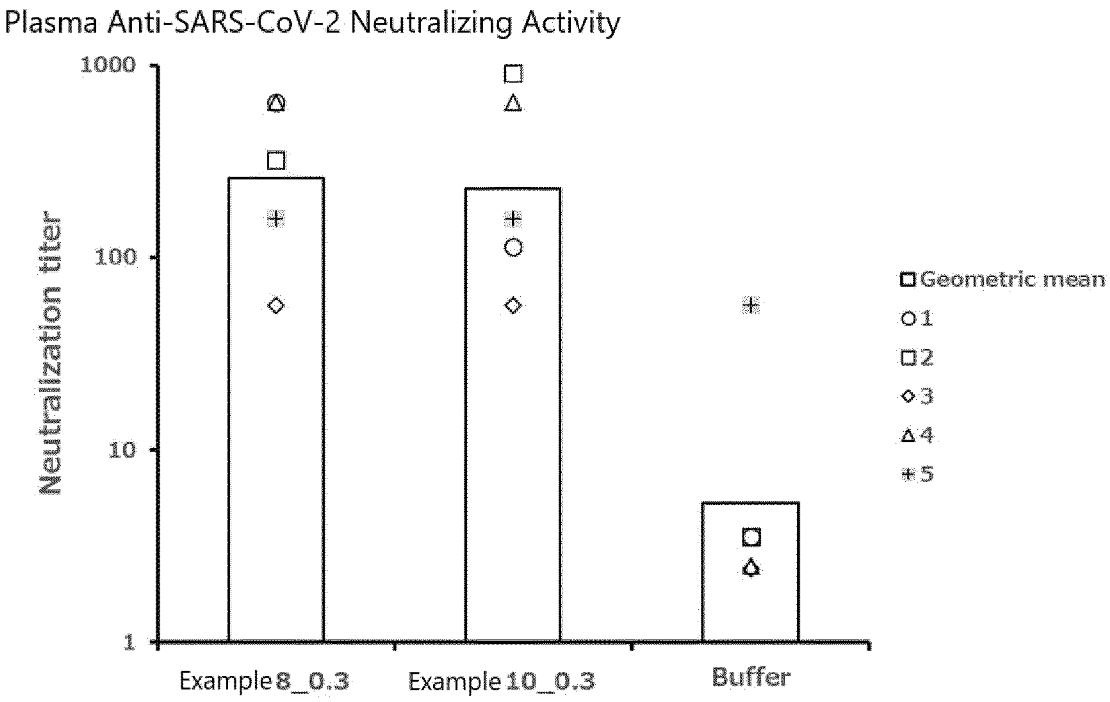
Plasma Anti-SARS-CoV-2 Neutralizing Activity
Neutralization titer
1000
100
10
1
Example 8_0.3
Example 10_0.3
Buffer
Geometric mean
1
2
3
4
5

[Fig. 9]

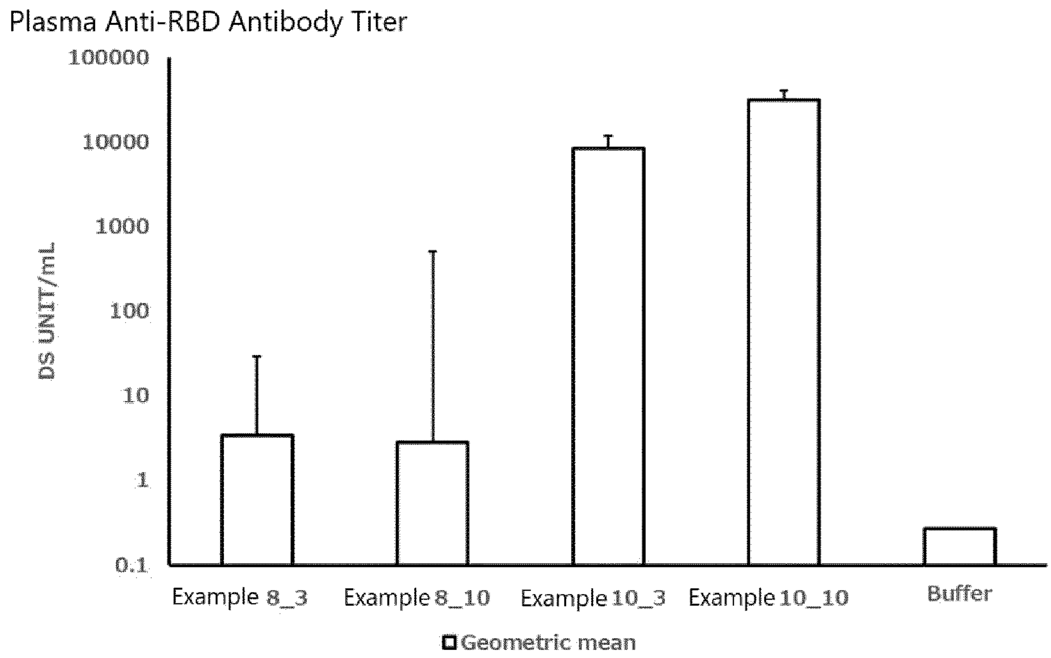
Plasma Anti-RBD Antibody Titer
100000
10000
1000
100
10
1
0.1
DS UNIT/mL
Example 8_3
Example 8_10
Example 10_3
Example 10_10
Buffer
Geometric mean

[Fig. 10]

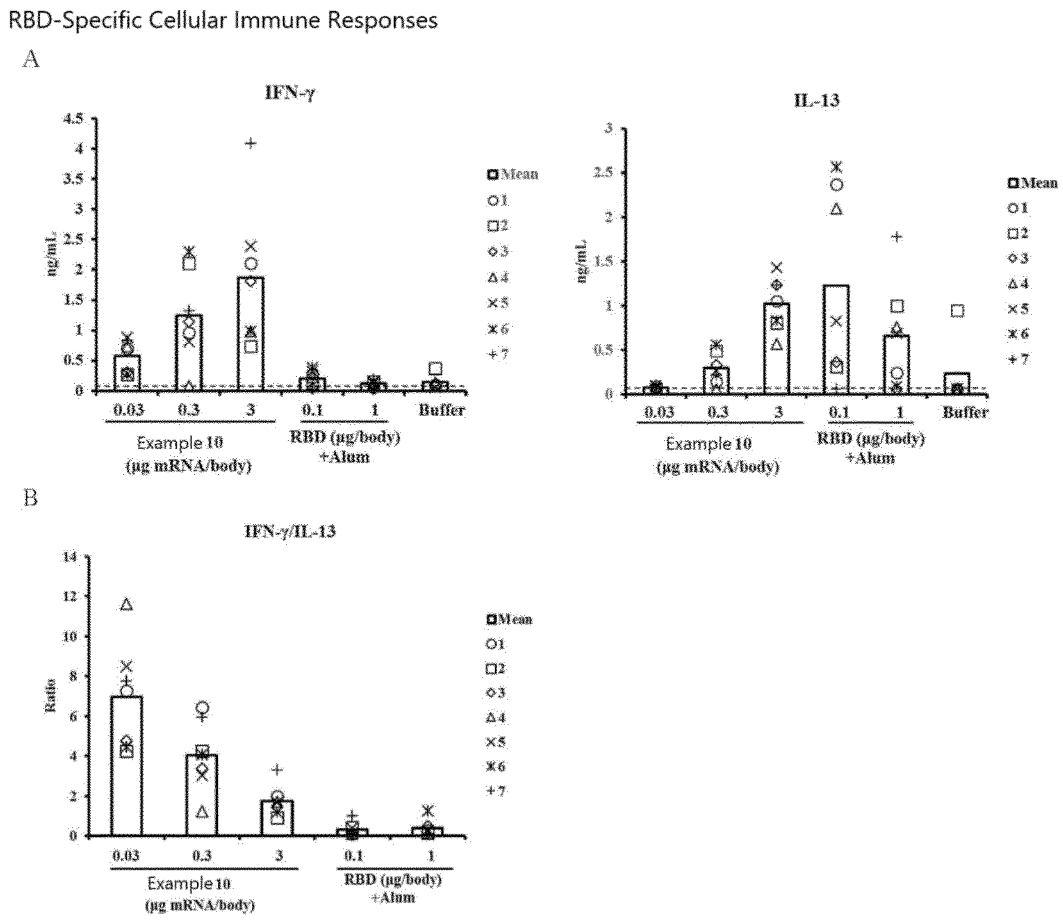
RBD-Specific Cellular Immune Responses
A
IFN-γ
ng/mL
0.03
0.3
3
0.1
1
Buffer
Example 10
(μg mRNA/body)
RBD (μg/body)
+Alum
Mean
1
2
3
4
5
6
7
IL-13
B
IFN-γ/IL-13
Ratio

[Fig. 11]

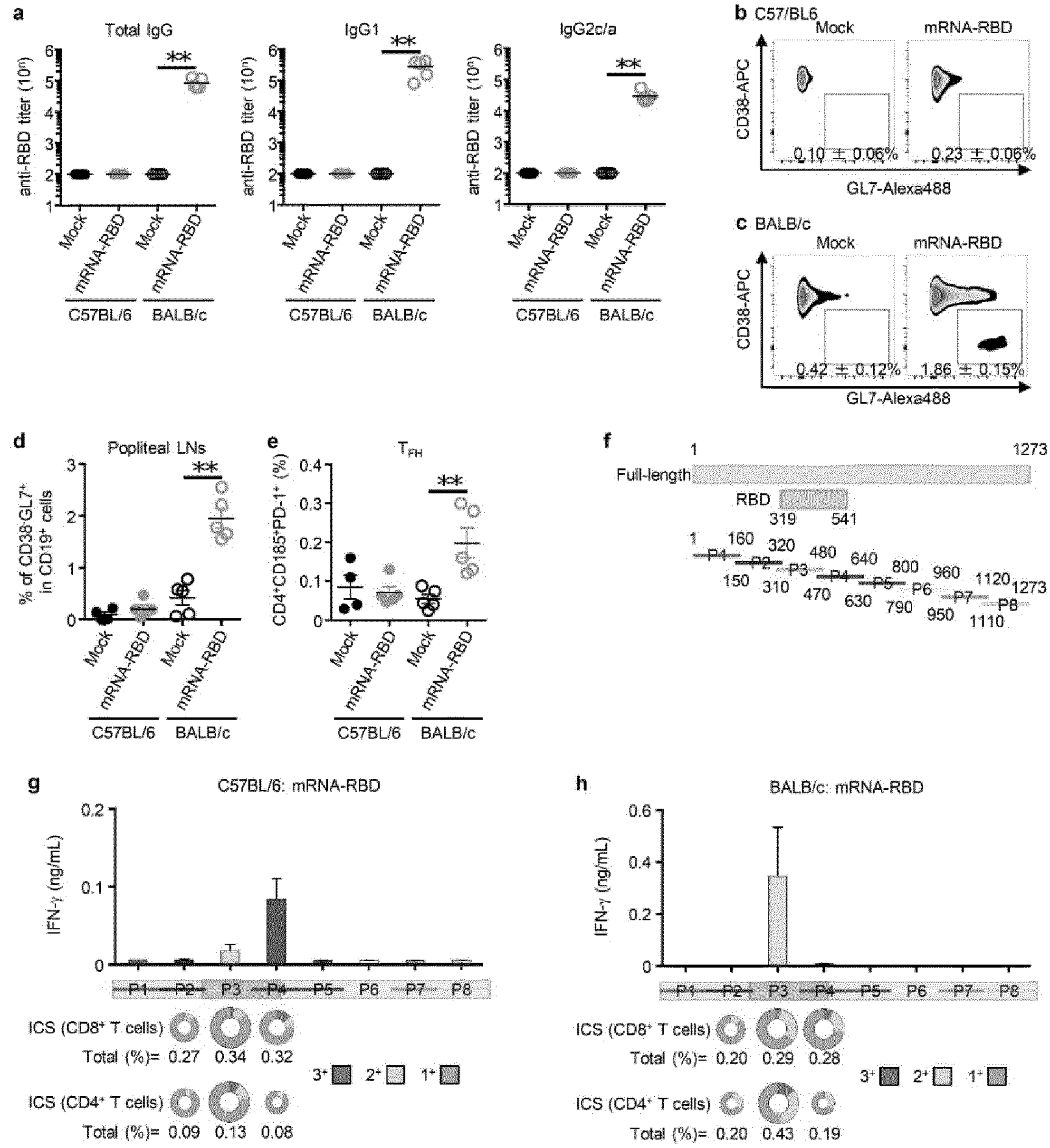
a
Total IgG
IgG1
IgG2c/a
anti-RBD titer (10ⁿ)
Mock
mRNA-RBD
C57BL/6
BALB/c
b C57/BL6
Mock
mRNA-RBD
CD38-APC
GL7-Alexa488
0.10 ± 0.06%
0.23 ± 0.06%
c BALB/c
0.42 ± 0.12%
1.86 ± 0.15%
d
Popliteal LNs
% of CD38⁻GL7⁺ in CD19⁺ cells
e
T_FH
CD4⁺CD185⁺PD-1⁺ (%)
f
Full-length
RBD
1
1273
319
541
P1
P2
P3
P4
P5
P6
P7
P8
g
C57BL/6: mRNA-RBD
IFN-γ (ng/mL)
ICS (CD8⁺ T cells)
Total (%)= 0.27 0.34 0.32
ICS (CD4⁺ T cells)
Total (%)= 0.09 0.13 0.08
3⁺ 2⁺ 1⁺
h
BALB/c: mRNA-RBD
ICS (CD8⁺ T cells)
Total (%)= 0.20 0.29 0.28
ICS (CD4⁺ T cells)
Total (%)= 0.20 0.43 0.19

[Fig. 12]

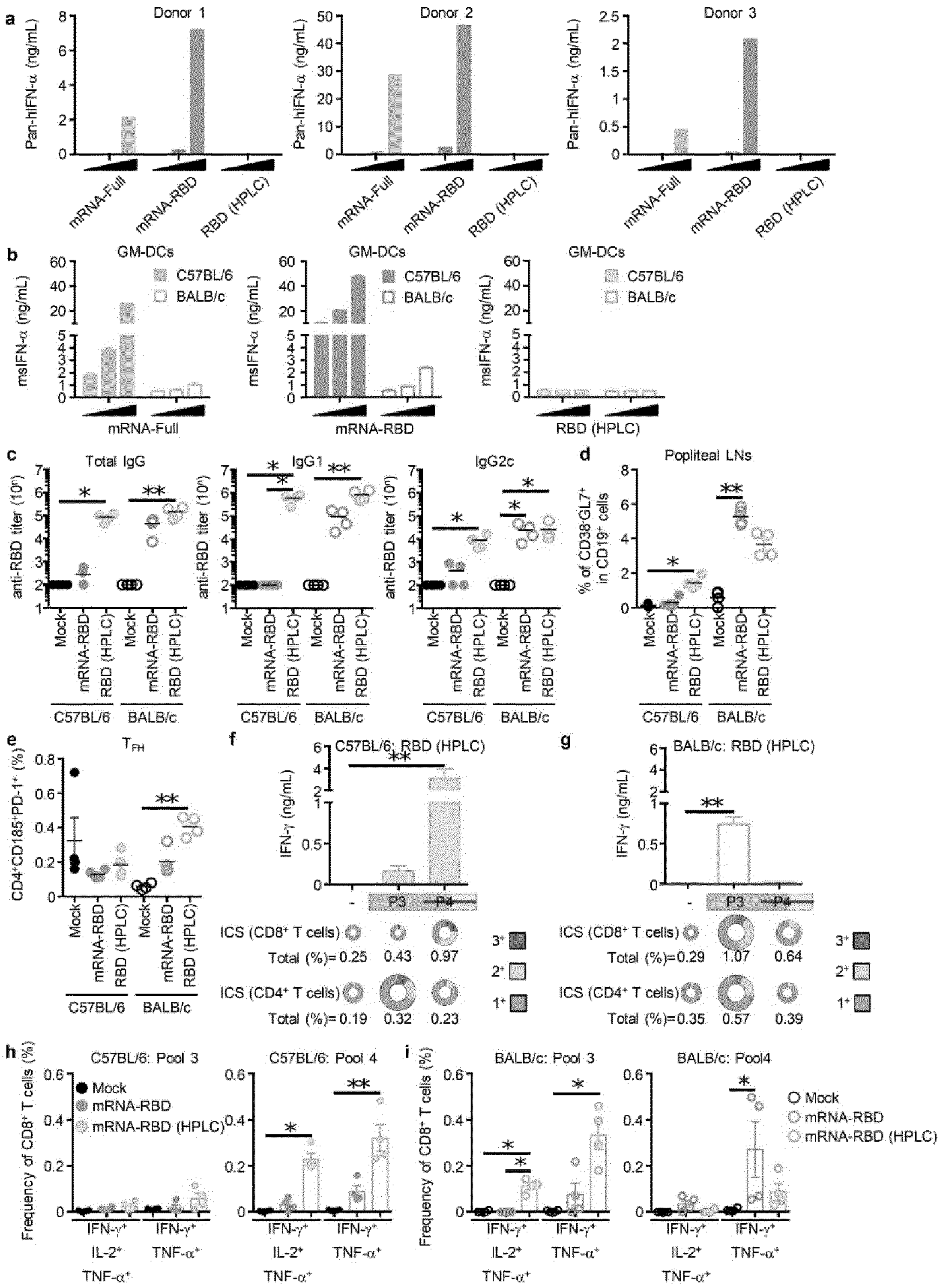
a
Donor 1
Donor 2
Donor 3
Pan-hIFN-α (ng/mL)
mRNA-Full
mRNA-RBD
RBD (HPLC)
b
GM-DCs
C57BL/6
BALB/c
msIFN-α (ng/mL)
mRNA-Full
mRNA-RBD
RBD (HPLC)
c
Total IgG
IgG1
IgG2c
anti-RBD titer (10ⁿ)
Mock
mRNA-RBD
RBD (HPLC)
C57BL/6
BALB/c
d
Popliteal LNs
% of CD38⁻GL7⁺ in CD19⁺ cells
e
T_FH
CD4⁺CD185⁺PD-1⁺ (%)
f
C57BL/6: RBD (HPLC)
IFN-γ (ng/mL)
P3
P4
ICS (CD8⁺ T cells)
Total (%)= 0.25 0.43 0.97
ICS (CD4⁺ T cells)
Total (%)= 0.19 0.32 0.23
3⁺
2⁺
1⁺
g
BALB/c: RBD (HPLC)
ICS (CD8⁺ T cells)
Total (%)=0.29 1.07 0.64
ICS (CD4⁺ T cells)
Total (%)=0.35 0.57 0.39
h
C57BL/6: Pool 3
C57BL/6: Pool 4
Frequency of CD8⁺ T cells (%)
Mock
mRNA-RBD
mRNA-RBD (HPLC)
IFN-γ⁺ IL-2⁺ TNF-α⁺
IFN-γ⁺ TNF-α⁺
i
BALB/c: Pool 3
BALB/c: Pool4

[Fig. 13]

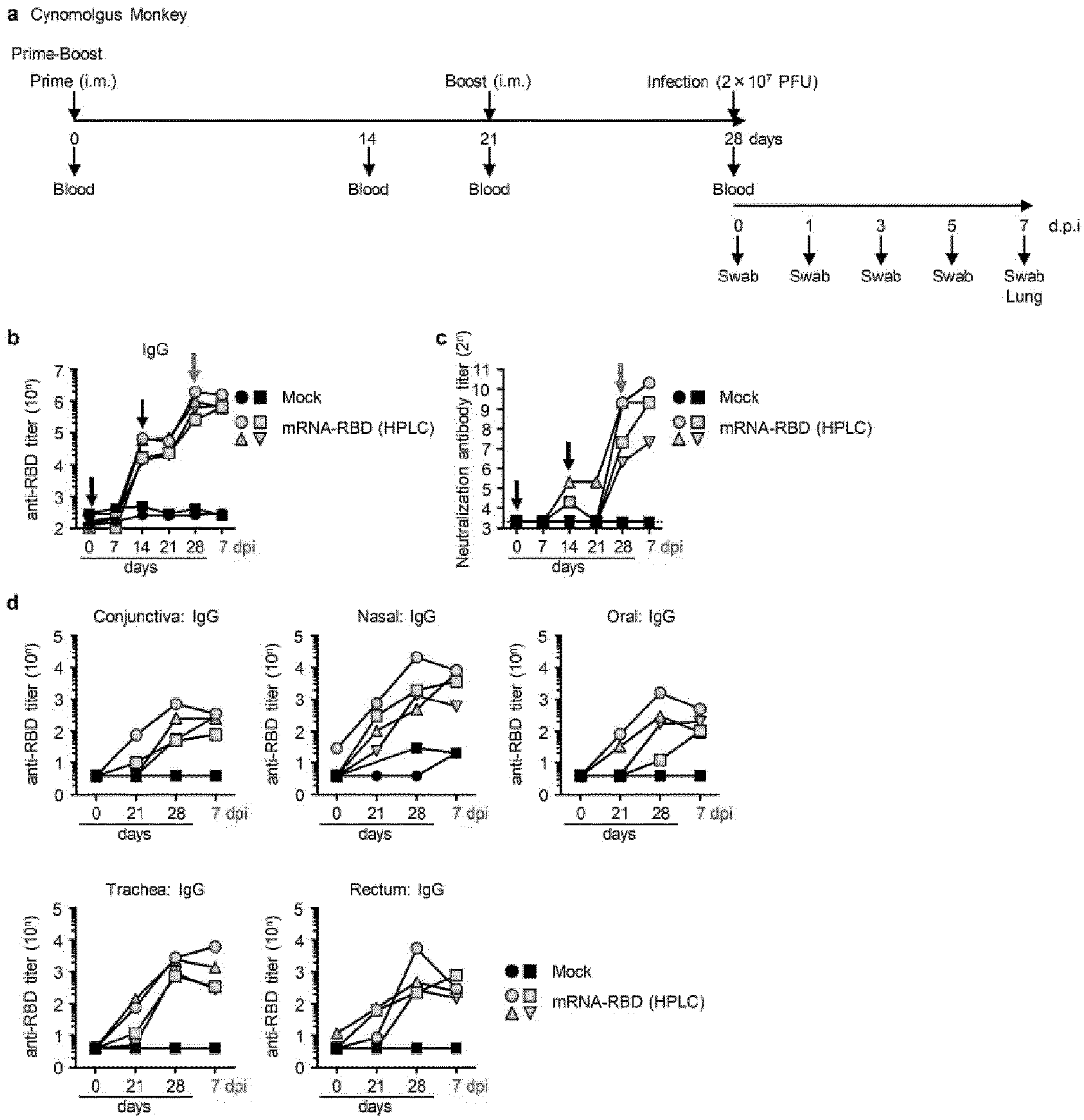
a Cynomolgus Monkey
Prime-Boost
Prime (i.m.)
Boost (i.m.)
Infection (2 × 10^7 PFU)
0
14
21
28 days
Blood
Blood
Blood
Blood
0
1
3
5
7
d.p.i
Swab
Swab
Swab
Swab
Swab
Lung
b
IgG
anti-RBD titer (10^n)
days
7 dpi
Mock
mRNA-RBD (HPLC)
c
Neutralization antibody titer (2^n)
days
7 dpi
Mock
mRNA-RBD (HPLC)
d
Conjunctiva: IgG
Nasal: IgG
Oral: IgG
Trachea: IgG
Rectum: IgG
anti-RBD titer (10^n)
days
7 dpi
Mock
mRNA-RBD (HPLC)

[Fig. 14]

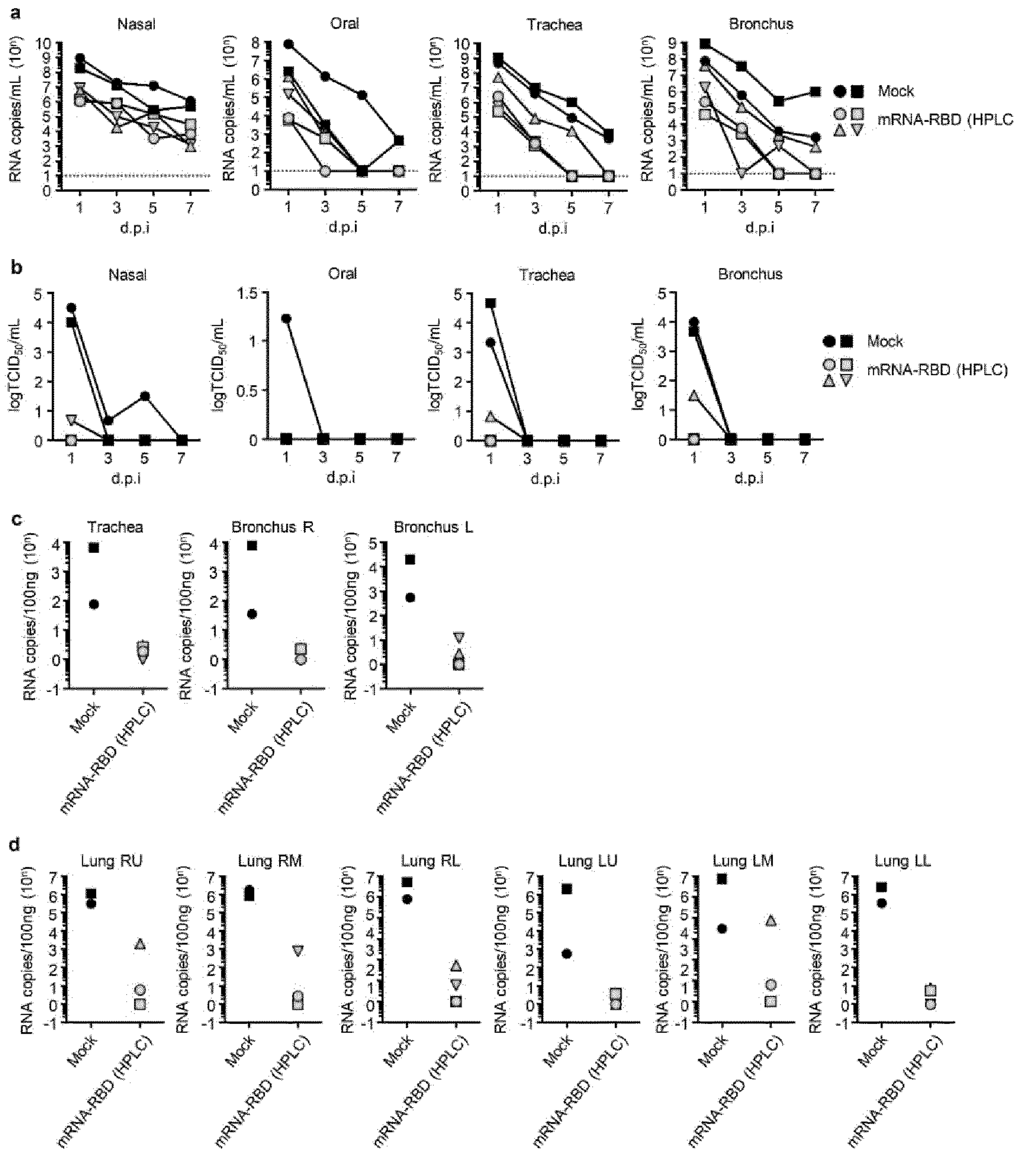
a
Nasal
Oral
Trachea
Bronchus
RNA copies/mL (10ⁿ)
d.p.i
Mock
mRNA-RBD (HPLC
b
Nasal
Oral
Trachea
Bronchus
logTCID50/mL
d.p.i
Mock
mRNA-RBD (HPLC)
c
Trachea
Bronchus R
Bronchus L
RNA copies/100ng (10ⁿ)
Mock
mRNA-RBD (HPLC)
d
Lung RU
Lung RM
Lung RL
Lung LU
Lung LM
Lung LL
RNA copies/100ng (10ⁿ)
Mock
mRNA-RBD (HPLC)

[Fig. 15]

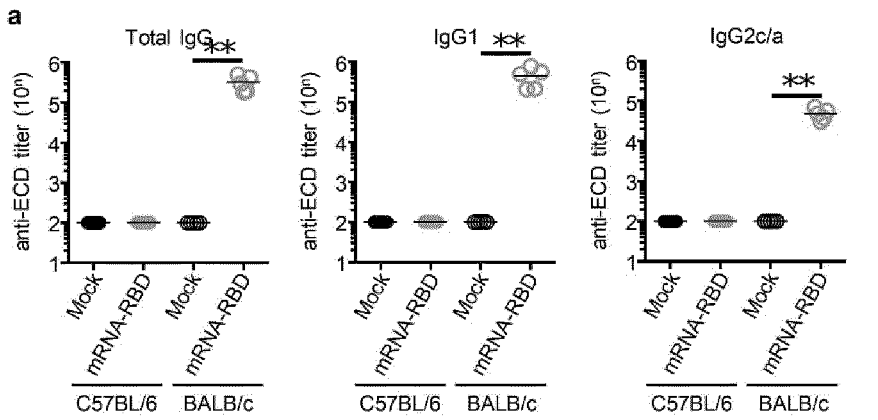
a
Total IgG
IgG1
IgG2c/a
**
anti-ECD titer (10^n)
Mock
mRNA-RBD
C57BL/6
BALB/c

[Fig. 16]

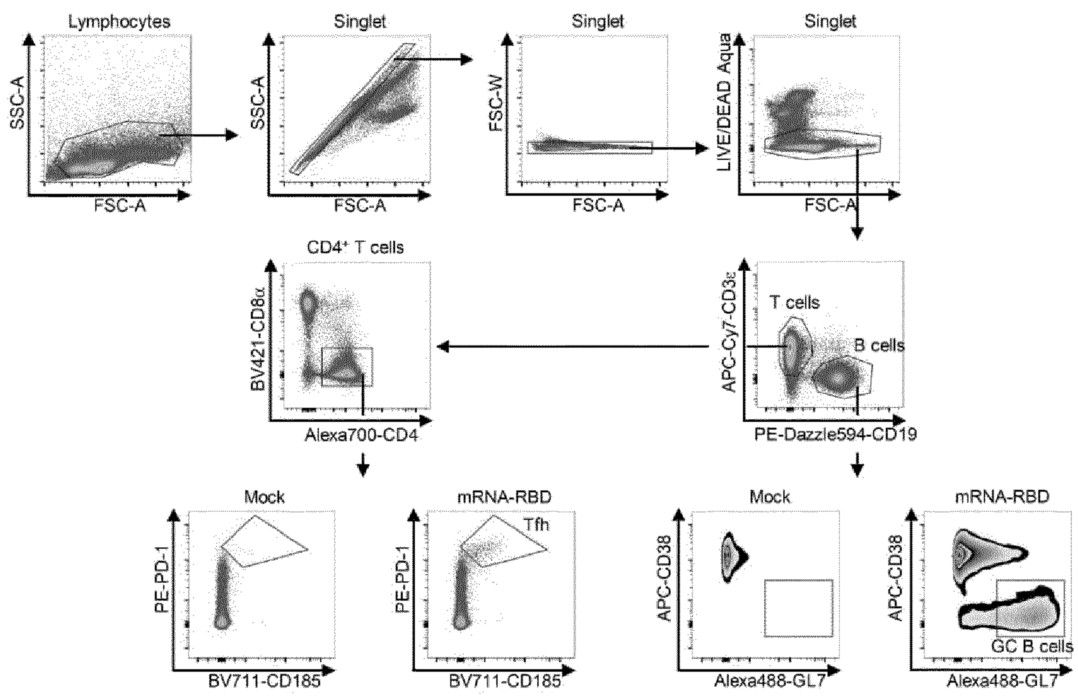
Lymphocytes
Singlet
SSC-A
FSC-A
FSC-W
LIVE/DEAD Aqua
CD4+ T cells
BV421-CD8α
Alexa700-CD4
APC-Cy7-CD3ε
T cells
B cells
PE-Dazzle594-CD19
Mock
mRNA-RBD
Tfh
PE-PD-1
BV711-CD185
APC-CD38
Alexa488-GL7
GC B cells

[Fig. 17]

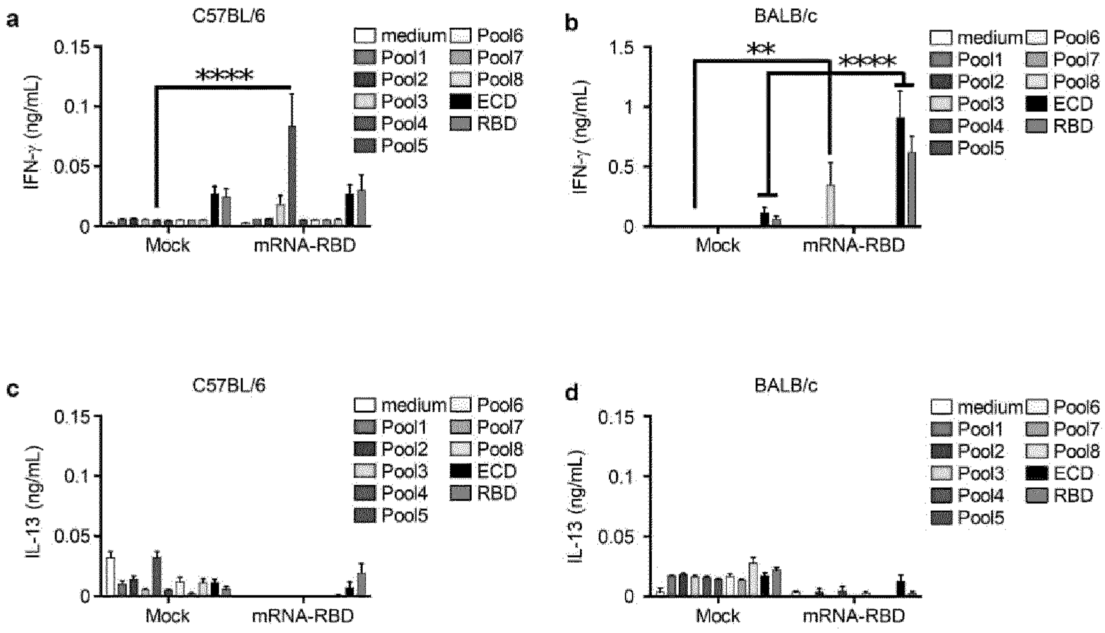
a
C57BL/6
IFN-γ (ng/mL)
0.15
0.1
0.05
0
****
Mock
mRNA-RBD
medium
Pool1
Pool2
Pool3
Pool4
Pool5
Pool6
Pool7
Pool8
ECD
RBD
b
BALB/c
IFN-γ (ng/mL)
1.5
1
0.5
0
**
****
c
C57BL/6
IL-13 (ng/mL)
d
BALB/c
IL-13 (ng/mL)

[Fig. 18]

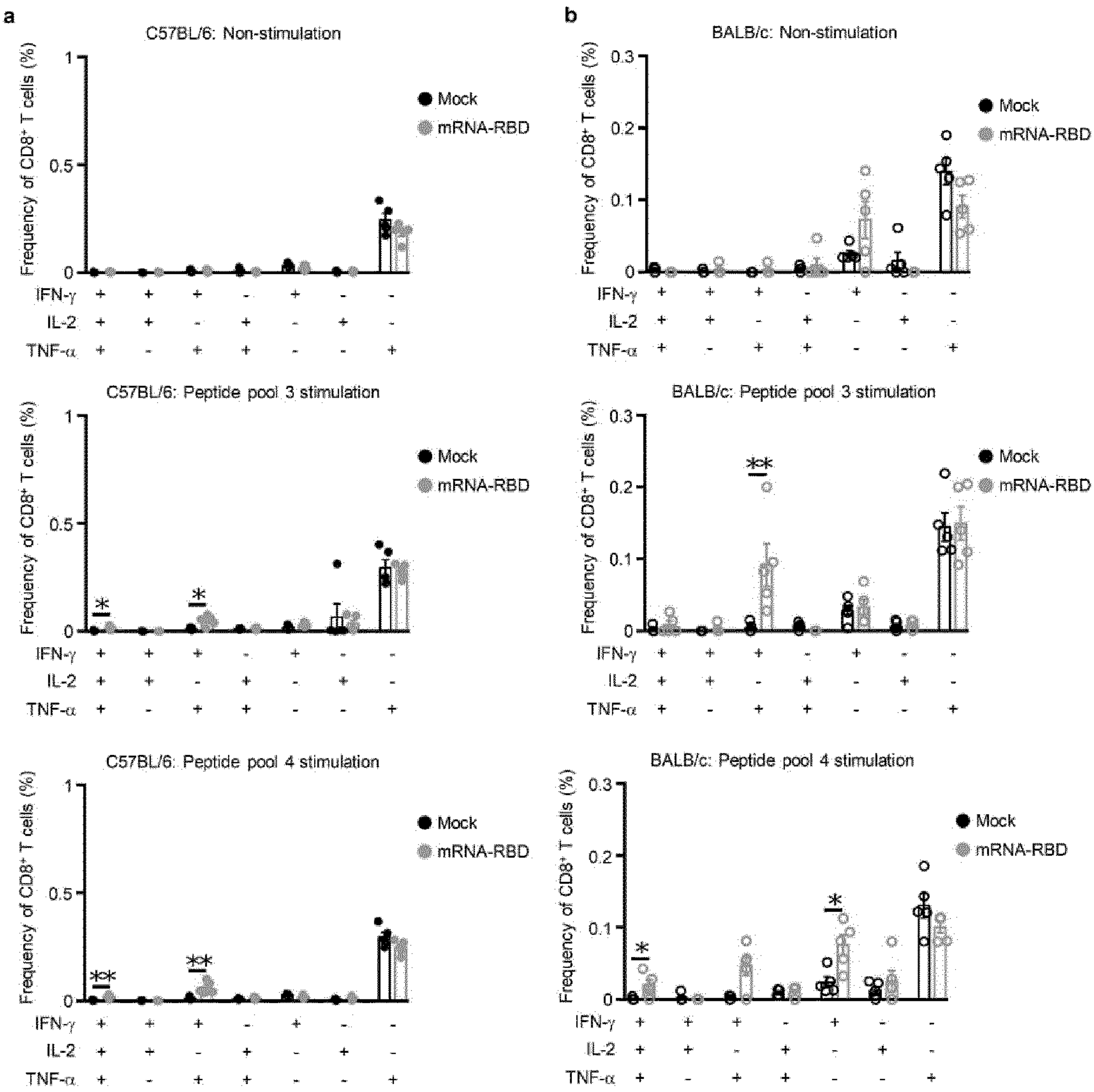
a
C57BL/6: Non-stimulation
C57BL/6: Peptide pool 3 stimulation
C57BL/6: Peptide pool 4 stimulation
b
BALB/c: Non-stimulation
BALB/c: Peptide pool 3 stimulation
BALB/c: Peptide pool 4 stimulation
Frequency of CD8⁺ T cells (%)
Mock
mRNA-RBD
IFN-γ
IL-2
TNF-α

[Fig. 19]

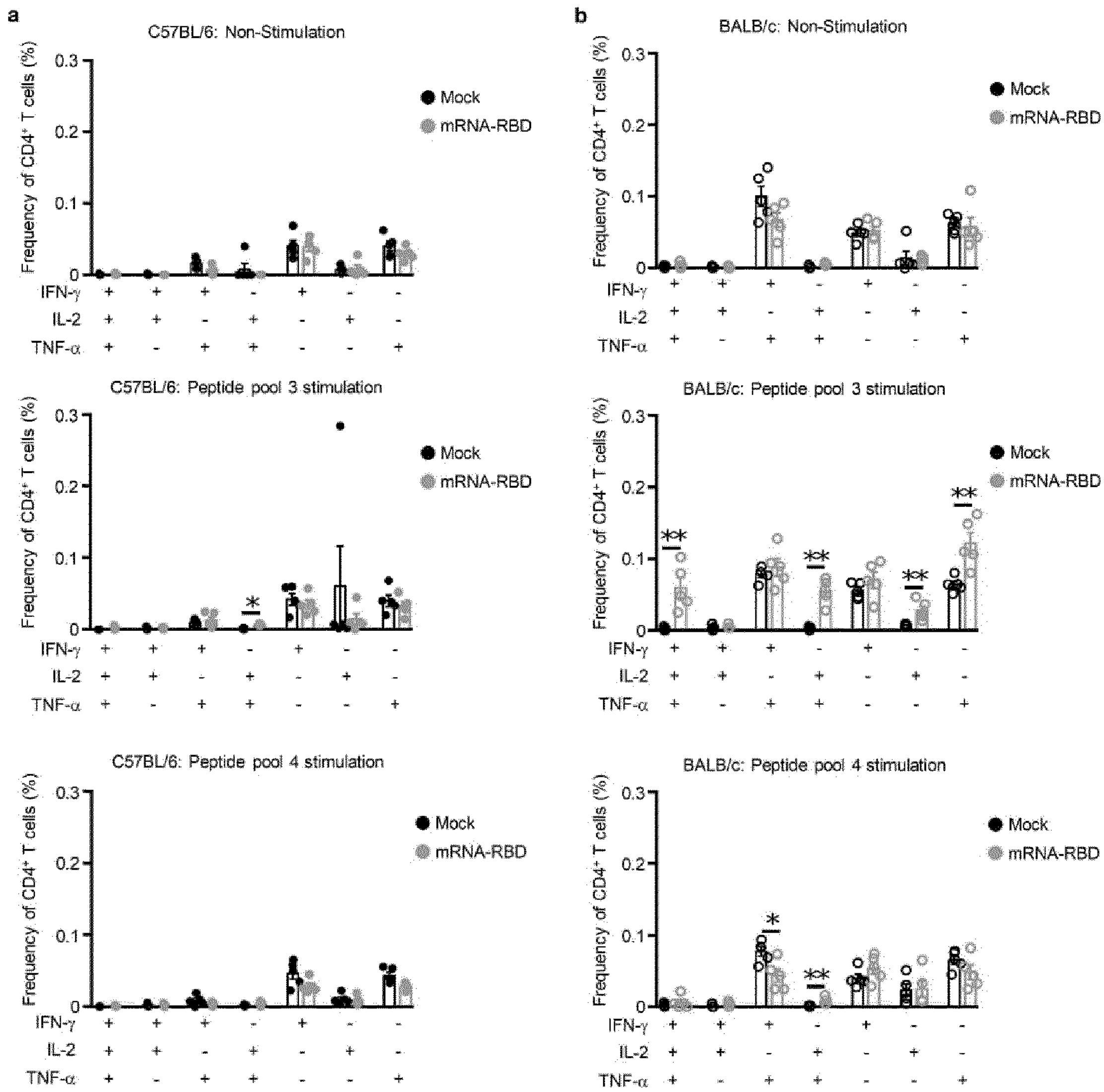
a
C57BL/6: Non-Stimulation
C57BL/6: Peptide pool 3 stimulation
C57BL/6: Peptide pool 4 stimulation
b
BALB/c: Non-Stimulation
BALB/c: Peptide pool 3 stimulation
BALB/c: Peptide pool 4 stimulation
Frequency of CD4⁺ T cells (%)
Mock
mRNA-RBD
IFN-γ
IL-2
TNF-α

[Fig. 20]

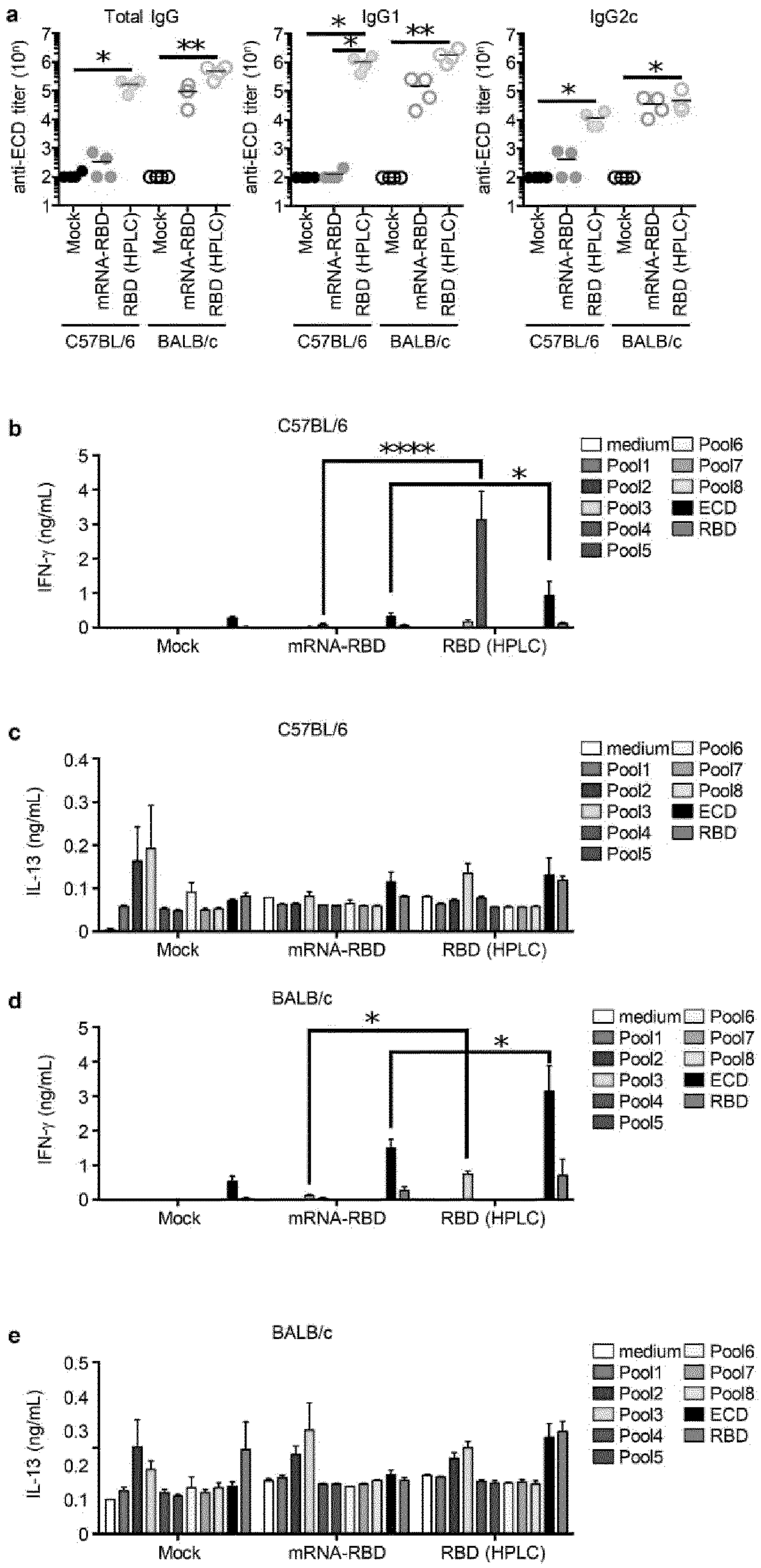
a
Total IgG
IgG1
IgG2c
anti-ECD titer (10ⁿ)
Mock
mRNA-RBD
RBD (HPLC)
C57BL/6
BALB/c
b
C57BL/6
IFN-γ (ng/mL)
medium
Pool1
Pool2
Pool3
Pool4
Pool5
Pool6
Pool7
Pool8
ECD
RBD
c
C57BL/6
IL-13 (ng/mL)
d
BALB/c
IFN-γ (ng/mL)
e
BALB/c
IL-13 (ng/mL)

[Fig. 21]

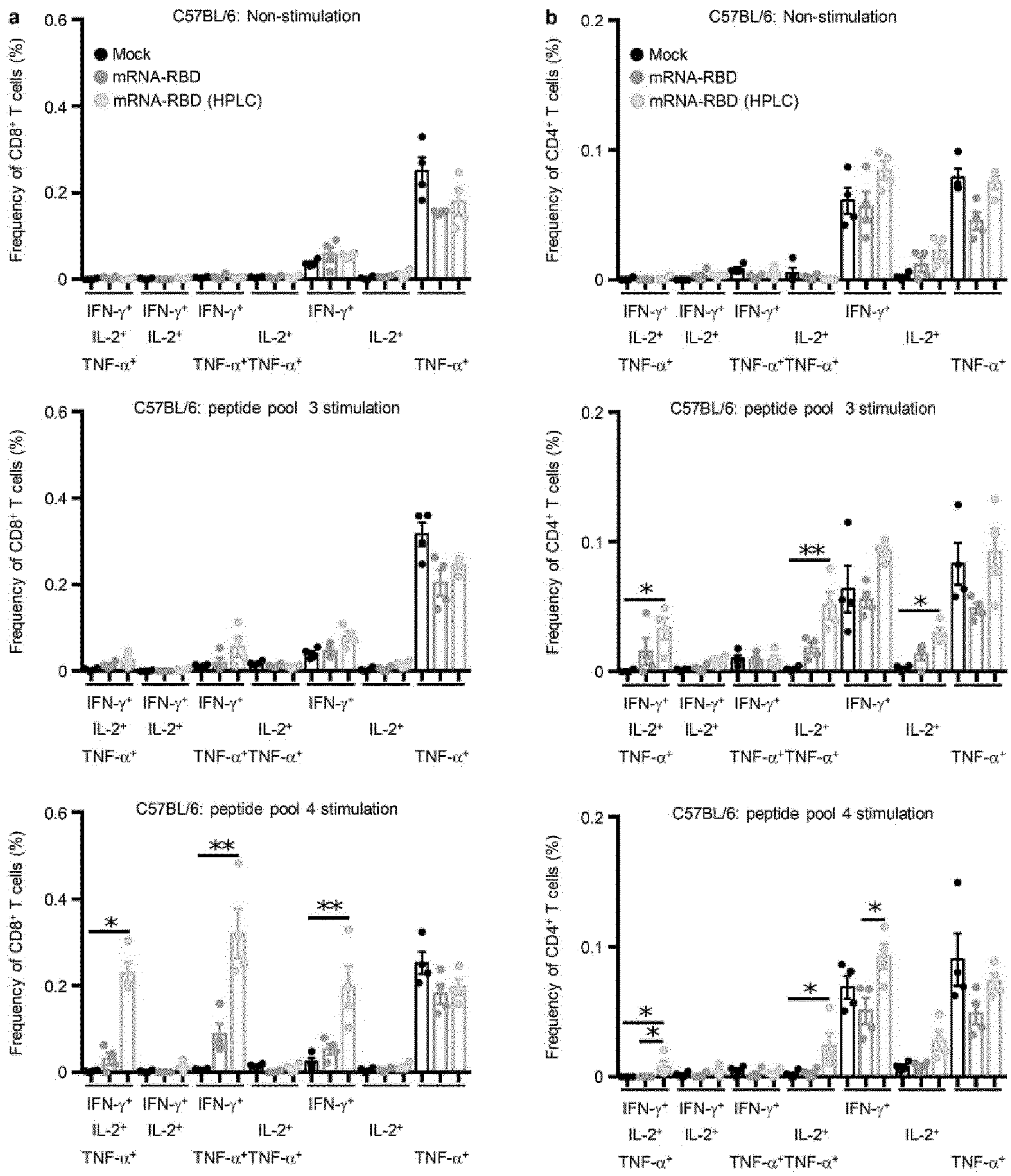
a
b
C57BL/6: Non-stimulation
C57BL/6: peptide pool 3 stimulation
C57BL/6: peptide pool 4 stimulation
Mock
mRNA-RBD
mRNA-RBD (HPLC)
Frequency of CD8+ T cells (%)
Frequency of CD4+ T cells (%)
IFN-γ+ IL-2+ TNF-α+
IFN-γ+ IL-2+
IFN-γ+ TNF-α+
IL-2+ TNF-α+
IFN-γ+
IL-2+
TNF-α+

[Fig. 22]

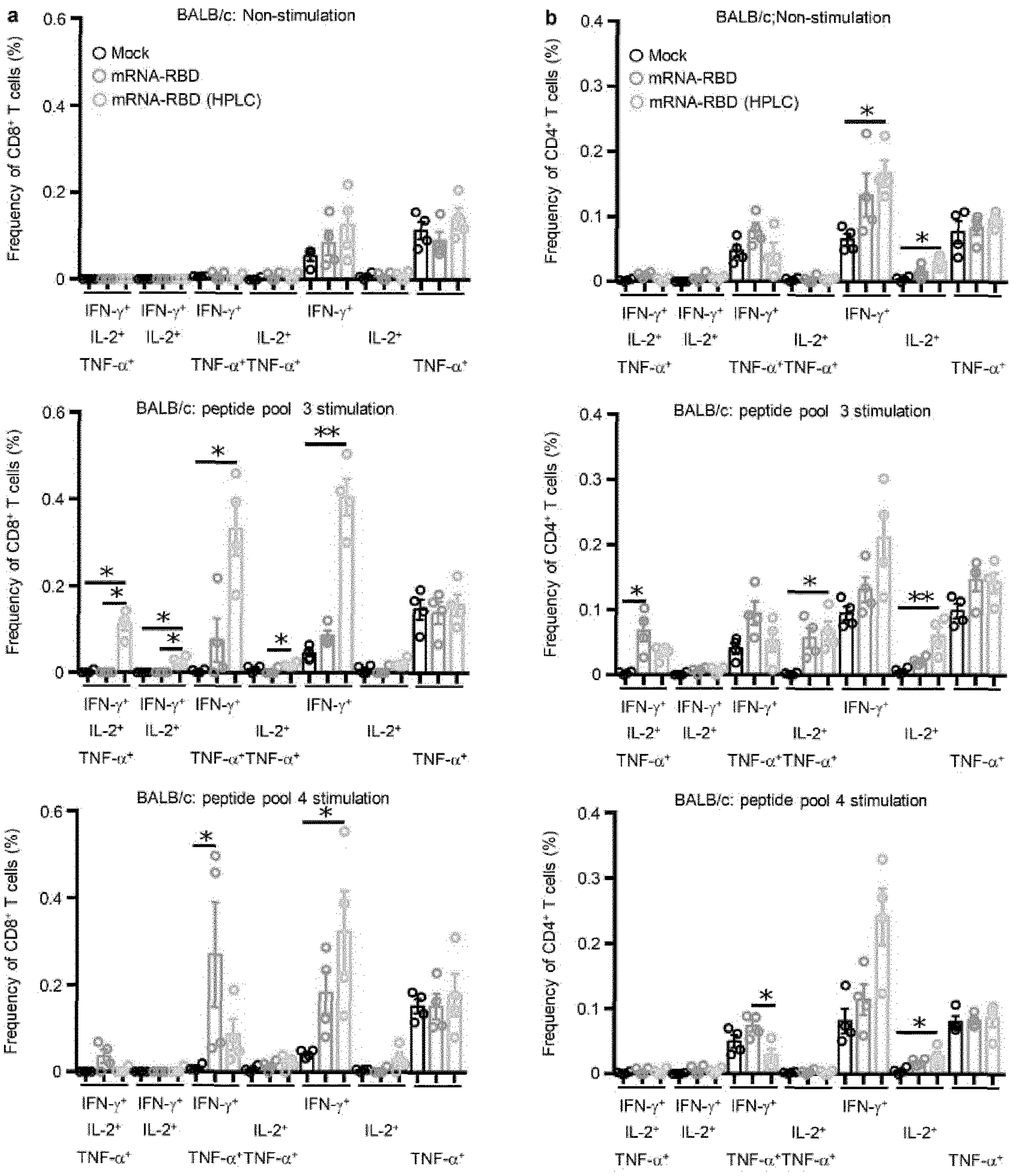
a
BALB/c: Non-stimulation
Frequency of CD8+ T cells (%)
Mock
mRNA-RBD
mRNA-RBD (HPLC)
IFN-γ+ IL-2+ TNF-α+
IFN-γ+ IL-2+
IFN-γ+ TNF-α+
IL-2+ TNF-α+
IFN-γ+
IL-2+
TNF-α+
b
BALB/c;Non-stimulation
Frequency of CD4+ T cells (%)
BALB/c: peptide pool 3 stimulation
BALB/c: peptide pool 4 stimulation

[Fig. 23]

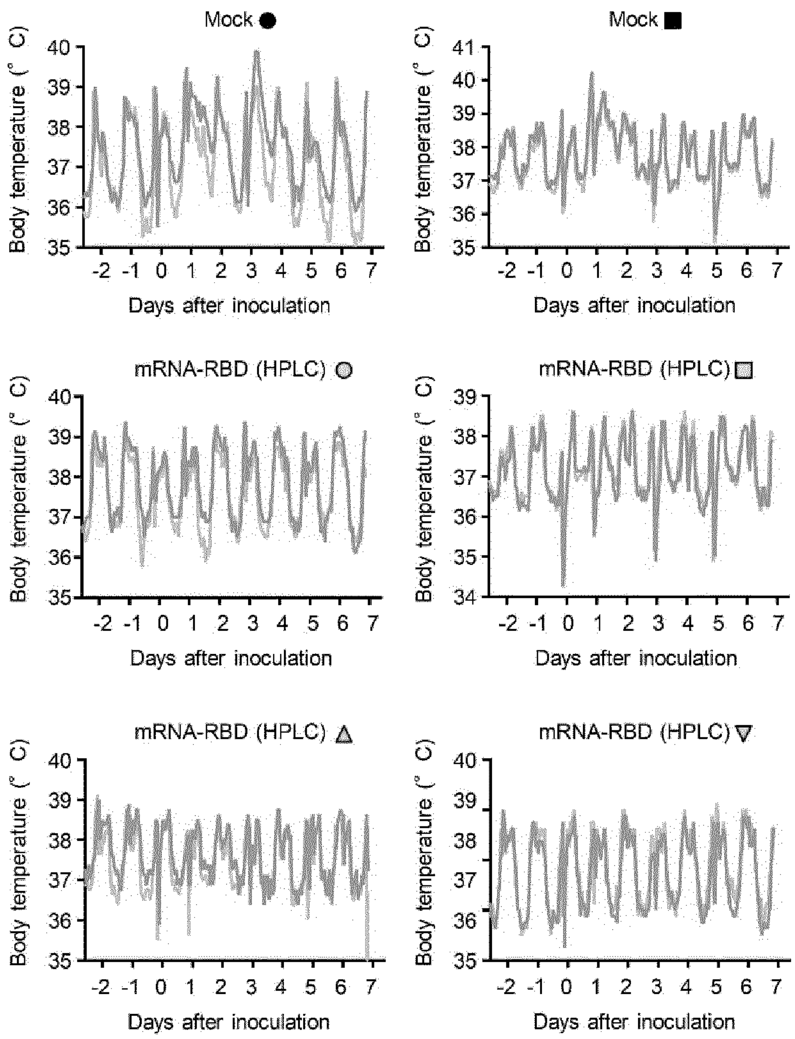
Mock ●
Body temperature (° C)
40
39
38
37
36
35
-2 -1 0 1 2 3 4 5 6 7
Days after inoculation
Mock ■
Body temperature (° C)
41
40
39
38
37
36
35
-2 -1 0 1 2 3 4 5 6 7
Days after inoculation
mRNA-RBD (HPLC) ○
Body temperature (° C)
40
39
38
37
36
35
-2 -1 0 1 2 3 4 5 6 7
Days after inoculation
mRNA-RBD (HPLC) □
Body temperature (° C)
39
38
37
36
35
34
-2 -1 0 1 2 3 4 5 6 7
Days after inoculation
mRNA-RBD (HPLC) △
Body temperature (° C)
40
39
38
37
36
35
-2 -1 0 1 2 3 4 5 6 7
Days after inoculation
mRNA-RBD (HPLC) ▽
Body temperature (° C)
40
39
38
37
36
35
-2 -1 0 1 2 3 4 5 6 7
Days after inoculation

[Fig. 24]

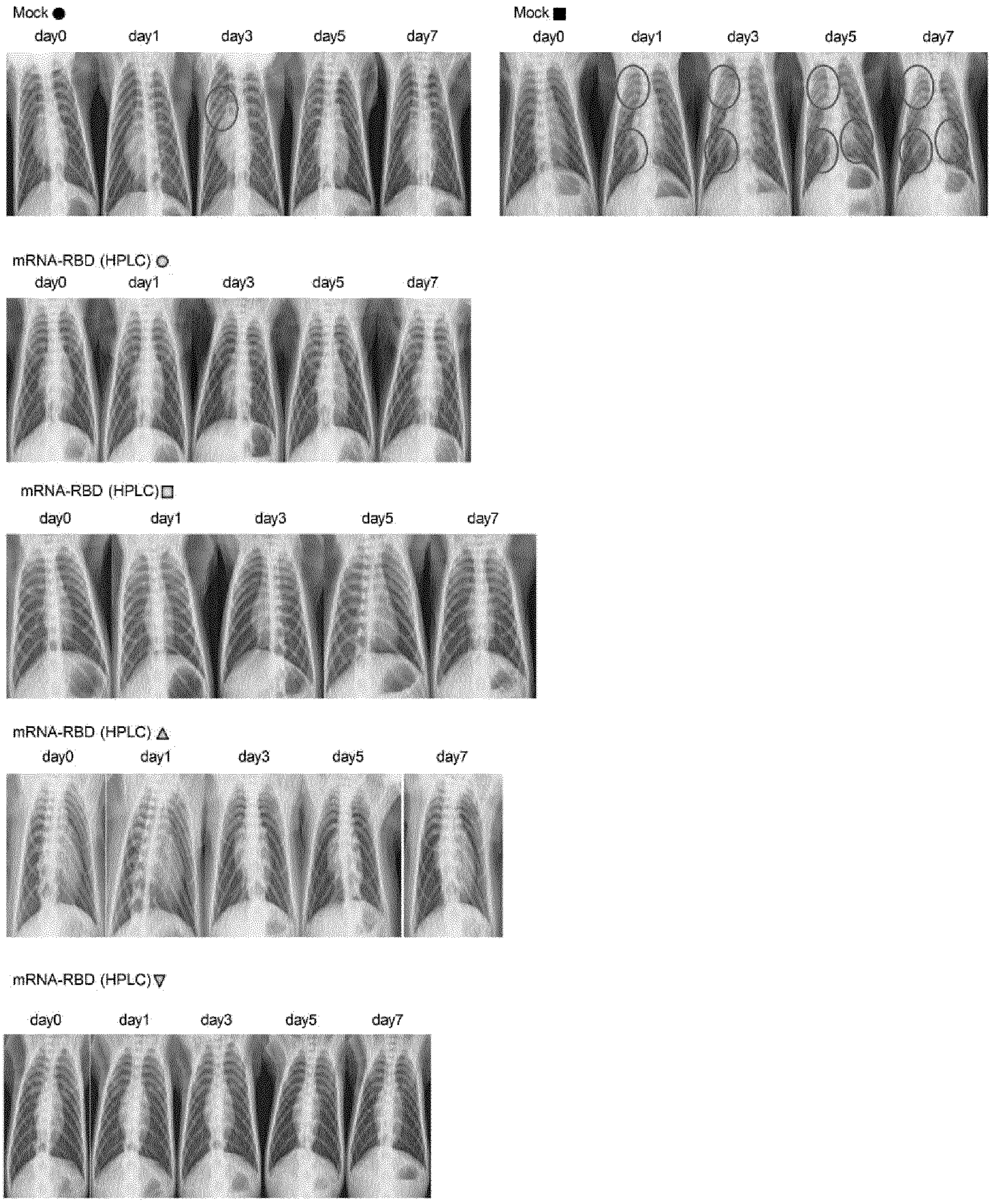
Mock ●
day0
day1
day3
day5
day7
Mock ■
day0
day1
day3
day5
day7
mRNA-RBD (HPLC) ○
day0
day1
day3
day5
day7
mRNA-RBD (HPLC) □
day0
day1
day3
day5
day7
mRNA-RBD (HPLC) △
day0
day1
day3
day5
day7
mRNA-RBD (HPLC) ▽
day0
day1
day3
day5
day7

[Fig. 25]

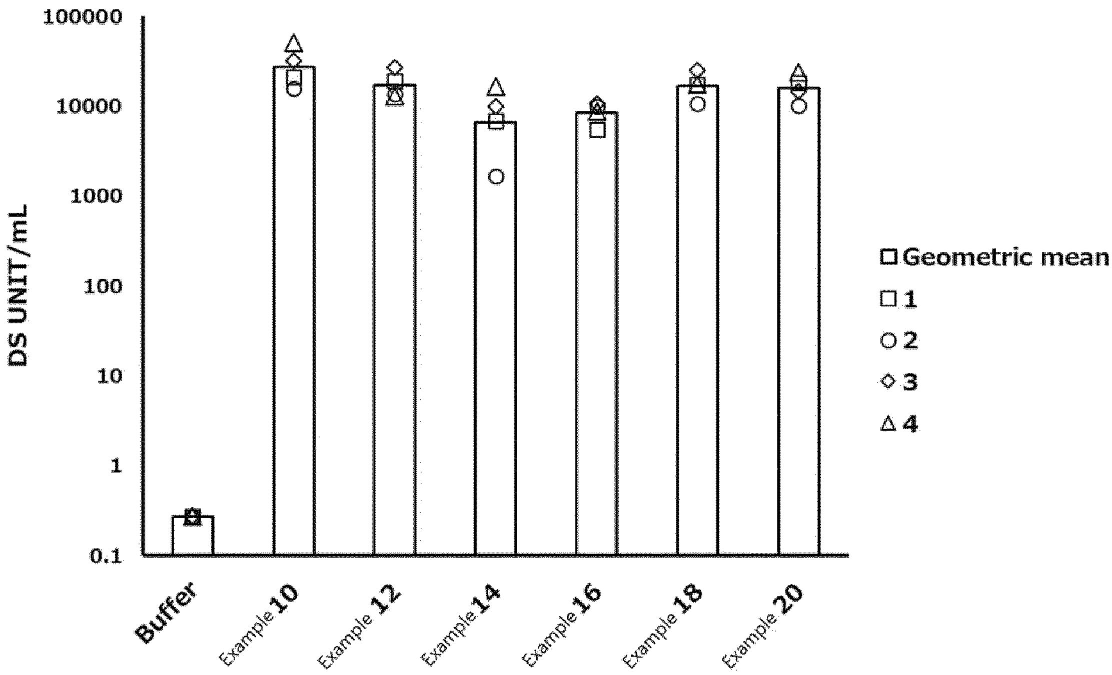
100000
10000
1000
100
10
1
0.1
DS UNIT/mL
Geometric mean
1
2
3
4
Buffer
Example 10
Example 12
Example 14
Example 16
Example 18
Example 20

[Fig. 26]

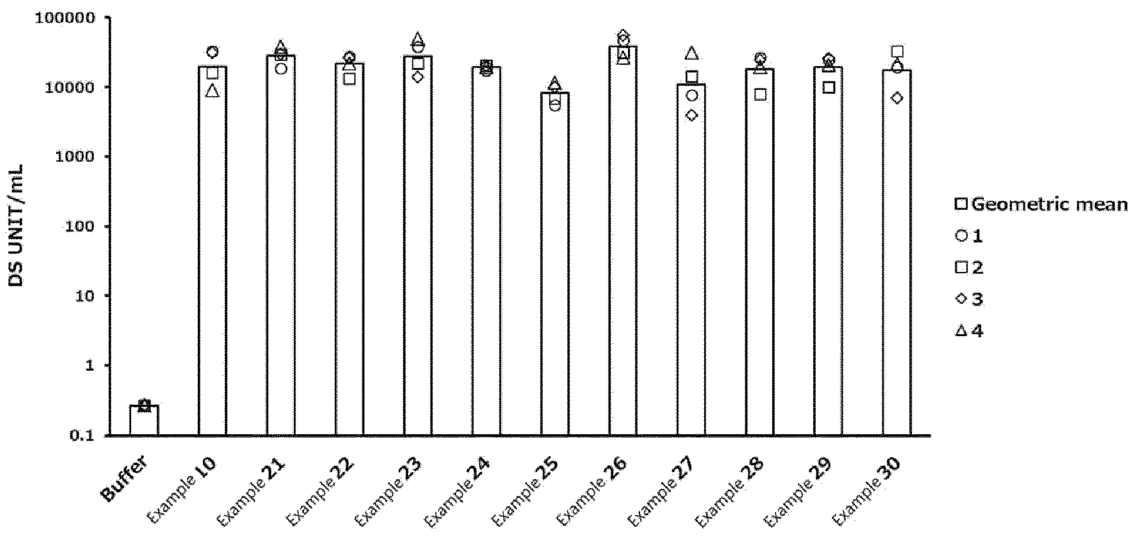
100000
10000
1000
100
10
1
0.1
DS UNIT/mL
Geometric mean
1
2
3
4
Buffer
Example 10
Example 21
Example 22
Example 23
Example 24
Example 25
Example 26
Example 27
Example 28
Example 29
Example 30

[Fig. 27]

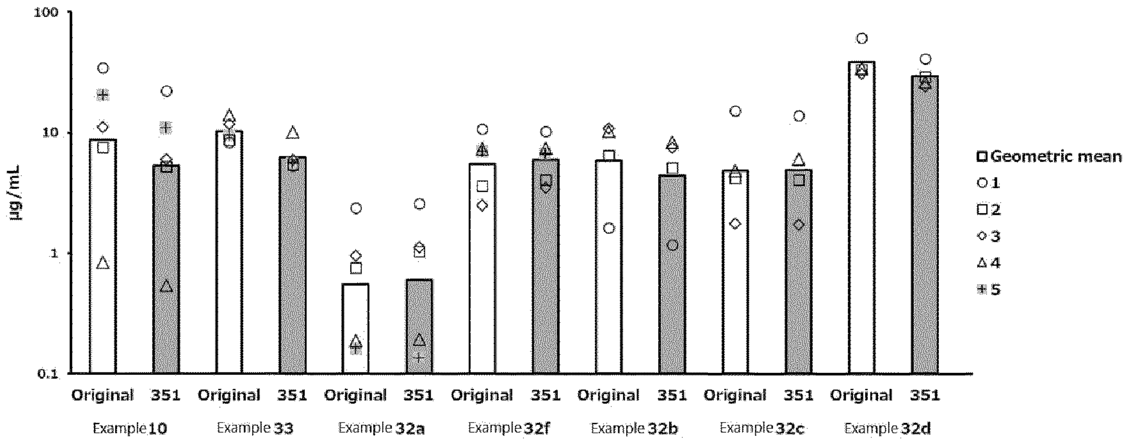
100
10
1
0.1
μg/mL
Geometric mean
1
2
3
4
5
Original
351
Example10
Example33
Example32a
Example32f
Example32b
Example32c
Example32d

[Fig. 28]

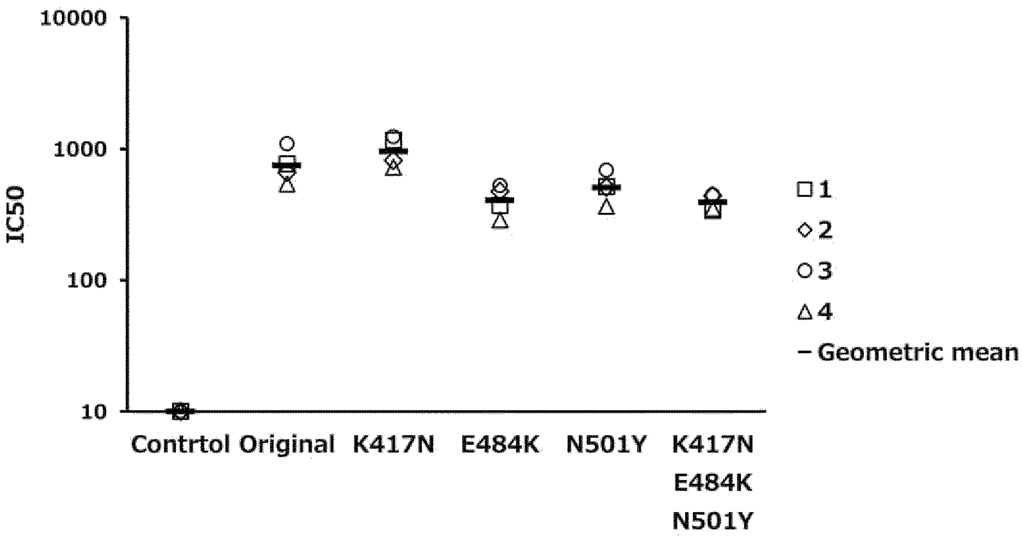
10000
1000
100
10
IC50
1
2
3
4
Geometric mean
Contrtol
Original
K417N
E484K
N501Y
K417N
E484K
N501Y

[Fig. 29]

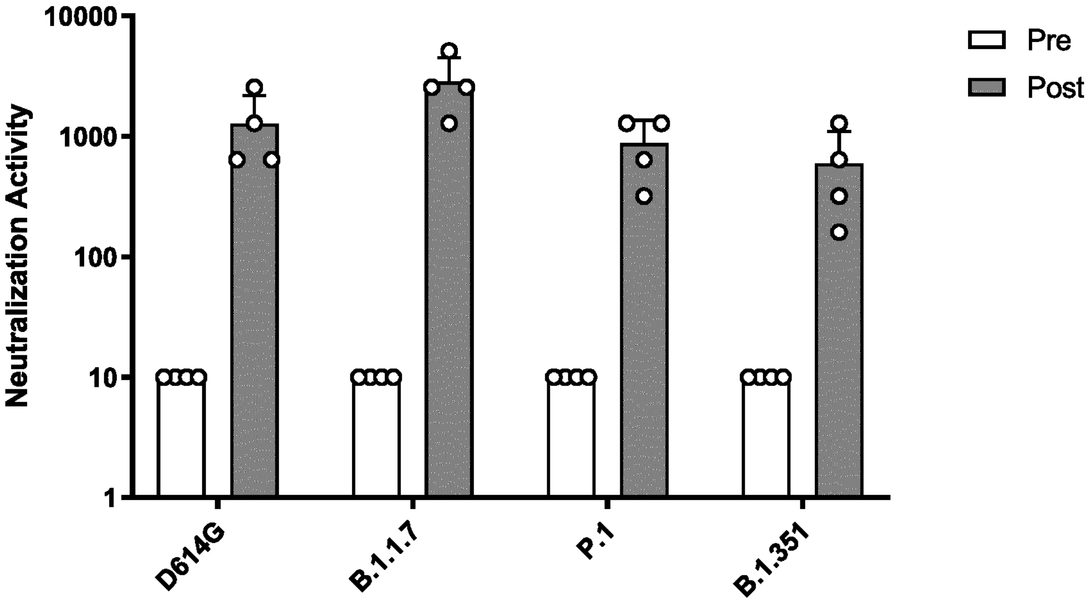
10000
1000
100
10
1
Neutralization Activity
D614G
B.1.1.7
P.1
B.1.351
Pre
Post

**INTERNATIONAL SEARCH REPORT**

International application No. PCT/JP2021/022057

A. CLASSIFICATION OF SUBJECT MATTER

A61K 39/00(2006.01)i; A61K 39/215(2006.01)i; A61K 48/00(2006.01)i; A61P 31/12(2006.01)i; A61P 31/14(2006.01)i; C07K 14/165(2006.01)i; A61K 9/107(2006.01)i; A61K 9/14(2006.01)i; A61K 47/14(2006.01)i; A61K 47/18(2006.01)i; A61K 47/24(2006.01)i; A61K 47/28(2006.01)i; C12N 15/50(2006.01)i; C12N 15/88(2006.01)i; A61K 31/7105(2006.01)i

FI: A61K39/00 H; A61K9/14; A61K47/18; A61K47/24; A61K47/28; A61K47/14; A61K9/107; A61P31/12; C12N15/88 Z; C12N15/50; A61K31/7105; A61P31/14; A61K48/00; C07K14/165; A61K39/215 ZNA

According to International Patent Classification (IPC) or to both national classification and IPC

B. FIELDS SEARCHED

Minimum documentation searched (classification system followed by classification symbols)

A61K39/00; A61K39/215; A61K48/00; A61P31/12; A61P31/14; C07K14/165; A61K9/107; A61K9/14; A61K47/14; A61K47/18; A61K47/24; A61K47/28; C12N15/50; C12N15/88; A61K31/7105

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

JSTPlus/JMEDPlus/JST7580 (JDreamIII); CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2015/005253 A1 (DAIICHI SANKYO COMPANY, LIMITED) 15 January 2015 (2015-01-15) claims, paragraphs [0283]-[0284], [0305]-[0307], examples, etc. | 1-52 |
| Y | BIONTECH, "BNT162 COVID-19 Vaccine", Program Update, April 2020, pp. 1-15, [retrieved on 10 August 2020], Retrieved from the Internet:<https://investors.biontech.de/static-files/398d9bd8-e2cb-49ca-9d6d-7dfd01c66b8a>, pp. 9-13, etc. | 1-52 |

☒ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 August 2021 (10.08.2021) | 17 August 2021 (17.08.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/022057

C (Continuation). DOCUMENTS CONSIDERED TO BE RELEVANT

| Category* | Citation of document, with indication, where appropriate, of the relevant passages | Relevant to claim No. |
|---|---|---|
| Y | WANG, F. et al., "An Evicence Based Perspective on mRNA-SARS-CoV-2 Vaccine Development", Med Sci Monit, May 2020, vol. 26, article no. e924700, pp. 1-8, ISSN 1643-3750, abstract, page 3, fig. 1, etc. | 1-52 |
| Y | MCKAY, P. F. et al., "Self-amplifying RNA SARS-CoV-2 lipid nanoparticle vaccine induces equivalent preclinical antibody titers and viral neutralization to recovered COVID-19 patients", bioRxiv, pp. 1-14, doi:https://doi.org/10.1101/2020.04.22.055608, abstract, MAIN, Discussion, etc. | 1-52 |

Form PCT/ISA/210 (continuation of second sheet) (January 2015)

**INTERNATIONAL SEARCH REPORT**
Information on patent family members

International application No.
PCT/JP2021/022057

| Patent Documents referred in the Report | Publication Date | Patent Family | Publication Date |
|---|---|---|---|
| WO 2015/005253 A1 | 15 Jan. 2015 | US 2016/0257951 A1<br>claims, paragraphs [0320]-[0321], [0338]-[0340], examples<br>EP 3020701 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020101420 A
- JP 2021033278 A
- WO 2015005253 A


### Non-patent literature cited in the description


- *Virology,* 2020, vol. 12, 372
- *Science,* 2020, vol. 367, 1260
- *Viruses,* 2020, vol. 12, 428
- *Virol J,* 2010, vol. 7, 299
- *Virology,* 2005, vol. 334, 74
- *Nat Commun,* 2020, vol. 11, 2251
- *Nature,* 2020, vol. 583, 290
- *Cell,* 2020, vol. 181, 1
- *Nat Med,* 2020, vol. 26, 1033
- *Nat Rev Immunol,* 2020, vol. 20, 347
- *J Immunol,* 2008, vol. 181, 5490
- *PLoS One,* 2012, vol. 7, e35421
- *Vaccine,* 2007, vol. 25, 2832
- *PNAS,* 2020, vol. 117, 8218
- **SAMBROOK, J. et al.** Molecular Cloning a Laboratory Manual. 1989
- **RASHTCHIAN, A.** *Current Opinion in Biotechnology,* 1995, vol. 6 (1), 30-36
- **GIBSON D. G. et al.** *Science,* 2008, vol. 319 (5867), 1215-1220
- **KORMANN, M.** *Nature Biotechnology,* 2011, vol. 29, 154-157
- **BADEN, L. R. et al.** Efficacy and Safety of the mRNA-1273 SARS-CoV-2 Vaccine. *N Engl J Med,* 2020
- **POLACK, F. P. et al.** Safety and Efficacy of the BNT162b2 mRNA Covid-19 Vaccine. *N Engl J Med,* 2020, vol. 383, 2603-2615
- **LAN, J. et al.** Structure of the SARS-CoV-2 spike receptor-binding domain bound to the ACE2 receptor. *Nature,* 2020, vol. 581, 215-220
- **RYDYZNSKI MODERBACHER, C. et al.** Antigen-Specific Adaptive Immunity to SARS-CoV-2 in Acute COVID-19 and Associations with Age and Disease Severity. *Cell,* 2020, vol. 183, 996-1012, e1019
- **MCMAHAN, K. et al.** Correlates of protection against SARS-CoV-2 in rhesus macaques. *Nature,* 2020
- **VOGEL, A. B. et al.** BNT162b vaccines are immunogenic and protect non-human primates against SARS-CoV-2. *bioRxiv, 2020.2012.2011.421008,* 2020
- **ELIA, U. et al.** Design of SARS-CoV-2 RBD mRNA Vaccine Using Novel Ionizable Lipids. *bioRxiv, 2020.2010.2015.341537,* 2020
- **TAI, W. et al.** A novel receptor-binding domain (RBD)-based mRNA vaccine against SARS-CoV-2. *Cell Res,* 2020, vol. 30, 932-935
- **LEDERER, K. et al.** SARS-CoV-2 mRNA Vaccines Foster Potent Antigen-Specific Germinal Center Responses Associated with Neutralizing Antibody Generation. *Immunity,* 2020, vol. 53, 1281-1295, e1285
- **ZHOU, P. et al.** A pneumonia outbreak associated with a new coronavirus of probable bat origin. *Nature,* 2020, vol. 579, 270-273
- **JACKSON, L. A. et al.** An mRNA Vaccine against SARS-CoV-2 - Preliminary Report. *N Engl J Med,* 2020, vol. 383, 1920-1931
- **ANDERSON, E. J. et al.** Safety and Immunogenicity of SARS-CoV-2 mRNA-1273 Vaccine in Older Adults. *N Engl J Med,* 2020, vol. 383, 2427-2438
- **WALSH, E. E. et al.** Safety and Immunogenicity of Two RNA-Based Covid-19 Vaccine Candidates. *N Engl J Med,* 2020, vol. 383, 2439-2450
- **DESMET, C. J. ; ISHII, K. J.** Nucleic acid sensing at the interface between innate and adaptive immunity in vaccination. *Nat Rev Immunol,* 2012, vol. 12, 479-491
- **COBAN, C. et al.** Novel strategies to improve DNA vaccine immunogenicity. *Curr Gene Ther,* 2011, vol. 11, 479-484
- **PARDI, N. ; HOGAN, M. J. ; PORTER, F. W. ; WEISSMAN, D.** mRNA vaccines - a new era in vaccinology. *Nat Rev Drug Discov,* 2018, vol. 17, 261-279
- **IAVARONE, C. ; O'HAGAN D, T. ; YU, D. ; DELAHAYE, N. F ; ULMER, J. B.** Mechanism of action of mRNA-based vaccines. *Expert Rev Vaccines,* 2017, vol. 16, 871-881

- **KARIKO, K. ; BUCKSTEIN, M. ; NI, H. ; WEISSMAN, D.** Suppression of RNA recognition by Toll-like receptors: the impact of nucleoside modification and the evolutionary origin of RNA. *Immunity,* 2005, vol. 23, 165-175
- **KARIKO, K. et al.** Incorporation of pseudouridine into mRNA yields superior nonimmunogenic vector with increased translational capacity and biological stability. *Mol Ther,* 2008, vol. 16, 1833-1840
- **KARIKO, K. ; MURAMATSU, H. ; LUDWIG, J. ; WEISSMAN, D.** Generating the optimal mRNA for therapy: HPLC purification eliminates immune activation and improves translation of nucleoside-modified, protein-encoding mRNA. *Nucleic Acids Res,* 2011, vol. 39, e142
- **DE BEUCKELAER, A. et al.** Type I Interferons Interfere with the Capacity of mRNA Lipoplex Vaccines to Elicit Cytolytic T Cell Responses. *Mol Ther,* 2016, vol. 24, 2012-2020
- **LINARES-FERNANDEZ, S. ; LACROIX, C. ; EXPOSITO, J. Y. ; VERRIER, B.** Tailoring mRNA Vaccine to Balance Innate/Adaptive Immune Response. *Trends Mol Med,* 2020, vol. 26, 311-323
- **CORBETT, K. S. et al.** Evaluation of the mRNA-1273 Vaccine against SARS-CoV-2 in Nonhuman Primates. *N Engl J Med,* 2020, vol. 383, 1544-1555
- **IMAI, M. et al.** Syrian hamsters as a small animal model for SARS-CoV-2 infection and countermeasure development. *Proc Natl Acad Sci U S A,* 2020, vol. 117, 16587-16595
- **ISHIGAKI, H. et al.** Neutralizing antibody-dependent and -independent immune responses against SARS-CoV-2 in cynomolgus macaques. *Virology,* 2021, vol. 554, 97-105